# EUROPEAN PATENT APPLICATION

(11) **EP 4 786 601 A1**
(43) Date of publication of application: **05.08.2026**
(21) Application number: 25382062.5
(22) Date of filing: 31.01.2025
(51) Int. Cl.: C12N 15/86, C07K 14/005

(54) **HELPER PLASMIDS AND USES THEREOF**

(71) Applicant: AskBio Inc., Research Triangle Park, NC 27709 (US)
(72) Inventor: FRANCOIS, Achille, Research Triangle Park, NC, 27709 (US)
(74) Representative: HGF

(57) **Abstract**

The technology described herein relates generally to compositions and methods for preparing viral, e.g., adeno-associated viral (AAV) particles.

## Description

### TECHNICAL FIELD

The technology described herein relates generally to compositions and methods for preparing viral, e.g., adeno-associated viral (AAV) particles.

### BACKGROUND

Production of recombinant adeno-associated vectors (rAAV) involves consideration of both efficiency and safety. The production of rAAVs usually requires the expression of and/or infection by both the desired vector as well as additional components necessary for robust production. In some cases, this may require the incorporation and expression of up to three large plasmids into one single cell. Successfully delivering three plasmids to one cell is a relatively inefficient process. For larger-scale manufacturing efforts, transient delivery of plasmid requires excess quantities of DNA, adding to the overall cost of production and purification.

In addition to delivery of the plasmids, the efficiency of the plasmids can also be considered. Helper viruses, such as adenovirus, a herpesvirus, or vaccinia, as well as adenovirus helper nucleic acids (Ad helpers) contain components, that assist in the production of rAAV. Ad helpers can contain proteins used for rAAV production. Efficient production of these proteins allows for the production of higher titers of rAAV.

Ad helpers have been regarded as safer alternatives to helper adenovirus infections because they only produce proteins for producing rAAV and not the infectious virus. Minimizing exposure to infectious virus is a consideration in the production of rAAV. Since this is a safer alternative, optimizing the delivery of Ad helpers can contribute to producing high titers of rAAV.

The present disclosure addresses these needs.

### SUMMARY

In one aspect, provided herein is a polynucleotide comprising a nucleotide sequence encoding: (a) an adenoviral E2a region; (b) an adenoviral E4 region; and (c) an adenoviral virus associated (VA) RNA region. Generally, the polynucleotide does not comprise a nucleotide sequence encoding at least 1 (e.g., 2, 3, 4, or all 5) of: (i) a full length adenoviral L4-100K protein or a portion, e.g., a functional portion thereof; (ii) a full length adenoviral L5 fiber protein or a portion, e.g., a functional portion thereof; (iii) a full length adenoviral pVIII protein or a portion, e.g., a functional portion thereof; (iv) a full length adenoviral L1-52K/55K protein or a portion, e.g., a functional portion thereof; or a full length adenoviral precursor terminal protein (pTP) or a portion, e.g., a functional portion thereof. In some embodiments, the polynucleotide comprises at least one (e.g., 1, 2, 3, 4, 5, 7 or all 7) of: (i) a deletion of G₂ and G₃ in a nucleotide sequence encoding an adenoviral L-22K protein (e.g., **SEQ ID NO: 103**) and/or a L-33K protein (e.g., **SEQ ID NO: 104**); (ii) a deletion of G₂ and G₃ in a nucleotide sequence encoding an adenoviral L4-100K protein (e.g., **SEQ ID NO: 105**); (iii) a deletion of G₂ in a nucleotide sequence encoding adenoviral pVIII protein (e.g., **SEQ ID NO: 106**); (iv) a AC → TT mutation at positions 149-150 of a nucleotide sequence encoding adenoviral pVIII protein (e.g., **SEQ ID NO: 106**); (v) a deletion of G₂ in a nucleotide sequence encoding adenoviral fiber protein (e.g., **SEQ ID NO: 107**); (vi) a C → T mutation at position 503 of a nucleotide encoding adenoviral L5 fiber protein (e.g., **SEQ ID NO: 103**); and (vii) a TA → CG mutation at positions 1692-1693 of a nucleotide encoding adenoviral L5 fiber protein (e.g., **SEQ ID NO: 107**).

In some embodiments, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 5.** For example, the polynucleotide comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 5.** In some embodiments, the polynucleotide comprises a nucleotide sequence having 100% identity to **SEQ ID NO: 5.**

In some embodiments, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 6.** For example, the polynucleotide comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 6.** In some embodiments, the polynucleotide comprises a nucleotide sequence having 100% identity to **SEQ ID NO: 6.**

In some embodiments, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 4.** For example, the polynucleotide comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 4.** In some embodiments, the polynucleotide comprises a nucleotide sequence having 100% identity to **SEQ ID NO: 4.**

In some embodiments, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 3.** For example, the polynucleotide comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 3.** In some embodiments, the polynucleotide comprises a nucleotide sequence having 100% identity to **SEQ ID NO: 3.**

In some embodiments, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 2.** For example, the polynucleotide comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 2.** In some embodiments, the polynucleotide comprises a nucleotide sequence having 100% identity to **SEQ ID NO: 2.**

In some embodiments, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 1.** For example, the polynucleotide comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 1.** In some embodiments, the polynucleotide comprises a nucleotide sequence having 100% identity to **SEQ ID NO: 1.**

In some embodiments, the polynucleotide does not comprise nucleotides 26197-26198 relative to Genbank Accession Number AC_000008.

In some embodiments, the the polynucleotide does not comprise nucleotides 24063-24064 relative to Genbank Accession Number AC_000008.

In some embodiments, the polynucleotide does not comprise nucleotide 27176 relative to Genbank Accession Number AC_000008.

In some embodiments, the polynucleotide comprises AC → TT mutation at positions 27322-27324 relative to Genbank Accession Number AC_000008.

In some embodiments, the polynucleotide does not comprise nucleotide 31024 relative to Genbank Accession Number AC_000008.

In some embodiments, the polynucleotide comprises C → T mutation at positions 31544 relative to Genbank Accession Number AC_000008.

In some embodiments, the polynucleotide comprises TA → CG mutation at positions 32733-32734 relative to Genbank Accession Number AC_000008.

In some embodiments, the polynucleotide does not comprise a nucleotide sequence encoding a protein comprising, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95.**

In some embodiments, the polynucleotide does not comprise a nucleotide sequence encoding: full length adenoviral L4-22K protein or a portion, e.g., a functional portion thereof. For example, the polynucleotide comprises a nucleotide sequence encoding a less than full length fragment of adenoviral L4-22K protein. In some embodiments, the polynucleotide comprises a nucleotide sequence encoding a truncated adenoviral L4-22K protein. For example, the polynucleotide does not comprise a nucleotide sequence encoding the first 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, or 76 amino acids that are present at the N-terminus of the full-length adenoviral L4-22K protein. In some embodiments, the polynucleotide does not comprise a nucleotide sequence encoding the first 10, 11, 12, 13, 14, 15, 16, 17, 18, 19, 20, 21, 22, 23, 24, 25, 26, 27, 28, 29, 30, 31, 32, 33, or 34 amino acids that are present at the N-terminus of the full-length adenoviral L4-22K protein. For example, the polynucleotide does not comprise a nucleotide sequence encoding the first 30, 31, 32, 33, or 34, preferably the first 33 or 34, more preferably the first 34 amino acids that are present at the N-terminus of the full-length adenoviral L4-22K protein. In some embodiments, the polynucleotide does not comprise a nucleotide sequence encoding the first 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, or 76 amino acids that are present at the N-terminus of the full-length adenoviral L4-22K protein. For example, the polynucleotide does not comprise a nucleotide sequence encoding the first 65, 66, 67, 68, 69, 70, 71, 72, 73, 74, 75, or 76, preferably the first 75 or 76, more preferably the first 76 amino acids that are present at the N-terminus of the full-length adenoviral L4-22K protein.

In some embodiments, the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the protein does not comprise, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95.** For example, the polynucleotide comprises a nucleotide sequence encoding a protein comprising an amino acid sequence having at least 90% (e.g., at least 91%, at least 92%, at least 93%, at least 94%, at least 95%, at least 96%, at least 97%, at least 98%, at least 99% or 100%) identity to **SEQ ID NO: 92 or 93,** and wherein the protein does not comprise, e.g., at its N-terminus an amino acid sequence having at least 90% (e.g., at least 91%, at least 92%, at least 93%, at least 94%, at least 95%, at least 96%, at least 97%, at least 98%, at least 99% or 100%) identity to **SEQ ID NO: 94 or 95.** In some embodiments, the polynucleotide comprises a nucleotide sequence encoding a protein comprising an amino acid sequence having 100% identity to **SEQ ID NO: 92 or 93,** and wherein the protein does not comprise, e.g., at its N-terminus an amino acid sequence having 100% identity to **SEQ ID NO: 94 or 95**.

In some embodiments, the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a full length adenoviral L4-22K protein having the amino acid sequence **SEQ ID NO: 7.** For example, , the polynucleotide comprises a nucleotide sequence encoding a protein comprising an amino acid sequence having at least 90% (e.g., at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a full length adenoviral L4-22K protein having the amino acid sequence **SEQ ID NO: 7**. In some embodiments, the polynucleotide comprises a nucleotide sequence encoding a protein comprising an amino acid sequence having 100% identity to **SEQ ID NO: 92 or 93,** and wherein the polynucleotide does not comprise a nucleotide sequence encoding a full length adenoviral L4-22K protein having the amino acid sequence **SEQ ID NO: 7.**

In some embodiments, the polynucleotide does not comprise a nucleotide sequence encoding: full length adenoviral L4-33K protein or a portion, e.g., a functional portion thereof. For example, the polynucleotide comprises a nucleotide sequence encoding a less than full length fragment of adenoviral L4-33K protein. In some embodiments, the polynucleotide comprises a nucleotide sequence encoding a truncated adenoviral L4-33K protein. For example, the polynucleotide does not comprise a nucleotide sequence encoding the first 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, or 76 amino acids that are present at the N-terminus of the full-length adenoviral L4-33K protein. In some embodiments, the polynucleotide does not comprise a nucleotide sequence encoding the first 10, 11, 12, 13, 14, 15, 16, 17, 18, 19, 20, 21, 22, 23, 24, 25, 26, 27, 28, 29, 30, 31, 32, 33, or 34 amino acids that are present at the N-terminus of the full-length adenoviral L4-33K protein. For example, the polynucleotide does not comprise a nucleotide sequence encoding the first 30, 31, 32, 33, or 34, preferably the first 33 or 34, more preferably the first 34 amino acids that are present at the N-terminus of the full-length adenoviral L4-33K protein. In some embodiments, the polynucleotide does not comprise a nucleotide sequence encoding the first 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, or 76 amino acids that are present at the N-terminus of the full-length adenoviral L4-33K protein. For example, the polynucleotide does not comprise a nucleotide sequence encoding the first 65, 66, 67, 68, 69, 70, 71, 72, 73, 74, 75, or 76, preferably the first 75 or 76, more preferably the first 76 amino acids that are present at the N-terminus of the full-length adenoviral L4-33K protein

In some embodiments, the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the protein does not comprise, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95.** For example, the polynucleotide comprises a nucleotide sequence encoding a protein comprising an amino acid sequence having at least 90% (e.g., at least 91%, at least 92%, at least 93%, at least 94%, at least 95%, at least 96%, at least 97%, at least 98%, at least 99% or 100%) identity to **SEQ ID NO: 90 or 91,** and wherein the protein does not comprise, e.g., at its N-terminus an amino acid sequence having at least 90% (e.g., at least 91%, at least 92%, at least 93%, at least 94%, at least 95%, at least 96%, at least 97%, at least 98%, at least 99% or 100%) identity to **SEQ ID NO: 94 or 95.** In some embodiments, the polynucleotide comprises a nucleotide sequence encoding a protein comprising an amino acid sequence having 100% identity to **SEQ ID NO: 90 or 91,** and wherein the protein does not comprise, e.g., at its N-terminus an amino acid sequence having 100% identity to **SEQ ID NO: 94 or 95**.

In some embodiments, the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91**, and wherein the polynucleotide does not comprise a nucleotide sequence encoding a full length adenoviral L4-33K protein having the amino acid sequence **SEQ ID NO: 8**. For example, , the polynucleotide comprises a nucleotide sequence encoding a protein comprising an amino acid sequence having at least 90% (e.g., at least 91%, at least 92%, at least 93%, at least 94%, at least 95%, at least 96%, at least 97%, at least 98%, at least 99% or 100%) identity to **SEQ ID NO: 90 or 91**, and wherein the polynucleotide does not comprise a nucleotide sequence encoding a full length adenoviral L4-33K protein having the amino acid sequence **SEQ ID NO: 8.** In some embodiments, the polynucleotide comprises a nucleotide sequence encoding a protein comprising an amino acid sequence having 100% identity to **SEQ ID NO: 90 or 91**, and wherein the polynucleotide does not comprise a nucleotide sequence encoding a full length adenoviral L4-33K protein having the amino acid sequence **SEQ ID NO: 8.**

In some embodiments, the polynucleotide does not comprise a nucleotide sequence encoding the L4-100K protein or a portion, e.g., a functional portion thereof. In some embodiments, the full length L4-100K protein has the amino acid sequence **SEQ ID NO: 9**. Thus, in some embodiments, the polynucleotide does not comprise a nucleotide sequence encoding a protein comprising the sequence of **SEQ ID NO: 9,** or at least a portion, e.g., a functional portion of **SEQ ID NO: 9.**

In some embodiments, the polynucleotide does not comprise a nucleotide sequence encoding the L5 fiber protein or a portion, e.g., a functional portion thereof. In some embodiments, the full length L5 fiber protein has the amino acid sequence **SEQ ID NO: 10.** Thus, in some embodiments, the polynucleotide does not comprise a nucleotide sequence encoding a protein comprising the sequence of **SEQ ID NO: 10,** or at least a portion, e.g., a functional portion of **SEQ ID NO: 10.**

In some embodiments, the polynucleotide does not comprise a nucleotide sequence encoding the pVIII protein or a portion, e.g., a functional portion thereof. In some embodiments, the full length pVIII protein has the amino acid sequence **SEQ ID NO: 11**. Thus, in some embodiments, the polynucleotide does not comprise a nucleotide sequence encoding a protein comprising the sequence of **SEQ ID NO: 11**, or at least a portion, e.g., a functional portion of **SEQ ID NO: 11**.

In some embodiments, the polynucleotide does not comprise a nucleotide sequence encoding the L1-52K/55K protein or a portion, e.g., a functional portion thereof. In some embodiments, the full length L1-52K/55K protein has the amino acid sequence **SEQ ID NO: 12.** Thus, in some embodiments, the polynucleotide does not comprise a nucleotide sequence encoding a protein comprising the sequence of **SEQ ID NO: 11**, or at least a portion, e.g., a functional portion of **SEQ ID NO: 12**.

In some embodiments, the polynucleotide does not comprise a nucleotide sequence encoding the pTP protein or a portion, e.g., a functional portion thereof. In some embodiments, the full length pTP protein has the amino acid sequence **SEQ ID NO: 13**. Thus, in some embodiments, the polynucleotide does not comprise a nucleotide sequence encoding a protein comprising the sequence of **SEQ ID NO: 11**, or at least a portion, e.g., a functional portion of **SEQ ID NO: 13**.

In some embodiments, the polynucleotide does not comprise a nucleotide sequence encoding: (i) the full length L4-100K protein or a portion, e.g., a functional portion thereof; (ii) the full length L1-52K/55K protein or a portion, e.g., a functional portion thereof; (iii) the full length L4-33K protein or a portion, e.g., a functional portion thereof; (iv) the full length L4-22K protein or a portion, e.g., a functional portion thereof; (v) the full length L5 fiber protein or a portion, e.g., a functional portion thereof; (vi) the full length pVIII protein or a portion, e.g., a functional portion thereof; and/or (vii) the full length pTP or a portion, e.g., a functional portion thereof. For example, the polynucleotide does not comprise a nucleotide sequence encoding (i) a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9**, or a portion, e.g., a functional portion thereof; (ii) a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion, e.g., a functional portion thereof; (iii) a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion, e.g., a functional portion thereof; (iv) a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion, e.g., a functional portion thereof; (v) a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion, e.g., a functional portion thereof; (vi) a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion, e.g., a functional portion thereof; and/or (vii) a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion, e.g., a functional portion thereof.

In some embodiments, the polynucleotide does not comprise a nucleotide sequence encoding: (i) a full length adenoviral E3 region or a portion, e.g., a functional portion thereof; (ii) a full length adenoviral U exon protein (UXP) exon 1 or a portion, e.g., a functional portion thereof; (iii) a full length L4-100K protein or a portion, e.g., a functional portion thereof; (iv) a full length L1-52K/55K protein or a portion, e.g., a functional portion thereof; (v) a full length L4-33K protein or a portion, e.g., a functional portion thereof; (vi) a full length L4-22K protein or a portion, e.g., a functional portion thereof; (vii) a full length L5 fiber protein or a portion, e.g., a functional portion thereof; (viii) a full length pVIII protein or a portion, e.g., a functional portion thereof; and/or (ix) a full length pTP or a portion, e.g., a functional portion thereof.

For example, the polynucleotide does not comprise a nucleotide sequence encoding: (i) a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion, e.g., functional portion thereof; (ii) a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion, e.g., a functional portion thereof; (iii) a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion, e.g., a functional portion thereof; (iv) a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion, e.g., a functional portion thereof; (v) a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion, e.g., a functional portion thereof; (vi) a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion, e.g., a functional portion thereof; (vii) a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion, e.g., a functional portion thereof; (viii) a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion, e.g., a functional portion thereof; and/or (ix) a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion, e.g., a functional portion thereof.

In some embodiments, the polynucleotide does not comprise a nucleotide sequence encoding: (i) a full length adenoviral E3 region or a portion, e.g., a functional portion thereof; (ii) a full length adenoviral U exon protein promoter or a portion, e.g., a functional portion thereof; (iii) a full length adenoviral U exon protein (UXP) exon 1 or a portion, e.g., a functional portion thereof; (iv) a full length L4-100K protein or a portion, e.g., a functional portion thereof; (v) a full length L1-52K/55K protein or a portion, e.g., a functional portion thereof; (vi) a full length L4-33K protein or a portion, e.g., a functional portion thereof; (vii) a full length L4-22K protein or a portion, e.g., a functional portion thereof; (viii) a full length L5 fiber protein or a portion, e.g., a functional portion thereof; (ix) a full length pVIII protein or a portion, e.g., a functional portion thereof; and/or (x) a full length pTP or a portion, e.g., a functional portion thereof.

For example, the polynucleotide does not comprise a nucleotide sequence encoding: (i) a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion, e.g., functional portion thereof; (ii) a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion, e.g., functional portion thereof; (iii) a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion, e.g., a functional portion thereof; (iv) a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion, e.g., a functional portion thereof; (v) a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion, e.g., a functional portion thereof; (vi) a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion, e.g., a functional portion thereof; (vii) a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion, e.g., a functional portion thereof; (viii) a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion, e.g., a functional portion thereof; (ix) a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion, e.g., a functional portion thereof; and/or (x) a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion, e.g., a functional portion thereof.

In some embodiments, the polynucleotide comprises a nucleotide sequence encoding a full length adenoviral L4-33K protein and a full length L4-22K protein, and wherein the polynucleotide does not comprise a nucleotide sequence encoding: (i) a full length adenoviral E3 region or a portion, e.g., a functional portion thereof; (ii) a full length adenoviral U exon protein promoter or a portion, e.g., a functional portion thereof; (iii) a full length adenoviral U exon protein (UXP) exon 1 or a portion, e.g., a functional portion thereof; (iv) a full length L4-100K protein or a portion, e.g., a functional portion thereof; (v) a full length L1-52K/55K protein or a portion, e.g., a functional portion thereof; (vi) a full length L5 fiber protein or a portion, e.g., a functional portion thereof; (vii) a full length pVIII protein or a portion, e.g., a functional portion thereof; and/or (viii) a full length pTP or a portion, e.g., a functional portion thereof.

For example, the polynucleotide comprises a nucleotide sequence encoding: (i) a full length L4-33K protein comprising an amino acid sequence having at least 80% (e.g., at least 85%, at least 90%, at least 91 %, at least 92%, at least 93%, at least 94%, at least 95%, at least 96%, at least 97%, at least 98%, at least 99% or 100%) identity to **SEQ ID NO: 8,** or a portion, e.g., a functional portion thereof; and (ii)) a full length L4-22K protein comprising an amino acid sequence having at least 80% (e.g., at least 85%, at least 90%, at least 91 %, at least 92%, at least 93%, at least 94%, at least 95%, at least 96%, at least 97%, at least 98%, at least 99% or 100%) identity to **SEQ ID NO: 7,** or a portion, e.g., a functional portion thereof, and wherein the polynucleotide does not comprise a nucleotide sequence encoding: (i) a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91 %, at least 92%, at least 93%, at least 94%, at least 95%, at least 96%, at least 97%, at least 98%, at least 99% or 100%) identity to a nucleotide sequence complementary to **SEQ ID NO: 21,** or a portion, e.g., functional portion thereof; (ii) a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion, e.g., functional portion thereof; (iii) a full length adenoviral UXP exon 1 comprising a nucleotide sequence having at least 80% (e.g., at least 85%, at least 90%, at least 91 %, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion, e.g., a functional portion thereof; (iv) a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion, e.g., a functional portion thereof; (v) a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion, e.g., a functional portion thereof; (vi) a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion, e.g., a functional portion thereof; (vii) a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion, e.g., a functional portion thereof; and/or (viii) a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion, e.g., a functional portion thereof.

In some embodiments, the polynucleotide does not comprise a nucleotide sequence encoding an adenoviral E3 region or a portion, e.g., a functional portion thereof.

In some embodiments, the polynucleotide does not comprise a nucleotide sequence encoding an adenoviral E3 12.5k protein or a portion, e.g., a functional portion thereof. In some embodiments, the full length E3 12.5k protein has the amino acid sequence **SEQ ID NO: 14.** Thus, in some embodiments, the polynucleotide does not comprise a nucleotide sequence encoding a protein comprising the sequence of **SEQ ID NO: 14,** or at least a portion, e.g., a functional portion of **SEQ ID NO: 14.**

In some embodiments, the polynucleotide does not comprise a nucleotide sequence encoding an adenoviral E3 CR1-alpha protein or a portion, e.g., a functional portion thereof. In some embodiments, the full length E3 CR1-alpha protein has the amino acid sequence **SEQ ID NO: 15.** Thus, in some embodiments, the polynucleotide does not comprise a nucleotide sequence encoding a protein comprising the sequence of **SEQ ID NO: 15,** or at least a portion, e.g., a functional portion of **SEQ ID NO: 15.**

In some embodiments, the polynucleotide does not comprise a nucleotide sequence encoding an adenoviral E3 gp19k protein or a portion, e.g., a functional portion thereof. In some embodiments, the full length E3 gp19k protein has the amino acid sequence **SEQ ID NO: 16.** Thus, in some embodiments, the polynucleotide does not comprise a nucleotide sequence encoding a protein comprising the sequence of **SEQ ID NO: 16,** or at least a portion, e.g., a functional portion of **SEQ ID NO: 16.**

In some embodiments, the polynucleotide does not comprise a nucleotide sequence encoding an adenoviral E3 10.5kd protein or a portion, e.g., a functional portion thereof. In some embodiments, the full length E3 10.5kd protein has the amino acid sequence **SEQ ID NO: 17.** Thus, in some embodiments, the polynucleotide does not comprise a nucleotide sequence encoding a protein comprising the sequence of **SEQ ID NO: 17,** or at least a portion, e.g., a functional portion of **SEQ ID NO: 17.**

In some embodiments, the polynucleotide does not comprise a nucleotide sequence encoding an adenoviral E3 RID-alpha protein or a portion, e.g., a functional portion thereof. In some embodiments, the full length E3 RID-alpha protein has the amino acid sequence **SEQ ID NO: 18.** Thus, in some embodiments, the polynucleotide does not comprise a nucleotide sequence encoding a protein comprising the sequence of **SEQ ID NO: 18,** or at least a portion, e.g., a functional portion of **SEQ ID NO: 18.**

In some embodiments, the polynucleotide does not comprise a nucleotide sequence encoding an adenoviral E3 RID-beta protein or a portion, e.g., a functional portion thereof. In some embodiments, the full length E3 RID-beta protein has the amino acid sequence **SEQ ID NO: 19.** Thus, in some embodiments, the polynucleotide does not comprise a nucleotide sequence encoding a protein comprising the sequence of **SEQ ID NO: 19,** or at least a portion, e.g., a functional portion of **SEQ ID NO: 19.**

In some embodiments, the polynucleotide does not comprise a nucleotide sequence encoding an adenoviral E3 14.7k protein or a portion, e.g., a functional portion thereof. In some embodiments, the full length E3 14.7k protein has the amino acid sequence **SEQ ID NO: 20.** Thus, in some embodiments, the polynucleotide does not comprise a nucleotide sequence encoding a protein comprising the sequence of **SEQ ID NO: 20,** or at least a portion, e.g., a functional portion of **SEQ ID NO: 20.**

In some embodiments, the polynucleotide comprises a nucleotide sequence encoding an adenoviral E3 region. For example, the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91 %, at least 92%, at least 93%, at least 94%, at least 95%, at least 96%, at least 97%, at least 98%, at least 99% or 100%) identity to a nucleotide sequence complementary to **SEQ ID NO: 21.**

The E3 region can be downstream or upstream of the E2a region. Thus, in some embodiments, the E3 region downstream of the E2a region. In some other embodiments, the E3 region is upstream of the E2a region. Preferably, the E3 region is downstream of the E2a region.

Similarly, the E3 region can be downstream or upstream of the E4 region. Thus, in the E3 region is downstream of the E4 region. In some other embodiments, wherein the E3 region upstream of the E4 region. Preferably, the E3 region is upstream of the E4 region.

In some embodiments, the E3 region can be between the E2a region and the E4 region.

It is noted that the VA region, the E2a region and the E4 region can be oriented independently in a 5'->3' direction or in a 3'->5' direction. In some embodiments, the VA region is oriented in a 5'->3' direction. In some embodiments, the E2a region is oriented in a 3'->5' direction. In some embodiments, the E4 region is oriented in a 3'->5' direction.

In some embodiments, the polynucleotide comprises in series: the poly A site, the VA RNA region, the E2a region, and the E4 region.

In some embodiments, VA RNA region is flanked on each side by at least one restriction site. For example, the polynucleotide comprises at least 2, e.g., 3, 4, 5 or more restriction sites upstream of the VA RNA region. In some embodiments, the polynucleotide comprises at least one restriction site downstream of the VA RNA region, and wherein the restriction site is between the VA region and the E2a region. In some embodiments, the polynucleotide comprises at least one restriction site downstream of the E4 region. The polynucleotide can also comprise a nucleotide sequence encoding a polyadenylation (poly A) site. For example, the polynucleotide comprises a nucleotide sequence encoding a polyadenylation (poly A) site, optionally, the poly A site is upstream of the VA RNA region. In some embodiments, the polynucleotide a polyadenylation (poly A) site having a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 22** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 22,** and optionally, the poly A site is upstream of the VA RNA region. In some embodiments, the poly A site is flanked by at least one restriction site on each side. In some embodiments, the polynucleotide comprises at least three restriction sites upstream of the poly A site. In some embodiments, the polynucleotide comprises at least one restriction site downstream of the poly A site. For example, the polynucleotide comprises at least three restriction sites upstream of the poly A site and the at least one restriction site is between the poly A site and the VA region.

The polynucleotide can also comprise one or more inverted terminal repeat (ITR) sequences selected independently from AAV ITR sequences and adenoviral ITR sequences. In some embodiments, the ITR comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 23** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 23.**

It is noted that the ITR sequence can be present anywhere in the polynucleotide. For example, the ITR sequence can be upstream or downstream of the E4 region. In some embodiments, the polynucleotide comprises at least one ITR sequence upstream of the E4 region. In some other embodiments, the polynucleotide comprises at least one ITR sequence downstream of the E4 region. Preferably, the ITR sequence is downstream of the E4 region.

Generally, the VA region comprises a VA RAN I region and/or a VA II region. In some embodiments, the VA RNA region comprises a VA RNA I region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 24** or having at least 80% (e.g., at least 85%, at least 90%, at least 91 %, at least 92%, at least 93%, at least 94%, at least 95%, at least 96%, at least 97%, at least 98%, at least 99% or 100%) identity to a sequence complementary to **SEQ ID NO: 24.** In some embodiments, the VA RNA region comprises a VA RNA II region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 25** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 25.**

In some preferred embodiments, the VA region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 26** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 26.**

In some embodiments, the polynucleotide comprises a E2a region comprising a nucleotide sequence encoding an adenoviral DNA binding protein (DBP). For example, the polynucleotide comprises a E2a region comprising a nucleotide sequence encoding an adenoviral DNA binding protein (DBP) and having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 27** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 27.** In some embodiments, the polynucleotide comprises a E2a region comprising a nucleotide sequence encoding a DBP having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 28** or a functional equivalent or ortholog thereof.

In some embodiments, the polynucleotide comprises a E2a region comprising an adenoviral U exon protein (UXP) exon 3 sequence. For example, the polynucleotide comprises a E2a region comprising UXP exon 3 having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 29** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 29.**

In some embodiments, the polynucleotide comprises a E2a region comprising an adenoviral E2a exon2/leader sequence. For example, the polynucleotide comprises a E2a region comprising E2a exon2/leader sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30.**

In some embodiments, the polynucleotide comprises a E2a region comprising an adenoviral UXP exon 2 sequence. For example, the polynucleotide comprises a E2a region comprising UXP exon 2 sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30.**

In some embodiments, the polynucleotide comprises a E2a region comprising an adenoviral E2a late promoter sequence. For example, the polynucleotide comprises a E2a region comprising E2a late promoter sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 31** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 31.**

In some embodiments, the polynucleotide comprises a E2a region comprising an adenoviral E2a exon 1 sequence. For example, the polynucleotide comprises a E2a region comprising E2a exon 1 sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 32** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 32.**

The E2a can be under the control of one or more promoters. In some embodiments, E2a is not under the control of a chicken β-actin promoter or a SV40 promoter.

In some embodiments, the polynucleotide comprises a E2a region comprising an adenoviral E2a early promoter sequence. For example, the polynucleotide comprises a E2a region comprising E2a early promoter sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 33** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 33.**

In some embodiments, the polynucleotide comprises a E2a region comprising an adenoviral E2a late primary transcript sequence. For example, the polynucleotide comprises a E2a region comprising E2a late primary transcript sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 34** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 34.**

In some embodiments, the polynucleotide comprises a E2a region comprising an adenoviral E2a early primary transcript sequence. For example, the polynucleotide comprises a E2a region comprising E2a early primary transcript sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 35** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 35.**

In some embodiments, the E2a region comprises a nucleotide having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 36** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 36.**

The E4 region can comprise one or more open reading frame (orf). Accordingly, in some embodiments, the polynucleotide comprises an E4 region comprising an adenoviral E4 orf6/7 sequence. For example, the polynucleotide comprises an E4 region comprising an adenoviral E4 orf6/7 sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 37** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 37.** In some embodiments, the polynucleotide comprises an E4 region encoding an adenoviral E4 orf6/7 protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 38** or a functional equivalent or ortholog thereof.

In some embodiments, the polynucleotide comprises an E4 region comprising a nucleotide sequence encoding e an adenoviral E4 34K protein having the amino acid sequence an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 39** or a functional equivalent or ortholog thereof.

In some embodiments, the polynucleotide comprises an E4 region comprising an adenoviral E4 orf4 sequence. For example, the polynucleotide comprises an E4 region comprising an adenoviral E4 orf4 sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 40** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 40.** In some embodiments, the polynucleotide comprises an E4 region encoding an adenoviral E4 orf4 protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 41** or a functional equivalent or ortholog thereof.

In some embodiments, the polynucleotide comprises an E4 region comprising an adenoviral E4 orf3 sequence. For example, the polynucleotide comprises an E4 region comprising an adenoviral E4 orf3sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 42** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 42.** In some embodiments, the polynucleotide comprises an E4 region encoding an adenoviral E4 orf3 protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 43** or a functional equivalent or ortholog thereof.

In some embodiments, the polynucleotide comprises an E4 region comprising an adenoviral E4 orf8 sequence. For example, the polynucleotide comprises an E4 region comprising an adenoviral E4 orf8 sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 44** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 44.** In some embodiments, the polynucleotide comprises an E4 region encoding an adenoviral E4 orf8 protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 45** or a functional equivalent or ortholog thereof.

In some embodiments, the polynucleotide comprises an E4 region comprising an adenoviral E4 orf1 sequence. For example, the polynucleotide comprises an E4 region comprising an adenoviral E4 orf1 sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 46** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 46.** In some embodiments, the polynucleotide comprises an E4 region encoding an adenoviral E4 orf1 protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 47** or a functional equivalent or ortholog thereof.

In some embodiments, the polynucleotide comprises an E4 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 48** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 48.** For example, the E4 region comprises a nucleotide sequence having at least 90% (e.g., at least 91%, at least 92%, at least 93%, at least 94%, at least 95%, at least 96%, at least 97%, at least 98%, at least 99% or 100%) identity to **SEQ ID NO: 48** or having at least 90% (e.g., at least 91%, at least 92%, at least 93%, at least 94%, at least 95%, at least 96%, at least 97%, at least 98%, at least 99% or 100%) identity to a sequence complementary to **SEQ ID NO: 48.**

In some embodiments, the polynucleotide comprises an E4 region comprising a nucleotide sequence having 100% identity to **SEQ ID NO: 48** or having 100% identity to a sequence complementary to **SEQ ID NO: 48.**

The E4 region can be under the control of one or more promoters. In some embodiments, E4 region isnot under the control of a chicken β-actin promoter or a SV40 promoter.

The polypeptide can also comprise a nucleotide sequence encoding an adenoviral E1 region or a functional portion thereof. In some embodiments, the polypeptide does not comprise a nucleotide sequence encoding an adenoviral E1 region or a functional portion thereof.

The polypeptide can also comprise a nucleotide sequence encoding an adenoviral protease or a functional portion thereof. In some embodiments, the polypeptide does not comprise a nucleotide sequence encoding an adenoviral protease or a functional portion thereof. In some embodiments, the adenoviral protease has the amino acid sequence **SEQ ID NO: 49.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 49** or a functional equivalent or ortholog thereof.

The polypeptide can also comprise a nucleotide sequence encoding an adenoviral E1a 13S protein or a functional portion thereof. In some embodiments, the polypeptide does not comprise a nucleotide sequence encoding an adenoviral protease or a functional portion thereof. In some embodiments, the adenoviral E1a 13S protein has the amino acid sequence **SEQ ID NO: 50.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 50** or a functional equivalent or ortholog thereof.

The polypeptide can also comprise a nucleotide sequence encoding an adenoviral E1a p12S protein or a functional portion thereof. In some embodiments, the polypeptide does not comprise a nucleotide sequence encoding an adenoviral protease or a functional portion thereof. In some embodiments, the adenoviral E1a 12S protein has the amino acid sequence **SEQ ID NO: 51.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 51** or a functional equivalent or ortholog thereof.

The polypeptide can also comprise a nucleotide sequence encoding an adenoviral E1a 11S protein or a functional portion thereof. In some embodiments, the polypeptide does not comprise a nucleotide sequence encoding an adenoviral protease or a functional portion thereof. In some embodiments, the adenoviral E1a 11S protein has the amino acid sequence **SEQ ID NO: 52.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 52** or a functional equivalent or ortholog thereof.

The polypeptide can also comprise a nucleotide sequence encoding an adenoviral E1a 10S protein or a functional portion thereof. In some embodiments, the polypeptide does not comprise a nucleotide sequence encoding an adenoviral protease or a functional portion thereof. In some embodiments, the adenoviral E1a 10S protein has the amino acid sequence **SEQ ID NO: 53.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 53** or a functional equivalent or ortholog thereof.

The polypeptide can also comprise a nucleotide sequence encoding an adenoviral E1a 9S protein or a functional portion thereof. In some embodiments, the polypeptide does not comprise a nucleotide sequence encoding an adenoviral protease or a functional portion thereof. In some embodiments, the adenoviral E1a 9S protein has the amino acid sequence **SEQ ID NO: 54.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 54** or a functional equivalent or ortholog thereof.

The polypeptide can also comprise a nucleotide sequence encoding an adenoviral E1b 19K protein or a functional portion thereof. In some embodiments, the polypeptide does not comprise a nucleotide sequence encoding an adenoviral protease or a functional portion thereof. In some embodiments, the adenoviral E1b 19K protein has the amino acid sequence **SEQ ID NO: 55.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 55** or a functional equivalent or ortholog thereof.

The polypeptide can also comprise a nucleotide sequence encoding an adenoviral E1b protein 55K protein or a functional portion thereof. In some embodiments, the polypeptide does not comprise a nucleotide sequence encoding an adenoviral protease or a functional portion thereof. In some embodiments, the adenoviral E1b protein 55K protein has the amino acid sequence **SEQ ID NO: 56.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 56** or a functional equivalent or ortholog thereof.

The polypeptide can also comprise a nucleotide sequence encoding an adenoviral hexon protein or a functional portion thereof. In some embodiments, the polypeptide does not comprise a nucleotide sequence encoding an adenoviral protease or a functional portion thereof. In some embodiments, the adenoviral E1b hexon protein has the amino acid sequence **SEQ ID NO: 57.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 57** or a functional equivalent or ortholog thereof.

The polypeptide can also comprise a nucleotide sequence encoding an adenoviral peripentonal hexon-associated protein or a functional portion thereof. In some embodiments, the polypeptide does not comprise a nucleotide sequence encoding an adenoviral protease or a functional portion thereof. In some embodiments, the adenoviral E1b peripentonal hexon-associated protein has the amino acid sequence **SEQ ID NO: 58.** Accordingly, in some embodiments, the polypeptide does not comprise a nucleotide sequence encoding a protein having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 58** or a functional equivalent or ortholog thereof.

In some embodiments, the polynucleotide is about 16,500 nucleotides or less in size. For example, the polynucleotide is about 15,500 nucleotides, about 14,000 nucleotides, about 12,500 nucleotides, about 12,000 nucleotides, about 11,000 nucleotides or less in size.

It is noted that the polynucleotide can be single-stranded or double-stranded. In some embodiments, the polypeptide is single-stranded. In some other embodiments, the polynucleotide is double-stranded. Further, the polynucleotide can be linear, circular, or close ended linear duplexed DNA (cIDNA).

In some preferred embodiments, the polynucleotide does not comprise a chicken β-actin promoter and/or an SV40 promoter. For example, the expression of E2a region and/or E4 open reading frames is not under the control of a chicken β-actin promoter and/or an SV40 promoter.

In another aspect provided herein is vector comprising a polynucleotide described herein. In some embodiments, the vector is viral vector, e.g., a retroviral vector, a lentiviral vector, an adenoviral vector, an adeno-associated viral vector, or a herpes simplex viral vector. Preferably, the viral vector is an adenoviral vector. In some embodiments, the vector does not comprise a nucleotide sequence encoding full length L-22K and/or a full length L-33K protein. For example, the vector does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 and/or 8,** or ortholog thereof.

In yet another aspect provided herein is plasmid comprising a polynucleotide described herein. In some embodiments, the plasmid does not comprise a nucleotide sequence encoding full length L-22K and/or a full length L-33K protein. For example, the vector does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 and/or 8,** or ortholog thereof.

In still another aspect, provided herein is a cell comprising a polynucleotide, vector or plasmid described herein. The cell can be an insect cell or a mammalian cell, preferably the cell is a mammalian cell. In some embodiments, the cell is a HeLa cell, COS cell, COS-1 cell, COS-7 cell, HEK293 cell, A549 cell, BHK cell, BSC-1 cell, BSC-40 cell, Vero cell, Sf"c9 cell, Sf -21 cell, Tn-368 cell, BTI-Tn-5B1-4 (High-Five) cell, Saos cell, C2C12 cell, L cell, HT1080 cell, HepG2 cell, WEHI cell, 3T3 cell, 10T1/2 cell, MDCK cell, BMT-10 cell, WI38 cell, or a primary fibroblast, hepatocyte or myoblast cells derived from a mammal. Preferably, the cell is a HEK293 cell or HeLa cell.

In some embodiments, the cell can be a suspension adapted cell.

In some embodiments, the cell is a producer cell.

In some embodiments, the cell further comprises a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR), and wherein the L-ITR and R-ITR are selected independently from AAV ITRs and adenoviral ITRs.

In some embodiments, the cell further comprises a polynucleotide encoding viral capsid (e.g., AAV capsid) and/or non-structural replication (e.g., AAV Rep) genes.

In some embodiments, the cell further comprises: (i) a polynucleotide, vector or plasmid described herein; (ii) a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR); and (iii) a polynucleotide encoding viral capsid (e.g., AAV capsid) and/or non-structural replication (e.g., AAV Rep) genes. In some embodiments, the polynucleotide of (i) the polynucleotide of (ii) are the same. Stated in another way, the the polynucleotide of (i) and the polynucleotide of (ii) are covalently linked, e.g., by an internucleotide linkage (e.g., phosphodiester bond) or by a nucleotide sequence to each other.

In some embodiments, the cell comprising a polypeptide, vector or plasmid described herein does not comprise a polynucleotide encoding a full length L-22K and/or a full length L-33K protein. For example, the cell does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8,** or ortholog thereof.

Without wishing to be bound by a theory, a polypeptide, vector, or plasmid described herein, or a cell comprising same can be used for producing recombinant adeno-associated viral (rAAV) particles. Accordingly, in yet still another aspect, the disclosure provides a method for producing recombinant adeno-associated viral (rAAV) particles, the method comprising: culturing a cell described herein in a culture medium under conditions in which rAAV particles are produced.

In one aspect, provided herein is a method for producing recombinant adeno-associated viral (rAAV) particles, the method comprising: culturing a host cell in a culture medium under conditions in which rAAV particles are produced, where the host cell comprises: (i) a polynucleotide, vector or a plasmid described herein; (ii) a polynucleotide encoding a virus genome (e.g., an AAV endogenous genome, optionally flanked by left inverted terminal repeat (L-AAV ITR) and/or right ITR (R-AAV ITR), or a recombinant AAV genome comprising polynucleotide encoding a transgene, optionally flanked by L-AAVITR and/or R-AAV ITR); and (iii) a polynucleotide encoding viral capsid (e.g., AAV capsid) and/or non-structural replication (e.g., AAV Rep) genes. In some embodiments, the polynucleotide of (i) the polynucleotide of (ii) are the same. Stated in another way, the the polynucleotide of (i) and the polynucleotide of (ii) are covalently linked, e.g., by an internucleotide linkage (e.g., phosphodiester bond) or by a nucleotide sequence to each other.

In some embodiments, the cell culture is substantially free of a polynucleotide encoding a full-length L-22K protein and/or a full-length L-33K protein. For example, the cell culture is substantially free of a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8,** or ortholog thereof.

As used herein, the term "substantially free" means that the cell culture does not comprise a polynucleotide encoding a full-length L-22K protein and/or a full-length L-33K protein in such an amount that a full-length L-22K protein and/or a full-length L-33K protein is produced in a detectable amount. Thus, in some embodiments, a full-length L-22K protein and/or a full-length L-33K protein is not expressed during the culturing of the cell. For example, a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8,** or ortholog thereof, is not expressed during the culturing of the cell.

In some embodiments, the method further comprises a step of transfecting the cell with a polynucleotide vector or plasmid described herein. For example, the method comprises a step of transfecting the cell with a polynucleotide vector or plasmid described herein, and transfecting the cell with: (i) a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR); and/or (ii) a polynucleotide encoding viral capsid (e.g., AAV capsid) and/or non-structural replication (e.g., AAV Rep) genes. In some embodiments, the polynucleotide of (i) the polynucleotide of (ii) are the same. Stated in another way, the the polynucleotide of (i) and the polynucleotide of (ii) are covalently linked, e.g., by an internucleotide linkage (e.g., phosphodiester bond) or by a nucleotide sequence to each other.

The cells can be cultured for at least 10 hours (e.g., at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours, at least 28 hours, at least 29 hours, at least 30 hours, at least 31 hours, at least 32 hours, at least 33 hours, at least 34 hours, at least 35 hours, at least 36 hours, at least 37 hours, at least 38 hours, at least 39 hours, at least 40 hours, at least 41 hours, at least 42 hours, at least 43 hours, at least 44 hours, at least 45 hours, at least 46 hours, at least 47 hours, at least 48 hours, at least 49 hours, at least 50 hours, at least 51 hours, at least 52 hours, at least 53 hours, at least 54 hours, at least 55 hours, at least 56 hours, at least 57 hours, at least 58 hours, at least 59 hours, at least 60 hours, at least 61 hours, at least 62 hours, at least 63 hours, at least 64 hours, at least 65 hours, at least 66 hours, at least 67 hours, at least 68 hours, at least 69 hours, at least 70 hours, at least 71 hours, at least 72 hours, at least 73 hours, at least 74 hours, at least 75 hours, at least 76 hours, at least 77 hours, at least 78 hours, at least 79 hours, at least 80 hours, at least 81 hours, at least 82 hours, at least 83 hours, at least 84 hours, at least 85 hours, at least 86 hours, at least 87 hours, at least 88 hours, at least 89 hours, at least 90 hours, at least 91 hours, at least 92 hours, at least 93 hours, at least 94 hours, at least 95 hours, at least 96 hours, at least 97 hours, at least 98 hours, at least 99 hours, at least 100 hours or more).

In some embodiments, the method further comprises a step, e.g., a post-lysis step of removing or reducing an amount of impurities, e.g., hcDNA from the cell culture or cell culture supernatant. Without limitation, the step of removing or reducing the amount of impurities can comprise adding a cationic amine or nuclease to the cell culture or cell culture supernatant. In some embodiments, the step of removing or reducing the amount of impurities comprises adding a selective precipitation agent to the cell culture or cell culture supernatant.

In some embodiments, the method further comprises a step of clarifying the cell culture or cell culture supernatant. Without limitations, the step of clarifying the cell culture or cell culture supernatant can comprise depth filtration.

In some embodiments, the method further comprises a step of concentrating the clarified cell culture or cell culture supernatant, e.g., via tangential flow filtration.

In some embodiments, the method further comprises a step of enriching or purifying rAAV particles. For example, the method further comprises a step of enriching or purifying rAAV particles by chromatography, e.g., affinity chromatography and/or anion exchange chromatography.

In some embodiments, wherein at least about 50%, (e.g., at least about 55%, at least about 60%, at least about 65%, at least about 70%, at least about 75%, at least about 80%, at least about 85%, at last about 95%, at least about 95% or a higher percentage) of the rAAV particles produced are full capsid particles.

In some embodiments, a method described herein produces at least about 1 x 10⁴ (e.g., e.g., at least about 2 x 10⁴, 3 x 10⁴, 4 x 10⁴, 5 x 10⁴, 7 x 10⁴, 7 x 10⁴, 8 x 10⁴, 9 x 10⁴, or 1 x 10⁵ or more) vector genome-containing particles per cell, e.g., prior to or after purification.

In some embodiments, a method described herein provides at least about 1e¹² vector genome per ml (vg/ml) (e.g., at least about 1.5e¹² vg/ml, at least about 2e¹² vg/ml, at least about 2.5e¹²vg/ml, at least about 3e¹² vg/ml, at least about 3.5e¹² vg/ml, at least about 4e¹² vg/ml, at least about 4.5e¹² vg/ml, at least about 5e¹² vg/ml, at least about 5.5e¹² vg/ml, at least about 6e¹² vg/ml, at least about 6.5e¹² vg/ml, at least about 7e¹² vg/ml, at least about 7.5e¹² vg/ml, at least about 8e¹² vg/ml, at least about 8.5e¹² vg/ml, at least about 9e¹² vg/ml, at least about 9.5e¹³ vg/ml, at least about 1e¹³vg/ml, at least about 1.5e¹³vg/ml, at least about 2e¹³vg/ml, at least about 2.5e¹³vg/ml, at least about 3e¹³vg/ml, at least about 3.5e¹³vg/ml, at least about 4e¹³ vg/ml, at least about 4.5e¹³ vg/ml, at least about 5e¹³ vg/ml, at least about 5.5e¹³ vg/ml, at least about 6e¹³ vg/ml, at least about 6.5e¹³ vg/ml, at least about 7e¹³vg/ml, at least about 7.5e¹³vg/ml, at least about 8e¹³vg/ml, at least about 8.5e¹³vg/ml, at least about 9e¹³vg/ml, at least about 9.5e¹³ vg/ml, at least about 1e¹⁴ vg/ml or more), e.g., prior to or after purification, such as after affinity purification.

In some embodiments, a method described herein provides at least about 1e¹² viral particles per ml (vp/ml) at least about 1e¹² viral particles per ml (vp/ml) (e.g., at least about 1.5e¹² vp/ml, at least about 2e¹² vp/ml, at least about 2.5e¹² vp/ml, at least about 3e¹² vp/ml, at least about 3.5e¹² vp/ml, at least about 4e¹² vp/ml, at least about 4.5e¹² vp/ml, at least about 5e¹² vp/ml, at least about 5.5e¹² vp/ml, at least about 6e¹² vp/ml, at least about 6.5e¹² vp/ml, at least about 7e¹² vp/ml, at least about 7.5e¹² vp/ml, at least about 8e¹² vp/ml, at least about 8.5e¹² vp/ml, at least about 9e¹² vp/ml, at least about 9.5e¹³ vp/ml, at least about 1e¹³vp/ml, at least about 1.5e¹³vp/ml, at least about 2e¹³vp/ml, at least about 2.5e¹³vp/ml, at least about 3e¹³vp/ml, at least about 3.5e¹³vp/ml, at least about 4e¹³ vp/ml, at least about 4.5e¹³ vp/ml, at least about 5e¹³ vp/ml, at least about 5.5e¹³ vp/ml, at least about 6e¹³ vp/ml, at least about 6.5e¹³ vp/ml, at least about 7e¹³vp/ml, at least about 7.5e¹³vp/ml, at least about 8e¹³vp/ml, at least about 8.5e¹³vp/ml, at least about 9e¹³vp/ml, at least about 9.5e¹³ vp/ml, at least about 1e¹⁴ vp/ml or more), e.g., prior to or after purification, such as after affinity purification.

Without limitations, the rAAV particles can be AAV-1, AAV-2, AAV-2i8, AAV-3, AAV-4, AAV-5, AAV-6, AAV-7, AAV-8, AAV-9, AAV-10, AAVrh10, AAV-11, AAV-12, AAV-13, AAV-14, AAV-15, AAV-16 or a chimera, derivative, modification, or pseudotype thereof.

In some embodiments, the rAAV particle comprises at least one capsid protein (e.g., VP1, VP2, or VP3) from the AAV serotypes listed in **Table 2.**

Without wishing to be bound by a theory, a polynucleotide described herein is capable of producing recombinant adeno-associated viral (rAAV) particles in an amount that is at least 80% of an amount produced under similar conditions with a similar polynucleotide that further comprises a nucleotide sequence encoding a full-length L-22K protein and/or a full-length L-33K protein.

Again, without wishing to be bound by a theory, a packaging efficiency of the polynucleotide in a method of producing rAAV is at least 80% of a packaging efficiency of a similar polypeptide that further comprises a nucleotide sequence encoding a full-length L-22K protein and/or a full-length L-33K protein.

In yet another aspect, the disclosure provides a recombinant adeno-associated virus in combination with a polynucleotide, vector, plasmid, or cell described herein.

Sequences are shown in **Table 3.**

### BRIEF DESCRIPTION OF THE DRAWINGS

The patent or application file contains at least one drawing executed in color. Copies of this patent or patent application publication with **color drawings** will be provided by the Office upon request and payment of the necessary fee.

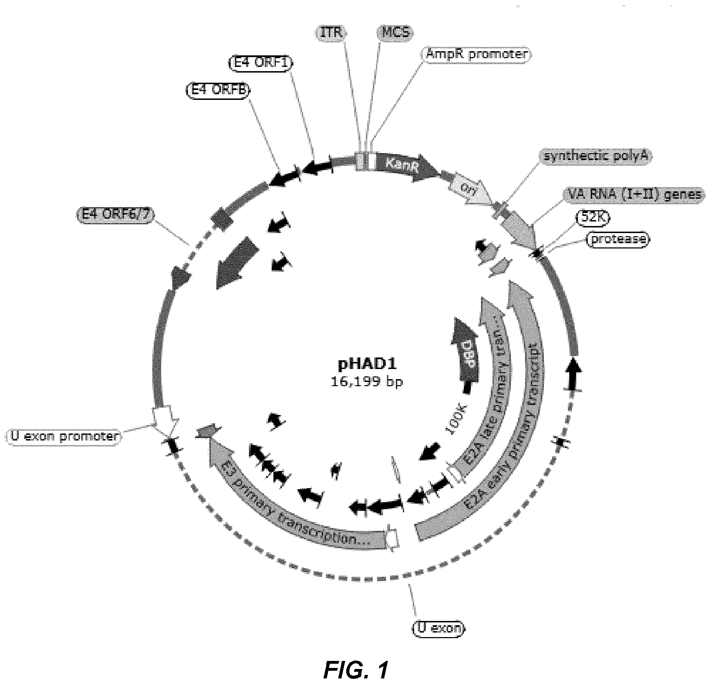

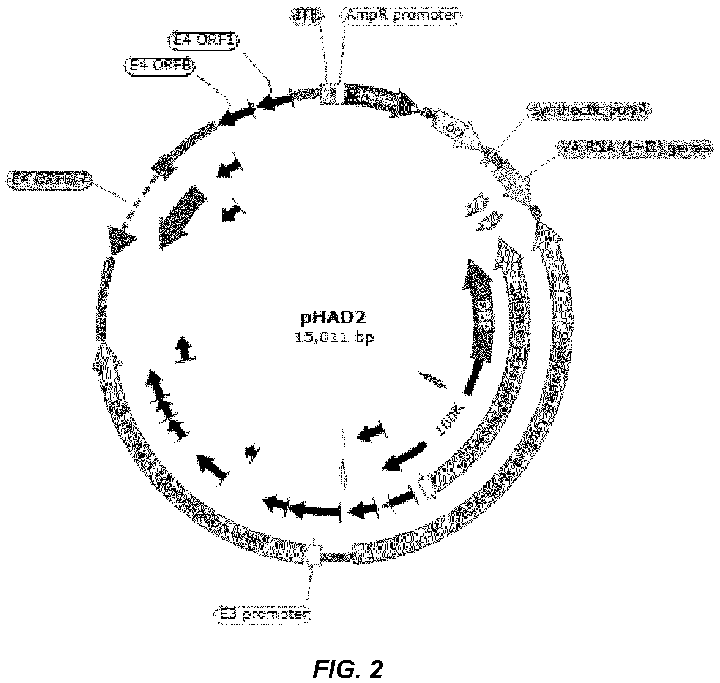

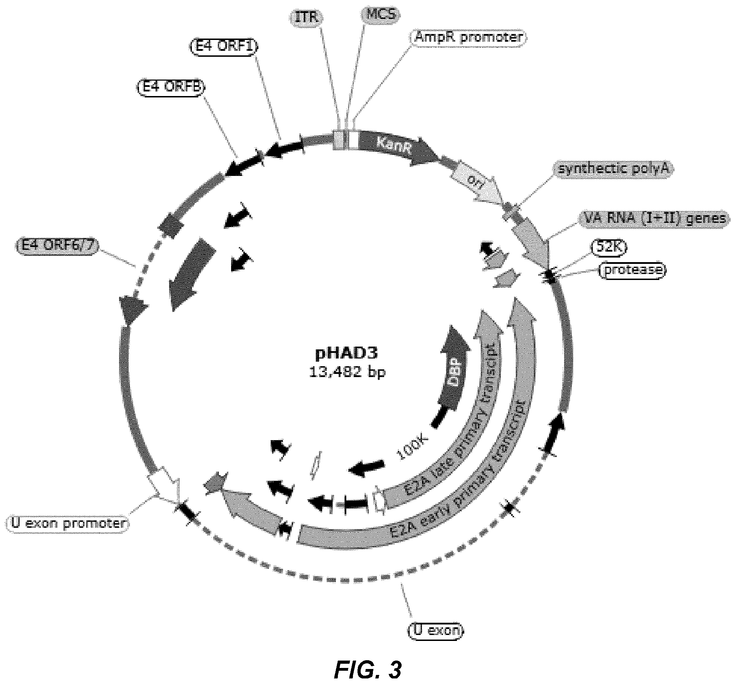

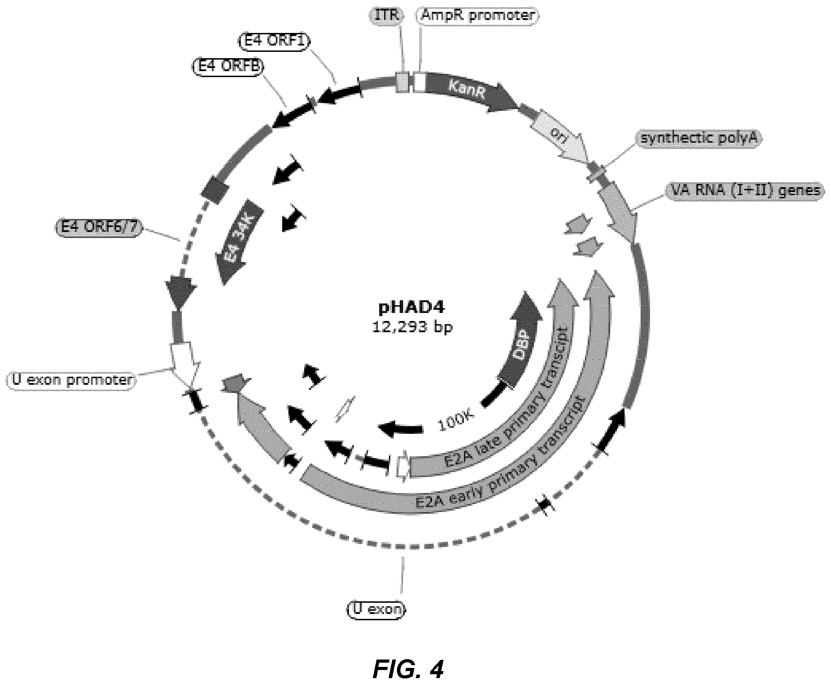

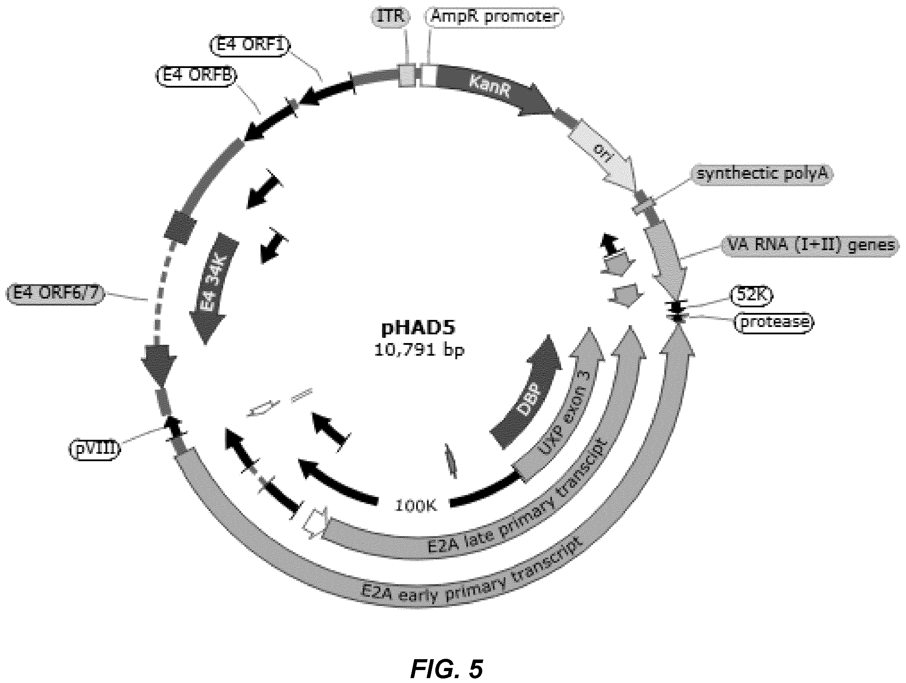

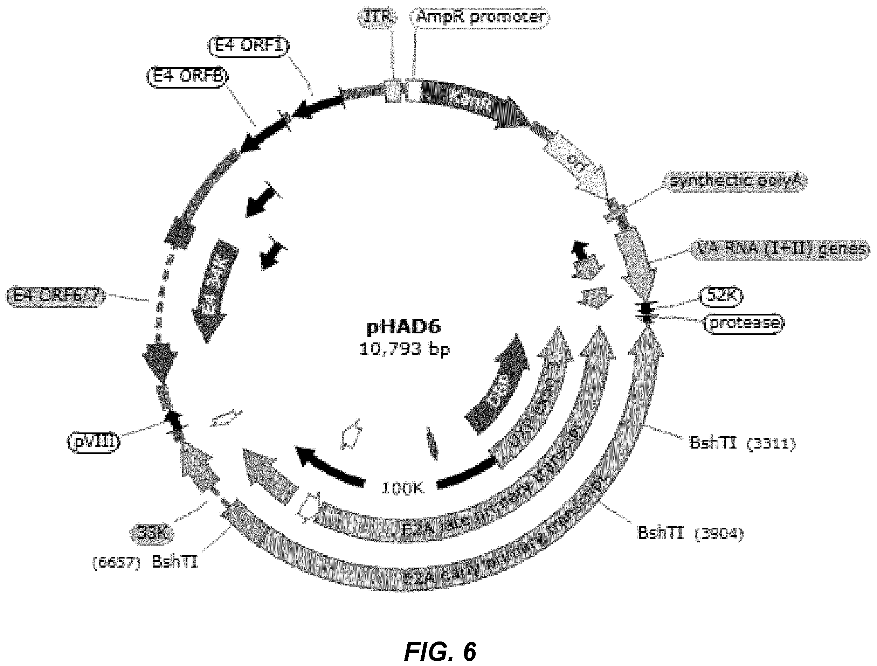

**FIGS. 1-6** are schematic sequence map of exemplary helper plasmids pHAD1 (**SEQ ID NO: 1,** **FIG. 1**), pHAD2 (**SEQ ID NO: 2,** **FIG. 2**), pHAD3 (**SEQ ID NO: 3,** **FIG. 3**), pHAD4 (**SEQ ID NO: 4,** **FIG. 4**), pHAD5 (**SEQ ID NO: 5,** **FIG. 5**), and pHAD6 (**SEQ ID NO: 6,** **FIG. 6**).

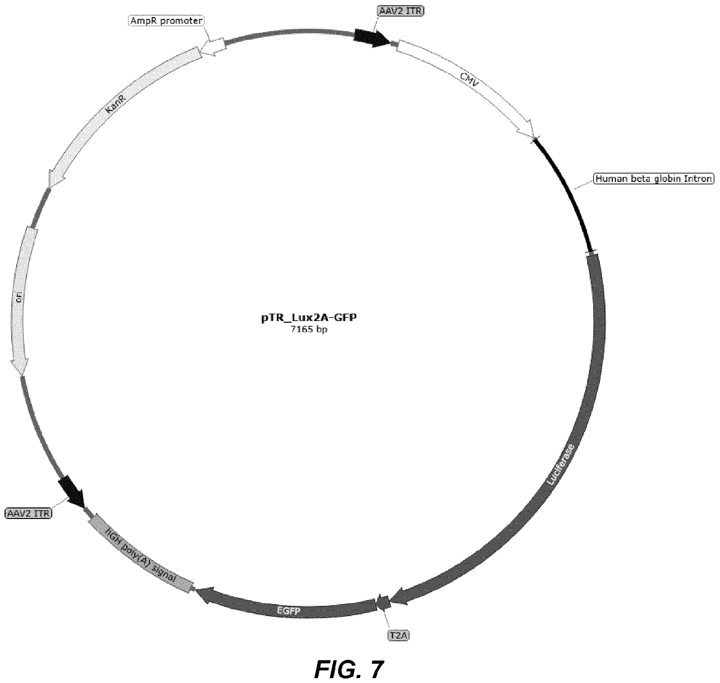

**FIG.** 7 is a schematic sequence map of TR_Lux2A-GFP recombinant AAV vector plasmid (**SEQ ID NO: 77**).
**FIG. 8** shows AAV8 total vector particles (VP) and vector genomes (VG) obtained through affinity purification from small-scale (62 mL) triple transfections with exemplary helper plasmids. Small-scale AAV productions were performed in duplicate with each helper plasmid. Vector particles titers were measured by SEC-HPLC and vector genomes titers by ITR qPCR.
**FIG. 9** shoes AAV6 total vector particles (VP) and vector genomes (VG) obtained through affinity purification from small-scale (62 mL) triple transfections with exemplary helper plasmids. Small-scale AAV productions were performed in duplicate with each helper plasmid. Vector particles titers were measured by SEC-HPLC and vector genomes titers by ITR qPCR.
**FIG. 10** shows AAV2, AAV8 and AAV9 vector particles (vp) and vector genomes (vg) titers obtained through affinity purification from small-scale (125 mL) triple transfections with exemplary helper plasmids. Small-scale AAV productions were performed in duplicate with each of the seven different helper plasmids. Vector particles titers were measured by capsid ELISA and vector genomes titers by ITR qPCR.
**FIG. 11** shows AAV8 vectors titers and total yields obtained after purification from 2-liter bioreactors with exemplary plasmids. Vector genomes were quantified by ITR qPCR and ITR ddPCR, and viral particles by SEC-HPLC.
**FIG. 12** shows residual plasmid sequences concentrations in purified AAV8 vectors produced in 2-liter bioreactors. Residual plasmid DNA concentration was measured by ddPCR targeting the E4 region in the helper plasmids, the Rep coding sequence in the rep-cap plasmid, and the kanamycin-resistance gene present in all three plasmid backbones. The PCR targets concentrations (copies/mL) are presented in logarithmic scale.
**FIG. 13** depicts AAV8 vector purity by SDS-PAGE and silver staining. Vectors produced with the different helper plasmids were loaded onto duplicate wells.
**FIG. 14** shows percentages of empty, partial, and full vector particles in AAV8 final products as determined by AUC.
**FIG. 15** shows luciferase transgene expression following transduction of GM16095 cells with purified AAV8 vectors. Vectors were added to the cells at a multiplicity of 5E+5 or 1E+6 VG/cell, and luciferase activity was measured after 48-hours and normalized to total proteins concentration.
**FIG. 16** shows AAV6 vectors titers and total yields obtained after purification from 2-liter bioreactors. Vector genomes were quantified by ITR qPCR and ITR ddPCR, and viral particles by SEC-HPLC.
**FIG. 17** shows residual plasmid sequences concentrations in purified AAV6 vectors produced in 2-liter bioreactors. Residual plasmid DNA concentration was measured by ddPCR targeting the E4 region in the helper plasmids, the Rep coding sequence in the rep-cap plasmid, and the kanamycin-resistance gene present in all three plasmid backbones. The PCR targets concentrations (copies/mL) are presented in logarithmic scale.
**FIG. 18** shows AAV6 vector purity by SDS-PAGE and silver staining. Vectors produced with the different helper plasmids were loaded onto duplicate wells.
**FIG. 19** shows percentages of empty, partial, and full vector particles in AAV6 final products as determined by AUC.
**FIG. 20** shows luciferase transgene expression following transduction of GM16095 cells with purified AAV6 vectors. Vectors were added to the cells at a multiplicity of 1E+5 or 5E+5 VG/cell, and luciferase activity was measured after 48-hours and normalized to total proteins concentration.
**FIG. 21** shows AAV9 vector particles (VP) and vector genomes (VG) titers obtained through affinity purification from small-scale (62 mL) triple transfections.VP were measured by SEC-HPLC and VG by ITR ddPCR and ITR qPCR. Statistical analysis was performed by one-way ANOVA with Kruskal Wallis test and Dunn's correction for multiple comparison.
**FIG. 22** shows percentages of empty, partial, and full vector particles in affinity-purified small-scale AAV9 vectors as determined by AUC. The graph represents mean values of triplicate transfections.
**FIG. 23** shows AAV9 vector particles (VP) and vector genomes (VG) titers obtained after purification from 2-liter bioreactors. Viral particles were quantified by SEC-HPLC and vector genomes by ITR qPCR and ITR ddPCR.
**FIG. 24** shows residual plasmid sequences concentrations in purified AAV9 vectors produced in 2-liter bioreactors. Residual plasmid DNA concentration was measured by ddPCR targeting the Rep coding sequence in the rep-cap plasmid, the E4 region in the helper plasmid, and the kanamycin-resistance gene present in all three plasmid backbones.
**FIG. 25** shows AAV9 vector purity by SDS-PAGE and silver staining. Vectors produced with the different helper plasmids were loaded onto duplicate wells.
**FIG. 26** shows percentages of empty, partial, and full vector particles in AAV9 vectors purified from 2-Liters bioreactors as determined by AUC.
**FIG. 27** shows luciferase transgene expression following transduction of HeLa cells with purified AAV9 vectors. Vectors were added to the cells at a multiplicity of 5E+5 or 1E+6 VG/cell, and luciferase activity was measured after 48-hours and normalized to total proteins concentration.
**FIG. 28** shows AAV8 vector genomes concentration (VG/mL) obtained in duplicate small-scale (62 mL) triple transfection in the transfected cells pool at harvest (TP), in the bulk lysate (BL), and in the bulk virus pool after affinity purification (BVP). VG were measured by ITR qPCR.
**FIG. 29** shows AAV6 vector genomes (VG/mL) concentration obtained in duplicate small-scale (62 mL) triple transfection in the transfected cells pool at harvest (TP), in the bulk lysate (BL), and in the bulk virus pool after affinity purification (BVP). VG were measured by ITR qPCR.
**FIG. 30** shows AAV9 vector genomes concentration (VG/L) obtained in triplicate small-scale (62 mL) triple transfection in the in the bulk lysate (BL), and in the bulk virus pool after affinity purification (BVP). VG were measured by ITR qPCR in the bulk lysate (BL), and in the bulk virus pool after affinity purification (BVP).

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise.

The section headings used herein are for organizational purposes only and are not to be construed as limiting the subject matter described. All documents, or portions of documents, cited in this application, including, but not limited to, patents, patent applications, articles, books, and treatises, are hereby expressly incorporated by reference in their entirety for any purpose.

In some embodiments, the polynucleotides described herein are smaller than the leading commercially available adenovirus helper plasmids. For example, the polynucleotides described herein have a reduced overall size relative to presently available adenoviral helper plasmids such as pXX680 at 18.932 kbp, pALD-X80 at 18.876 kbp, pFAdDeltaF6 at 15.420 kbp, and/or pHelper at 11.635 kbp.

In some embodiments, a polynucleotide of the present disclosure is approximately between about 6 kb and 17 kb. In some embodiments, a polynucleotide of the present disclosure has a size that is about 6 kb, about 6.5 kb, about 7 kb, about 7.5 kb, about 8 kb, about 8.5 kb, about 9 kb, about 9.5 kb, about 10 kb, about 10.5 kb, about 11 kb, about 11.5 kb, about 12 kb, about 12.5 kb, about 13 kb, about 13.5 kb, about 14 kb, about 14.5 kb, about 15 kb, about 15.5 kb, about 16 kb, about 16.5 kb or about17kb. In some embodiments, a polynucleotide of the present disclosure has a size that is about 6-7 kb; about 6.5-7.5 kb; about 7-8 kb; about 7.5-8.5 kb; about 8-9 kb; about 8.5-9.5 kb; about 9-10 kb; about 9.5-10.5 kb; about 10-11 kb; about 10.5-11.5 kb; about 11-12 kb; about 11.5-12.5 kb; about 12-13 kb; about 12.5-13.5 kb; about 13-14 kb; about 13.5-14.5 kb; about 14-15 kb; about 14.5-15.5kb; about 15-16kb; about 15.5-16.5kb; or about 16-17kb.

In some embodiments, a polynucleotide described herein can be at most about 7900, at most about 8000, at most about 8100, at most about 8200, at most about 8300, at most about 8400, at most about 8500, at most about 8600, at most about 8700, at most about 8800, at most about 8900, at most about 9000, at most about 9100, at most about 9200, at most about 9300, at most about 9400, at most about 9500, at most about 9600, at most about 9700, at most about 9800, at most about 9900, at most about 10000, at most about 10100, at most about 10200, at most about 10300, at most about 10400, at most about 10500, at most about 10600, at most about 10700, at most about 10800, at most about 10900, at most about 11000, at most about 11100, at most about 11200, at most about 11300, at most about 11400, at most about 11500, at most about 11600, at most about 11700, at most about 11800, at most about 11900, at most about 12000, at most about 12100, at most about 12200, at most about 12300, at most about 12400, at most about 12500, at most about 12600, at most about 12700, at most about 12800, at most about 12900, at most about 13000, at most about 13100, at most about 13200, at most about 13300, at most about 13400, at most about 13500, at most about 13600, at most about 13700, at most about 13800, at most about 13900, at most about 14000, at most about 14100, at most about 14200, at most about 14300, at most about 14400, at most about 14500, at most about 14600, at most about 14700, at most about 14800, at most about 14900, at most about 15000, at most about 15100, at most about 15200, at most about 15300, at most about 15400, at most about 15500, at most about 15600, at most about 15700, at most about 15800, at most about 15900, at most about 16000, at most about 16100, at most about 16200, at most about 16300, at most about 16400, at most about 16500, at most about 16600, at most about 16700, at most about 16,800, at most about 16,900, or at most about 17,000 nucleotides in length.

In some embodiments, a polynucleotide described herein is less than about 17,000 nucleotides, or less than about 16,750 nucleotides, or less than about 16,500 nucleotides, or less than about 16,250 nucleotides, or less than about 16,000 nucleotides, or less than about 15,750 nucleotides, or less than about 15,500 nucleotides, or less than about 15,250 nucleotides, or less than about 15,000, or less than about 14,750 nucleotides, or less than about 14,500 nucleotides, or less than about 14,250 nucleotides, or less than about 14,000 nucleotides, or less than about 13,750 nucleotides, or less than about 13,500 nucleotides, or less than about 13,250 nucleotides, or less than about 13,000 nucleotides, or less than about 12,750 nucleotides, or less than about 12,500 nucleotides, or less than about 12,250 nucleotides, or less than about 12,000 nucleotides, or less than about 11,750 nucleotides, or less than about 11,500 nucleotides, or less than about 11,250 nucleotides, or less than about 11,000 nucleotides, or less than about 10,750 nucleotides, or less than about 10,500 nucleotides, or less than about 10,250 nucleotides, or less than about 10,000 nucleotides, or less than about 9,750 nucleotides, or less than about 9,500 nucleotides, or less than about 9,250 nucleotides, or less than about 9,000 nucleotides, or less than about 8,750 nucleotides, or less than about 8,500 nucleotides, or less than about 8,250 nucleotides, or less than about 8,000 nucleotides, or less than about 7,750 nucleotides, or less than about 7,500 nucleotides, in length.

Without wishing to be bound by a theory, the polynucleotides described herein allow for safer and less costly production of rAAVs in producer cell expression systems. The smaller size of the polynucleotides describe herein enables the simpler and less costly production of AAV at the quantities necessary for large-scale manufacturing of AAV. In addition, removing genes and/or portions of genes makes a polynucleotide described herein can be safer because the producing cells would not produce the adenovirus structural proteins (e.g., fiber), that could co-purify with AAV during downstream processing and would therefore present a lower risk of inadvertently introducing adenovirus structural proteins to patients. Further, removal of helper genes resulting in a smaller size enables addition of supplementary genes to further improve AAV quality and yield. Although these supplementary genes increase the size of the polypeptide relative to the smallest versions, they enable comparable or higher AAV productivity and are therefore worth the additional cost to produce. Importantly, these polynucleotides are still smaller than commercially available helper plasmids such as, for example pXX6-80, pALD-X80, pFAdDeltaF6, and pHelper.

It is known in the art that the adenovirus late proteins, L4-22K/L4-33K, are essential for recombinant adeno-associated viral (rAAV) production. See for example, Adsero et al., "A Novel Role for the Adenoviral L4 region 22K and 33K Proteins in Adeno-Associated Virus Production." Human Gene Therapy (2024), 35(1-2):59); (stating: "the adenoviral 22K protein is essential for rAAV production" and "the 33K protein synergistically increases rAAV yield" (Abstract)) and Su et al., "AAV production in stable packaging cells requires expression of adenovirus 22/33K protein to allow episomal amplification of integrated rep/cap genes." Scientific Reports (2023), 13:21670 (stating: "expression of the adenovirus L4 22/33K unit is essential for rep/cap amplification" (Abstract)). See also WO2024107985 (stating: "these data indicate that the L4 region (containing 33K and 22K ORFs), JEP/GEP regional requirements, and adequate E2a production is important for the production of AAV" (para. [0120]). However, contrary to what the prior teaches, polypeptides described herein that do not encode for a full length L-22K protein and/or a full length L-33K protein unexpectedly and surprisingly can produce rAAV in an amount substantially similar to the amount of rAAV produced with a similar polynucleotide that also encodes for a full-length L-22K protein and/or a full-length L-33K protein. In addition, the packaging efficiency of the polynucleotides that don't encode for a full-length length L-22K protein and a full-length L-33K protein is also unexpectedly and surprisingly substantially similar to the packaging efficiency of a similar polynucleotide that also encodes for a full-length L-22K protein and/or a full-length L-33K protein.

Thus, without wishing to be bound by a theory, a polypeptide described herein that does not include a nucleotide sequence encoding a full length L-22K protein and/or a full length L-33K protein can produce recombinant adeno-associated viral (rAAV) particles in an amount that is at least 80% (e.g., at least 85%, at least 90%, at least 95%, at least 100% (i.e., substantially similar), at least 105%, at least 110%, at least 115%, at least 120%, at least 125%, at least 130%, at least 135%, at least 140%, at least 145%, at least 150%) or higher of an amount produced under similar conditions with a similar polynucleotide that further comprises a nucleotide sequence encoding a full-length L-22K protein and/or a full-length L-33K protein.

Without wishing to be bound by a theory, a packaging efficiency of a polypeptide described herein that does not include a nucleotide sequence encoding a full length L-22K protein and/or a full length L-33K protein in a method of producing rAAV can be at least 80% (e.g., at least 85%, at least 90%, at least 95%, at least 100% (i.e., substantially similar), at least 105%, at least 110%, at least 115%, at least 120%, at least 125%, at least 130%, at least 135%, at least 140%, at least 145%, at least 150%) or more of a packaging efficiency of a similar polypeptide that further comprises a nucleotide sequence encoding a full-length L-22K protein and/or a full-length L-33K protein.

A polynucleotide described herein can be single-stranded or double-stranded. In some embodiments of any one of the aspects described herein, the polynucleotide is linear, e.g., a linear DNA. In some embodiments of any one of the aspects described herein the polynucleotide is circular. For example, the polynucleotide is a closed ended linear duplex DNA (cIDNA). Alternatively, clDNA is termed as no-end DNA or, neDNA. Exemplary closed linear duplexed DNA molecules, or no-end DNA molecules include, but are not limited to, doggybone DNA (dbDNA), and dumbbell shaped DNA.

### L4-22K protein

In some embodiments, a polynucleotide described herein does not comprise a nucleotide sequence encoding at least a portion of L4-22K protein. For example, a polynucleotide described herein does not comprise a nucleotide sequence encoding at least a portion of L4-22K protein from an adenovirus, e.g., Ad5. In some embodiments, a polynucleotide described herein does not comprise a nucleotide sequence encoding a full-length L4-22K protein. For example, a polynucleotide described herein does not comprise a nucleotide sequence encoding a full-length L4-22K protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 7** (Ad5 L4-22K).

In some embodiments, the polynucleotide does not comprise a nucleotide sequence encoding a protein having, e.g., at its N-terminus an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 94 or 95.**

In some embodiments, the polynucleotide comprises a deletion of G₃ and G₄ in a nucleotide sequence encoding an adenoviral L-22K protein. For example, the polynucleotide does not comprise nucleotides 26197-26198 relative to Genbank Accession Number AC_000008.

Inventors have discovered *inter alia* that AAV production with polynucleotides described herein that lack a sequence encoding a full length L4-22K and/or L4-33K protein unexpectedly and surprisingly is better compared to similar polypeptides that encode for a full length L4-22K protein and/or L4-33K protein. In other words, polypeptides that encode only for a fragment of L4-22K and/or L4-33K protein unexpectedly and surprisingly can provide a more efficient helper function for AAV production.

Thus, in some embodiments, a polynucleotide described herein comprises a nucleotide sequence encoding a fragment, i.e., less than full-length of **SEQ ID NO: 7** (Ad5 L4-22K) or a functional equivalent thereof. For example, the polynucleotide comprises nucleotide sequence encoding a truncated L4-22K protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 92 or 93.** In some embodiments, the truncated L4-22K protein is at least 2 (e.g., 3, 4, 5, 6, 7, 8, 9, 10 or more) amino acids shorter than the full-length L4-22K protein. For example, the truncated L4-22K protein encoded by the polypeptide is at least 11 (e.g., 12, 13, 14, 15, 16, 17, 18, 19, 20 or more) amino acids shorter than the full-length L4-22K protein. In some embodiments, the truncated L4-22K protein encoded by the polypeptide is at least 21 (e.g., 22, 23, 24, 25, 26, 27, 28, 29, 30, or more) amino acids shorter than the full-length L4-22K protein. For example, the truncated L4-22K protein encoded by the polypeptide is at least 31 (e.g., 32, 33, 34, 35 or more) amino acids shorter than the full-length L4-22K protein.

In some embodiments, the truncated L4-22K protein is no more than 180 (e.g., no more than 179, 178, 176, 175, 174, 173, 172, 171, 170, 169, 168, 167, 166, 165 or less) amino acids in length. In some embodiments, the truncated L4-22K protein is about 155 to about 165 amino acids in length. For example, the truncated L4-22K protein is about 156, 157, 158, 159, 160, 161, 162, 163 or 164 amino acids in length. In some embodiments, the truncated L4-22K protein is 160, 161, 162, 163, or 164 amino acids in length. In a preferred embodiment, the truncated L4-22K protein is about 162 amino acids in length.

In some embodiments, the truncated L4-22K protein is about 115 to about 125 amino acids in length. For example, the truncated L4-22K protein is about 116, 117, 118, 119, 120, 111, 122, 123 or 144 amino acids in length. In some embodiments, the truncated L4-22K protein is 118, 119, 120, 121, or 122 amino acids in length. In a preferred embodiment, the truncated L4-22K protein is about 120 amino acids in length.

In some embodiments, the polynucleotide comprises nucleotide sequence encoding a truncated L4-22K protein comprising or consisting of an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a L4-22K protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.**

In some embodiments, the polynucleotide comprises nucleotide sequence encoding a truncated L4-22K protein comprising consisting of an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a L4-22K protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7,** and having a length of at least 175 (e.g., 176, 177, 178, 179, 180, 181, 182, 183, 184, 185, 186, 187, 188, 189, 190, 191, 192, 193, 194, 195 or more) amino acids.

In some embodiments, the truncated L4-22K protein does not comprise an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 94 or 95,** e.g., at its N-terminus.

In some embodiments, the polynucleotide comprises nucleotide sequence encoding a truncated L4-22K protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 92,** and wherein the truncated L4-22K protein does not comprise an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 94,** e.g., at its N-terminus.

In some embodiments, the polynucleotide comprises nucleotide sequence encoding a truncated L4-22K protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 93,** and wherein the truncated L4-22K protein does not comprise an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 95,** e.g., at its N-terminus.

The term "truncated" in relation to a protein relates to a shortened form of the protein, e.g., where at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more) amino acids from the N- and/or C-terminus of the protein are deleted. It is noted that the truncated form of the protein may or may not retain the function of the parent protein.

### L4-33K protein

In some embodiments, a polynucleotide described herein does not comprise a nucleotide sequence encoding at least a portion of L4-33k protein. For example, a polynucleotide described herein does not comprise a nucleotide sequence encoding at least a portion of L4-33k protein from an adenovirus, e.g., Ad5. In some embodiments, a polynucleotide described herein does not comprise a nucleotide sequence encoding a full-length L4-33k protein. For example, a polynucleotide described herein does not comprise a nucleotide sequence encoding a full-length L4-33k protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 8** (Ad5 L4-33k).

In some embodiments, the polynucleotide does not comprise a nucleotide sequence encoding a protein having, e.g., at its N-terminus an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 94 or 95.**

In some embodiments, the polynucleotide comprises a deletion of G₃ and G₄ in a nucleotide sequence encoding an adenoviral L-33K protein. For example, the polynucleotide does not comprise nucleotides 26197-26198 relative to Genbank Accession Number AC_000008.

In some embodiments, a polynucleotide described herein comprises a nucleotide sequence encoding a fragment, i.e., less than full-length of **SEQ ID NO: 8** (Ad5 L4-33k) or a functional equivalent thereof. For example, the polynucleotide comprises nucleotide sequence encoding a truncated L4-33k protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 90 or 91.** In some embodiments, the truncated L4-33k protein is at least 2 (e.g., 3, 4, 5, 6, 7, 8, 9, 10 or more) amino acids shorter than the full-length L4-33k protein. For example, the truncated L4-33k protein encoded by the polypeptide is at least 11 (e.g., 12, 13, 14, 15, 16, 17, 18, 19, 20 or more) amino acids shorter than the full-length L4-33k protein. In some embodiments, the truncated L4-33k protein encoded by the polypeptide is at least 21 (e.g., 22, 23, 24, 25, 26, 27, 28, 29, 30, or more) amino acids shorter than the full-length L4-33k protein. For example, the truncated L4-33k protein encoded by the polypeptide is at least 31 (e.g., 32, 33, 34, 35 or more) amino acids shorter than the full-length L4-33k protein.

In some embodiments, the truncated L4-33k protein is no more than 215 (e.g., no more than 214, 213, 212, 211, 210, 209, 208, 206, 205 or less) amino acids in length. In some embodiments, the truncated L4-33k protein is about 195 to about 205 amino acids in length. For example, the truncated L4-33k protein is about 196, 197, 198, 199, 200, 201, 202, 203 or 204 amino acids in length. In some embodiments, the truncated L4-33k protein is 198, 199, 200, 201, or 202 amino acids in length. In a preferred embodiment, the truncated L4-33k protein is about 200 amino acids in length.

In some embodiments, the truncated L4-33k protein is about 150 to about 160 amino acids in length. For example, the truncated L4-33k protein is about 151, 152, 153, 154, 155, 156, 157, 158, 158, or 159 amino acids in length. In some embodiments, the truncated L4-33k protein is 151, 152, 153, 154 or 155amino acids in length. In a preferred embodiment, the truncated L4-33k protein is about 153 amino acids in length.

In some embodiments, the polynucleotide comprises nucleotide sequence encoding a truncated L4-33k protein comprising or consisting of an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the polynucleotide does not comprise a nucleotide sequence encoding a L4-33k protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.**

In some embodiments, the polynucleotide comprises nucleotide sequence encoding a truncated L4-33k protein comprising consisting of an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the polynucleotide does not comprise a nucleotide sequence encoding a L4-33k protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8,** and having a length of at least 175 (e.g., 176, 177, 178, 179, 180, 181, 182, 183, 184, 185, 186, 187, 188, 189, 190, 191, 192, 193, 194, 195 or more) amino acids.

In some embodiments, the truncated L4-33k protein does not comprise an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 94 or 95,** e.g., at its N-terminus.

In some embodiments, the polynucleotide comprises nucleotide sequence encoding a truncated L4-33k protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 90,** and wherein the truncated L4-33k protein does not comprise an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 94,** e.g., at its N-terminus.

In some embodiments, the polynucleotide comprises nucleotide sequence encoding a truncated L4-33k protein comprising an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 91,** and wherein the truncated L4-33k protein does not comprise an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 95,** e.g., at its N-terminus.

### VA RNA Region

A polynucleotide described herein comprises a nucleotide sequence encoding a VA RNA region. As used herein, the term "VA RNA region" refers to a nucleotide sequence that produces virus or viral associated RNAs (VA RNAs). VA RNAs interfere with one or more host systems that interface with double-stranded RNAs (dsRNAs), such as their sensing by protein kinase R (PKR), export by Exportin-5, processing by Dicer, editing by ADAR, or activation of oligoadenylate synthetases (OAS), etc. Without wishing to be bound by a theory, VA RNAs improve the efficiency of translation, increase the stability of capsid mRNA, and/or help prevent degradation of one or more gene products, e.g., a Rep protein. It is noted that the VA RNA region can comprises a nucleotide sequence encoding a wild-type VA RNA, a derivative thereof, or an artificial VA RNA having an equivalent function to a wild-type VA RNA. Generally, the VA RNA region comprises a nucleotide sequence encoding an adenovirus VA RNA or a functional equivalent thereof. For example, the VA RNA region comprises a nucleotide sequence encoding a VA RNA from adenovirus serotype 5 (Ad5) or a functional equivalent thereof.

In some embodiments, the VA RNA region comprises a nucleotide sequence encoding a VA RNA I or a functional equivalent thereof. For example, the VA RNA region comprises a nucleotide sequence encoding a VA RNA I from Ad5 or a functional equivalent thereof. Accordingly, in some embodiments, the VA RNA region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 24.** For example, the VA RNA region comprises a nucleotide sequence that is 100% identical (e.g., is completely identical) to **SEQ ID NO: 24.**

In some embodiments, the VA RNA region comprises a nucleotide sequence encoding a VA RNA II or a functional equivalent thereof. For example, the VA RNA region comprises a nucleotide sequence encoding a VA RNA II from Ad5 or a functional equivalent thereof. Accordingly, in some embodiments, the VA RNA region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 25.** For example, the VA RNA region comprises a nucleotide sequence that is 100% identical (e.g., is completely identical) to **SEQ ID NO: 25.**

In some embodiments, the VA RNA region comprises a nucleotide sequence encoding: (i) a VA RNA I or a functional equivalent thereof; and (ii) a VA RNA II or a functional equivalent thereof. For example, the VA RNA region comprises a nucleotide sequence encoding: (i) a VA RNA I from Ad5 or a functional equivalent thereof; and (ii) a VA RNA II from Ad5 or a functional equivalent thereof. Accordingly, in some embodiments, the VA RNA region comprises: (i) a first nucleotide sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO:** 24; and (ii) second nucleotide sequence having at least 80% (e.g., at least 85%, at least 90%, at least 90%, at least 91 %, at least 92%, at least 93%, at least 94%, at least 95%, at least 96%, at least 97%, at least 98%, or at least 99%) identity to **SEQ ID NO: 25.** For example, the VA RNA region comprises: (i) a first nucleotide sequence having 100% identity (e.g., is completely identical) to **SEQ ID NO:** 24; and (ii) a first nucleotide sequence having 100% identity (e.g., is completely identical) to **SEQ ID NO: 25.**

In some embodiments, the VA RNA region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO:** 26. For example, the VA RNA region comprises a nucleotide sequence that is 100% identical (e.g., is completely identical) to **SEQ ID NO:** 26.

### Polyadenylation sequence

In some embodiments, the polynucleotide comprises a polyadenylation sequence or polyadenylation signal sequence. As used herein, the term "polyadenylation sequence" or "polyadenylation signal sequence" refers to a nucleotide sequence that contains a transcription termination signal and which, when it appears in an RNA transcript (e.g., mRNA), allows for said RNA transcript to be polyadenylated. As used herein, the term "polyadenylation", as used herein, refers to the addition of a polyadenine stretch to the 3'-end of an RNA transcript, e.g., an mRNA. The polyadenylation sequence or polyadenylation signal sequence is also referred to as a polyadenylation (poly A) site herein.

Any polyadenylation sequence which is functional in a host cell of choice, e.g., a eukaryotic cell can be used. For example, the polyadenylation sequence can be a mammalian or a viral polyadenylation sequence. Exemplary polyadenylation sequences include, but are not limited to, the polyadenylation signal of adenovirus 5 Eib, the SV40 early-late polyadenylation signal, the polyadenylation signal of HSV thymidine kinase, the polyadenylation signal of the protamine gene, the polyadenylation signal of the bovine growth hormone, the polyadenylation signal of the human variant of the growth hormone, the rabbit beta-globin polyadenylation signal, and similar ones.

In some embodiments, the polyadenylation sequence comprises the nucleotide sequence aataaaatatctttattttcattacatctgtgtgttggttttttgtgtg (SEQ **ID** NO: 22).

The polyadenylation signal sequence can be present on the 5'-end or the 3'-end of the VA RNA region. In some embodiments, the polyadenylation signal sequence can be present at the 5'-end of the VA RNA region.

The polyadenylation signal sequence and the VA RNA region can be linked directly to each other, i.e., the polyadenylation signal sequence and the VA RNA region can be linked to each other via a single internucleotide linkage, e.g., a phosphodiester bond. Alternatively, the polyadenylation signal sequence and the VA RNA region can be linked to each other via a nucleotide sequence comprising a few nucleotides to 100's of nucleotides. For example, there can be from 1 to about 250 nucleotides between the polyadenylation signal sequence and the VA RNA region. In some embodiments, the polyadenylation signal sequence and the VA RNA region can be linked to each other via a nucleotide sequence comprising from about 20 to about 250 nucleotides, e.g., from about 30 to about 200 nucleotides or from about 40 to about 190 nucleotides, or from about 50 to about 180 nucleotides, or from about 60 to about 160 nucleotides, or from about 70 to about 150 nucleotides, or from about 80 to about 140 nucleotides, or from about 90 to about 130 nucleotides, or from about 100 to about 125 nucleotides, or from about 110 to about 120 nucleotides. In some embodiments, the polyadenylation signal sequence and the VA RNA region can be linked to each other via a nucleotide sequence comprising about 10, about 15, about 20, about 25, about 30, about 35, about 40, about 45, about 50, about 55, about 60, about 65, about 70, about 75, about 80, about 85, about 90, about 95, about 100, about 110, about 115, about 120, about 125, about 130, about 135, about 140, about 145, about 150, about 155, about 160, about 165, about 170, about 175, about 180, about 185, about 190, about 195, about 200, about 210, about 215, about 220, about 225, about 230, about 235, about 240, about 245, or about 250 nucleotides. For example, the polyadenylation signal sequence and the VA RNA region can be linked to each other via a nucleotide sequence comprising about 100, about 110, about 115, about 120, or about 125 nucleotides. In some embodiments, the polyadenylation signal sequence and the VA RNA region can be linked to each other via a nucleotide sequence comprising about 115 nucleotides.

It is noted that the nucleotide sequence linking the polyadenylation signal sequence and the VA RNA region can be a random sequence. In other words, the nucleotide sequence linking the polyadenylation signal sequence and the VA RNA region may not be encoding a particular polypeptide sequence. In some embodiments, the nucleotide sequence linking the polyadenylation signal sequence and the VA RNA region can be derived from an adenovirus, e.g., adenovirus serotype 5 (Ad5) or similar.

In some embodiments, the nucleotide sequence linking the polyadenylation signal sequence and the VA RNA region does not comprise an open reading frame.

In some embodiments, the nucleotide sequence linking the polyadenylation signal sequence and the VA RNA region comprises a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO: 69.** For example, the nucleotide sequence linking the polyadenylation signal sequence and the VA RNA region comprises a sequence that is 100% identical (e.g., is completely identical) to **SEQ ID NO: 69.**

In some embodiments, the polynucleotide comprises, linked to 5'-end of the polyadenylation signal sequence, a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO: 80.** For example, the polynucleotide comprises, linked to 5'-end of the polyadenylation signal sequence, a sequence that is 100% identical (e.g., is completely identical) to **SEQ ID NO: 80.**

### E2a region

In some embodiments, a polynucleotide described herein comprises a nucleotide sequence encoding an E2a region or a functional equivalent thereof. As used herein, "E2a region" refers to a nucleotide sequence that encodes a 72-kDa DNA-binding protein (DBP) or a functional equivalent thereof. The DBP plays a crucial role during the elongation phase of Ad DNA replication. It regulates the promoter of AAV, helps AAV genome replication and is involved in increased capsid protein production through splicing of Rep mRNA and enhanced stability of capsid mRNA.

It is noted that the E2a region can comprises a nucleotide sequence encoding a wild-type DBP, a derivative thereof, or an artificial DBP having an equivalent function to a wild-type DBP. Generally, the DBP region comprises a nucleotide sequence encoding an adenovirus DBP or a functional equivalent thereof. For example, the E2 region comprises a nucleotide sequence encoding a DBP from adenovirus serotype 5 (Ad5) or functional equivalent thereof.

In some embodiments, the E2a region comprises a nucleotide sequence encoding a DBP and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 27.** For example, the E2a region comprises a nucleotide sequence encoding a DBP and that is 100% identical (e.g., is completely identical) to **SEQ ID NO: 27.**

In some embodiments, the E2 region comprises a nucleotide sequence encoding a polypeptide comprising the amino acid sequence of **SEQ ID NO: 28** or a functional equivalent thereof. For example, the E2 region comprises a nucleotide sequence encoding a polypeptide comprising an amino acid sequence encoded by **SEQ ID NO: 27** or an ortholog of **SEQ ID NO: 27.**

### UXP exon 3

In some embodiments, the E2a region can comprises a nucleotide sequence encoding UXP exon 3 or a functional equivalent thereof. For example, the E2a region comprises a nucleotide sequence encoding UXP exon 3 from adenovirus serotype 5 (Ad5) or functional equivalent thereof. For example, the E2a region comprises a nucleotide sequence encoding UXP exon 3 and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 29**. For example, the E2a region comprises a nucleotide sequence encoding UXP exon 3 and that is 100% identical (e.g., is completely identical) to **SEQ ID NO: 29.**

### E2a exon2/leader sequence

In some embodiments, the E2a region can comprises a nucleotide sequence encoding E2a exon2/leader sequence or a functional equivalent thereof. For example, the E2a region comprises a nucleotide sequence encoding E2a exon2/leader sequence from adenovirus serotype 5 (Ad5) or functional equivalent thereof. For example, the E2a region comprises a nucleotide sequence encoding E2a exon2/leader sequence and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 30.** For example, the E2a region comprises a nucleotide sequence encoding E2a exon2/leader sequence and that is 100% identical (e.g., is completely identical) to **SEQ ID NO: 30.**

### UXP exon 2

In some embodiments, the E2a region can comprises a nucleotide sequence encoding UXP exon 2 or a functional equivalent thereof. For example, the E2a region comprises a nucleotide sequence encoding UXP exon 2 from adenovirus serotype 5 (Ad5) or functional equivalent thereof. For example, the E2a region comprises a nucleotide sequence encoding UXP exon 2 and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 30**. For example, the E2a region comprises a nucleotide sequence encoding UXP exon 2 and that is 100% identical (e.g., is completely identical) to **SEQ ID NO: 30.**

### E2a late promoter

In some embodiments, the E2a region can comprises a nucleotide sequence encoding E2a late promoter or a functional equivalent thereof. For example, the E2a region comprises a nucleotide sequence encoding E2a late promoter from adenovirus serotype 5 (Ad5) or functional equivalent thereof. For example, the E2a region comprises a nucleotide sequence encoding E2a late promoter and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 31.** For example, the E2a region comprises a nucleotide sequence encoding E2a late promoter and that is 100% identical (e.g., is completely identical) to **SEQ ID NO: 31.**

### E2a exon 1

In some embodiments, the E2a region can comprises a nucleotide sequence encoding E2a exon 1 or a functional equivalent thereof. For example, the E2a region comprises a nucleotide sequence encoding E2a exon 1 from adenovirus serotype 5 (Ad5) or functional equivalent thereof. For example, the E2a region comprises a nucleotide sequence encoding E2a exon 1 and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 32**. For example, the E2a region comprises a nucleotide sequence encoding E2a exon 1 and that is 100% identical (e.g., is completely identical) to **SEQ ID NO: 32.**

### E2a early promoter

In some embodiments, the E2a region is under the control of a promoter. In some embodiments, the E2a region is under the control of a promoter and wherein the promoter is not a chicken β-actin promoter or a SV40 promoter.

In some embodiments, the E2a region can comprises a nucleotide sequence encoding E2a early promoter or a functional equivalent thereof. For example, the E2a region comprises a nucleotide sequence encoding E2a early promoter from adenovirus serotype 5 (Ad5) or functional equivalent thereof. For example, the E2a region comprises a nucleotide sequence encoding E2a early promoter and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 33.** For example, the E2a region comprises a nucleotide sequence encoding E2a early promoter and that is 100% identical (e.g., is completely identical) to **SEQ ID NO: 33.**

### E2a late primary transcript sequence

In some embodiments, the E2a region can comprises a nucleotide sequence encoding E2a late primary transcript sequence or a functional equivalent thereof. For example, the E2a region comprises a nucleotide sequence encoding E2a late transcript primary sequence from adenovirus serotype 5 (Ad5) or functional equivalent thereof. For example, the E2a region comprises a nucleotide sequence encoding E2a late transcript primary sequence and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 34.** For example, the E2a region comprises a nucleotide sequence encoding E2a late primary transcript sequence and that is 100% identical (e.g., is completely identical) to **SEQ ID NO: 34.**

### E2a early primary transcript sequence

In some embodiments, the E2a region can comprises a nucleotide sequence encoding E2a early primary transcript sequence or a functional equivalent thereof. For example, the E2a region comprises a nucleotide sequence encoding E2a early primary transcript sequence from adenovirus serotype 5 (Ad5) or functional equivalent thereof. For example, the E2a region comprises a nucleotide sequence encoding E2a early primary transcript sequence and having at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 35.** For example, the E2a region comprises a nucleotide sequence encoding E2a early primary transcript sequence and that is 100% identical (e.g., is completely identical) to **SEQ ID NO: 35.**

In some embodiments, the E2a region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 36.** For example, the E2a region comprises a nucleotide sequence that is 100% identical (e.g., is completely identical) to **SEQ ID NO: 36.**

In some embodiments, the E2a region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 76.** For example, the E2a region comprises a nucleotide sequence that is 100% identical (e.g., is completely identical) to **SEQ ID NO: 76.**

The E2a region can be on the 5'-end or the 3'-end of the VA RNA region. In some embodiments, the E2a region is present at the 3'-end of the VA RNA region.

The E2a region and the VA RNA region can be linked directly to each other, i.e., the E2a region and the VA RNA region can be linked to each other via a single internucleotide linkage, e.g., a phosphodiester bond. Alternatively, the E2a region and the VA RNA region can be linked to each other via a nucleotide sequence comprising a few nucleotides to 100's of nucleotides. For example, there can be from 1 to about 250 nucleotides between the E2a region and the VA RNA region. In some embodiments, the E2a region and the VA RNA region can be linked to each other via a nucleotide sequence comprising from about 10 to about 200 nucleotides, e.g., from about 20 to about 190 nucleotides, or from about 30 to about 180 nucleotides, or from about 40 to about 160 nucleotides, or from about 50 to about 150 nucleotides, or from about 60 to about 140 nucleotides, or from about 70 to about 130 nucleotides, or from about 80 to about 120 nucleotides, or from about 90 to about 110 nucleotides.

In some embodiments, the polyadenylation signal sequence and the VA RNA region can be linked to each other via a nucleotide sequence comprising about 10, about 15, about 20, about 25, about 30, about 35, about 40, about 45, about 50, about 55, about 60, about 65, about 70, about 75, about 80, about 85, about 90, about 95, about 100, about 110, about 115, about 120, about 125, about 130, about 135, about 140, about 145, about 150, about 155, about 160, about 165, about 170, about 175, about 180, about 185, about 190, about 195, about 200, about 210, about 215, about 220, about 225, about 230, about 235, about 240, about 245, or about 250 nucleotides. For example, the E2a region and the VA RNA region can be linked to each other via a nucleotide sequence comprising about 85, about 90, about 95, about 100, or about 110 nucleotides. In some embodiments, the E2a region and the VA RNA region can be linked to each other via a nucleotide sequence comprising about 95 nucleotides.

It is noted that the nucleotide sequence linking the E2a region and the VA RNA region can be a random sequence. In other words, the nucleotide sequence linking the E2a region and the VA RNA region may not be encoding a particular polypeptide sequence. In some embodiments, the nucleotide sequence linking the E2a region and the VA RNA region is derived from an adenovirus, e.g., adenovirus serotype 5 (Ad5) or similar.

In some embodiments, the nucleotide sequence linking the E2a region and the VA RNA region does not comprise an open reading frame.

In some embodiments, the nucleotide sequence linking the E2a region and the VA RNA region comprises a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO: 70.** For example, the nucleotide sequence linking the E2a region and the VA RNA region comprises a sequence that is 100% identical (e.g., is completely identical) to **SEQ ID NO: 70.**

### E4 region

In some embodiments, a polynucleotide described herein comprises a nucleotide sequence encoding an E4 region or a functional equivalent thereof. As used herein, an "E4 region" refers to a group of genes that are present in the Adenovirus genome next to the right ITR and are expressed in the early phase of the virus replication cycle. The E4 region includes at least six open reading frames (ORFs). The E4 region encodes at least six proteins involved in several distinct functions related to viral mRNA splicing and transport, host-cell mRNA transport, viral and cellular transcription and transformation. The products of the E4 region promote viral gene expression and replication, interact with host cell components, and participate in lytic infection and oncogenesis. Generally, the E4 region comprises one or more ORF nucleotide sequences from an E4 region of an adenovirus, e.g., E4 region of Ad5 or an ortholog thereof. For example, the E4 region comprises one or more of E4orf1, E4orf3, E4orf4, E4orf6 (E4 34K), Eorf6/7 and/or E4orf8 from an adenovirus, e.g., Ad5.

In some embodiments, the E4 region comprises a E4orf6/7 nucleotide sequence from adenovirus serotype 5 (Ad5) or an ortholog thereof. For example, the E4 region comprises E4orf6/7 with a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 37.** In some embodiments the E4 region comprises E4orf6/7 with a nucleotide sequence that is 100% identical (e.g., is completely identical) to **SEQ**

### ID NO: 37.

In some embodiments, the E4 region comprises a nucleotide sequence encoding a polypeptide comprising the amino acid sequence of **SEQ ID NO: 38** (E4orf6/7 protein) or a functional equivalent thereof. For example, the E4 region comprises a nucleotide sequence encoding a polypeptide comprising an amino acid sequence encoded by **SEQ ID NO: 37** or an ortholog of **SEQ ID NO: 37.**

In some embodiments, the E4 region comprises a E4orf6 (E4 34K) nucleotide sequence from adenovirus serotype 5 (Ad5) or an ortholog thereof. For example, the E4 region comprises E4orf6 with a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 60.** In some embodiments the E4 region comprises E4orf6 with a nucleotide sequence that is 100% identical (e.g., is completely identical) to **SEQ ID NO: 59.**

In some embodiments, the E4 region comprises a nucleotide sequence encoding a polypeptide comprising the amino acid sequence of **SEQ ID NO: 39** (E4orf6 or E4 34K protein) or a functional equivalent thereof.

In some embodiments, the E4 region comprises a E4orf4 nucleotide sequence from adenovirus serotype 5 (Ad5) or an ortholog thereof. For example, the E4 region comprises E4orf4 with a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 40.** In some embodiments the E4 region comprises E4orf4 with a nucleotide sequence that is 100% identical (e.g., is completely identical) to **SEQ ID NO: 40.**

In some embodiments, the E4 region comprises a nucleotide sequence encoding a polypeptide comprising the amino acid sequence of **SEQ ID NO: 41** (E4orf4 protein) or a functional equivalent thereof. For example, the E4 region comprises a nucleotide sequence encoding a polypeptide comprising an amino acid sequence encoded by **SEQ ID NO: 40** or an ortholog of **SEQ ID NO: 40.**

In some embodiments, the E4 region comprises a E4orf3 nucleotide sequence from adenovirus serotype 5 (Ad5) or an ortholog thereof. For example, the E4 region comprises E4orf3 with a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 42.** In some embodiments the E4 region comprises E4orf3 with a nucleotide sequence that is 100% identical (e.g., is completely identical) to **SEQ ID NO: 42.**

In some embodiments, the E4 region comprises a nucleotide sequence encoding a polypeptide comprising the amino acid sequence of **SEQ ID NO: 43** (E4orf3 protein) or a functional equivalent thereof. For example, the E4 region comprises a nucleotide sequence encoding a polypeptide comprising an amino acid sequence encoded by **SEQ ID NO: 42** or an ortholog of **SEQ ID NO: 42.**

In some embodiments, the E4 region comprises a E4orf8 nucleotide sequence from adenovirus serotype 5 (Ad5) or an ortholog thereof. For example, the E4 region comprises E4orf8 with a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 44.** In some embodiments the E4 region comprises E4orf8 with a nucleotide sequence that is 100% identical (e.g., is completely identical) to **SEQ ID NO: 44.**

In some embodiments, the E4 region comprises a nucleotide sequence encoding a polypeptide comprising the amino acid sequence of **SEQ ID NO: 45** (E4orf8 protein) or a functional equivalent thereof. For example, the E4 region comprises a nucleotide sequence encoding a polypeptide comprising an amino acid sequence encoded by **SEQ ID NO: 44** or an ortholog of **SEQ ID NO: 44.**

In some embodiments, the E4 region comprises a E4orf1 nucleotide sequence from adenovirus serotype 5 (Ad5)or an ortholog thereof. For example, the E4 region comprises E4orf1 with a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 46.** In some embodiments the E4 region comprises E4orf1 with a nucleotide sequence that is 100% identical (e.g., is completely identical) to **SEQ ID NO: 46.**

In some embodiments, the E4 region comprises a nucleotide sequence encoding a polypeptide comprising the amino acid sequence of **SEQ ID NO: 47** (E4orf1 protein) or a functional equivalent thereof. For example, the E4 region comprises a nucleotide sequence encoding a polypeptide comprising an amino acid sequence encoded by **SEQ ID NO: 46** or an ortholog of **SEQ ID NO: 46.**

In some embodiments, the E4 region is operably linked to a promoter. For example, the E4 region is operably linked to a E4 mini promoter comprising the sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 100** or at least 80% (e.g., at least 85%, at least 90%, at least 90%, at least 91%, at least 92%, at least 93%, at least 94%, at least 95%, at least 96%, at least 97%, at least 98%, or at least 99%) identical to a sequence complementary to **SEQ ID NO: 100.** In some embodiments, the E4 region is operably linked to a promoter and wherein the promoter is not a chicken β-actin promoter or a SV40 promoter.

In some embodiments, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 48.** For example, the E4 region comprises a nucleotide sequence that is 100% identical (e.g., is completely identical) to **SEQ ID NO: 48**

The E4 region can be on the 5'-end or the 3'-end of the E2A region. In some embodiments, the E4 region is present at the 3'-end of the E2A region.

The E4 region and the E2a region can be linked directly to each other, i.e., the E4 region and the E2a region can be linked to each other via a single internucleotide linkage, e.g., a phosphodiester bond. Alternatively, the E4 region and the E2a region can be linked to each other via a nucleotide sequence comprising a few nucleotides to 1000's of nucleotides. For example, there can be from about 100 to about 10000 nucleotides between the E4 region and the E2a region. In some embodiments, the E4 region and the E2a region can be linked to each other via a nucleotide sequence comprising from about 150 to about 9000 nucleotides, e.g., from about 200 to about 800 nucleotides, or from about 300 to about 7500 nucleotides, or from about 400 to about 7000 nucleotides, , or from about 5000 to about 7000 nucleotides, or from about 5100 to about 6900 nucleotides, or from about 5200 to about 6700 nucleotides, or from about 5300 to about 6700 nucleotides, or from about 5400 to about 6600 nucleotides, or from 5500 to about 6500 nucleotides, or from about 5600 to about 6400 nucleotides, or from about 5700 to about 6000 nucleotides, or from about 4000 to about 5200 nucleotides, or from about 4100 to about 5100 nucleotides, or from 4200 to about 5000 nucleotides, or from 4300 to about 4900 nucleotides, or from about 4400 to about 4800 nucleotides, or from about 4500 to about 4700 nucleotides, or from about 2500 to about 3600 nucleotide, from about 2600 to about 3500 nucleotide, or from about 2700 to about 3400 nucleotides, or from about 2800 to about 3300 nucleotide, or from about 2900 to about 3200 nucleotides, or from about 1500 to about 2500 nucleotides, or from about 1600 to about 2400 nucleotides, or from about 1700 to about 2300 nucleotides, or from about 1800 to about 2200 nucleotides, or from about 1900 to about 2100 nucleotides, or from about 200 to about 700 nucleotides, or from about 300 to about 600 nucleotides, or from about 400 to about 500 nucleotides.

In some embodiments, the polyadenylation signal sequence and the E2a region can be linked to each other via a nucleotide sequence comprising about 200, about 210, about 215, about 220, about 225, about 230, about 235, about 240, about 245, about 250 nucleotides, about 250, about 255, about 260, about 265, about 270, about 275, about 280, about 285, about 290, about 295, about 300, about 310, about 315, about 320, about 325, about 330, about 335, about 340, about 345, about 350 nucleotides, about 350, about 355, about 360, about 365, about 370, about 375, about 380, about 385, about 390, about 395, about 400, about 410, about 415, about 420, about 425, about 430, about 435, about 440, about 445, about 450 nucleotides, about 450, about 455, about 460, about 465, about 470, about 475, about 480, about 485, about 490, about 495, about 500, about 510, about 515, about 520, about 525, about 530, about 535, about 540, about 545, about 550 nucleotides, about 550, about 555, about 560, about 565, about 570, about 575, about 580, about 585, about 590, about 595, about 600, about 610, about 615, about 620, about 625, about 630, about 635, about 640, about 645, about 650 nucleotides, about 650, about 655, about 660, about 665, about 670, about 675, about 680, about 685, about 690, about 695, about 700, about 710, about 715, about 720, about 725, about 730, about 735, about 740, about 745, about 750 nucleotides, about 750, about 755, about 760, about 765, about 770, about 775, about 780, about 785, about 790, about 795, about 800, about 810, about 815, about 820, about 825, about 830, about 835, about 840, about 845, about 850 nucleotides, about 850, about 855, about 860, about 865, about 870, about 875, about 880, about 885, about 890, about 895, about 900, about 910, about 915, about 920, about 925, about 930, about 935, about 940, about 945, about 950 nucleotides, about 950, about 955, about 960, about 965, about 970, about 975, about 980, about 985, about 990, about 995, or about 1000 nucleotides. For example, the E4 region and the E2a region can be linked to each other via a nucleotide sequence comprising about 400, about 410, about 415, about 420, about 425, about 430, about 435, about 440, about 445, about 450 nucleotides, about 450, about 455, about 460, about 465, about 470, about 475, about 480, about 485, about 490, about 495, or about 500, nucleotides. In some embodiments, the E4 region and the E2a region can be linked to each other via a nucleotide sequence comprising about 450 nucleotides.

It is noted that the nucleotide sequence linking the E4 region and the E2a region can be a random sequence. In other words, the nucleotide sequence linking the E4 region and the E2a region may not be encoding a particular polypeptide sequence. In some embodiments, the nucleotide sequence linking the E4 region and the E2a region is derived from an adenovirus, e.g., adenovirus serotype 5 (Ad5) or similar.

In some embodiments, the nucleotide sequence linking the E2 region and the E4 region does not comprise an open reading frame.

In some embodiments, the E2a region is located between the VA RNA region and the E4 region. For example, the polynucleotide comprises from 5' to 3' direction the VA RNA region, the E2a region and the E4 region.

In some embodiments, the nucleotide sequence linking the E2a region and the E4 region comprises a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity one of **SEQ ID NO: 71-75.** For example, the nucleotide sequence linking the E2a region and the VA RNA region comprises a sequence that is 100% identical (e.g., is completely identical) to one of **SEQ ID NO: 71-75.** In some preferred embodiments, the nucleotide sequence linking the E2a region and the E4 region comprises a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO: 75.** More preferably, the nucleotide sequence linking the E2a region and the VA RNA region comprises a sequence that is 100% identical (e.g., is completely identical) to one of **SEQ ID NO: 75.**

### Fiber

In some embodiments, a polynucleotide described herein does not comprise a nucleotide sequence encoding at least a portion of fiber protein or a functional equivalent thereof. For example, a polynucleotide described herein does not comprise a nucleotide sequence encoding at least a portion of fiber protein from an adenovirus, e.g., Ad5, or a functional equivalent thereof. In some embodiments, a polynucleotide described herein does not comprise a nucleotide sequence encoding a full-length fiber protein or a functional equivalent thereof. For example, a polynucleotide described herein does not comprise a nucleotide sequence encoding a full-length fiber protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 10** (Ad5 fiber) or a functional equivalent thereof.

In some embodiments, the polynucleotide comprises a deletion of G₃ in a nucleotide sequence encoding an adenoviral fiber protein. For example, the polynucleotide does not comprise nucleotide 31024 relative to Genbank Accession Number AC_000008.

In some embodiments, the polynucleotide comprises a restriction enzyme site in a nucleotide sequence encoding an adenoviral fiber protein. For example, the polynucleotide comprises C → T mutation at positions 31544 relative to Genbank Accession NumberAC_000008. In another non-limiting example, the polynucleotide comprises TA → CG mutation at positions 32733-32734 relative to Genbank Accession Number AC_000008

### L1-52/55K protein

In some embodiments, a polynucleotide described herein does not comprise a nucleotide sequence encoding at least a portion of L1-52/55K (Packaging Protein 3) or a functional equivalent thereof. For example, a polynucleotide described herein does not comprise a nucleotide sequence encoding at least a portion of L1-52/55K protein from an adenovirus, e.g., Ad5, or a functional equivalent thereof. In some embodiments, a polynucleotide described herein does not comprise a nucleotide sequence encoding a full-length L1-52/55K protein or a functional equivalent thereof. For example, a polynucleotide described herein does not comprise a nucleotide sequence encoding a full-length L1-52/55K protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 12** (Ad5 L1-52/55K) or a functional equivalent thereof.

In some embodiments, a polynucleotide described herein comprises a nucleotide sequence encoding a fragment, i.e., less than full-length of **SEQ ID NO: 10** (Ad5 L1-52/55K).

### L4-100K protein

In some embodiments, a polynucleotide described herein does not comprise a nucleotide sequence encoding at least a portion of L4-100K protein or a functional equivalent thereof. For example, a polynucleotide described herein does not comprise a nucleotide sequence encoding at least a portion of L4-100K protein from an adenovirus, e.g., Ad5, or a functional equivalent thereof. In some embodiments, a polynucleotide described herein does not comprise a nucleotide sequence encoding a full-length L4-100K protein or a functional equivalent thereof. For example, a polynucleotide described herein does not comprise a nucleotide sequence encoding a full-length L4-100K protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 9** (Ad5 L4-100K) or a functional equivalent thereof.

In some embodiments, the polynucleotide comprises a deletion of G₃ and G₄ in a nucleotide sequence encoding an adenoviral L4-100K protein. For example, the polynucleotide does not comprise nucleotides 24063-24064 relative to Genbank Accession Number AC_000008.

In some embodiments, the polynucleotide comprises AC → TT mutation at positions 27322-27324 relative to Genbank Accession Number AC_000008.

In some embodiments, a polynucleotide described herein comprises a nucleotide sequence encoding a fragment, i.e., less than full-length of **SEQ ID NO: 9** (Ad5 L4-100K) or a functional equivalent thereof.

### Protein VIII (pVIII)

In some embodiments, a polynucleotide described herein does not comprise a nucleotide sequence encoding at least a portion of protein VIII or a functional equivalent thereof. For example, a polynucleotide described herein does not comprise a nucleotide sequence encoding at least a portion of protein VIII from an adenovirus, e.g., Ad5, or a functional equivalent thereof. In some embodiments, a polynucleotide described herein does not comprise a nucleotide sequence encoding a full-length protein VIII or a functional equivalent thereof. For example, a polynucleotide described herein does not comprise a nucleotide sequence encoding a full-length protein VIII from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 11** (pVIII) or a functional equivalent thereof.

In some embodiments, a polynucleotide described herein comprises a nucleotide sequence encoding a fragment, i.e., less than full-length of **SEQ ID NO: 11** (pVIII) or a functional equivalent thereof.

### Terminal Protein (TP)

In some embodiments, a polynucleotide described herein does not comprise a nucleotide sequence encoding at least a portion of precursor terminal protein (pTP) or a functional equivalent thereof. For example, a polynucleotide described herein does not comprise a nucleotide sequence encoding at least a portion of a precursor terminal protein from an adenovirus, e.g., Ad5, or a functional equivalent thereof. In some embodiments, a polynucleotide described herein does not comprise a nucleotide sequence encoding a full-length precursor terminal protein or a functional equivalent thereof. For example, a polynucleotide described herein does not comprise a nucleotide sequence encoding a full-length precursor terminal protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 13** (pTP) or a functional equivalent thereof.

In some embodiments, a polynucleotide described herein comprises a nucleotide sequence encoding a fragment, i.e., less than full-length of SEQ ID NO: 13 (pTP) or a functional equivalent thereof.

### 23K endoprotease

In some embodiments, a polynucleotide described herein does not comprise a nucleotide sequence encoding at least a portion of an endoprotease or a functional equivalent thereof. For example, a polynucleotide described herein does not comprise a nucleotide sequence encoding at least a portion of an endoprotease (e.g., 23K endoprotease) from an adenovirus, e.g., Ad5, or a functional equivalent thereof. In some embodiments, a polynucleotide described herein does not comprise a nucleotide sequence encoding a full-length an endoprotease (e.g., 23K endoprotease) or a functional equivalent thereof. For example, a polynucleotide described herein does not comprise a nucleotide sequence encoding a full-length 23K endoprotease from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 49** (23K endoprotease) or a functional equivalent thereof.

In some embodiments, a polynucleotide described herein comprises a nucleotide sequence encoding a fragment, i.e., less than full-length of **SEQ ID NO: 49** (23k endoprotease) or a functional equivalent thereof.

### E3 region

In some embodiments, a polynucleotide described herein comprises a nucleotide sequence encoding an E3 region or a functional equivalent thereof. In some other embodiments, a polynucleotide described herein comprises a nucleotide sequence encoding an E3 region or a functional equivalent thereof.

As used herein, an "E3 region" refers to a group of genes that are present in the Adenovirus genome next to the right ITR and are expressed in the early phase of the virus replication cycle. The E3 region includes at least 7 open reading frames (ORFs). The E3 region encodes at least six proteins involved in several distinct functions related to viral mRNA splicing and transport, host-cell mRNA transport, viral and cellular transcription and transformation. The products of the E3 region promote viral gene expression and replication, interact with host cell components, and participate in lytic infection and oncogenesis. Generally, the E3 region comprises one or more ORF nucleotide sequences from an E3 region of an adenovirus, e.g., E3 region of Ad5 or an ortholog thereof. For example, the E3 region comprises a nucleotide sequence encoding one or more E3 protein 12.5K, E3 protein CR1-alpha, E3 protein gp19k, E3 protein CR1-beta, E3 protein RID-alpha, E3 protein RID-beta, and E3 protein 14.7k from an adenovirus, e.g., Ad5.

In some embodiments, the E3 region comprises an E3 12K nucleotide sequence from Ad5 or an ortholog thereof. For example, the E3 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 60** (E3 12K nt). In some embodiments the E3 region comprises (E3 12K nt) with a nucleotide sequence that is 100% identical (e.g., is completely identical) to **SEQ ID NO: XX.**

In some embodiments, the E3 region comprises a nucleotide sequence encoding a polypeptide comprising an amino acid sequence encoded by **SEQ ID NO: 60** (E3 12K nt sequence) or by an ortholog of **SEQ ID NO: 60.** For example, the E3 region comprises a nucleotide sequence encoding a polypeptide comprising the amino acid sequence of **SEQ ID NO: 14** (E3 12K protein) or a functional equivalent thereof.

In some embodiments, the E3 region comprises an E3 CR1-alpha nucleotide sequence from Ad5 or an ortholog thereof. For example, the E3 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 61** (E3 CR1-alpha nt). In some embodiments the E4 region comprises (E3 CR1-alpha nt) with a nucleotide sequence that is 100% identical (e.g., is completely identical) to **SEQ ID NO: 61.**

In some embodiments, the E3 region comprises a nucleotide sequence encoding a polypeptide comprising an amino acid sequence encoded by **SEQ ID NO: 61** (E3 CR1-alpha nt sequence) or an ortholog of **SEQ ID NO: XX.** For example, the E3 region comprises a nucleotide sequence encoding a polypeptide comprising the amino acid sequence of **SEQ ID NO: 15** (E3 CR1-alpha protein) or a functional equivalent thereof.

In some embodiments, the E3 region comprises an E3 gp19K nucleotide sequence from Ad5 or an ortholog thereof. For example, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 62** (E3 gp19 K nt). In some embodiments the E3 region comprises (E3 gp19K nt) with a nucleotide sequence that is 100% identical (e.g., is completely identical) to **SEQ ID NO: 62.**

In some embodiments, the E3 region comprises a nucleotide sequence encoding a polypeptide comprising an amino acid sequence encoded by **SEQ ID NO: 62** (E3 gp19 K nt sequence) or an ortholog of **SEQ ID NO: 62.** For example, the E3 region comprises a nucleotide sequence encoding a polypeptide comprising the amino acid sequence of **SEQ ID NO: 16** (E3 gp19 K protein) or a functional equivalent thereof.

In some embodiments, the E3 region comprises an E3 10.5K nucleotide sequence from Ad5 or an ortholog thereof. For example, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 63** (E3 10.5K nt). In some embodiments the E3 region comprises (E3 10.5 nt) with a nucleotide sequence that is 100% identical (e.g., is completely identical) to **SEQ ID NO: 63.**

In some embodiments, the E3 region comprises a nucleotide sequence encoding a polypeptide comprising an amino acid sequence encoded by **SEQ ID NO: 63** (E3 10.5K nt sequence) or an ortholog of **SEQ ID NO: 63.** For example, the E3 region comprises a nucleotide sequence encoding a polypeptide comprising the amino acid sequence of **SEQ ID NO: 17** (E3 10.5K protein) or a functional equivalent thereof.

In some embodiments, the E3 region comprises an E3 RID-alpha nucleotide sequence from Ad5 or an ortholog thereof. For example, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 64** (E3 RID-alpha nt). In some embodiments the E3 region comprises (E3 RID-alpha nt) with a nucleotide sequence that is 100% identical (e.g., is completely identical) to **SEQ ID NO: 64.**

In some embodiments, the E3 region comprises a nucleotide sequence encoding a polypeptide comprising an amino acid sequence encoded by **SEQ ID NO: 64** (E3 RID-alpha nt sequence) or an ortholog of **SEQ ID NO: 64**. For example, the E3 region comprises a nucleotide sequence encoding a polypeptide comprising the amino acid sequence of **SEQ ID NO: 18** (E3 RID-alpha protein) or a functional equivalent thereof.

In some embodiments, the E3 region comprises an E3 RID-beta nucleotide sequence from Ad5 or an ortholog thereof. For example, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 65** (E3 RID-beta nt). In some embodiments the E3 region comprises (E3 RID-beta nt) with a nucleotide sequence that is 100% identical (e.g., is completely identical) to **SEQ ID NO: 65.**

In some embodiments, the E3 region comprises a nucleotide sequence encoding a polypeptide comprising an amino acid sequence encoded by **SEQ ID NO: 65** (E3 RID-beta nt sequence) or an ortholog of **SEQ ID NO: 65.** For example, the E3 region comprises a nucleotide sequence encoding a polypeptide comprising the amino acid sequence of **SEQ ID NO: 19** (E3 RID-beta protein) or a functional equivalent thereof.

In some embodiments, the E3 region comprises an E3 14.7K nucleotide sequence from Ad5 or an ortholog thereof. For example, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 66** (E3 14.7K nt). In some embodiments the E3 region comprises (E3 14.7K nt) with a nucleotide sequence that is 100% identical (e.g., is completely identical) to **SEQ ID NO: 66.**

In some embodiments, the E3 region comprises a nucleotide sequence encoding a polypeptide comprising an amino acid sequence encoded by **SEQ ID NO: 66** (E3 14.7K nt sequence) or an ortholog of **SEQ ID NO: 66.** For example, the E3 region comprises a nucleotide sequence encoding a polypeptide comprising the amino acid sequence of **SEQ ID NO: 20** (E3 14.7K protein) or a functional equivalent thereof.

In some embodiments, the E3 region comprises an E3 CR1-beta nucleotide sequence from Ad5 or an ortholog thereof. For example, the E4 region comprises a nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 67** (E3 CR1-beta nt). In some embodiments the E3 region comprises (E3 CR1-beta nt) with a nucleotide sequence that is 100% identical (e.g., is completely identical) to **SEQ ID NO: 67.**

In some embodiments, the E3 region comprises a nucleotide sequence encoding a polypeptide comprising an amino acid sequence encoded by **SEQ ID NO: 67** (E3 CR1-beta nt sequence) or an ortholog of **SEQ ID NO: 67.** For example, the E3 region comprises a nucleotide sequence encoding a polypeptide comprising the amino acid sequence of **SEQ ID NO: 68** (E3 CR1-beta protein) or a functional equivalent thereof.

In some embodiments, a polynucleotide described herein does not comprise an E3 region or a portion thereof. For example, the polynucleotide does not comprise a nucleotide sequence encoding one or more of E3 12K, E3 CR1-alpha, E3 gp19K, E3 CR1-beta, E3 RID-alpha, E3 RID-beta, E3 14.7K, or a functional equivalent thereof.

In some embodiments, a polynucleotide described herein does not comprise a nucleotide sequence encoding at least a portion of E3 protein 12K or a functional equivalent thereof. For example, a polynucleotide described herein does not comprise a nucleotide sequence encoding at least a portion of E3 protein 12K from an adenovirus, e.g., Ad5, or a functional equivalent thereof. In some embodiments, a polynucleotide described herein does not comprise a nucleotide sequence encoding a full-length E3 protein 12K or a functional equivalent thereof. For example, a polynucleotide described herein does not comprise a nucleotide sequence encoding a full-length E3 protein 12K from an adenovirus, e.g., Ad5, or a functional equivalent thereof. Thus, in some embodiments, a polynucleotide described herein does not comprise a nucleotide sequence encoding a polypeptide comprising the amino acid sequence of **SEQ ID NO: 14** (E3 12Kprotein) or a functional equivalent thereof.

In some embodiments, a polynucleotide described herein does not comprise a nucleotide sequence encoding at least a portion of E3 protein CR1-alpha or a functional equivalent thereof. For example, a polynucleotide described herein does not comprise a nucleotide sequence encoding at least a portion of E3 protein CR1-alpha from an adenovirus, e.g., Ad5, or a functional equivalent thereof. In some embodiments, a polynucleotide described herein does not comprise a nucleotide sequence encoding a full-length E3 protein CR1-alpha or a functional equivalent thereof. For example, a polynucleotide described herein does not comprise a nucleotide sequence encoding a full-length E3 protein CR1-alpha from an adenovirus, e.g., Ad5, or a functional equivalent thereof. Thus, in some embodiments, a polynucleotide described herein does not comprise a nucleotide sequence encoding a polypeptide comprising the amino acid sequence of **SEQ ID NO: 15** (E3 CR1-alpha protein) or a functional equivalent thereof.

In some embodiments, a polynucleotide described herein does not comprise a nucleotide sequence encoding at least a portion of E3 protein gp19K or a functional equivalent thereof. For example, a polynucleotide described herein does not comprise a nucleotide sequence encoding at least a portion of E3 protein gp19K from an adenovirus, e.g., Ad5, or a functional equivalent thereof. In some embodiments, a polynucleotide described herein does not comprise a nucleotide sequence encoding a full-length E3 protein gp19K or a functional equivalent thereof. For example, a polynucleotide described herein does not comprise a nucleotide sequence encoding a full-length E3 protein gp19K from an adenovirus, e.g., Ad5, or a functional equivalent thereof Thus, in some embodiments, a polynucleotide described herein does not comprise a nucleotide sequence encoding a polypeptide comprising the amino acid sequence of **SEQ ID NO: 16** (E3 gp19K protein) or a functional equivalent thereof.

In some embodiments, a polynucleotide described herein does not comprise a nucleotide sequence encoding at least a portion of E3 protein 10.59K or a functional equivalent thereof. For example, a polynucleotide described herein does not comprise a nucleotide sequence encoding at least a portion of E3 protein 10.5K from an adenovirus, e.g., Ad5, or a functional equivalent thereof. In some embodiments, a polynucleotide described herein does not comprise a nucleotide sequence encoding a full-length E3 protein 10.5K or a functional equivalent thereof. For example, a polynucleotide described herein does not comprise a nucleotide sequence encoding a full-length E3 protein 10.5K from an adenovirus, e.g., Ad5, or a functional equivalent thereof Thus, in some embodiments, a polynucleotide described herein does not comprise a nucleotide sequence encoding a polypeptide comprising the amino acid sequence of **SEQ ID NO: 17** (E3 10.5K protein) or a functional equivalent thereof.

In some embodiments, a polynucleotide described herein does not comprise a nucleotide sequence encoding at least a portion of E3 protein RID-alpha or a functional equivalent thereof. For example, a polynucleotide described herein does not comprise a nucleotide sequence encoding at least a portion of E3 protein RID-alpha from an adenovirus, e.g., Ad5, or a functional equivalent thereof. In some embodiments, a polynucleotide described herein does not comprise a nucleotide sequence encoding a full-length E3 protein RID-alpha or a functional equivalent thereof. For example, a polynucleotide described herein does not comprise a nucleotide sequence encoding a full-length E3 protein RID-alpha from an adenovirus, e.g., Ad5, or a functional equivalent thereof. Thus, in some embodiments, a polynucleotide described herein does not comprise a nucleotide sequence encoding a polypeptide comprising the amino acid sequence of **SEQ ID NO: 18** (E3 RID-alpha protein) or a functional equivalent thereof.

In some embodiments, a polynucleotide described herein does not comprise a nucleotide sequence encoding at least a portion of E3 protein RID-beta or a functional equivalent thereof. For example, a polynucleotide described herein does not comprise a nucleotide sequence encoding at least a portion of E3 protein RID-beta from an adenovirus, e.g., Ad5, or a functional equivalent thereof. In some embodiments, a polynucleotide described herein does not comprise a nucleotide sequence encoding a full-length E3 protein RID-beta or a functional equivalent thereof. For example, a polynucleotide described herein does not comprise a nucleotide sequence encoding a full-length E3 protein RID-beta from an adenovirus, e.g., Ad5, or a functional equivalent thereof. Thus, in some embodiments, a polynucleotide described herein does not comprise a nucleotide sequence encoding a polypeptide comprising the amino acid sequence of **SEQ ID NO: 19** (E3 RID-beta protein) or a functional equivalent thereof.

In some embodiments, a polynucleotide described herein does not comprise a nucleotide sequence encoding at least a portion of E3 protein 14.7K or a functional equivalent thereof. For example, a polynucleotide described herein does not comprise a nucleotide sequence encoding at least a portion of E3 protein 14.7K from an adenovirus, e.g., Ad5, or a functional equivalent thereof. In some embodiments, a polynucleotide described herein does not comprise a nucleotide sequence encoding a full-length E3 protein 14.7K or a functional equivalent thereof. For example, a polynucleotide described herein does not comprise a nucleotide sequence encoding a full-length E3 protein 14.7K from an adenovirus, e.g., Ad5, or a functional equivalent thereof. Thus, in some embodiments, a polynucleotide described herein does not comprise a nucleotide sequence encoding a polypeptide comprising the amino acid sequence of **SEQ ID NO: 20** (E3 14.7K protein) or a functional equivalent thereof.

In some embodiments, a polynucleotide described herein does not comprise a nucleotide sequence encoding at least a portion of E3 protein CR1-beta or a functional equivalent thereof. For example, a polynucleotide described herein does not comprise a nucleotide sequence encoding at least a portion of E3 protein CR1-beta from an adenovirus, e.g., Ad5, or a functional equivalent thereof. In some embodiments, a polynucleotide described herein does not comprise a nucleotide sequence encoding a full-length E3 protein CR1-beta or a functional equivalent thereof. For example, a polynucleotide described herein does not comprise a nucleotide sequence encoding a full-length E3 protein CR1-beta from an adenovirus, e.g., Ad5, or a functional equivalent thereof. Thus, in some embodiments, a polynucleotide described herein does not comprise a nucleotide sequence encoding a polypeptide comprising the amino acid sequence of **SEQ ID NO: 68** (E3 CR1-beta protein) or a functional equivalent thereof.

In some embodiments, a polynucleotide described herein does not comprise a nucleotide sequence encoding E3 protein 12K or a portion thereof, E3 protein CR1-alpha or a portion thereof, E3 protein gp19K or a portion thereof, E3 protein CR1-beta or a portion thereof, and E3 protein RID-alpha or a portion thereof.

### ITR

In some embodiments, a polynucleotide described herein comprises an inverted terminal repeat (ITR) nucleotide sequence. It is noted that the ITR sequence can be selected independently from an ITR sequence of an adenoviral ITR and/or AAV ITR. For example, the ITR sequence can be selected independently from an ITR sequence of AAV1, AAV2, AAV3, AAV4, AAV5, AAV6, AAV7, AAV8, AAV9, AAV10, AAV11, AAV12, AAV13, AAVrh74, AAVrh10, po1, AAV9-PHP.B, AAV9-ePHP.B, AAV LK03, AAV Anc80L65, AAVDJ, AAV1A6ii, AAV1P5ii, AAV4A1ii, AAV7P4i, AAV9A1i, AAV9A2i, AAV9A6i, AAV9P1i, AAV9P2i, AAV9P5i, AAVrh10A1i, AAVrh10A2i, AAVrh10P1i, AAV12P2ii, AAVS10P1i, AAV JEA, AAV2 3xA P2i, AAVDJ P2i, AAV 2i8, AAV2G9, AAV2.5i82g9, AAV2.5, AAVr10pLDB_L2, AAVr10pLDB_P31, AAV4E, and AAV4A and/or any chimeras thereof. It is noted when polynucleotide described herein comprises two or more ITR sequences, the ITR sequences can be from the same AAV serotype or from differ AAV serotypes. Thus, in some embodiments of any one of the aspects described herein, the ITR sequences are from the same AAV serotype. In some other embodiments of any one of the aspects described herein, the ITR sequences are from the different AAV serotypes. In some embodiments, when polynucleotide described herein comprises two or more ITR sequences, one of the ITR sequence can be from an AAV ITR and one of the ITR sequence can be from an adenoviral ITR.

In some embodiments, the polynucleotide comprises an ITR nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 23 or 81-89** (ITR nt). In some embodiments, the polynucleotide comprises an ITR nucleotide sequence that is 100% identical (e.g., is completely identical) to **SEQ ID NO: 23 or 81-89.**

In some embodiments, the polynucleotide comprises an ITR nucleotide sequence that is at least 80% (e.g., at least 85%, at least 90%, at least 90%, at least 91%, at least 92%, at least 93%, at least 94%, at least 95%, at least 96%, at least 97%, at least 98%, or at least 99%) identical to **SEQ ID NO: 23** (ITR nt). In some embodiments, the polynucleotide comprises an ITR nucleotide sequence that is 100% identical (e.g., is completely identical) to **SEQ ID NO: 23.**

The ITR nucleotide sequence can be 5' or 3' of the E4 region. In some embodiments, the ITR sequence can be located 3'-end of the E4 region

The ITR sequence and the E4 region can be linked directly to each other, i.e., the ITR sequence and the E4 region can be linked to each other via a single internucleotide linkage, e.g., a phosphodiester bond. Alternatively, the ITR sequence and the E4 region can be linked to each other via a nucleotide sequence comprising a few nucleotides to 100's of nucleotides. For example, there can be from 1 to about 250 nucleotides between the ITR sequence and the E4 region. In some embodiments, the ITR sequence and the E4 region can be linked to each other via a nucleotide sequence comprising from about 10 to about 1000 nucleotides, e.g., from about 25 to about 600 nucleotides, or from about 50 to about 575 nucleotides, or from 75 to about 550 nucleotides, or from about 100 to about 525 nucleotides, or from about 125 to about 500 nucleotides, or from about 150 to about 475 nucleotide, or from about 175 to about 450 nucleotides, or from about 200 to about 400 nucleotides, or from about 225 to about 375 nucleotides, or from about 250 to about 350 nucleotides.

In some embodiments, the ITR sequence and the E4 region can be linked to each other via a nucleotide sequence comprising about 10, about 15, about 20, about 25, about 30, about 35, about 40, about 45, about 50, about 55, about 60, about 65, about 70, about 75, about 80, about 85, about 90, about 95, about 100, about 110, about 115, about 120, about 125, about 130, about 135, about 140, about 145, about 150, about 155, about 160, about 165, about 170, about 175, about 180, about 185, about 190, about 195, about 200, about 210, about 215, about 220, about 225, about 230, about 235, about 240, about 245, about 250, about 255, about 260, about 265, about 270, about 275, about 280, about 285, about 290, about 295, about 300, about 310, about 315, about 320, about 325, about 330, about 335, about 340, about 345, about 350, about 355, about 360, about 365, about 370, about 375, about 380, about 385, about 390, about 395, about 400, about 410, about 415, about 420, about 425, about 430, about 435, about 440, about 445, about 450, about 455, about 460, about 465, about 470, about 475, about 480, about 485, about 490, about 495, about 500, about 510, about 515, about 520, about 525, about 530, about 535, about 540, about 545, about 550, about 555, about 560, about 565, about 570, about 575, about 580, about 585, about 590, about 595, about 600, about 610, about 615, about 620, about 625, about 630, about 635, about 640, about 645, about 650, about 655, about 660, about 665, about 670, about 675, about 680, about 685, about 690, about 695, about 700, about 710, about 715, about 720, about 725, about 730, about 735, about 740, about 745, about 750, about 755, about 760, about 765, about 770, about 775, about 780, about 785, about 790, about 795, about 800, about 810, about 815, about 820, about 825, about 830, about 835, about 840, about 845, about 850, about 855, about 860, about 865, about 870, about 875, about 880, about 885, about 890, about 895, about 900, about 910, about 915, about 920, about 925, about 930, about 935, about 940, about 945, about 950, about 955, about 960, about 965, about 970, about 975, about 980, about 985, about 990, about 995, or about 1000 nucleotides. For example, the ITR sequence and the E4 region can be linked to each other via a nucleotide sequence comprising about 200, about 210, about 215, about 220, about 225, about 230, about 235, about 240, about 245, about 250, about 255, about 260, about 265, about 270, about 275, about 280, about 285, about 290, about 295, about 300, about 310, about 315, about 320, about 325, about 330, about 335, about 340, about 345, about 350, about 355, about 360, about 365, about 370, about 375, about 380, about 385, about 390, about 395, or about 400 nucleotides. In some embodiments, the ITR sequence and the E4 region can be linked to each other via a nucleotide sequence comprising about 275, about 280, about 285, about 290, about 295, about 300, about 310, about 315, about 320 or about 325 nucleotides, e.g., about 310 nucleotides.

It is noted that the nucleotide sequence linking the ITR sequence and the E4 region can be a random sequence. In other words, the nucleotide sequence linking the ITR sequence and the E4 region may not be encoding a particular polypeptide sequence. In some embodiments, the nucleotide sequence linking the ITR sequence and the E4 region can be derived from an adenovirus, e.g., adenovirus serotype 5 (Ad5) or similar.

In some embodiments, the nucleotide sequence linking the ITR sequence and the E4 region does not comprise an open reading frame.

In some embodiments, the nucleotide sequence linking the ITR sequence and the E4 region comprises a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO: 78.** For example, the nucleotide sequence linking the ITR sequence and the E4 region comprises a sequence that is 100% identical (e.g., is completely identical) to **SEQ ID NO: 78.**

In some embodiments, the polynucleotide comprises, linked to 3'-end of the ITR, a sequence having at least 80% (e.g., at least 85%, at least 90%, at least 90%, at least 91%, at least 92%, at least 93%, at least 94%, at least 95%, at least 96%, at least 97%, at least 98%, or at least 99%) identity to **SEQ ID NO: 79.** For example, the polynucleotide comprises, linked to 3'-end of the ITR, a sequence that is 100% identical (e.g., is completely identical) to **SEQ ID NO: 79.**

### E1 region

### E1A protein 13S

In some embodiments, a polynucleotide described herein does not comprise a nucleotide sequence encoding at least a portion of E1A protein 13S or a functional equivalent thereof. For example, a polynucleotide described herein does not comprise a nucleotide sequence encoding at least a portion of E1A protein 13S from an adenovirus, e.g., Ad5, or a functional equivalent thereof. In some embodiments, a polynucleotide described herein does not comprise a nucleotide sequence encoding a full-length E1A protein 13S or a functional equivalent thereof. For example, a polynucleotide described herein does not comprise a nucleotide sequence encoding a full-length E1A protein 13S from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 50** (E1A 13S) or a functional equivalent thereof.

In some embodiments, a polynucleotide described herein comprises a nucleotide sequence encoding a fragment, i.e., less than full-length of **SEQ ID NO: 50** (E1A 13S) or a functional equivalent thereof.

In some embodiments, a polynucleotide described herein comprises a nucleotide sequence encoding a polypeptide of **SEQ ID NO: 50** (E1A 13S) or a functional equivalent thereof.

### E1A protein 12S

In some embodiments, a polynucleotide described herein does not comprise a nucleotide sequence encoding at least a portion of E1A protein 12S or a functional equivalent thereof. For example, a polynucleotide described herein does not comprise a nucleotide sequence encoding at least a portion of E1A protein 12S from an adenovirus, e.g., Ad5, or a functional equivalent thereof. In some embodiments, a polynucleotide described herein does not comprise a nucleotide sequence encoding a full-length E1A protein 12S or a functional equivalent thereof. For example, a polynucleotide described herein does not comprise a nucleotide sequence encoding a full-length E1A protein 12S from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 51** (E1A 12S) or a functional equivalent thereof.

In some embodiments, a polynucleotide described herein comprises a nucleotide sequence encoding a fragment, i.e., less than full-length of **SEQ ID NO: 51** (E1A 12S) or a functional equivalent thereof.

In some embodiments, a polynucleotide described herein comprises a nucleotide sequence encoding a polypeptide of **SEQ ID NO: 51** (E1A 12S) or a functional equivalent thereof.

### E1A protein 11S

In some embodiments, a polynucleotide described herein does not comprise a nucleotide sequence encoding at least a portion of E1A protein 11S or a functional equivalent thereof. For example, a polynucleotide described herein does not comprise a nucleotide sequence encoding at least a portion of E1A protein 11S from an adenovirus, e.g., Ad5, or a functional equivalent thereof. In some embodiments, a polynucleotide described herein does not comprise a nucleotide sequence encoding a full-length E1A protein 11S or a functional equivalent thereof. For example, a polynucleotide described herein does not comprise a nucleotide sequence encoding a full-length E1A protein 11S from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 52** (E1A 11S) or a functional equivalent thereof.

In some embodiments, a polynucleotide described herein comprises a nucleotide sequence encoding a fragment, i.e., less than full-length of **SEQ ID NO: 52** (E1A 11S) or a functional equivalent thereof.

In some embodiments, a polynucleotide described herein comprises a nucleotide sequence encoding a polypeptide of **SEQ ID NO: 52** (E1A 11S) or a functional equivalent thereof.

### E1A protein 10S

In some embodiments, a polynucleotide described herein does not comprise a nucleotide sequence encoding at least a portion of E1A protein 10S or a functional equivalent thereof. For example, a polynucleotide described herein does not comprise a nucleotide sequence encoding at least a portion of E1A protein 10S from an adenovirus, e.g., Ad5, or a functional equivalent thereof. In some embodiments, a polynucleotide described herein does not comprise a nucleotide sequence encoding a full-length E1A protein 10S or a functional equivalent thereof. For example, a polynucleotide described herein does not comprise a nucleotide sequence encoding a full-length E1A protein 10S from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 53** (E1A 10S) or a functional equivalent thereof.

In some embodiments, a polynucleotide described herein comprises a nucleotide sequence encoding a fragment, i.e., less than full-length of **SEQ ID NO: 53** (E1A 10S) or a functional equivalent thereof.

In some embodiments, a polynucleotide described herein comprises a nucleotide sequence encoding a polypeptide of **SEQ ID NO: 53** (E1A 10S) or a functional equivalent thereof.

### E1A protein 9S

In some embodiments, a polynucleotide described herein does not comprise a nucleotide sequence encoding at least a portion of E1A protein 9S or a functional equivalent thereof. For example, a polynucleotide described herein does not comprise a nucleotide sequence encoding at least a portion of E1Aprotein 9S from an adenovirus, e.g., Ad5, or a functional equivalent thereof. In some embodiments, a polynucleotide described herein does not comprise a nucleotide sequence encoding a full-length E1A protein 9S or a functional equivalent thereof. For example, a polynucleotide described herein does not comprise a nucleotide sequence encoding a full-length E1A protein 9S from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 54** (E1A 9S) or a functional equivalent thereof.

In some embodiments, a polynucleotide described herein comprises a nucleotide sequence encoding a fragment, i.e., less than full-length of **SEQ ID NO: 54** (E1A 9S) or a functional equivalent thereof.

In some embodiments, a polynucleotide described herein comprises a nucleotide sequence encoding a polypeptide of **SEQ ID NO: 54** (E1A 9S) or a functional equivalent thereof.

### E1B protein 19K

In some embodiments, a polynucleotide described herein does not comprise a nucleotide sequence encoding at least a portion of E1B protein 19K or a functional equivalent thereof. For example, a polynucleotide described herein does not comprise a nucleotide sequence encoding at least a portion of E1B protein 19K from an adenovirus, e.g., Ad5, or a functional equivalent thereof. In some embodiments, a polynucleotide described herein does not comprise a nucleotide sequence encoding a full-length E1B protein 19K or a functional equivalent thereof. For example, a polynucleotide described herein does not comprise a nucleotide sequence encoding a full-length E1B protein 19K from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 55** (E1B 19K) or a functional equivalent thereof.

In some embodiments, a polynucleotide described herein comprises a nucleotide sequence encoding a fragment, i.e., less than full-length of **SEQ ID NO: 55** (E1B 19K) or a functional equivalent thereof.

In some embodiments, a polynucleotide described herein comprises a nucleotide sequence encoding a polypeptide of **SEQ ID NO: 55** (E1B 19K) or a functional equivalent thereof.

### E1B protein 55K

In some embodiments, a polynucleotide described herein does not comprise a nucleotide sequence encoding at least a portion of E1B protein 55K or a functional equivalent thereof. For example, a polynucleotide described herein does not comprise a nucleotide sequence encoding at least a portion of E1B protein 55K from an adenovirus, e.g., Ad5, or a functional equivalent thereof. In some embodiments, a polynucleotide described herein does not comprise a nucleotide sequence encoding a full-length E1B protein 55K or a functional equivalent thereof. For example, a polynucleotide described herein does not comprise a nucleotide sequence encoding a full-length E1B protein 55K from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 56** (E1B 55K) or a functional equivalent thereof.

In some embodiments, a polynucleotide described herein comprises a nucleotide sequence encoding a fragment, i.e., less than full-length of **SEQ ID NO: 56** (E1B 55K) or a functional equivalent thereof.

In some embodiments, a polynucleotide described herein comprises a nucleotide sequence encoding a polypeptide of **SEQ ID NO: 56** (E1B 55K) or a functional equivalent thereof.

### Hexon protein

In some embodiments, a polynucleotide described herein does not comprise a nucleotide sequence encoding at least a portion of a hexon protein or a functional equivalent thereof. For example, a polynucleotide described herein does not comprise a nucleotide sequence encoding at least a portion of hexon protein from an adenovirus, e.g., Ad5, or a functional equivalent thereof. In some embodiments, a polynucleotide described herein does not comprise a nucleotide sequence encoding a full-length hexon protein or a functional equivalent thereof. For example, a polynucleotide described herein does not comprise a nucleotide sequence encoding a full-length hexon protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 57** (hexon protein) or a functional equivalent thereof.

In some embodiments, a polynucleotide described herein comprises a nucleotide sequence encoding a fragment, i.e., less than full-length of **SEQ ID NO: 57** (hexon protein) or a functional equivalent thereof.

In some embodiments, a polynucleotide described herein comprises a nucleotide sequence encoding a polypeptide of **SEQ ID NO: 57** (hexon protein) or a functional equivalent thereof.

### Peripentonal hexon-associated protein

In some embodiments, a polynucleotide described herein does not comprise a nucleotide sequence encoding at least a portion of a peripentonal hexon-associated protein or a functional equivalent thereof. For example, a polynucleotide described herein does not comprise a nucleotide sequence encoding at least a portion of peripentonal hexon-associated protein from an adenovirus, e.g., Ad5, or a functional equivalent thereof. In some embodiments, a polynucleotide described herein does not comprise a nucleotide sequence encoding a full-length peripentonal hexon-associated protein or a functional equivalent thereof. For example, a polynucleotide described herein does not comprise a nucleotide sequence encoding a full-length peripentonal hexon-associated protein from an adenovirus, e.g., Ad5, or a functional equivalent thereof. In some embodiments, a polynucleotide described herein does not comprise a nucleotide sequence encoding a polypeptide comprising the amino acid sequence of **SEQ ID NO: 58** (peripentonal hexon-associated protein) or a functional equivalent thereof.

In some embodiments, a polynucleotide described herein comprises a nucleotide sequence encoding a fragment, i.e., less than full-length of **SEQ ID NO: 58** (peripentonal hexon-associated protein) or a functional equivalent thereof.

In some embodiments, a polynucleotide described herein comprises a nucleotide sequence encoding a polypeptide of **SEQ ID NO: 58** (peripentonal hexon-associated protein) or a functional equivalent thereof.

### Restriction sites

In some embodiments, the polynucleotide comprises a restriction site or restriction recognition site for a restriction enzyme. As used herein, the term "restriction site" or "restriction recognition site" refers to a recognition nucleotide sequence that is necessary for the manifestation of the action of a restriction enzyme, and includes a site of catalytic cleavage. It is appreciated that a site of cleavage may or may not be contained within a portion of a restriction site that comprises a low ambiguity sequence (i.e., a sequence containing the principal determinant of the frequency of occurrence of the restriction site). When an enzyme (e.g. a restriction enzyme) is said to "cleave" a polynucleotide, it is understood to mean that the restriction enzyme catalyzes or facilitates a cleavage of a polynucleotide. Exemplary restriction enzymes and their recognition sequences are provided in **Table 1**.

| **Table 1: Exemplary restriction enzymes and their recognition sequences.** | |
|---|---|
| **Enzyme** | **Recognition site / sequence** |
| Acc65I | G/GTACC |
| Accl | GT/MKAC |
| Acil | CCGC(-3/-1) |
| AclI | AA/CGTT |
| AcuI | CTGAAG(16/14) |
| AfeI | AGC/GCT |
| AflII | C/TTAAG |
| AflIII | A/CRYGT |
| Agel AgeI-HFO | A/CCGGT |
| AhdI | GACNNN/NNGTC |
| AleI | CACNN/NNGTG |
| AluI | AG/CT |
| AlwI | GGATC(4/5) |
| AlwNI | CAGNNN/CTG |
| ApaLI | G/TGCAC |
| ApoI Apol-HF | R/AATTY |
| Ascl | GG/CGCGCC |
| Asel | AT/TAAT |
| AsiSI | GCGAT/CGC |
| Aval BsoBI | C/YCGRG |
| AvaII | G/GWCC |
| AvrIl | C/CTAGG |
| BaeGI | GKGCM/C |
| Bael | (10/15)ACNNNNGTAYC(12/7) |
| BamHI BamHI-HF^{®} | G/GATCC |
| Banl | G/GYRCC |
| Banll | GRGCY/C |
| Bbsl BbsI-HF^{®} | GAAGAC(2/6) |
| BbvCI | CCTCAGC(-5/-2) |
| Bbvl | GCAGC(8/12) |
| Bccl | CCATC(4/5) |
| BceAI | ACGGC(12/14) |
| Bcgl | (10/12)CGANNNNNNTGC(12/10) |
| BciVI | GTATCC(6/5) |
| Bell | T/GATCA |
| Bfal | C/TAG |
| BgII | GCCNNNN/NGGC |
| BglII | A/GATCT |
| Blpl | GC/TNAGC |
| BmgBI | CACGTC(-3/-3) |
| Bmrl | ACTGGG(5/4) |
| Bmtl BmtI-HF^{®} | GCTAG/C |
| Bpml | CTGGAG(16/14) |
| Bpu10I | CCTNAGC(-5/-2) |
| BpuEI | CTTGAG(16/14) |
| BsaAI | YAC/GTR |
| BsaBI | GATNN/NNATC |
| BsaHI | GR/CGYC |
| Bsal Bsal-HF^{®} | GGTCTC(1/5) |
| BsaJI | C/CNNGG |
| BsaWI | W/CCGGW |
| BsaXI | (9/12)ACNNNNNCTCC(10/7) |
| BseRI | GAGGAG(10/8) |
| BseYI | CCCAGC(-5/-1) |
| Bsgl | GTGCAG(16/14) |
| BsiEI | CGRY/CG |
| BsiHKAI | GWGCW/C |
| BsiWI BsiWI-HF^{®} | C/GTACG |
| Bsll | CCNNNNN/NNGG |
| BsmAI BcoDI | GTCTC(1/5) |
| BsmBI | CGTCTC(1/5) |
| BsmFI | GGGAC(10/14) |
| Bsml | GAATGC(1/-1) |
| Bsp1286I | GDGCH/C |
| BspCNI | CTCAG(9/7) |
| BspEI | T/CCGGA |
| BspHI | T/CATGA |
| BspMI BfuAI | ACCTGC(4/8) |
| BsrBI | CCGCTC(-3/-3) |
| BsrDI | GCAATG(2/0) |
| BsrFI BsrFαI | R/CCGGY |
| BsrGI BsrGI-HF^{®} | T/GTACA |
| Bsrl | ACTGG(1/-1) |
| BssHII | G/CGCGC |
| BssKI StyD4I | /CCNGG |
| BssSI BssSαI | CACGAG(-5/-1) |
| BstAPI | GCANNNN/NTGC |
| BstBI | TT/CGAA |
| BstEII BstEII-HF^{®} | G/GTNACC |
| BstNI | CC/WGG |
| BstUI | CG/CG |
| BstXI | CCANNNNN/NTGG |
| BstZ17I | GTA/TAC |
| BstZ17I-HF^{®} | GTATAC |
| Bsu36I | CC/TNAGG |
| Btgl | C/CRYGG |
| BtgZI | GCGATG(10/14) |
| BtsCI | GGATG(2/0) |
| Btsl BtsαI | GCAGTG(2/0) |
| BtsIMutl | CAGTG(2/0) |
| Cac8I | GCN/NGC |
| Clal BspDI | AT/CGAT |
| CspCI | (11/13)CAANNNNNGTGG(12/10) |
| CviAII | C/ATG |
| CviKI-1 | RG/CY |
| CviQI | G/TAC |
| Ddel | C/TNAG |
| Dpnl | GA/TC |
| Dral | TTT/AAA |
| DraIII DraIII-HF^{®} | CACNNN/GTG |
| Drdl | GACNNNN/NNGTC |
| Eael | Y/GGCCR |
| Eagl Eagl-HF^{®} | C/GGCCG |
| Earl | CTCTTC(1/4) |
| Ecil | GGCGGA(11/9) |
| Eco53kl | GAG/CTC |
| EcoNI | CCTNN/NNNAGG |
| Eco0109l | RG/GNCCY |
| EcoP15I | CAGCAG(25/27) |
| EcoRI EcoRI-HF^{®} | G/AATTC |
| EcoRV EcoRV-HF^{®} | GAT/ATC |
| Fatl | /CATG |
| Faul | CCCGC(4/6) |
| Fnu4HI | GC/NGC |
| Fokl | GGATG(9/13) |
| Fsel | GGCCGG/CC |
| FspEI | CC(12/16) |
| Fspl | TGC/GCA |
| Haell | RGCGC/Y |
| Haelll Phol | GG/CC |
| Hgal | GACGC(5/10) |
| Hhal | GCG/C |
| Hincll | GTY/RAC |
| Hindlll HindIII-HF^{®} | A/AGCTT |
| Hinfl | G/ANTC |
| HinP1I | G/CGC |
| Hpal | GTT/AAC |
| Hphl | GGTGA(8/7) |
| Hpy166II | GTN/NAC |
| Hpy88I | TCN/GA |
| Hpy188III | TC/NNGA |
| HpyAV | CCTTC(6/5) |
| HpyCH4III | ACN/GT |
| HpyCH4IV | A/CGT |
| HpyCH4V | TG/CA |
| I-Ceul | TAACTATAACGGTCCTAAGGTAGCGAA (-9/-13) |
| I-Scel | TAGGGATAACAGGGTAAT(-9/-13) |
| Kasl | G/GCGCC |
| Kpnl KpnI-HF^{®} | GGTAC/C |
| LpnPI | CCDG(10/14) |
| Mbol Sau3AI Dpnll BfuCI | /GATC |
| Mboll | GAAGA(8/7) |
| Mfel MfeI-HF^{®} | C/AATTG |
| MIuCI Tsp509I | /AATT |
| Mlul MluI-HF^{®} | A/CGCGT |
| Mlyl | GAGTC(5/5) |
| Mmel | TCCRAC(20/18) |
| Mscl | TGG/CCA |
| Msel | T/TAA |
| Msll | CAYNN/NNRTG |
| MspA1I | CMG/CKG |
| Mspl Hpall | C/CGG |
| MspJl | CNNR(9/13) |
| Mull | CCTC(7/6) |
| Mwol | GCNNNNN/NNGC |
| Nael | GCC/GGC |
| Narl | GG/CGCC |
| Nb. BbvCI | CCTCAGC |
| Nb. Bsml | GAATGC |
| Nb. BssSI | CACGAG |
| Neil | CC/SGG |
| Ncol NcoI-HF^{®} | C/CATGG |
| Ndel | CA/TATG |
| NgoMIV | G/CCGGC |
| Nhel NheI-HF^{®} | G/CTAGC |
| Nlalll | CATG/ |
| NlaIV | GGN/NCC |
| NmeAIII | GCCGAG(21/19) |
| Notl NotI-HF^{®} | GC/GGCCGC |
| Nrul NruI-HF^{®} | TCG/CGA |
| Nsil NsiI-HF^{®} | ATGCA/T |
| Nspl | RCATG/Y |
| Nt. Alwl | GGATC(4/-5) |
| Nt. BbvCI | CCTCAGC(-5/-7) |
| Nt. BsmAI | GTCTC(1/-5) |
| Nt. BspQI | GCTCTTC(1/-7) |
| Nt. BstNBI | GAGTC(4/-5) |
| Nt. CviPII | (0/-1)CCD |
| PacI | TTAAT/TAA |
| Pcil | A/CATGT |
| PflMI | CCANNNN/NTG |
| PI-Pspl | TGGCAAACAGCTATTATGGGTATTATGGGT (-13/-17) |
| PI-Scel | ATCTATGTCGGGTGCGGAGAAAGAGGTAAT (-15/-19) |
| Plel | GAGTC(4/5) |
| Pmel | GTTT/AAAC |
| Pmll | CAC/GTG |
| PpuMI | RG/GWCCY |
| PshAI | GACNN/NNGTC |
| Psil | TTA/TAA |
| | /CCWGG |
| PspOMI | G/GGCCC |
| PspXI | VC/TCGAGB |
| Pstl PstI-HF^{®} | CTGCA/G |
| Pvul PvuI-HF^{®} | CGAT/CG |
| Pvull PvuII-HF^{®} | CAG/CTG |
| Rsal | GT/AC |
| Rsrll | CG/GWCCG |
| Sacll | CCGC/GG |
| Sadl SadI-HF^{®} | GAGCT/C |
| Sapl BspQI | GCTCTTC(1/4) |
| Sau96I | G/GNCC |
| Sbfl SbfI-HF^{®} | CCTGCA/GG |
| Seal ScaI-HF^{®} | AGT/ACT |
| ScrFI | CC/NGG |
| SexAl | A/CCWGGT |
| SfaNI | GCATC(5/9) |
| Sfil | GGCCNNNN/NGGCC |
| SgrAI | CR/CCGGYG |
| Smal | CCC/GGG |
| Smll | C/TYRAG |
| SnaBI | TAC/GTA |
| Spel SpeI-HF^{®} | A/CTAGT |
| Sphl SphI-HF^{®} | GCATG/C |
| Srfl | GCCC/GGGC |
| Sspl SspI-HF^{®} | AAT/ATT |
| Stul | AGG/CCT |
| Styl StyI-HF^{®} | C/CWWGG |
| Swal | ATTT/AAAT |
| TaqaI | T/CGA |
| Tfil | G/AWTC |
| Tsel ApeKI | G/CWGC |
| Tsp45I | /GTSAC |
| TspRI | NNCASTGNN/ |
| Tth111I PfIFI | GACN/NNGTC |
| Xbal | T/CTAGA |
| Xcml | CCANNNNN/NNNNTGG |
| Xhol PaeR7I Tlil | C/TCGAG |
| Xmal TspMI | C/CCGGG |
| Xmnl | GAAN N/N NTTC |
| Zral | GAC/GTC |
| | |

In some embodiments, the polynucleotide comprises a restriction site or restriction recognition site upstream of the VA RNA region. For example, the polynucleotide comprises a restriction site for an enzyme selected from the enzyme listed in **Table 1,** and wherein the restriction site is upstream of the VA RNA region. In some embodiments, the polynucleotide comprises a restriction site for Sal1 and wherein the restriction site is upstream of the VA RNA region.

In some embodiments, the polynucleotide comprises a restriction site or restriction recognition site upstream of the VA RNA region and downstream of a polyadenylation sequence. In other words, the restriction site is located between the polyadenylation sequence and 5'-end of the VA RNA region. For example, the polynucleotide comprises a restriction site for an enzyme selected from the enzyme listed in **Table 1,** and wherein the restriction site is upstream of the VA RNA region and downstream from a polyadenylation sequence. In some embodiments, the polynucleotide comprises restriction site for Sal1 located upstream of the VA RNA region and located between a polyadenylation sequence and the VA RNA region.

In some embodiments, the polynucleotide comprises a restriction site or restriction recognition site downstream of the VA RNA region. For example, the polynucleotide comprises a restriction site for an enzyme selected from the enzyme listed in **Table 1,** and wherein the restriction site is downstream of the VA RNA region. In some embodiments, the polynucleotide comprises a restriction site for Nsil and wherein the restriction site is downstream of the VA RNA region.

In some embodiments, the polynucleotide comprises a restriction site downstream of the VA RNA region and upstream of the E2A region. In other words, the restriction site is located between the VA RNA region and the E2A region. For example, the polynucleotide comprises a restriction site for an enzyme selected from the enzyme listed in **Table 1,** and wherein the restriction site is downstream from the VA RNA region and is located between the VA RNA region and the E2A region. In some embodiments, the polynucleotide comprises restriction site for Nsil downstream from the VA RNA region and located between the VA RNA region and the E2A region.

### Vectors

In another aspect, provided herein is a composition comprising a polynucleotide described herein.

In some embodiments of any one of the aspects described herein, the polynucleotide described herein is a vector.

As used herein, a "vector" refers to a compound used as a vehicle to carry foreign genetic material into another cell, where it can be replicated and/or expressed. A cloning vector containing foreign nucleic acid is termed a recombinant vector. Exemplary vectors include, but are not limited to, plasmids, phagemids, bacmids, cosmids, viral vectors, and artificial chromosomes (e.g., bacterial or yeast artificial chromosome). In some embodiments of any one of the aspects described herein, the polynucleotide described herein is a plasmid. In some other embodiments, the polynucleotide described herein is a bacmid. In yet other embodiments, the polynucleotide described herein is a cosmid. Recombinant vectors typically contain an origin of replication, a multicloning site, and a selectable marker. The nucleic acid sequence typically consists of an insert (recombinant nucleic acid or transgene) and a larger sequence that serves as the "backbone" of the vector. The purpose of a vector which transfers genetic information to another cell is typically to isolate, multiply, or express the insert in the target cell. Expression vectors (expression constructs) are for the expression of the exogenous gene in the target cell, and generally have a promoter sequence that drives expression of the exogenous gene/ORF. Insertion of a vector into the target cell is referred to transformation or transfection for bacterial and eukaryotic cells, although insertion of a viral vector is often called transduction. The term "vector" may also be used in general to describe items that serve to carry foreign genetic material into another cell, such as, but not limited to, a transformed cell or a nanoparticle.

In some embodiments, the vector is an expression vector. As used herein, the term "expression vector" refers to a vector that directs expression of an RNA or polypeptide from sequences linked to transcriptional regulatory sequences on the vector. The sequences expressed will often, but not necessarily, be heterologous to the cell. An expression vector can comprise additional elements. For example, the expression vector can have two replication systems; thus, allowing it to be maintained in two organisms, for example in human cells for expression and in a prokaryotic host for cloning and amplification. Examples of eukaryotic expression vectors include, but are not limited to, pW-LNEO, pSV2CAT, pOG44, pXTl and pSG available from Stratagene; pSVK3, pBPV, pMSG and pSVL available from Amersham Pharmacia Biotech; and pCMVDsRed2-express, pIRES2-DsRed2, pDsRed2-Mito, pCMV-EGFP available from Clontech. Many other vectors are well-known and commercially available. For mammalian cells adenoviral vectors, the pSV and the pCMV series of vectors are particularly well-known non-limiting examples. There are many well-known yeast expression vectors including, without limitation, yeast integrative plasmids (YIp) and yeast replicative plasmids (YRp). For plants the Ti plasmid of agrobacterium is an exemplary expression vector, and plant viruses also provide suitable expression vectors, e.g., tobacco mosaic virus (TMV), potato virus X, and cowpea mosaic virus.

In some embodiments, the vector does not comprise a nucleotide sequence encoding a full length L-22K and/or a full length L-33K protein. For example, the vector does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the vector does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the vector does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.**

In some embodiments, the expression vector is a plasmid. Such a plasmid can include a variety of other functional nucleic acid sequences, such as one or more selectable markers, one or more origins of replication, polycloning sites and the like.

In some embodiments, the polynucleotide is a helper plasmid. As used herein, the term "helper plasmid" refers to a plasmid used when producing copies of a helper virus-dependent viral vector, such as adeno-associated virus, which does not have the ability to replicate on its own. The helper plasmid is used to co-infect cells alongside the viral vector and provides the necessary proteins for replication of the genome of the viral vector.

In some embodiments, the plasmid does not comprise a nucleotide sequence encoding a full length L-22K and/or a full length L-33K protein. For example, the plasmid does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the plasmid does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the plasmid does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.** In some embodiments, the plasmid does not comprise a nucleotide sequence encoding a full length L-22K and/or a full length L-33K protein. For example, the plasmid does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the plasmid does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the plasmid does not comprise a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.**

### Compositions

In another aspect, provided herein is a composition comprising a polynucleotide described herein.

In some embodiments, the composition further comprises a transfection agent. The term "transfection agent" as used herein generally refers to a molecule or composition capable of delivering molecules, e.g., nucleic acids to cells. Exemplary classes of transfection agents include, but are not limited to, cationic compounds (compounds having a net positive charge) and charge neutral compounds. By way of example, suitable transfection agents can include cationic and non-cationic polymers and cationic and non-cationic lipids. Exemplary cationic lipids include, but are not limited to, 3β-[N-(N',N'-dimethylaminoethane)-carbamoyl]cholesterol hydrochloride (DC-cholesterol); 1,2-dioleoyl-3-trimethylammonium-propane (DOTAP); dimethyldioctadecylammonium (DDAB); 1,2-dioleoyl-sn-glycero-3-ethylphosphocholine (EPC); 1,2-di-O-octadecenyl-3-trimethylammonium propane (DOTMA); 1,2-di-(9Z-octadecenoyl)-3-dimethylammonium-propane (DODAP); 1,2-dilinoleyloxy-3-dimethylaminopropane (DLinDMA); 1,2-dioleoyl-sn-glycero-3-phosphoethanolamine (DOPE); cholesterol; 1,2-dioctadecanoyl-sn-glycero-3-phosphocholine (DSPC); 1,2-distearoyl-sn-glycero-3-phosphoethanolamine (DSPE); and derivatives thereof. Other exemplary lipids can include, but are not limited to, lipidoids, atuplex formulations, and PEGylated forms of lipids described above. Transfection agents can also include polycation containing cyclodextrin, histones, cationized human serum albumin, aminopolysaccharides such as chitosan, peptides such as poly-L-lysine, poly-L-ornithine, and poly(4-hydroxy-L-proline ester, and polyamines such as polyethylenimine (PEI), polypropylenimine, polyamidoamine dendrimers, and poly(beta-aminoesters). In some preferred embodiments, the transfection agent is PEI.

In some embodiments of the any one of the aspects described herein, the composition further comprises a nucleic acid comprising a nucleotide sequence encoding viral capsid (e.g., AAV capsid) and/or non-structural replication (e.g., AAV Rep) genes.

In some other embodiments of the any one of the aspects described herein, the composition further comprises a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR).

In some embodiments of any one of the aspects described herein, the composition comprises: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR). It is noted that the nucleic acid encoding the viral capsid (e.g., AAV capsid) and/or non-structural replication (e.g., AAV Rep) gene viral capsid (e.g., AAV capsid) and/or non-structural replication (e.g., AAV Rep) gene can be in a separate polynucleotide. Alternatively, the nucleic acid encoding the viral capsid (e.g., AAV capsid) and/or non-structural replication (e.g., AAV Rep) gene viral capsid (e.g., AAV capsid) and/or non-structural replication (e.g., AAV Rep) gene can be a part of the polynucleotide described herein. In other words, the polynucleotide described herein and the nucleic acid encoding the viral capsid and non-structural replication genes are the same, i.e., the polynucleotide further comprises a nucleotide sequence encoding the viral capsid (e.g., AAV capsid) and/or non-structural replication (e.g., AAV Rep) genes.

In some embodiments, the composition does not comprise a polynucleotide comprising a nucleotide sequence encoding a full length L-22K and/or a full length L-33K protein. For example, the composition does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the composition does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the composition does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.** In some embodiments, the composition does not comprise a polynucleotide comprising a nucleotide sequence encoding a full length L-22K and/or a full length L-33K protein. For example, the composition does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the composition does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the composition does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.**

### Cells

In another aspect, provided herein is a cell comprising a polynucleotide described herein. As used herein, the term "cell" refers to a single cell as well as to a population of (i.e., more than one) cells. Further, unless otherwise indicated, the terms "cell" or "cell line" are understood to include modified or engineered variants of the indicated cell or cell line.

A cell comprising a polynucleotide described herein can be a mammalian, bacterial, insect or yeast cell. Some exemplary cells include, but are not limited to HeLa cell, COS cell, COS-1 cell, COS-7 cell, HEK293 cell, A549 cell, BHK cell, BSC-1 cell, BSC-40 cell, Vero cell, Sf"c9 cell, Sf -21 cell, Tn-368 cell, BTI-Tn-5B1-4 (High-Five) cell, Saos cell, C2C12 cell, L cell, HT1080 cell, HepG2 cell, WEHI cell, 3T3 cell, 10T1/2 cell, MDCK cell, BMT-10 cell, WI38 cell, or primary fibroblast, hepatocyte or myoblast cells derived from mammals.

In some embodiments, the cell comprising a polynucleotide described herein is a mammalian cell. Exemplary mammalian cells include, but are not limited to, Chinese hamster ovary (CHO) cell for example of K1 lineage (ATCC CCL 61) including the Pro5 variant (ATCC CRL 1281); the fibroblast-like cells derived from SV40-transformed African Green monkey kidney of the CV-1 lineage (ATCC CCL 70), of the COS-1 lineage (ATCC CRL 1650) and of the COS-7 lineage (ATCC CRL 1651; murine L-cells, murine 3T3 cells (ATCC CRL 1658), murine C127 cells, human embryonic kidney cells of the 293 lineage (ATCC CRL 1573), human carcinoma cells including those of the HeLa lineage (ATCC CCL 2), and neuroblastoma cells of the lines IMR-32 (ATCC CCL 127), SK-N-MC (ATCC HTB 10) and SK-N-SH (ATCC HTB 11). In some embodiments, the cell comprising a polynucleotide described herein is a HEK293 cell. In another embodiment, the cell comprising a polynucleotide described herein is a HeLa cell.

In some embodiments, the cell comprising a polynucleotide described herein is a microbial cell. For example, the cell comprising a polynucleotide described herein is a bacterial cell such as a *E*. *coli* cell, or yeast cell such as *S*. *cerevisiae* cell.

In some embodiments, the cell comprising a polynucleotide described herein is a host cell or a producer cell. The terms "host cell" and "producer cell" are used interchangeably herein and refer to any cell or cells capable of producing a recombinant virus, e.g., recombinant adeno-associated virus particles. Host cell can be a mammalian, bacterial, or yeast cell. In some embodiments, the host cell is a mammalian cell.

In some embodiments, the cell comprising a polynucleotide described herein is a modified or engineered cell. For example, the cell comprising a polynucleotide described herein is a suspension adapted cell. In some embodiments, the cell comprising a polynucleotide described herein is a suspension-adapted cell derived from the human embryonic kidney cell line 293 (HEK293). For example, the cell comprising a polynucleotide described herein is a PRO10^{™} cell line (AskBio, NC, USA), a suspension-adapted and serum-free cell line derived from the human embryonic kidney cell line 293 (HEK293).

In some embodiments of the any one of the aspects described herein, the cell further comprises a nucleic acid comprising a nucleotide sequence encoding viral capsid (e.g., AAV capsid) and/or non-structural replication (e.g., AAV Rep) genes.

In some other embodiments of the any one of the aspects described herein, the cell further comprises a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR).

In some embodiments of any one of the aspects described herein, the cell comprises: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR).

It is noted that the nucleic acid encoding the viral capsid (e.g., AAV capsid) and/or non-structural replication (e.g., AAV Rep) gene viral capsid (e.g., AAV capsid) and/or non-structural replication (e.g., AAV Rep) gene can be in a separate polynucleotide. Alternatively, the nucleic acid encoding the viral capsid (e.g., AAV capsid) and/or non-structural replication (e.g., AAV Rep) gene viral capsid (e.g., AAV capsid) and/or non-structural replication (e.g., AAV Rep) gene can be a part of the polynucleotide described herein. In other words, the polynucleotide described herein and the nucleic acid encoding the viral capsid and non-structural replication genes are the same, i.e., the polynucleotide further comprises a nucleotide sequence encoding the viral capsid (e.g., AAV capsid) and/or non-structural replication (e.g., AAV Rep) genes.

In some embodiments, the cell comprises an endogenous or exogenous polynucleotide encoding at least one recombinase. The term "recombinase" refers to an enzyme that catalyzes DNA exchange at a specific target site, for example, a palindromic sequence, by excision/insertion, inversion, translocation and exchange. Exemplary recombinases include, but are not limited to, TelN, Tel, Tel (gp26 K02 phage) Cre, Flp, phiC31, Int and other lambdoid phage integrases, *e.g.*, phi 80, HK022 and HP1 recombinases.

In some embodiments, the cell does not comprise a polynucleotide comprising a nucleotide sequence encoding a full length L-22K and/or a full length L-33K protein. For example, the cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.** In some embodiments, the cell does not comprise a polynucleotide comprising a nucleotide sequence encoding a full length L-22K and/or a full length L-33K protein. For example, the cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.**

### Kits

In another aspect, provided herein is a kit comprising a polynucleotide described herein. The kit is an assemblage of materials or components, including at least one of the polynucleotides described herein. Optionally, the kit can also contain other useful components and reagents, such as, transfection agents, cells, other nucleic acids, measuring tools, diluents, buffers, instructions for use, and/or other useful paraphernalia as will be readily recognized by those of skill in the art. It is noted that the exact nature of the components configured in the kit depends on its intended purpose. In some embodiments, the kit is configured for industrial applications. In some embodiments, the kit is configured for research applications.

In some embodiments, the kit includes instructions for use. "Instructions for use" typically include a tangible expression describing the technique to be employed in using the components of the kit.

In some embodiments of any one of the aspects described herein, the kit further comprises a transfection agent.

In some embodiments of any one of the aspects described herein, the kit further comprises a nucleic acid comprising a nucleotide sequence encoding viral capsid (e.g., AAV capsid) and/or non-structural replication (e.g., AAV Rep) genes.

In some embodiments of any one of the aspects described herein, the kit further comprises a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR).

In some embodiments of any one of the aspects described herein, the kit comprises: i) a polynucleotide described herein; ii) a transfection agent; iii) a nucleic acid comprising a nucleotide sequence encoding viral capsid (e.g., AAV capsid) and/or non-structural replication (e.g., AAV Rep) genes; and iv) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR).

In some embodiments, the kit comprises a polynucleotide described herein and a cell, e.g., a host cell. In some embodiments, the kit comprises a cell, e.g., a host cell comprising a polynucleotide described herein.

The materials or components assembled in the kit can be provided to the practitioner stored in any convenient and suitable ways that preserve their operability and utility. For example, the components can be in dissolved, dehydrated, or lyophilized form; they can be provided at room, refrigerated or frozen temperatures. The components are typically contained in suitable packaging material(s). As employed herein, the phrase "packaging material" refers to one or more physical structures used to house the contents of the kit, such as inventive compositions and the like. The packaging material is constructed by well-known methods, preferably to provide a sterile, contaminant-free environment. The packaging may also preferably provide an environment that protects from light, humidity, and oxygen. As used herein, the term "package" refers to a suitable solid matrix or material such as glass, plastic, paper, foil, polyester (such as polyethylene terephthalate, or Mylar) and the like, capable of holding the individual kit components in a format suitable for use. Thus, for example, a package can be a glass vial or a plastic vial used to contain suitable quantities of a component included in the kit therein. The packaging material generally has an external label which indicates the contents and/or purpose of the kit and/or its components.

In some embodiments, the kit does not comprise a polynucleotide comprising a nucleotide sequence encoding a full length L-22K and/or a full length L-33K protein. For example, the kit does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the kit does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the kit does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.** In some embodiments, the kit does not comprise a polynucleotide comprising a nucleotide sequence encoding a full length L-22K and/or a full length L-33K protein. For example, the kit does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the kit does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the kit does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.**

### Uses and methods

The polynucleotides and cells, e.g. host or producer cells described herein can be used in manufacturing recombinant virus e.g., recombinant adeno-associated virus (rAAV) particles. For example, polynucleotides described herein can be used in a method of producing a plurality of viral particles, e.g., rAAV particles. Exemplary methods for producing rAAV are described in PCT/US2022/013279 (published as WO2022159679), and in PCT/US2021/013689 (published as WO/2021/146591), contents of both of which are incorporated herein by reference in their entireties. It is noted that virus particle (e.g., rAAV particle) and virus capsid (e.g., rAAV capsid) are used interchangeably herein.

Thus, in another aspect, provided herein is a method of producing recombinant virus e.g., rAAV particles. Generally, the method comprises culturing a host cell comprising a polynucleotide described herein in a culture medium under conditions in which viral particles, e.g., rAAV particles are produced. In some embodiments, the host cell further comprises a nucleic acid encoding viral capsid (e.g., AAV capsid) and/or non-structural replication (e.g., AAV Rep) genes and/or a nucleic acid encoding a virus genome. For example, the host cell comprises: (i) a polynucleotide described herein; (ii) a nucleic acid encoding viral capsid (e.g., AAV capsid) and/or non-structural replication (e.g., AAV Rep) genes; and (iii) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR).

In some embodiments, the cell, e.g., the host cell or a producer cell does not comprise a polynucleotide comprising a nucleotide sequence encoding a full length L-22K and/or a full length L-33K protein. For example, the cell, e.g., the host cell or a producer cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the cell, e.g., the host cell or a producer cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the cell, e.g., the host cell or a producer cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.** In some embodiments, the cell, e.g., the host cell or a producer cell does not comprise a polynucleotide comprising a nucleotide sequence encoding a full length L-22K and/or a full length L-33K protein. For example, the cell, e.g., the host cell or a producer cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7.** In another example, the cell, e.g., the host cell or a producer cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 8.** In yet another example, the cell, e.g., the host cell or a producer cell does not comprise a polynucleotide comprising a nucleotide sequence encoding a protein having an amino acid sequence having at least 80% (e.g., at least 85%, at least 90%, at least 90%, at least 91%, at least 92%, at least 93%, at least 94%, at least 95%, at least 96%, at least 97%, at least 98%, at least 99% or 100%) identity to **SEQ ID NO: 7 or 8.**

The host cell can be a mammalian, bacterial, or yeast cell. In some embodiments of any one of the aspect, the host cell can be HeLa cell, COS cell, COS-1 cell, COS-7 cell, HEK293 cell, A549 cell, BHK cell, BSC-1 cell, BSC-40 cell, Vero cell, Sf"c9 cell, Sf -21 cell, Tn-368 cell, BTI-Tn-5B1-4 (High-Five) cell, Saos cell, C2C12 cell, L cell, HT1080 cell, HepG2 cell, WEHI cell, 3T3 cell, 10T1/2 cell, MDCK cell, BMT-10 cell, WI38 cell, or primary fibroblast, hepatocyte or myoblast cells derived from mammals. In some embodiments, the host cell is a mammalian cell. In some embodiments, PRO10^{™} cell line (AskBio, NC, USA), a suspension-adapted and serum-free cell line derived from the human embryonic kidney cell line 293 (HEK293), can be used to produce the recombinant virus, e.g., rAAV particles.

It is noted that a recombinant viral particle, e.g., rAAV can be produced from a host or producer cell using any suitable method known in the art. In order to achieve maximum production of viral, e.g., rAAV capsid particles, the host cells can be cultured for a few hours to several days. In some embodiments, the host cells are cultured for at least 10 hours. For example, the host cells can be cultured for at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, at least 24 hours, at least 25 hours, at least 26 hours, at least 27 hours, at least 28 hours, at least 29 hours, at least 30 hours, at least 31 hours, at least 32 hours, at least 33 hours, at least 34 hours, at least 35 hours, at least 36 hours, at least 37 hours, at least 38 hours, at least 39 hours, at least 40 hours, at least 41 hours, at least 42 hours, at least 43 hours, at least 44 hours, at least 45 hours, at least 46 hours, at least 47 hours, at least 48 hours, at least 49 hours, at least 50 hours, at least 51 hours, at least 52 hours, at least 53 hours, at least 54 hours, at least 55 hours, at least 56 hours, at least 57 hours, at least 58 hours, at least 59 hours, at least 60 hours, at least 61 hours, at least 62 hours, at least 63 hours, at least 64 hours, at least 65 hours, at least 66 hours, at least 67 hours, at least 68 hours, at least 69 hours, at least 70 hours, at least 71 hours, at least 72 hours, at least 73 hours, at least 74 hours, at least 75 hours, at least 76 hours, at least 77 hours, at least 78 hours, at least 79 hours, at least 80 hours, at least 81 hours, at least 82 hours, at least 83 hours, at least 84 hours, at least 85 hours, at least 86 hours, at least 87 hours, at least 88 hours, at least 89 hours, at least 90 hours, at least 91 hours, at least 92 hours, at least 93 hours, at least 94 hours, at least 95 hours, at least 96 hours, at least 97 hours, at least 98 hours, at least 99 hours, at least 100 hours or more.

In some embodiments, the host cells can be cultured in suspension and/or under serum free conditions. The host cells can be cultured in animal component-free conditions. The animal component-free medium can be any animal component-free medium (e.g., serum-free medium) compatible with a given cell line, for example, HEK293 cells. Examples include, without limitation, SFM4Transfx-293 (HYCLONE), Ex-Cell 293 (JRH BIOSCIENCES), LC-SFM (INVITROGEN), and Pro293-S (LONZA) Pro-10 cells (as described in US Patent No. 9,441,206, content of which is incorporated herein by reference in its entirety.

Generally, the copy number of the polynucleotide described herein, that is used to produce recombinant virus e.g., rAAV, is at least about 2000 copies per cell to at least about 20,000 copies per cell. For example, the copy number of the polynucleotide, that is used to produce recombinant virus e.g., rAAV, is at least about 5000 copies per cell to at least about 12000 copies per cell. In some embodiments, the copy number of the polynucleotide, that is used to produce recombinant virus e.g., rAAV, is at least about 1000 copies per cell, at least about 1500 copies per cell, at least about 2000 copies per cell, at least about 2500 copies per cell, at least about 3000 copies per cell, at least about 3500 copies per cell, at least about 4000 copies per cell, at least about 4500 copies per cell, at least about 5000 copies per cell, at least about 5500 copies per cell, at least about 6000 copies per cell, at least about 6500 copies per cell, at least about 7000 copies per cell, at least about 7500 copies per cell, at least about 8000 copies per cell, at least about 8500 copies per cell, at least about 9000 copies per cell, at least about 9500 copies per cell, at least about 10000 copies per cell, at least about 12000 copies per cell, at least about 14000 copies per cell, at least about 16000 copies per cell, at least about 18000 copies per cell, at least about 20000 copies per cell or higher.

The host cells can be transfected with one or more nucleic acids, e.g., polynucleotide described herein to produce the recombinant virus, e.g., rAAV virus particles. Thus, in some embodiments, the method of manufacturing viral, e.g., rAAV particles comprises: (a) transfecting host cells with: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR); and (b) culturing the transfected host cells for a sufficient period of time to produce rAAV capsid particles.

As used herein, "transfection" refers to the insertion of a nucleic acid into a target cell, e.g., a host cell. There are two different types of transfection: (i) stable transfection and (ii) transient transfection. Stable transfection incorporates exogenous nucleic acids into the transfected cell's genome whereas in transient transfection, the exogenous nucleic acids are present only for a limited time in the cell and do not integrate with the transfected cell's genome. In some embodiments, the transfection method used is transient transfection. In some embodiments, the transfection method used is stable transfection. Transfection can be performed with a variety of methods including, but not limited to, calcium phosphate, electroporation, and/or cationic lipid-mediated methods (e.g., LIPOFECTAMINE, polyethylenimine (PEI)). In some embodiments, the transfection method uses polyethylenimine. Transfection can require an optimal cell density based on the cell type, application, and/or transfection technology. Additional description of transfection can be found in Shin et al. Recombinant Adeno-Associated Viral Vector Production and Purification. Methods Mol Biol. 2012; 798: 267-284; Grieger et al. Production of Recombinant Adeno-associated Virus Vectors Using Suspension HEK293 Cells and Continuous Harvest of Vector from the Culture Media for GMP FIX and FLT1 Clinical Vector. Mol Ther. 2016 Feb; 24(2): 287-297; and Meier et al. The Interplay between Adeno-Associated Virus and Its Helper Viruses. Viruses. 2020 Jun; 12(6): 662, contents of each of which are incorporated herein by reference in their entireties.

Transfection of multiple nucleic acids into the same cells can occur simultaneously or it can occur within 5 minutes, within 10 minutes, within 15 minutes, within 20 minutes, within 25 minutes, within 30 minutes, within 35 minutes, within 40 minutes, within 45 minutes, within 50 minutes, within 60 minutes, within 65 minutes, within 70 minutes, within 75 minutes, within 80 minutes, within 85 minutes, within 90 minutes, within 95 minutes, within 100 minutes, within 110 minutes, within 120 minutes, within 130 minutes, within 140 minutes, within 150 minutes, within 160 minutes, within 170 minutes, within 180 minutes, within 190 minutes, within 200 minutes, within 210 minutes, within 220 minutes, within 230 minutes, within 240 minutes, within 250 minutes, within 260 minutes, within 270 minutes, within 280 minutes, within 290 minutes, within 300 minutes, within 310 minutes, within 320 minutes, within 330 minutes, within 340 minutes, within 350 minutes, within 360 minutes or more between transfection of the first nucleic acid and transfection of subsequent nucleic acids.

Without wishing to be bound by a theory, where the polynucleotides described herein are used as one of the nucleic acids (e.g., plasmids) in transient transfection system using two, three, or more nucleic acids (e.g., plasmids), the polynucleotide or component thereof can be stably integrated in the host cell, e.g., AAV producer cell.

As one of skill in the art is aware, a sufficient cell mass is needed for transfection. As used herein, "sufficient cell mass" refers to an optimal cell density for transfection, based on the cell type, application, and/or transfection technology. In some embodiments, the cells can be transfected at a cell density of from about 1 ×10⁶ to about 8×10⁷ viable cells/ml. In some embodiments, transfected cells are expanded to produce sufficient cell mass to seed a bioreactor from at least a 25L scale.

In some embodiments, the method further comprises harvesting the cell culture or cell culture supernatant and/or clarifying the cell culture or cell culture supernatant to produce a harvest media comprising recombinantly expressed virus, e.g., rAAV particles. In some embodiments, the method comprises a step of lysing a host cell in the cell culture or cell culture supernatant prior to clarification. Thus, in some embodiments, the method comprises: (a) transfecting host cells with: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinantAAV genome comprising nucleic acid encoding a transgene, optionally flanked by L-ITR and/or R-ITR); (b) culturing the transfected host cells for a sufficient period of time to produce rAAV capsid particles; and (c) lysing a host cell in the cell culture or cell culture supernatant.

Methods and compositions for lysing host cells are well known in the art. For example, a surfactant, e.g., a non-ionic surfactant, can be added to the cell culture or cell culture supernatant for lysing a host cell present therein. Generally, the non-ionic surfactant is added to the cell culture or cell culture supernatant to a final concentration of at least about 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1% (w/v, w/w or v/v) or higher. For example, the non-ionic surfactant is added to the cell culture or cell culture supernatant to a final concentration of from about 0.05% to about 1%, from about 0.1% to about 0.95%, from about 0.15% to about 0.9%, from about 0.2% to about 0.85%, from about 0.25% to about 0.8%, from about 0.3% to about 0.75%, from about 0.35% to about 0.65% from about 0.4% to about 0.6% or from 0.45% to about 0.55%. In some embodiments, the non-ionic surfactant is added to the cell culture or cell culture supernatant to a final concentration of about 0.05%, 0.1%., about 0.15%, about 0.2%, about 0.25%, about 0.3%, about 0.35%, about 0.4%, about 0.45%, about 0.5%, about 0.55%, about 0.6%, about 0.65%, about.7%, about 0.75%, about 0.8%, about 0.85%, about 0.9%, about 0.95%, or about 1%. For example, the non-ionic surfactant can be added to the cell culture or cell culture supernatant to a final concentration of about 0.5%.

After addition, the the non-ionic surfactant is allowed to mix with the cell culture or cell culture supernatant for a sufficient period of time to lyse host cells present in the cell culture or cell culture supernatant. For example, the non-ionic surfactant is mixed with the cell culture or cell culture supernatant for a period of from about 15 minutes to about 2 hours. In some embodiments, the non-ionic surfactant is mixed with the cell culture or cell culture supernatant for a period of from about 30 minutes to about 60 minutes. The mixing can be at ambient temperature or an elevated temperature. For example, the mixing with the non-ionic surfactant can be at a temperature from about 15°C to about 37°C. In some embodiments, the mixing with the non-ionic surfactant can be at a temperature of about 18°C, 19°C, 20°C, 21°C, 22°C, 23°C, 24°C, 25°C, 26°C, 27°C, 28°C, 28°C, 30°C, 31°C, 32°C, 33°C, 34°C, 35°C, 36°C, or 37°C.

It is noted that, any desired non-ionic surfactant can be used for lysing the host cells. Exemplary non-ionic surfactants and classes of non-ionic surfactants for lysing host cells can include polyarylphenol polyethoxy ethers; polyalkylphenol polyethoxy ethers; polyglycol ether derivatives of saturated fatty acids; polyglycol ether derivatives of unsaturated fatty acids; polyglycol ether derivatives of aliphatic alcohols; polyglycol ether derivatives of cycloaliphatic alcohols; fatty acid esters of polyoxyethylene sorbitan; alkoxylated vegetable oils; alkoxylated acetylenic dials; polyalkoxylated alkylphenols; fatty acid alkoxylates; sorbitan alkoxylates; sorbitol esters; C8 to C22 alkyl or alkenyl polyglycosides; polyalkoxy styrylaryl ethers; alkylamine oxides; block copolymer ethers; polyalkoxylated fatty glyceride; polyalkylene glycol ethers; linear aliphatic or aromatic polyesters; organo silicones; polyaryl phenols; sorbitol ester alkoxylates; and mono- and diesters of ethylene glycol and mixtures thereof; ethoxylated tristyrylphenol; ethoxylated fatty alcohol; ethoxylated lauryl alcohol; ethoxylated castor oil; and ethoxylated nonylphenol; alkoxylated alcohols, amines or acids. In some embodiments of any one of the aspects, the the non-ionic surfactant for lysing the host cells is selected from the group consisting of polyoxyethylene fatty alcohol ethers, polyoxyethylene alkylphenyl ethers, polyoxyethylene-polyoxypropylene block copolymers, alkylglucosides, alkylphenol ethoxylates, preferably polysorbates, polyoxyethylene alkyl phenyl ethers, and any combinations thereof. Specific exemplary non-ionic surfactants for lysing host cells include, but are not limited to, ECOSURF EH-9, polysorbates (such as polysorbate 20 (TWEEN 20), polysorbate 28, polysorbate 40, polysorbate 60, polysorbate 65, polysorbate 80, polysorbate 81, and polysorbate 85), ECOSURF EH-14, TWEEN 60 nonionic detergent, PPG-PEG-PPG Pluronic 10R5, Polyoxyethylene (18) tridecyl ether, Polyoxyethylene (12) tridecyl ether, MERPOL SH surfactant,MERPOL OJ surfactant, MERPOL HCS surfactant, IGEPAL CO-720, IGEPAL CO-630, IGEPAL CA-720, Brij S20, BrijS10, Brij 010, Brij C10, BRIJ 020, TERGITOL 15-S-7, ECOSURF SA-15, TERGITOL15-S-9, TERGITOL 15-S-12, TERGITOL L-64, TERGITOLNP-7, TERGITOL NP-8, TERGITOL NP-9, TERGITOL NP-9.5,TERGITOL NP-10, TERGITOL NP-11, TERGITOL NP-12, and TERGITOLNP-13 and any combinations thereof. Preferably, the non-onic surfactant for lysing host cells is not Triton X-100.

In some embodiments, a zwitterionic surfactant can be added to the cell culture or cell culture supernatant for lysing the host cell. Exemplary zwitterionic surfactants include, but are not limited to, sulfonates, such as CHAPS (3-[(3-Cholamidopropyl)dimethylammonio]-1-propanesulfonate), CHAPSO (3-{(3-cholamidopropyl)dimethylammonio}-2-hydroxy-I-propane- sulfonate), 3-(decyldimethylammonio)propanesulfonate, 3-(dodecyldimethylammonio) propanesulfonate, 3-(N,N-dimethylmyristylammonio)propanesulfonate, 3-(N,N-dimethyl octadecylammonio)propanesulfonate, 3-(N,N-dimethyloctylammonio) propanesulfonate, and 3-(N,N dimethylpalmitylammonio)propanesulfonate; sultaines, such as cocamidopropyl hydroxysultaine; betaines, e.g., cocamidopropyl betaine; and phosphates, such as lecithin.

In some embodiments, the surfactant, e.g., the zwitterionic surfactant can be an amine oxide surfactant. For example, an amine oxide surfactant can be added to the cell culture or cell culture supernatant for lysing the host cell. An amine oxide surfactant that can be used in methods described herein can be a trialkyl amine N-oxide, e.g., an amine oxide of formula R¹R²R³NO, wherein R¹ is a substituted or unsubstituted alkyl or alkenyl containing from about 8 to about 30 carbon atoms; and R² and R³ are independently substituted or unsubstituted alkyl or alkenyl groups containing from about 1 to about 18 carbon atoms. Non limiting examples of trialkyl amine N-oxide and trialkyl amine N-oxide surfactants of use are described in WO1998055581, which is incorporated herein by reference in its entirety.

In some embodiments, the method does not comprise a step of lysing a host cell prior to clarification.

The cell culture or cell culture supernatant may comprise impurities, e.g., host cell DNA (hcDNA). Therefore, the method can comprise a step, e.g., a post-lysis step of removing or reducing the amount of impurities, e.g., hcDNA from the cell culture or cell culture supernatant. Methods and compositions for reducing the amount of host cell DNA cell cultures or cell culture supernatants are well known in the art. For example, a cationic amine or nuclease can be added to the cell culture or cell culture supernatant. In some embodiments, the step of removing or reducing the amount of impurities comprise adding a selective precipitation agent to reduce or remove impurities such as hcDNA from the cell culture or cell culture supernatant. As used herein, a "selective precipitation agent" refers to any agent, compound or such which, when added to a preparation comprising a population of recombinant virus particles and contaminating nucleic acid molecules, will affect the selective precipitation of at least a substantial amount of contaminating nucleic acid molecules away from the recombinant virus particles. Exemplary agents for adding to the cell culture or cell culture supernatant in the post-lysis step include, but are not limited to cetyl trimethylammonium bromide, cetylpyridinium chloride, benzethonium chloride, tetradecyltrimethyl-ammonium chloride, polyethylene imine and combinations thereof.

In some embodiments, a nuclease, e.g., an endonuclease is added to the cell culture or cell culture supernatant for reducing or removing impurities such as hcDNA. Exemplary endonucleases include endonucleases derived from both Prokaryotes and Eukaryotes. In some embodiments, the nuclease is BENZONASE^{®} or a salt active nuclease (SAN). Generally, the nuclease is added to the cell culture or cell culture supernatant to a final concentration of at least about 0.05%, 0.1%., 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1% (w/v, w/w or v/v) or higher. For example, the nuclease is added to the cell culture or cell culture supernatant to a final concentration of from about 0.05% to about 1 %, from about 0.1 % to about 0.95%, from about 0.15% to about 0.9%, from about 0.2% to about 0.85%, from about 0.25% to about 0.8%, from about 0.3% to about 0.75%, from about 0.35% to about 0.65% from about 0.4% to about 0.6%, from 0.45% to about 0.55% from about 0.05% to about 0.4%, or from about 0.2% to about 0.4%. In some embodiments, the nuclease is added to the cell culture or cell culture supernatant to a final concentration of about 0.05%, 0.1%., about 0.15%, about 0.2%, about 0.25%, about 0.3%, about 0.35%, about 0.4%, about 0.45%, about 0.5%, about 0.55%, about 0.6%, about 0.65%, about.7%, about 0.75%, about 0.8%, about 0.85%, about 0.9%, about 0.95%, or about 1%. For example, the nuclease can be added to the cell culture or cell culture supernatant to a final concentration of about 0.2%. In some embodiments, the nuclease can be added to the cell culture or cell culture supernatant to a final concentration of about 0.05% to about 0.4%.

After adding, the agent or nuclease is allowed to mix with the cell culture or cell culture supernatant for a period of about 15, 20, 30, 35, 40, 45, 50, 55 minutes or longer. In some embodiments, the agent or nuclease is allowed to mix with the cell culture or cell culture supernatant for a period of from about 10 minutes to about 4 hours. For example, the agent or nuclease is mixed with the cell culture or cell culture supernatant for a period of from about 15 minutes to about 3 hours. In some embodiments, the agent or nuclease is mixed with the cell culture or cell culture supernatant for a period of from about 30 minutes to about 120 minutes. For example, the agent or nuclease is mixed with the cell culture or cell culture supernatant for a period of about 30 minutes.

In some embodiment, the method further comprises a step of clarifying the cell culture or cell culture supernatant. For example, the method comprises a step of clarifying the cell culture or cell culture supernatant by depth filtration to produce the clarified composition (e.g., harvest media) comprising recombinantly expressed virus, e.g., rAAV particles. Thus, in some embodiments, the method comprises: (a) transfecting host cells with: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR); (b) culturing the transfected host cells for a sufficient period of time to produce rAAV capsid particles; and (c) optionally, lysing a host cell in the cell culture or cell culture supernatant; and (d) clarifying the cell culture or cell culture supernatant, e.g., by depth filtration to produce a harvest media comprising recombinantly expressed virus, e.g., rAAV particles

Exemplary depth filters for use include, but are not limited to, CUNO^{®} Zeta PLUS^{®} Delipid filters, CUNO^{®} Emphaze AEX filters, CUNO^{®} 30/60ZA filters, CUNO^{®} 90ZB08A filters, CUNO^{®} DELI08A Delipid filters, and CUNO^{®} DELIP08A Delipid plus filters (3M, St. Paul, Minn.), Clarisolve^{®} grade 60HX, 40MS, 20MS, Millistak+^{®} HC grade C0HC, D0HC, A1HC, B1HC, X0HC, F0HC, Millistak+^{®} HC Pro grade D0SP, C0SP, and X0SP Millipore filters (EMD Millipore, Billerica, Mass.), and Sartopore^{®} bi-layer filter cartridges.

Without wishing to be bound by a theory, at least about 15% of the viral, e.g., rAAV capsid particles in the harvest media (e.g., the clarified cell culture or clarified cell culture supernatant) comprising recombinantly expressed virus, e.g., rAAV particles are full capsid particles. In certain embodiments, at least about 18% of the viral, e.g., rAAV capsid particles in the harvest media are full capsid particles. For example, at least about 20%, at least about 22%, at least about 25%, at least about 30%, at least about 35%, at least about 40%, at least about 45%, at least about 50%, or a higher percentage of the viral, e.g., rAAV capsid particles in the harvest media are full capsid particles.

A "filled particle" or "full particle" (also interchangeably referred to as "full AAV particle," "full AAV capsid particle", or "full rAAV capsid particle") refers to a viral particle that comprises an intact viral particle (*e.g*., complete capsid) comprising a genome (*e.g.*, the viral genome or the recombinant genome, which can comprise a heterologous polynucleotide such as a transgene, i.e., a polynucleotide other than a wild-type virus genome). A "filled" or "full" particle can also be interchangeably referred to as a "packaged particle," "packaged virus," "packaged AAV," or "recombinantly expressed AAV". It is noted that the terms "particle" and "capsid" can be used interchangeably and/or redundantly herein.

An "empty particle," which is also interchangeably referred to as "empty AAV particle," refers to a viral particle that comprises at least one viral protein but lacks all of the genome, *e.g*., virus genome or recombinant genome. Empty particles do not include, *e.g*., an intact viral particle comprising a heterologous polynucleotide.

A "partially full particle," which is also interchangeably referred to as "partially full AAV particle" or "partially filled AAV particle," refers to a viral particle that comprises at least one viral protein but lacks at least part of the genome, e.g., virus genome or recombinant genome. As used herein, "partially full particle" also include particles containing DNA from the host cell or pDNA used in transfection.

The percentage of full AAV particles ("% AAV full" or "% full") can be expressed as the number of "full" AAV particles over the total number of AAV particles (including "full," partially full," and "empty" AAV particles).

In some embodiments, the method further comprises a step of concentrating the clarified cell culture or cell culture supernatant. For example, the method comprises a step of concentrating the clarified cell culture or cell culture supernatant by tangential flow filtration. Thus, in some embodiments, the method comprises: (a) transfecting host cells with: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR); (b) culturing the transfected host cells for a sufficient period of time to produce rAAV capsid particles; (c) optionally, lysing a host cell in the cell culture or cell culture supernatant; (d) clarifying the cell culture or cell culture supernatant, e.g., by depth filtration to produce a harvest media comprising recombinantly expressed virus, e.g., rAAV particles; and (e) concentrating the clarified cell culture or cell culture supernatant, e.g., by tangential flow filtration.

The recombinant viral particles (e.g., rAAV particles) in the harvest media can be enriched, isolated or purified. Thus, in some embodiments, the method further comprises a step of enriching or purifying) the virus, e.g., rAAV capsid particles in the harvest media. For example, the method comprises: (a) transfecting host cells with: i) a polynucleotide described herein; ii) a nucleic acid comprising a nucleotide sequence encoding viral capsid (e.g., AAV capsid) and/or non-structural replication (e.g., AAV Rep) genes; and iii) a nucleic acid encoding a virus genome (e.g., an AAV endogenous genome, optionally flanked by left inverted terminal repeat (L-ITR) and/or right ITR (R-ITR), or a recombinantAAV genome comprising nucleic acid encoding a transgene, optionally flanked by L-ITR and/or R-ITR); (b) culturing the transfected host cells for a sufficient period of time to produce rAAV capsid particles; (c) optionally, lysing a host cell in the cell culture or cell culture supernatant; (d) clarifying the cell culture or cell culture supernatant, e.g., by depth filtration to produce a harvest media comprising recombinantly expressed virus, e.g., rAAV particles; (e) optionally, concentrating the clarified cell culture or cell culture supernatant, e.g., by tangential flow filtration; and (f) enriching, isolating or purifying the recombinantly expressed virus, e.g., rAAV particles from the clarified cell culture or clarified cell culture supernatant.

Methods for enriching or purifying recombinantly produced e.g., rAAV capsid particles are well known in the art. Exemplary methods include, but are not limited to chromatography, e.g., affinity chromatography and/or anion exchange chromatography. See, for example, PCT Publication WO2022159679, contents of which are incorporated herein by reference in their entireties. Without wishing to be bound by a theory, the step of enriching or purifying increases the percentage of full viral particles (e.g., by removing at least some of the partially full or empty viral particles). It is noted that the the purified or enriched rAAV particles (e.g., as measured by % full AAV particles, % full rAAV particles) may still comprise partially full viral particles and/or empty viral particles; however, the percentage of partially full viral particles and/or empty viral particles is substantially decreased compared to a clarified lysate that is not enriched. Stated in another way, the percentage of full viral particles is substantially increased compared to the percentage in the clarified lysate.

In some embodiments, the purified or enriched rAAV particles comprise at least about 65% full capsid particles. For example, the purified or enriched rAAV particles comprise at least about 70% full capsid particles, at least about 75% full capsid particles, at least about 80% full capsid particles, at least about 85% full capsid particles, at least about 90% full capsid particles, at least about 95% full capsid particles, at least about 98% full capsid particles, at least about 99% full capsid particles or at least about 99.5% full capsid particles or higher. In some embodiments, the purified or enriched rAAV particles comprise 100% full capsid particles.

Generally, the purified or enriched rAAV particles comprise less than about 10% empty capsid particles. For example, the purified or enriched rAAV particles comprise less than about 8% empty capsid particles, less than about 6% empty capsid particles, less than about 5% empty capsid particles, less than about 5% empty capsid particles, less than about 3% empty capsid particles, less than about 2% empty capsid particles, less than about 1% empty capsid particles, less than about 0.8% empty capsid particles, less than about 0.6% empty capsid particles, less than about 0.5% empty capsid particles, less than about 0.4% empty capsid particles, less than about 0.3% empty capsid particles, less than about 0.2% empty capsid particles, less than about 0.1% empty capsid particles, less than about 0.08% empty capsid particles, less than about 0.06% empty capsid particles, less than about 0.05% empty capsid particles, less than about 0.03% empty capsid particles, less than about 0.02% empty capsid particles, or less than about 0.01% empty capsid particles, or fewer % empty capsid particles. In some embodiments, the purified or enriched rAAV particles are substantially devoid of empty capsid particles.

In certain embodiments, the method provides at least about 1 × 10⁴ vector genome-containing particles per cell prior to purification, e.g., at least about 2 × 10⁴, 3 × 10⁴, 4 × 10⁴, 5 × 10⁴, 6 × 10⁴, 7 × 10⁴, 8 × 10⁴, 9 × 10⁴, or 1 × 10⁵ or more vector genome-containing particles per cell prior to purification. In other embodiments, the method provides at least about 1 × 10¹² purified vector genome-containing particles per liter of cell culture, e.g., at least about 5 × 10¹², 1 × 10¹³, 5 × 10¹³, or 1 × 10¹⁴ or more purified vector genome-containing particles per liter of cell culture.

In some embodiments, at least about 50%, (e.g., at least about 55%, at least about 60%, at least about 65%, at least about 70%, at least about 75%, at least about 80%, at least about 85%, at last about 95%, at least about 95% or a higher percentage) of the rAAV particles produced using a polynucleotide described herein are full capsid particles.

In some embodiments, less than about 10%, less than about 8%, less than about 6%, less than about 5%, less than about 5%, less than about 3%, less than about 2%, less than about 1%, less than about 0.8%, less than about 0.6%, less than about 0.5%, less than about 0.4%, less than about 0.3%, less than about 0.2%, less than about 0.1%, less than about 0.08%, less than about 0.06%, less than about 0.05%, less than about 0.03%, less than about 0.02%, or less than about 0.01%, or fewer of the rAAV particles produced using a polynucleotide described herein are empty capsid particles.

Surprisingly and unexpectedly, methods using a polynucleotide described herein produces rAAV with higher % full capsid than that the rAAV produced with the helper plasmid xx680 **(SEQ ID NO: 97**) under the same conditions. Without wishing to be bound by a theory, this indicates, the polynucleotide described herein has a higher packaging efficiency than the helper plasmid xx680 nucleic. Accordingly, in some embodiments, method using a polynucleotide described herein produces rAAV particles that comprises at least about 1.1 fold higher, e.g., at least about 1.2 fold higher, at least about 1.3 fold higher, at least about 1.4 fold higher, at least about, 1.5 fold higher, at least about 1.6 fold higher, at least about 1.7 fold higher, at least about 1.8 fold higher, at least about 2 fold higher, at least about 2.2 fold higher, at least about 2.5 fold higher, at least about 2.8 fold higher, at least about 3 fold higher or a greater fold higher full capsid particles compared to rAAV particles produced with the helper plasmid xx680 **(SEQ ID NO: 97)** under the same conditions.

In some embodiments, the method produces at least about 1 × 10⁴ (e.g., e.g., at least about 2 × 10⁴, 3 × 10⁴, 4 × 10⁴, 5 × 10⁴, 6 × 10⁴, 7 × 10⁴, 8 × 10⁴, 9 × 10⁴, or 1 × 10⁵ or more) vector genome-containing particles per cell.

Surprisingly and unexpectedly, the method using a polynucleotide described herein produces rAAV an higher amount of vector genome-containing particles per cell compared to with the helper plasmid xx680 **(SEQ ID NO: 97)** under the same conditions. Accordingly, in some embodiments, method using a polynucleotide described herein produces at least about 1.1 fold higher, e.g., at least about 1.2 fold higher, at least about 1.3 fold higher, at least about 1.4 fold higher, at least about, 1.5 fold higher, at least about 1.6 fold higher, at least about 1.7 fold higher, at least about 1.8 fold higher, at least about 2 fold higher, at least about 2.2 fold higher, at least about 2.5 fold higher, at least about 2.8 fold higher, at least about 3 fold higher or a greater fold higher vector genome-containing particles per cell compared to the helper plasmid xx680 **(SEQ ID NO: 97)** under the same conditions.

In some embodiments, the method provides at least about 1e¹² vector genome per ml (vg/ml) (e.g., at least about 1.5e¹² vg/ml, at least about 2e¹² vg/ml, at least about 2.5e¹² vg/ml, at least about 3e¹² vg/ml, at least about 3.5e¹² vg/ml, at least about 4e¹² vg/ml, at least about 4.5e¹² vg/ml, at least about 5e¹² vg/ml, at least about 5.5e¹² vg/ml, at least about 6e¹² vg/ml, at least about 6.5e¹² vg/ml, at least about 7e¹² vg/ml, at least about 7.5e¹²vg/ml, at least about 8e¹²vg/ml, at least about 8.5e¹²vg/ml, at least about 9e¹² vg/ml, at least about 9.5e¹³ vg/ml, at least about 1e¹³vg/ml, at least about 1.5e¹³vg/ml, at least about 2e¹³vg/ml, at least about 2.5e¹³vg/ml, at least about 3e¹³vg/ml, at least about 3.5e¹³vg/ml, at least about 4e¹³ vg/ml, at least about 4.5e¹³ vg/ml, at least about 5e¹³ vg/ml, at least about 5.5e¹³ vg/ml, at least about 6e¹³ vg/ml, at least about 6.5e¹³ vg/ml, at least about 7e¹³vg/ml, at least about 7.5e¹³vg/ml, at least about 8e¹³vg/ml, at least about 8.5e¹³vg/ml, at least about 9e¹³vg/ml, at least about 9.5e¹³ vg/ml, at least about 1e¹⁴ vg/ml or more). The vector genome per cell can be determined prior to or after purification (e.g., after affinity purification).

Surprisingly and unexpectedly, the method using a polynucleotide described herein produces a higher amount of vector genome per ml compared to with the helper plasmid xx680 **(SEQ ID NO: 97)** under the same conditions. Accordingly, in some embodiments, method using a polynucleotide described herein produces at least about 1.1 fold higher, e.g., at least about 1.2 fold higher, at least about 1.3 fold higher, at least about 1.4 fold higher, at least about, 1.5 fold higher, at least about 1.6 fold higher, at least about 1.7 fold higher, at least about 1.8 fold higher, at least about 2 fold higher, at least about 2.2 fold higher, at least about 2.5 fold higher, at least about 2.8 fold higher, at least about 3 fold higher or a greater fold higher vector genome per ml compared to the helper plasmid xx680 **(SEQ ID NO: 97)** under the same conditions.

In some embodiments of any one of the aspects described herein, the method produces at least about 1e¹² viral particles per ml (vp/ml) at least about 1e¹² viral particles per ml (vp/ml) (e.g., at least about 1.5e¹² vp/ml, at least about 2e¹² vp/ml, at least about 2.5e¹² vp/ml, at least about 3e¹² vp/ml, at least about 3.5e¹² vp/ml, at least about 4e¹² vp/ml, at least about 4.5e¹² vp/ml, at least about 5e¹² vp/ml, at least about 5.5e¹² vp/ml, at least about 6e¹² vp/ml, at least about 6.5e¹² vp/ml, at least about 7e¹² vp/ml, at least about 7.5e¹²vp/ml, at least about 8e¹²vp/ml, at least about 8.5e¹²vp/ml, at least about 9e¹² vp/ml, at least about 9.5e¹³ vp/ml, at least about 1e¹³vp/ml, at least about 1.5e¹³vp/ml, at least about 2e¹³vp/ml, at least about 2.5e¹³vp/ml, at least about 3e¹³vp/ml, at least about 3.5e¹³vp/ml, at least about 4e¹³ vp/ml, at least about 4.5e¹³ vp/ml, at least about 5e¹³ vp/ml, at least about 5.5e¹³ vp/ml, at least about 6e¹³ vp/ml, at least about 6.5e¹³ vp/ml, at least about 7e¹³vp/ml, at least about 7.5e¹³vp/ml, at least about 8e¹³vp/ml, at least about 8.5e¹³vp/ml, at least about 9e¹³vp/ml, at least about 9.5e¹³ vp/ml, at least about 1e¹⁴vp/ml or more).. The vector particles per cell can be determined prior to or after purification (e.g., after affinity purification).

Surprisingly and unexpectedly, the method using a polynucleotide described herein produces an higher amount of viral particles per ml compared to with the helper plasmid xx680 **(SEQ ID NO: 97)** under the same conditions. Accordingly, in some embodiments, method using a polynucleotide described herein produces at least about 1.1 fold higher, e.g., at least about 1.2 fold higher, at least about 1.3 fold higher, at least about 1.4 fold higher, at least about, 1.5 fold higher, at least about 1.6 fold higher, at least about 1.7 fold higher, at least about 1.8 fold higher, at least about 2 fold higher, at least about 2.2 fold higher, at least about 2.5 fold higher, at least about 2.8 fold higher, at least about 3 fold higher or a greater fold higher viral particles per ml compared to the helper plasmid xx680 **(SEQ ID NO: 97)** under the same condition.

Those skilled in the art will appreciate that it may be advantageous to provide the AAV cap and rep sequences and the polynucleotide sequences on a single helper construct. Thus, in some cases, the polynucleotide described herein and the nucleic acid encoding the viral capsid and non-structural replication genes are the same, i.e., the polynucleotide further comprises a nucleotide sequence encoding the viral capsid (e.g., AAV capsid) and/or non-structural replication (e.g., AAV Rep) genes.

In some embodiments, the nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR) is a plasmid DNA, a closed ended linear duplexed DNA (clDNA), or a no-end DNA (neDNA). In some embodiments, the nucleic acid encoding a virus genome is a no-end DNA (neDNA). In some embodiments, the nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR) comprises one or both of the ITRs each of which independently is 145 nucleotides long, or less than 145 nucleotides long. In some embodiments, the nucleic acid encoding a virus genome (e.g., an AAV endogenous genome, optionally flanked by left inverted terminal repeat (L-ITR) and/or right ITR (R-ITR), or a recombinant AAV genome comprising nucleic acid encoding a transgene, optionally flanked by L-ITR and/or R-ITR) comprises one or both of the ITRs each of which independently is 140 nucleotides long, 135 nucleotides long, 130 nucleotides long, 125 nucleotides long or less than 125 nucleotides long.

### Serotypes

The method described herein is easily adaptable for different viruses and serotypes. Thus, in some embodiments of any one of the aspects, the recombinant virus particles are recombinant adeno associated virus (rAAV) particles. For example, the rAAV particles can be AAV-1, AAV-2, AAV-2i8, AAV-3, AAV-4, AAV-5, AAV-6, AAV-7, AAV-8, AAV-9, AAV-10, AAVrh10, AAV-11, AAV-12, AAV-13, AAV-14, AAV-15, AAV-16 or a chimera, derivative, modification, or pseudotype thereof. In some embodiments, the rAAV is a rational polyploid (also referred as haploid) AAV. In some embodiments, the method described herein is used to prepare rAAV, wherein the rAAV comprises at least one capsid protein (e.g., VP1, VP2, or VP3) from the AAV serotypes listed in **Table 2.**

| **TABLE 2: Exemplary descriptions of capsids and sequences** *(patent and non-patent references are incorporated herein by reference in their entireties)* | |
|---|---|
| **Serotype and where capsid sequence is published and/or described** | **Serotype and where capsid sequence is published and/or described** |
| AAV1 (seeSEQ IDNO:11 in US20150159173 and SEQ ID NO:202 in US20150315612) | AAV1 (see SEQ ID NO:1 in US20160017295, SEQ ID NO:64 in US20030138772, SEQ ID NO:27 in US20150159173, and SEQ ID NO:5 in US7198951) |
| AAV1 (see SEQ ID NO:6 in US20030138772) | AAV1.3 (see SEQ ID NO:14 in US20030138772) |
| AAV2 (see SEQ ID NO:7 in US20150159173 and SEQ ID NO:211 in US20150315612) | AAV2 (see SEQ ID NO:70 in US20030138772, SEQ ID NO:23 in US20150159173, and SEQ ID NO:4 in US6156303) |
| AAV2 (see SEQ ID NO:8 in US6156303) | AAV2 (see SEQ ID NO:7 in US20030138772) |
| AAV2 (see SEQ ID NO:3 in US6156303) | AAV2.5T (see SEQ ID NO:42 in US9233131) |
| AAV3 (see SEQ ID NO:12 in US20150159173) | AAV3 (see SEQ ID NO: 71 in US20030138772, SEQ ID NO:28 in US20150159173, and SEQ ID NO:6 in US7198951) |
| AAV3.3b (See SEQ ID NO:72 in US20030138772) | AAV3-3 (See SEQ ID NO: 200 US20150315612) |
| AAV3-3 (See SEQ ID NO:217 US20150315612) | AAV3a ((See SEQ ID NO: 5 in US6156303) |
| AAV3a (See SEQ ID NO: 9 in US6156303) | AAV3b (See SEQ ID NO: 6 in US6156303) |
| AAV3b (See SEQ ID NO: 10 in US6156303) | AAV3b (See SEQ ID NO: 1 in US6156303) |
| AAV4 (See SEQ ID NO:17 US20140348794) | AAV4 ((See SEQ ID NO:5 in US20140348794) |
| AAV4 (See SEQ ID NO: 3 in US20140348794) | AAV4 (See SEQ ID NO:14 in US20140348794) |
| AAV4 (See SEQ ID NO: 15 in US20140348794) | AAV4 (See SEQ ID NO: 1 in US20140348794) |
| AAV4 (See SEQ ID NO: 12 in US20140348794) | AAV4 (See SEQ ID NO: 1 in US20140348794) |
| AAV4 (See SEQ ID NO: 7 in US20140348794) | AAV4 (See SEQ ID NO 8 in US20140348794) |
| AAV4 (See SEQ ID NO: 9 in US20140348794) | AAV4 (See SEQ ID NO: 2 in US20140348794) |
| AAV4 (See SEQ ID NO: 10 in US20140348794) | AAV4 (See SEQ ID NO: 11 in US20140348794) |
| AAV4 (See SEQ ID NO: 18 in US20140348794) | AAV4 (See SEQ ID NO:63 in US20030138772 and SEQ ID NO:4 in US20160017295) |
| AAV4 (See SEQ ID NO: 4 in US20140348794) | AAV4 (See SEQ ID NO: 16 in US20140348794) |
| AAV4 (See SEQ ID NO: 20 in US20140348794) | AAV4 (See SEQ ID NO: 6 in US20140348794) |
| AAV4 (See SEQ ID NO: 1 in US20140348794) | AAV42.2 (See SEQ ID NO: 9 in US20030138772) |
| AAV42.2 (See SEQ ID NO: 102 in US20030138772) | AAV42.3b (See SEQ ID NO: 36 in US20030138772) |
| AAV42.3B (See SEQ ID NO: 107 in US20030138772) | AAV42.4 (See SEQ ID NO: 33 in US20030138772) |
| AAV42.4 (See SEQ ID NO: 88 in US20030138772) | AAV42.8 (See SEQ ID NO: 27 in US20030138772) |
| AAV42.8 (See SEQ ID NO: 85 in US20030138772) | AAV43.1 (See SEQ ID NO: 39 in US20030138772) |
| AAV43.1 (See SEQ ID NO: 92 in US20030138772) | AAV43.12 (See SEQ ID NO: 41 in US20030138772) |
| AAV43.12 (See SEQ ID NO: 93 in US20030138772) | AAV5 (see SEQ ID NO:1 in US7427396) |
| AAV5 (see SEQ ID NO:114 in US20030138772) | AAV5 (see SEQ ID NO:5 in US20160017295, SEQ ID NO:2 in US7427396, and SEQ ID NO:216 in US20150315612) |
| AAV5 (see SEQ ID NO:199 in US20150315612) | AAV6 (see SEQ ID NO:65 in US20030138772, SEQ ID NO:29 in US20150159173, SEQ ID NO:6 in US20160017295, and SEQ ID NO:7 in US6156303) |
| AAV6 (see SEQ ID NOS:2, 7, and 11 in US6156303) | AAV6 (see SEQ ID NOS:203 and 220 in US20150315612) |
| AAV7 (see SEQ ID NO:14 in US20150159173) | AAV7 (see SEQ ID NO:183 in US20150315612) |
| AAV7 (see SEQ ID NO:2 in US20030138772, SEQ ID NO:30 in US20150159173, SEQ ID NO:181 in US20150315612, and SEQ ID NO:7 in US20160017295) | AAV7 (see SEQ ID NO:3 in US20030138772) |
| AAV7 (see SEQ ID NO:1 in US20030138772 and SEQ ID NO:180 in US20150315612) | AAV7 (see SEQ ID NO:213 US20150315612) |
| AAV7 (see SEQ ID NO:222 in US20150315612) | AAV7 (see SEQ ID NO:2 in US8906675) |
| AAV8 (See SEQ ID NO: 15 in US20150159173) | AAV8 (See SEQ ID NO: 7 in US20150376240) |
| AAV8 (See SEQ ID NO:4 in US20030138772; SEQ ID NO: 182 in US20150315612) | AAV8 (See SEQ ID NO: 95 in US20030138772, SEQ ID NO:1 in US20140359799) |
| AAV8 (See SEQ ID NO: 31 in US20150159173) | AAV8 (See, e.g., SEQ ID NO: 8 in US20160017295, or SEQ ID NO:7 in US7198951, or SEQ ID NO: 223 in US20150315612) |
| AAV8 (See SEQ ID NO: 8 in US20150376240) | AAV8 (See SEQ ID NO: 214 in US20150315612) |
| AAV-8b (See SEQ ID NO: 5 in US20150376240) | AAV-8b (See SEQ ID NO: 3 in US20150376240) |
| AAV-8h (See SEQ ID NO: 6 in US20150376240) | AAV-8h (See SEQ ID NO: 4 in US20150376240) |
| AAV9 (See SEQ ID NO: 5 in US20030138772) | AAV9 (See SEQ ID NO: 1 in US7198951) |
| AAV9 (See SEQ ID NO: 9 in US20160017295) | AAV9 (See SEQ ID NO: 100 in US20030138772 and SEQ ID NO:2 in US7198951) |
| AAV9 (See SEQ ID NO: 3 in US7198951) | AAV9 (AAVhu.14) (See SEQ ID NO: 3 in US20150315612) |
| AAV9 (AAVhu.14) (See SEQ ID NO: 123 in US20150315612) | AAV10 (see SEQ ID NO:117 in US20030138772) |
| AAV10 (SEQ ID NO:9 in WO2015121501) | AAV10 (SEQ ID NO:8 in WO2015121501) |
| AAV11 (SEQ ID NO:118 in US20030138772) | AAV12 (SEQ ID NO:119 in US20030138772) |
| AAVA3.1 (See SEQ ID NO: 120 in US20030138772) | AAVA3.3 (See SEQ ID NO: 57 in US20030138772) |
| AAVA3.3 (See SEQ ID NO: 66 in US20030138772) | AAVA3.4 (See SEQ ID NO: 54 in US20030138772) |
| AAVA3.4 (See SEQ ID NO: 68 in US20030138772) | AAVA3.5 (See SEQ ID NO: 55 in US20030138772) |
| AAVA3.5 (See SEQ ID NO: 69 in US20030138772) | AAVA3.7 (See SEQ ID NO: 56 in US20030138772) |
| AAVA3.7 (See SEQ ID NO: 67 in US20030138772) | AAV29. (See SEQ ID NO: 11 in (AAVbb. I) 161 US20030138772) |
| AAVC2 (See SEQ ID NO: 61 in US20030138772) | AAVCh.5 (See SEQ ID NO:46 in US20150159173); SEQ ID NO: 234 in US20150315612) |
| AAV24.1 (See SEQ ID NO: 101 in US20030138772) | AAVcy.2 (AAV13.3) (See SEQ ID NO: 15 in US20030138772) |
| AAV27.3 (See SEQ ID NO: 104 in US20030138772) | AAVcy.3 (AAV24.1) (See SEQ ID NO: 16 in US20030138772) |
| AAVcy.5 (See SEQ ID NO: 227 in US20150315612) | AAVcy.4 (AAV27.3) (See SEQ ID NO: 17 in US20030138772) |
| AAVcy.5 (AAV7.2) (See SEQ ID NO: 18 in US20030138772) | AAV7.2 (See SEQ ID NO: 103 in US20030138772) |
| AAVcy.6 (AAV16.3) (See SEQ ID NO: 10 in US20030138772) | AAV16.3 (See SEQ ID NO: 105 in US20030138772) |
| AAVcy.5 (See SEQ ID NO: 24 in US20150159173) | AAVcy.5 (See SEQ ID NO: 8 in US20150159173) |
| AAVCy.5R2 (See SEQ ID NO: 24 modified with G13D and D403N in US20150159173) | AAVCy.5Rl (See SEQ ID NO: 24 modified with G13D in US20150159173) |
| AAVCy.5R4 (See SEQ ID NO: 24 modified with G13D, D403N, R51K, N158K, and P161Q in US20150159173) | AAVCy.5R3 (See SEQ ID NO: 24 modified with G13D, D403N, and R51K in US20150159173) |
| AAVDJ (See SEQ ID NO: 2 in US20140359799) | AAVDJ (See SEQ ID NO: 3 in US20140359799 and SEQ ID NO: 2 in US7588772) |
| AAVDJ-8 (See SEQ ID NO: 1 modified with R587Q and R590T in US7588772) | AAVDJ (See SEQ ID NO:1 in US7588772) |
| AAVH2 (See SEQ ID NO: 26 in US20030138772) | AAVF5 (See SEQ ID NO: 110 in US20030138772) |
| AAVhEl. I (See SEQ ID NO: 44 in US9233131) | AAVH6 (See SEQ ID NO: 25 in US20030138772) |
| AAVhErl.16 (See SEQ ID NO: 48 in US9233131) | AAVhErl.14 (See SEQ ID NO: 46 in US9233131) |
| AAVhErl.23 (AAVhEr2.29) (See SEQ ID NO: 53 in US9233131) | AAVhErl.18 (See SEQ ID NO: 49 in US9233131) |
| AAVhErl.36 (See SEQ ID NO: 52 in US9233131) | AAVhErl.35 (See SEQ ID NO: 50 in US9233131) |
| AAVhErl.7 (See SEQ ID NO: 51 in US9233131) | AAVhErl.5 (See SEQ ID NO: 45 in US9233131) |
| AAVhEr2.16 (See SEQ ID NO: 55 in US9233131) | AAVhErl.8 (See SEQ ID NO: 47 in US9233131) |
| AAVhEr2.31 (See SEQ ID NO: 58 in US9233131) | AAVhEr2.30 (See SEQ ID NO: 56 in US9233131) |
| AAVhEr2.4 (See SEQ ID NO: 54 in US9233131) | AAVhEr2.36 (See SEQ ID NO: 57 in US9233131) |
| AAVhu.I (See SEQ ID NO 46 in US20150315612) | AAVhEr3.1 (See SEQ ID NO: 59 in US9233131) |
| AAVhu.IO (AAV16.8) (See SEQ ID NO: 56 in US20150315612) | AAVhu.I (See SEQ ID NO: 144 in US20150315612) |
| AAVhu.I I (AAV16.12) (See SEQ ID NO: 57in US20150315612) | AAVhu.IO (AAV16.8) (See SEQ ID NO: 156 in US20150315612) |
| AAVhu. 12 (See SEQ ID NO: 59 in US20150315612) | AAVhu.I I (AAV16.12) (See SEQ ID NO: 153 in US20150315612) |
| AAVhu. 13 (See SEQ ID NO: 16 in US2015015917 and ID NO: 71 in US20150315612) | AAVhu.12 (See SEQ ID NO: 154 in US20150315612) |
| AAVhu. 13 (See SEQ ID NO: 32 in US20150159173 and ID NO: 129 US20150315612) | AAVhu.136.1 (See SEQ ID NO: 165 in US20150315612) |
| AAVhu.140.2 (See SEQ ID NO: 167 in US20150315612) | AAVhu.140.1 (See SEQ ID NO: 166 in US20150315612) |
| AAVhu. 15 (See SEQ ID NO: 147 in US20150315612) | AAVhu.145.6 (See SEQ ID NO: 178 in US20150315612) |
| AAVhu. 156.1 (See SEQ ID NO: 179 in US20150315612) | AAVhu.15 (AAV33.4) (See SEQ ID NO: 50 in US20150315612) |
| AAVhu.l6 (AAV33.8) (See SEQ ID NO: 51 in US20150315612) | AAVhu.16 (See SEQ ID NO: 148 in US20150315612) |
| AAVhu.l7 (AAV33.12) (See SEQ ID NO: 4 in US20150315612) | AAVhu.17 (See SEQ ID NO: 83 in US20150315612) |
| AAVhu. 172.2 (See SEQ ID NO: 172 in US20150315612) | AAVhu.172.1 (See SEQ ID NO: 171 in US20150315612) |
| AAVhu. 173.8 (See SEQ ID NO: 175 in US20150315612) | AAVhu.173.4 (See SEQ ID NO: 173 in US20150315612) |
| AAVhu. 18 (See SEQ ID NO: 149 in US20150315612) | AAVhu.18 (See SEQ ID NO: 52 in US20150315612) |
| AAVhu. 19 (See SEQ ID NO: 133 in US20150315612) | AAVhu.19 (See SEQ ID NO: 62 in US20150315612) |
| AAVhu.2 (See SEQ ID NO: 143 in US20150315612) | AAVhu.2 (See SEQ ID NO: 48 in US20150315612) |
| AAVhu.20 (See SEQ ID NO: 134 in US20150315612) | AAVhu.20 (See SEQ ID NO: 63 in US20150315612) |
| AAVhu.21 (See SEQ ID NO: 135 in US20150315612) | AAVhu.21 (See SEQ ID NO: 65 in US20150315612) |
| AAVhu.22 239 (See SEQ ID NO: 138 in US20150315612) | AAVhu.22 (See SEQ ID NO: 67 in US20150315612) |
| AAVhu.23.2 (See SEQ ID NO: 137 in US20150315612) | AAVhu.23 (See SEQ ID NO: 60 in US20150315612) |
| AAVhu.24 (See SEQ ID NO: 136 in US20150315612) | AAVhu.24 (See SEQ ID NO: 66 in US20150315612) |
| AAVhu.25 (See SEQ ID NO: 146 in US20150315612) | AAhu.25 (See SEQ ID NO: 49 in US20150315612) |
| AAVhu.26 (See SEQ ID NO: 33 in US20150159173, and SEQ ID NOS: 61 and 139 in US20150315612) | AAVhu.26 (See SEQ ID NO: 17 in US20150159173 and SEQ ID NO: 61 in US20150315612) |
| AAVhu.27 (See SEQ ID NO: 140 in US20150315612) | AAVhu.27 (See SEQ ID NO: 64 in US20150315612) |
| AAVhu.28 (See SEQ ID NO: 130 in US20150315612) | AAVhu.28 (See SEQ ID NO: 68 in US20150315612) |
| AAVhu.29 (See SEQ ID NO: 42 in US20150159173 and SEQ ID NO: 132 in US20150315612) | AAVhu.29 (See SEQ ID NO: 69 in US20150315612) |
| AAVhu.29 (See SEQ ID NO: 225 in US20150315612) | AAVhu.29R (See SEQ ID NO: 42 with G396E in US20150159173) |
| AAVhu.3 (See SEQ ID NO: 44 in US20150315612) | AAVhu.3 (See SEQ ID NO: 145 in US20150315612) |
| AAVhu.30 (See SEQ ID NO: 70 in US20150315612) | AAVhu.30 (See SEQ ID NO: 131 in US20150315612) |
| AAVhu.31 (See SEQ ID NO: 1 in US20150315612) | AAVhu.31 (See SEQ ID NO: 121 in US20150315612) |
| AAVhu.32 (See SEQ ID NO: 2 in US20150315612) | AAVhu.32 (See SEQ ID NO: 122 in US20150315612) |
| AAVhu.33 (See SEQ ID NO: 75 in US20150315612) | AAVhu.33 (See SEQ ID NO: 124 in US20150315612) |
| AAVhu.34 (See SEQ ID NO: 72 in US20150315612) | AAVhu.34 (See SEQ ID NO: 125 in US20150315612) |
| AAVhu.35 (See SEQ ID NO: 73 in US20150315612) | AAVhu.35 (See SEQ ID NO: 164 in US20150315612) |
| AAVhu.36 (See SEQ ID NO: 74 in US20150315612) | AAVhu.36 (See SEQ ID NO: 126 in US20150315612) |
| AAVhu.37 (See SEQ ID NO: 34 in US20150159173 and SEQ ID NO: 88 in US20150315612) | AAVhu.37 (AAV106.1) (See SEQ ID NO: 10 in US20150315612 and SEQ ID NO: 18 in US20150159173) |
| AAVhu.38 (See SEQ ID NO: 161 in US20150315612) | AAVhu.39 (See SEQ ID NO: 102 in US20150315612) |
| AAVhu.39 (AAVLG-9) (See SEQ ID NO: 24 in US20150315612 | AAVhu.4 (See SEQ ID NO: 47 in US20150315612) |
| AAVhu.4 (See SEQ ID NO: 141 in US20150315612) | AAVhu.40 (See SEQ ID NO: 87 in US20150315612) |
| AAVhu.40 (AAV114.3) (See SEQ ID NO: 11 in US20150315612) | AAVhu.41 (See SEQ ID NO: 91 in US20150315612) |
| AAVhu.41 (AAV127.2) (See SEQ ID NO: 6 in US20150315612) | AAVhu.42 (See SEQ ID NO: 85 in US20150315612) |
| AAVhu.42 (AAV127.5) (See SEQ ID NO:8 in US20150315612) | AAVhu.43 (See SEQ ID NO: 160 in US20150315612) |
| AAVhu.43 (See SEQ ID NO: 236 in US20150315612) | AAVhu.43 (AAV128.1) (See SEQ ID NO: 80 in US20150315612) |
| AAVhu.44 (See SEQ ID NO: 45 in US20150159173 and SEQ ID NO: 158 in US20150315612) | AAVhu.44RI (See SEQ ID NO: 45 modified with E137K in US20150159173) |
| AAVhu.44 (AAV128.3) (See SEQ ID NO: 81 in US20150315612) | AAVhu.44R3 (See SEQ ID NO: 45 modified with E137K, P446L, and G609D in US20150159173) |
| AAVhu.44R2 (See SEQ ID NO: 45 modified with E137K and P446L in US20150159173) | AAVhu.45 (See SEQ ID NO: 127 in US20150315612) |
| AAVhu.45 (See SEQ ID NO: 76 in US20150315612) | AAVhu.46 (See SEQ ID NO: 159 in US20150315612) |
| AAVhu.46 (See SEQ ID NO: 82 in US20150315612) | AAVhu.47 (See SEQ ID NO: 77 in US20150315612) |
| AAVhu.46 (See SEQ ID NO: 224 in US20150315612) | AAVhu.48 (See SEQ ID NO: 38 in US20150159173) |
| AAVhu.47 (See SEQ ID NO: 128 in US20150315612) | AAVhu.48 (AAV130.4) (See SEQ ID NO: 78 in US20150315612) |
| AAVhu.48 (See SEQ ID NO: 157 in US20150315612) | AAVhu.48R2 (See SEQ ID NO: 38 modified with G277S and E322K in US20150159173) |
| AAVhu.48RI (See SEQ ID NO: 38 modified with G277S in US20150159173) | AAVhu.49 (See SEQ ID NO: 209 in US20150315612) |
| AAVhu.48R3 (See SEQ ID NO: 38 modified with G277S, E322K, and S552N in US20150159173) | AAVhu.5 (See SEQ ID NO: 45 in US20150315612) |
| AAVhu.49 (See SEQ ID NO: 189 in US20150315612) | AAVhu.51 (See SEQ ID NO: 208 in US20150315612) |
| AAVhu.5 (See SEQ ID NO: 142 in US20150315612) | AAVhu.52 (See SEQ ID NO: 210 in US20150315612) |
| AAVhu.51 (See SEQ ID NO: 190 in US20150315612) | AAVhu.53 (See SEQ ID NO: 19 in US20150159173) |
| AAVhu.52 (See SEQ ID NO: 191 in US20150315612) | AAVhu.53 (AAV145.1) (See SEQ ID NO: 176 in US20150315612) |
| AAVhu.53 (See SEQ ID NO: 35 in US20150159173) | AAVhu.54 (AAV145.5) (See SEQ ID NO: 177 in US20150315612) |
| AAVhu.54 (See SEQ ID NO: 188 in US20150315612) | AAVhu.56 (See SEQ ID NO: 205 in US20150315612) |
| AAVhu.55 (See SEQ ID NO: 187 in US20150315612) | AAVhu.56 (AAV145.6) (See SEQ ID NO: 192 in US20150315612) |
| AAVhu.56 (AAV145.6) (See SEQ ID NO: 168 in US20150315612) | AAVhu.57 (See SEQ ID NO: 169 in US20150315612) |
| AAVhu.57 (See SEQ ID NO: 206 in US20150315612) | AAVhu.58 (See SEQ ID NO: 207 in US20150315612) |
| AAVhu.57 (See SEQ ID NO: 193 in US20150315612) | AAVhu.6 (AAV3.1) (See SEQ ID NO: 5 in US20150315612) |
| AAVhu.58 (See SEQ ID NO: 194 in US20150315612) | AAVhu.60 ID NO: 184 in (See SEQ US20150315612) |
| AAVhu.6 (AAV3.1) (See SEQ ID NO: 84 in US20150315612) | AAVhu.61 (See SEQ ID NO: 185 in US20150315612) |
| AAVhu.60 (AAV161.10) (See SEQ ID NO: 170 in US20150315612) | AAVhu.63 (See SEQ ID NO: 204 in US20150315612) |
| AAVhu.61 (AAV161.6) (See SEQ ID NO: 174 in US20150315612) | AAVhu.64 (See SEQ ID NO: 212 in US20150315612) |
| AAVhu.63 (See SEQ ID NO: 195 in US20150315612) | AAVhu.66 (See SEQ ID NO: 197 in US20150315612) |
| AAVhu.64 (See SEQ ID NO: 196 in US20150315612) | AAVhu.67 (See SEQ ID NO: 198 in US20150315612) |
| AAVhu.67 (See SEQ ID NO: 215 in US20150315612) | AAVhu.7 (See SEQ ID NO: 150 in US20150315612) |
| AAVhu.7 (See SEQ ID NO: 226 in US20150315612) | AAVhu.71 (See SEQ ID NO: 79 in US20150315612) |
| AAVhu.7 (AAV7.3) (See SEQ ID NO: 55 in US20150315612) | AAVhu.8 (See SEQ ID NO: 12 in US20150315612) |
| AAVhu.8 (See SEQ ID NO: 53 in US20150315612) | AAVhu.9 (AAV3.1) (See SEQ ID NO: 58 in US20150315612) |
| AAVhu.8 (See SEQ ID NO: 151 in US20150315612) | AAV-LK01 (See SEQ ID NO: 2 in US20150376607) |
| AAVhu.9 (AAV3.1) (See SEQ ID NO: 155 in US20150315612) | AAV-LK02 (See SEQ ID NO: 3 in US20150376607) |
| AAV-LK01 (See SEQ ID NO: 29 in US20150376607) | AAV-LK03 (See SEQ ID NO: 4 in US20150376607) |
| AAV-LK02 (See SEQ ID NO: 30 in US20150376607) | AAV-LK04 (See SEQ ID NO: 32 in US20150376607) |
| AAV-LK03 (See SEQ ID NO: 12 in WO2015121501 and SEQ ID NO: 31 in US20150376607) | AAV-LK05 (See SEQ ID NO: 33 in US20150376607) |
| AAV-LK04 (See SEQ ID NO: 5 in US20150376607) | AAV-LK06 (See SEQ ID NO: 34 in US20150376607) |
| AAV-LK05 (See SEQ ID NO: 6 in US20150376607) | AAV-LK07 (See SEQ ID NO: 35 in US20150376607) |
| AAV-LK06 (See SEQ ID NO: 7 in US20150376607) | AAV-LK08 (See SEQ ID NO: 36 in US20150376607) |
| AAV-LK07 (See SEQ ID NO 8 in US20150376607) | AAV-LK09 (See SEQ ID NO: 37 in US20150376607) |
| AAV-LK08 (See SEQ ID NO: 9 in US20150376607) | AAV-LK10 (See SEQ ID NO: 38 in US20150376607) |
| AAV-LK09 (See SEQ ID NO: 10 in US20150376607) | AAV-LK11 (See SEQ ID NO: 39 in US20150376607) |
| AAV-LK10 (See SEQ ID NO: 11 in US20150376607) | AAV-LK12 (See SEQ ID NO: 40 in US20150376607) |
| AAV-LK11 (See SEQ ID NO: 12 in US20150376607) | AAV-LK13 (See SEQ ID NO: 41 in US20150376607) |
| AAV-LK12 (See SEQ ID NO: 13 in US20150376607) | AAV-LK14 (See SEQ ID NO: 42 in US20150376607) |
| AAV-LK13 (See SEQ ID NO: 14 in US20150376607) | AAV-LK15 (See SEQ ID NO: 43 in US20150376607) |
| AAV-LK14 (See SEQ ID NO: 15 in US20150376607) | AAV-LK16 (See SEQ ID NO: 44 in US20150376607) |
| AAV-LK15 (See SEQ ID NO: 16 in US20150376607) | AAV-LK17 (See SEQ ID NO: 45 in US20150376607) |
| AAV-LK16 (See SEQ ID NO: 17 in US20150376607) | AAV-LK18 (See SEQ ID NO: 46 in US20150376607) |
| AAV-LK17 (See SEQ ID NO: 18 in US20150376607) | AAV-LK19 (See SEQ ID NO: 47 in US20150376607) |
| AAV-LK18 (See SEQ ID NO: 19 in US20150376607) | AAV-PAEC (See SEQ ID NO: 48 in US20150376607) |
| AAV-LK19 (See SEQ ID NO: 20 in US20150376607) | AAV-PAEC11 (See SEQ ID NO: 54 in US20150376607) |
| AAV-PAEC (See SEQ ID NO: 1 in US20150376607) | AAV-PAEC 12 (See SEQ ID NO: 51 in US20150376607) |
| AAV-PAEC11 (See SEQ ID NO: 26 in US20150376607) | AAV-PAEC 13 (See SEQ ID NO: 49 in US20150376607) |
| AAV-PAEC 12 (See SEQ\|E) NO: 27 in US20150376607) | AAV-PAEC2 (See SEQ ID NO: 56 in US20150376607) |
| AAV-PAEC 13 (See SEQ ID NO: 28 in US20150376607) | AAV-PAEC4 (See SEQ ID NO: 55 in US20150376607) |
| AAV-PAEC2 (See SEQ ID NO: 21 in US20150376607) | AAV-PAEC6 (See SEQ ID NO: 52 in US20150376607) |
| AAV-PAEC4 (See SEQ ID NO: 22 in US20150376607) | AAV-PAEC7 (See SEQ ID NO: 53 in US20150376607) |
| AAV-PAEC6 (See SEQ ID NO: 23 in US20150376607) | AAV-PAEC8 (See SEQ ID NO: 50 in US20150376607) |
| AAV-PAEC7 (See SEQ ID NO: 24 in US20150376607) | AAVpi.I (See SEQ ID NO: 93 in US20150315612) |
| AAV-PAEC8 (See SEQ ID NO: 25 in US20150376607) | AAVpi.2 (see SEQ ID NO: 30 in US20150315612) |
| AAVpi.I (See SEQ ID NO: 28 in US20150315612) | AAVpi.3 (See SEQ ID NO: 29 in US20150315612) |
| AAVpi.2 (See SEQ ID NO: 95 in US20150315612) | AAVrh.10 (See SEQ ID NO: 9 in US20150159173) |
| AAVpi.3 (See SEQ ID NO: 94 in US20150315612) | AAV44.2 (See SEQ ID NO: 59 in US20030138772) |
| AAVrh.10 (See SEQ ID NO: 25 in US20150159173) | AAV42.1B (See SEQ ID NO: 90 in US20030138772) |
| AAVrh.10 (AAV44.2) (See SEQ ID NO: 81 in US20030138772) | AAVrh.13 (See SEQ ID NO: 10 in US20150159173) |
| AAVrh.I2 (AAV42.1b) (See SEQ ID NO: 30 in US20030138772) | AAVrh.13 (See SEQ ID NO: 228 in US20150315612) |
| AAVrh.13 (See SEQ ID NO: 26 in US20150159173) | AAV42.3A (See SEQ ID NO: 87 in US20030138772) |
| AAVrh.13R (See SEQ ID NO: 26 modified with E538K in US20150159173 | AAV42.5A (See^{.}SEQ ID NO: 89 in US20030138772) |
| AAVrh.I4 (AAV42.3a) (See SEQ ID NO: 32 in US20030138772) | AAV42.5B (See SEQ ID NO: 91 in US20030138772) |
| AAVrh.I7 (AAV42.5a) (See SEQ ID NO: 34 in US20030138772) | AAV42.6B (See SEQ ID NO: 112 in US20030138772) |
| AAVrh.I8 (AAV42.5b) (See SEQ ID NO: 29 in US20030138772) | AAVrh.2 (See SEQ ID NO: 39 in US20150159173) |
| AAVrh.I9 (AAV42.6b) (See SEQ ID NO: 38 in US20030138772) | AAVrh.20 (See SEQ ID NO: 1 in US20150159173) |
| AAVrh.2 (See SEQ ID NO: 231 in US20150315612) | AAVrh.21 (AAV42.10) (See SEQ ID NO: 35 in US20030138772) |
| AAV42.10 (See SEQ ID NO: 106 in US20030138772) | AAVrh.22 (AAV42.11) (See SEQ ID NO: 37 in US20030138772) |
| AAV42.11 (See SEQ ID NO: 108 in US20030138772) | AAVrh.23 (AAV42.12) (See SEQ ID NO: 58 in US20030138772) |
| AAV42.12 SEQ ID NO: 113 in US20030138772) | AAVrh.24 (AAV42.13) (See SEQ ID NO: 31in US20030138772) |
| AAV42.13 (See SEQ ID NO: 86 in US20030138772) | AAVrh.25 (AAV42.15) (See SEQ ID NO: 28 in US20030138772) |
| AAV42.15 (See SEQ ID NO: 84 in US20030138772) | AAVrh.31 (AAV223.1) (See SEQ ID NO: 48 in US20030138772) |
| AAVrh.2R (See SEQ ID NO: 39 modified with V651I in US20150159173) | AAVrh.32 (AAVC1) (See SEQ ID NO: 19 in 446 US20030138772) |
| AAVC1 (See SEQ ID NO: 60 in US20030138772) | AAVrh.51 (AAV2-5) (See SEQ ID NO: 104 in US20150315612) |
| AAVrh.32/33 (See SEQ ID NO: 2 in US20150159173) | AAVrh.52 (AAV3-9) (See SEQ ID NO: 96 in US20150315612) |
| AAVrh.52 (AAV3-9) (See SEQ ID NO: 18 in US20150315612) | AAVrh.53 (AAV3-11) (See SEQ ID NO: 17 in US20150315612) |
| AAVrh.53 (See SEQ ID NO: 97 in US20150315612) | AAVrh.54 (See SEQ ID NO: 40 in US20150315612) |
| AAVrh.53 (AAV3-11) (See SEQ ID NO: 186 in US20150315612) | AAVrh.55 (AAV4-19) (See SEQ ID NO: 117 in US20150315612) |
| AAVrh.54 (See SEQ ID NO: 49 in US20150159173 and SEQ ID NO: 116 in US20150315612) | AAVrh.56 (See SEQ ID NO: 152 in US20150315612) |
| AAVrh.55 (See SEQ ID NO: 37 in US20150315612) | AAVrh.57 (See SEQ ID NO: 105 in US20150315612) |
| AAVrh.56 (See SEQ ID NO: 54 in US20150315612) | AAVrh.58 (See SEQ ID NO: 48 in US20150159173 and SEQ ID NO: 106 in US20150315612) |
| AAVrh.57 (See SEQ ID NO: 26 in US20150315612) | AAVrh.58 (See SEQ ID NO: 232 in US20150315612) |
| AAVrh.58 (See SEQ ID NO: 27 in US20150315612) | AAVrh.59 (See SEQ ID NO: 110 in US20150315612) |
| AAVrh.59 (See SEQ ID NO: 42 in US20150315612) | AAVrh.60 (See SEQ ID NO: 120 in US20150315612) |
| AAVrh.60 (See SEQ ID NO: 31 in US20150315612) | AAVrh.61 (AAV2-3) (See SEQ ID NO: 21 in US20150315612) |
| AAVrh.61 (See SEQ ID NO 107 in US20150315612) | AAVrh.62 (AAV2-15) (See SEQ ID NO: 114 in US20150315612) |
| AAVrh.62 (AAV2-15) (See SEQ ID NO: 33 in US20150315612) | AAVrh.64 (See SEQ ID NO: 43 in US20150159173 and SEQ ID NO: 99 in US20150315612) |
| AAVrh.64 (See SEQ ID NO: 15 in US20150315612) | AAVrh.64 (See SEQ ID NO: 233 in US20150315612) |
| AAVRh.64RI (See SEQ ID NO: 43 modified with R697W in US20150159173 | AAVRh.64R2 (See SEQ ID NO: 43 modified with R697W and V686E in US20150159173 |
| AAVrh.65 (See SEQ ID NO: 35 in US20150315612) | AAVrh.65 (See SEQ ID NO: 112 in US20150315612) |
| AAVrh.67 (See SEQ ID NO: 36 in US20150315612) | AAVrh.67 (See SEQ ID NO: 230 in US20150315612) |
| AAVrh.67 (See SEQ ID NO: 47 in US20150159173 and SEQ ID NO: 47 in US20150315612) | AAVrh.68 (See SEQ ID NO: 100 in US20150315612) |
| AAVrh.68 (See SEQ ID NO: 16 in US20150315612) | AAVrh.69 (See SEQ ID NO: 119 in US20150315612) |
| AAVrh.69 (See SEQ ID NO: 39 in US20150315612) | AAVrh.70 (See SEQ ID NO: 98 in US20150315612) |
| AAVrh.70 (See SEQ ID NO: 20 in US20150315612) | AAVrh.72 (See SEQ ID NO: 9 in US20150315612) |
| AAVrh.71 (See SEQ ID NO: 162 in US20150315612) | AAVrh.74 (See SEQ ID NO: 6 in US20150159173) |
| AAVrh.73 (See SEQ ID NO: 5 in US20150159173) | AAVrh.8 (See SEQ ID NO: 235 in US20150315612) |
| AAVrh.8 (See SEQ ID NO: 41 in US20150159173) | AAVrh.8R A586R mutant (See SEQ ID NO: 10 in WO2015168666) |
| AAVrh.8R (See SEQ ID NO: 9 in US20150159173, WO2015168666) | BAAV (bovine AAV) (See SEQ ID NO: 8 in US9193769) |
| AAVrh.8R R533A mutant(See SEQ ID NO: 11 in WO2015168666) | BAAV (bovine AAV) (See SEQ ID NO: 4 in US9193769) |
| BAAV (bovine AAV) (See SEQ ID NO: 10 in US9193769) | BAAV (bovine AAV) (See SEQ ID NO: 6 in US9193769) |
| BAAV (bovine AAV) (See SEQ ID NO: 2 in US9193769) | BAAV (bovine AAV) (See SEQ ID NO: 5 in US9193769) |
| BAAV (bovine AAV) (See SEQ ID NO: 1 in US9193769) | BAAV (bovine AAV) (See SEQ ID NO: 11 in US9193769) |
| BAAV (bovine AAV) (See SEQ ID NO: 3 in US9193769) | BAAV (bovine AAV) (See SEQ ID NO: 6 in US7427396) |
| BAAV (bovine AAV) (See SEQ ID NO: 5 in US7427396) | BAAV (bovine AAV) (See SEQ ID NO: 9 in US9193769) |
| BAAV (bovine AAV) (See SEQ ID NO: 7 in US9193769) | BNP61 AAV (See SEQ ID NO: 2 in US20150238550) |
| BNP61 AAV (See SEQ ID NO: 1 in US20150238550) | BNP63 AAV (See SEQ ID NO: 4 in US20150238550) |
| BNP62 AAV (See SEQ ID NO: 3 in US20150238550) | caprine AAV (See SEQ ID NO: 4 in US7427396) |
| caprine AAV (See SEQ ID NO: 3 in US7427396) | AAAV (Avian AAV) (See SEQ ID NO: 12 in US9238800) |
| true type AAV (ttAAV) (See SEQ ID NO: 2 in WO2015121501) | AAAV (Avian AAV) (See SEQ ID NO: 6 in US9238800) |
| AAAV (Avian AAV) (See SEQ ID NO: 2 in US9238800) | AAAV (Avian AAV) (See SEQ ID NO: 8 in US9238800) |
| AAAV (Avian AAV) (See SEQ ID NO: 4 in US9238800) | AAAV (Avian AAV) (See SEQ ID NO: 10 in US9238800) |
| AAAV (Avian AAV) (See SEQ ID NO: 14 in US9238800) | AAAV (Avian AAV) (See SEQ ID NO: 5 in US9238800) |
| AAAV (Avian AAV) (See SEQ ID NO: 15 in US9238800) | AAAV (Avian AAV) (See SEQ ID NO: 3 in US9238800) |
| AAAV (Avian AAV) (See SEQ ID NO: 9 in US9238800) | AAAV (Avian AAV) (See SEQ ID NO: 11 in US9238800) |
| AAAV (Avian AAV) (See SEQ ID NO: 7 in US9238800) | AAAV (Avian AAV) (See SEQ ID NO: 1 in US9238800) |
| AAAV (Avian AAV) (See SEQ ID NO: in US9238800) | AAV Shuffle 100-1 (See SEQ ID NO: 11 in US20160017295) |
| AAV Shuffle 100-1 (See SEQ ID NO: 23 in US20160017295) | AAV Shuffle 100-2 (See SEQ ID NO: 29 in US20160017295) |
| AAV Shuffle 100-2 (See SEQ ID NO: 37 in US20160017295) | AAV Shuffle 100-3 (See SEQ ID NO: 12 in US20160017295) |
| AAV Shuffle 100-3 (See SEQ ID NO: 24 in US20160017295) | AAV Shuffle 100-7 (See SEQ ID NO 13 in US20160017295) |
| AAV Shuffle 100-7 (See SEQ ID NO: 25 in US20160017295) | AAV Shuffle 10-2 (See SEQ ID NO: 26 in US20160017295) |
| AAV Shuffle 10-2 (See SEQ ID NO: 34 in US20160017295) | AAV Shuffle 10-6 (See SEQ ID NO: 27 in US20160017295) |
| AAV Shuffle 10-6 (See SEQ ID NO: 35 in US20160017295) | AAV Shuffle 10-8 (See SEQ ID NO: 28 in US20160017295) |
| AAV Shuffle 10-8 (See SEQ ID NO: 36 in US20160017295) | AAV SM 100-10 (See SEQ ID NO: 33 in US20160017295) |
| AAV SM 100-10 (See SEQ ID NO: 41 in US20160017295) | AAV SM 100-3 (See SEQ ID NO: 32 in US20160017295) |
| AAV SM 100-3 (See SEQ IDNO: 40 in US20160017295) | AAV SM 10-1 (See SEQ ID NO: 30 in US20160017295) |
| AAV SM 10-1 (See SEQ ID NO: 38 in US20160017295) | AAV SM 10-2 (See SEQ ID NO: 22 in US20160017295) |
| AAV SM 10-2 (See SEQ ID NO: 10 in US20160017295) | AAV SM 10-8 (See SEQ ID NO: 31 in US20160017295) |
| AAV SM 10-8 (See SEQ ID NO: 39 in US20160017295) | AAV CBr-7.1 (See SEQ ID NO: 54 in WO2016065001) |
| AAV CBr-7.1 (See SEQ ID NO: 4 in WO2016065001) | AAV CBr-7.10 (See SEQ ID NO: 61 in WO2016065001) |
| AAV CBr-7.10 (See SEQ ID NO: 11 in WO2016065001) | AAV CBr-7.2 (See SEQ ID NO: 55 in WO2016065001) |
| AAV CBr-7.2 (See SEQ ID NO: 5 in WO2016065001) | AAV CBr-7.3 (See SEQ ID NO: 56 in WO2016065001) |
| AAV CBr-7.3 (See SEQ ID NO: 6 in WO2016065001) | AAV CBr-7.4 (See SEQ ID NO: 57 in WO2016065001) |
| AAV CBr-7.4 (See SEQ ID NO: 7 WO2016065001) | AAV CHt-6.6 (See SEQ ID NO: 35 in WO2016065001) |
| AAV CBr-7.5 (See SEQ ID NO: 8 in WO2016065001) | AAV CHt-6.7 (See SEQ ID NO: 36 in WO2016065001) |
| AAV CHt-6.6 (See SEQ ID NO: 85 in WO2016065001) | AAV CHt-6.8 (See SEQ ID NO: 37 in WO2016065001) |
| AAV CHt-6.7 (See SEQ ID NO: 86 in WO2016065001) | AAV CHt-PI (See SEQ ID NO: 29 in WO2016065001) |
| AAV CHt-6.8 (See SEQ ID NO: 87 in WO2016065001) | AAV CHt-P2 (See SEQ ID NO: 1 in WO2016065001) |
| AAV CHt-PI (See SEQ ID NO: 79 in WO2016065001) | AAV CHt-P5 (See SEQ ID NO: 2 in WO2016065001) |
| AAV CHt-P2 (See SEQ ID NO: 51 in WO2016065001) | AAV CHt-P6 (See SEQ ID NO: 30 in WO2016065001) |
| AAV CHt-P5 (See SEQ ID NO: 52 in WO2016065001) | AAV CHt-P8 (See SEQ ID NO: 31 in WO2016065001) |
| AAV CHt-P6 (See SEQ ID NO: 80 in WO2016065001) | AAV CHt-P9 (See SEQ ID NO: 3 in WO2016065001) |
| AAV CHt-P8 (See SEQ ID NO: 81 in WO2016065001) | AAV CKd-1 (See SEQ ID NO: 57 in US8734809) |
| AAV CHt-P9 (See SEQ ID NO: 53 in WO2016065001) | AAV CKd-10 (See SEQ ID NO: 58 in US8734809) |
| AAV CKd-1 (See SEQ ID NO: 131 in US8734809) | AAV CKd-2 (See SEQ ID NO: 59 in US8734809) |
| AAV CKd-10 (See SEQ ID NO: 132 in US8734809) | AAV CKd-3 (See SEQ ID NO: 60 in US8734809) |
| AAV CKd-2 (See SEQ ID NO: 133 in US8734809) | AAV CKd-4 (See SEQ ID NO: 61 in US8734809) |
| AAV CKd-3 (See SEQ ID NO: 134 in US8734809) | AAV CKd-6 (See SEQ ID NO: 62 in US8734809) |
| AAV CKd-4 (See SEQ ID NO: 135 in US8734809) | AAV CKd-7 (See SEQ ID NO: 63 in US8734809) |
| AAV CKd-6 (See SEQ ID NO: 136 in US8734809) | AAV CKd-8 (See SEQ ID NO: 64 in US8734809) |
| AAV CKd-7 (See SEQ ID NO: 137 in US8734809) | AAV CKd-B 1 (See SEQ ID NO: 73 in US8734809) |
| AAV CKd-8 (See SEQ ID NO: 138 in US8734809) | AAV CKd-B2 (See SEQ ID NO: 74 in US8734809) |
| AAV CKd-B 1 (See SEQ ID NO: 147 in US8734809) | AAV CKd-B3 (See SEQ ID NO: 75 in US8734809) |
| AAV CKd-B2 (See SEQ ID NO: 148 in US8734809) | AAV CKd-B3 (See SEQ ID NO: 149 in US8734809) |
| AAV CKd-B3 (See SEQ ID NO: in US8734809 | AAV CLv-1 (See SEQ ID NO: 139 in US8734809) |
| AAV CLv-1 (See SEQ ID NO: 65 in US8734809) | AAV Civ 1-10 (See SEQ ID NO: 178 in US8734809) |
| AAV CLvl-1 (See SEQ ID NO: 171 in US8734809) | AAV CLv-12 (See SEQ ID NO: 66 in US8734809) |
| AAV CLvl-2 (See SEQ ID NO: 172 in US8734809) | AAV CLvl-3 (See SEQ ID NO: 173 in US8734809) |
| AAV CLv-12 (See SEQ ID NO: 140 in US8734809) | AAV CLv-13 (See SEQ ID NO: 141 in US8734809) |
| AAV CLv-13 (See SEQ ID NO: 67 in US8734809) | AAV Civ 1-7 (See SEQ ID NO: 175 in US8734809) |
| AAV CLvl-4 (See SEQ ID NO: 174 in US8734809) | AAV Civ 1-9 (See SEQ ID NO: 177 in US8734809) |
| AAV Civ 1-8 (See SEQ ID NO: 176 in US8734809) | AAV CLv-2 (See SEQ ID NO: 142 in US8734809) |
| AAV CLv-2 (See SEQ ID NO: 68 in US8734809) | AAV CLv-3 (See SEQ ID NO: 143 in US8734809) |
| AAV CLv-3 (See SEQ ID NO: 69 in US8734809) | AAV CLv-4 (See SEQ ID NO: 144 in US8734809) |
| AAV CLv-4 (See SEQ ID NO: 70 in US8734809) | AAV CLv-6 (See SEQ ID NO: 145 in US8734809) |
| AAV CLv-6 (See SEQ ID NO: 71 in US8734809) | AAV CLv-8 (See SEQ ID NO: 146 in US8734809) |
| AAV CLv-8 (See SEQ ID NO: 72 in US8734809) | AAV CLv-DI (See SEQ ID NO: 96 in US8734809) |
| AAV CLv-DI (See SEQ ID NO: 22 in US8734809) | AAV CLv-D2 (See SEQ ID NO: 97 in US8734809) |
| AAV CLv-D2 (See SEQ ID NO: 23 in US8734809) | AAV CLv-D3 (See SEQ ID NO: 98 in US8734809) |
| AAV CLv-D3 (See SEQ ID NO: 24 in US8734809) | AAV CLv-D4 (See SEQ ID NO: 99 in US8734809) |
| AAV CLv-D4 (See SEQ ID NO: 25 in US8734809) | AAV CLv-D5 (See SEQ ID NO: 100 in US8734809) |
| AAV CLv-D5 (See SEQ ID NO: 26 in US8734809) | AAV CLv-D6 (See SEQ ID NO: 101 in US8734809) |
| AAV CLv-D6 (See SEQ ID NO: 27 in US8734809) | AAV CLv-D7 (See SEQ ID NO: 102 in US8734809) |
| AAV CLv-D7 (See SEQ ID NO: 28 in US8734809) | AAV CLv-D8 (See SEQ ID NO: 103 in US8734809); AAV CLv-KI 762, see SEQ ID NO: 18 in WO2016065001) |
| AAV CLv-D8 (See SEQ ID NO: 29 in US8734809) | AAV CLv-K3 (See SEQ ID NO: 19 in WO2016065001) |
| AAV CLv-KI (See SEQ ID NO: 68 in WO2016065001) | AAV CLv-K6 (See SEQ ID NO: 20 in WO2016065001) |
| AAV CLv-K3 (See SEQ ID NO: 69 in WO2016065001) | AAV CLv-L4 (See SEQ ID NO: 15 in WO2016065001) |
| AAV CLv-K6 (See SEQ ID NO: 70 in WO2016065001) | AAV CLv-L5 (See SEQ ID NO: 16 in WO2016065001) |
| AAV CLv-L4 (See SEQ ID NO: 65 in WO2016065001) | AAV CLv-L6 (See SEQ ID NO: 17 in WO2016065001) |
| AAV CLv-L5 (See SEQ ID NO: 66 in WO2016065001) | AAV CLv-MI (See SEQ ID NO: 21 in WO2016065001) |
| AAV CLv-L6 (See SEQ ID NO: 67 in WO2016065001) | AAV CLv-MII (See SEQ ID NO: 22 in WO2016065001) |
| AAV CLv-MI (See SEQ ID NO: 71 in WO2016065001) | AAV CLv-M2 (See SEQ ID NO: 23 in WO2016065001) |
| AAV CLv-MI 1 (See SEQ ID NO: 72 in WO2016065001) | AAV CLv-M5 (See SEQ ID NO: 24 in WO2016065001) |
| AAV CLv-M2 (See SEQ ID NO: 73 in WO2016065001) | AAV CLv-M6 (See SEQ ID NO: 25 in WO2016065001) |
| AAV CLv-M5 (See SEQ ID NO: 74 in WO2016065001) | AAV CLv-M7 (See SEQ ID NO: 26 in WO2016065001) |
| AAV CLv-M6 (See SEQ ID NO: 75 in WO2016065001) | AAV CLv-M8 (See SEQ ID NO: 27 in WO2016065001) |
| AAV CLv-M7 (See SEQ ID NO: 76 in WO2016065001) | AAV CLv-M9 (See SEQ ID NO: 28 in WO2016065001) |
| AAV CLv-M8 (See SEQ ID NO: 77 in WO2016065001) | AAV CLv-RI (See SEQ ID NO: 30 in US8734809) |
| AAV CLv-M9 (See SEQ ID NO: 78 in WO2016065001) | AAV CLv-R2 (See SEQ ID NO: 31 in US8734809) |
| AAV CLv-RI (See SEQ ID NO: 104 in US8734809) | AAV CLv-R3 (See SEQ ID NO: 32 in US8734809) |
| AAV CLv-R2 (See SEQ ID NO: 105 in US8734809) | AAV CLv-R4 (See SEQ ID NO: 33 in US8734809) |
| AAV CLv-R3 (See SEQ ID NO: 106 in US8734809) | AAV CLv-R5 (See SEQ ID NO: 34 in US8734809) |
| AAV CLv-R4 (See SEQ ID NO: 107 in US8734809) | AAV CLv-R6 (See SEQ ID NO: 35 in US8734809) |
| AAV CLv-R5 (See SEQ ID NO: 108 in US8734809) | AAV CLv-R7 (See SEQ ID NO: 110 in US8734809) |
| AAV CLv-R6 (See SEQ ID NO: 109 in US8734809); AAV CLv-R7 802 (see SEQ ID NO: 36 in US8734809) | AAV CLv-R8 (See SEQ ID NO: 111 in US8734809) |
| AAV CLv-R8 (See SEQ ID NO: 37 in US8734809) | AAV CLv-R9 (See SEQ ID NO: 112 in US8734809) |
| AAV CLv-R9 (See SEQ ID NO: 38 in US8734809) | AAV CSp-1 (See SEQ ID NO: 119 in US8734809) |
| AAV CSp-1 (See SEQ ID NO: 45 in US8734809) | AAV CSp-10 (See SEQ ID NO: 120 in US8734809) |
| AAV CSp-10 (See SEQ ID NO: 46 in US8734809) | AAV CSp-11 (See SEQ ID NO: 121 in US8734809) |
| AAV CSp-11 (See SEQ ID NO: 47 in US8734809) | AAV CSp-2 (See SEQ ID NO: 122 in US8734809) |
| AAV CSp-2 (See SEQ ID NO: 48 in US8734809) | AAV CSp-3 (See SEQ ID NO: 123 in US8734809) |
| AAV CSp-3 (See SEQ ID NO: 49 in US8734809) | AAV CSp-4 (See SEQ ID NO: 124 in US8734809) |
| AAV CSp-4 (See SEQ ID NO: 50 in US8734809) | AAV CSp-6 (See SEQ ID NO: 125 in US8734809) |
| AAV CSp-6 (See SEQ ID NO: 51 in US8734809) | AAV CSp-7 (See SEQ ID NO: 126 in US8734809) |
| AAV CSp-7 (See SEQ IDNO: 52 in US8734809) | AAV CSp-8 (See SEQ ID NO 127 in US8734809) |
| AAV CSp-8 (See SEQ ID NO: 53 in US8734809) | AAV CSp-8.10 (See SEQ ID NO: 88 in WO2016065001) |
| AAV CSp-8.10 (See SEQ ID NO: 38 in WO2016065001) | AAV CSp-8.2 (See SEQ ID NO: 89 in WO2016065001) |
| AAV CSp-8.2 (See SEQ ID NO: 39 in WO2016065001) | AAV CSp-8.4 (See SEQ ID NO: 90 in WO2016065001) |
| AAV CSp-8.4 (See SEQ ID NO: 40 in WO2016065001) | AAV CSp-8.5 (See SEQ ID NO: 91 in WO2016065001) |
| AAV CSp-8.5 (See SEQ ID NO: 41 in WO2016065001) | AAV CSp-8.6 (See SEQ ID NO: 92 in WO2016065001) |
| AAV CSp-8.6 (See SEQ ID NO: 42 in WO2016065001) | AAV CSp-8.7 (See SEQ ID NO: 93 in WO2016065001) |
| AAV CSp-8.7 (See SEQ ID NO: 43 in WO2016065001) | AAV CSp-8.8 (See SEQ ID NO: 94 in WO2016065001) |
| AAV CSp-8.8 (See SEQ ID NO: 44 in WO2016065001) | AAV CSp-8.9 (See SEQ ID NO: 95 in WO2016065001) |
| AAV CSp-8.9 (See SEQ ID NO: 45 in WO2016065001) | AAV CSp-9 (See SEQ ID NO: 128 in US8734809) |
| AAV CSp-9 842 (See SEQ ID NO: 54 in US8734809) | AAV.VR-355 (See SEQ ID NO: 181 in US8734809) |
| AAV.hu.48R3 (See SEQ ID NO: 183 in US8734809) | AAV3B (See SEQ ID NO: 98 in WO2016065001) |
| AAV3B (See SEQ ID NO: 48 in WO2016065001) | AAV4 (See SEQ ID NO: 99 in WO2016065001) |
| AAV4 (See SEQ ID NO: 49 in WO2016065001) | AAV5 (See SEQ ID NO: 100 in WO2016065001) |
| AAV5 (See SEQ ID NO: 50 in WO2016065001) | AAVF1/HSC1 (See SEQ ID NO: 2 in WO2016049230) |
| AAVF1/HSC1 (See SEQ ID NO: 20 in WO2016049230) | AAVF11/HSC11 (See SEQ ID NO: 4 in WO2016049230) |
| AAVF11/HSC11 (See SEQ ID NO: 26 in WO2016049230) | AAVF12/HSC12 (See SEQ ID NO: 12 in WO2016049230) |
| AAVF12/HSC12 (See SEQ ID NO: 30 in WO2016049230) | AAVF13/HSC13 (See SEQ ID NO: 14 in WO2016049230) |
| AAVF13/HSC13 (See SEQ ID NO: 31 in WO2016049230) | AAVF14/HSC14 (See SEQ ID NO: 15 in WO2016049230) |
| AAVF14/HSC14 (See SEQ ID NO: 32 in WO2016049230) | AAVF15/HSC15 (See SEQ ID NO: 16 in WO2016049230) |
| AAVF15/HSC15 (See SEQ ID NO: 33 in WO2016049230) | AAVF16/HSC16 (See SEQ ID NO: 17 in WO2016049230) |
| AAVF16/HSC16 (See SEQ ID NO: 34 in WO2016049230) | AAVF17/HSC17 (See SEQ ID NO: 13 in WO2016049230) |
| AAVF17/HSC17 (See SEQ ID NO: 35 in WO2016049230) | AAVF2/HSC2 (See SEQ ID NO: 3 in WO2016049230) |
| AAVF2/HSC2 (See SEQ ID NO: 21 in WO2016049230) | AAVF3/HSC3 (See SEQ ID NO: 5 in WO2016049230) |
| AAVF3/HSC3 (SeeSEQ ID NO: 22 in WO2016049230) | AAVF4/HSC4 (See SEQ ID NO: 6 in WO2016049230) |
| AAVF4/HSC4 (See SEQ ID NO: 23 in WO2016049230) | AAVF5/HSC5 (See SEQ IDNO: 11 in WO2016049230) |
| AAVF5/HSC5 (See SEQ ID NO: 25 in WO2016049230) | AAVF6/HSC6 (See SEQ ID NO: 7 in WO2016049230) |
| AAVF6/HSC6 (See SEQ ID NO: 24 in WO2016049230) | AAVF7/HSC7 (See SEQ ID NO: 8 in WO2016049230) |
| AAVF7/HSC7 (See SEQ ID NO: 27 in WO2016049230) | AAVF8/HSC8 (See SEQ ID NO:9 in WQ2016049230) |
| AAVF8/HSC8 (See SEQ ID NO: 28 in WO2016049230) | AAVF9/HSC9 882 (see SEQ ID NO: 29 in WO2016049230) |
| AAVF9/HSC9 (See SEQ ID NO: 10 in WO2016049230) | AAVAnc80L65 (see SEQ ID NO:23 in WO/2017/019994) |
| AAVpo1 (see SEQ ID NO: 24 in US8420372) | AAV1P5ii (see Mol. Ther. 2020 April 8; 28(4):1016-1032 and SEQ ID NOS:5 and 11 in US20200370039 with corresponding description) |
| AAV1A6ii (see Mol. Ther. 2020 April 8; 28(4):1016-1032 and SEQ ID NOS:8 and 14 in US20200370039 with corresponding description) | AAV7P4i (see Mol. Ther. 2020 April 8; 28(4):1016-1032 and SEQ ID NOS:4 and 10 in US20200370039 with corresponding description) |
| AAV4A1ii (see Mol. Ther. 2020 April 8; 28(4):1016-1032 and SEQ ID NOS:6 and 12 in US20200370039 with corresponding description) | AAV9A2i (see Mol. Ther. 2020 April 8; 28(4):1016-1032 and SEQ ID NOS:7 and 13 in US20200370039 with corresponding description) |
| AAV9A1i (see Mol. Ther. 2020 April 8; 28(4):1016-1032 and SEQ ID NOS:6 and 12 in US20200370039 with corresponding description) | AAV9P1i (see Mol. Ther. 2020 April 8; 28(4):1016-1032) |
| AAV9A6i (see Mol. Ther. 2020 April 8; 28(4):1016-1032 and SEQ ID NOS:8 and 14 in US20200370039 with corresponding description) | AAV9P5i (see Mol. Ther. 2020 April 8; 28(4):1016-1032 and SEQ ID NOS:5 and 11 in US20200370039 with corresponding description) |
| AAV9P2i (see Mol. Ther. 2020 April 8; 28(4):1016-1032 and SEQ ID NOS:3 and 9 in US20200370039 with corresponding description) | AAVrh10A2i (see Mol. Ther. 2020 April 8; 28(4):1016-1032 and SEQ ID NOS:7 and 13 in US20200370039 with corresponding description) |
| AAVrh10A1i (see Mol. Ther. 2020 April 8; 28(4):1016-1032 and SEQ ID NOS:6 and 12 in US20200370039 with corresponding description) | AAVS10P1i (see Mol. Ther. 2020 April 8; 28(4):1016-1032) |
| AAVrh10P1i (see Mol. Ther. 2020 April 8; 28(4):1016-1032) | AAV2 3xA P2i (see Mol. Ther. 2020 April 8; 28(4):1016-1032 and SEQ ID NOS:3 and 9 in US20200370039 with corresponding description) |
| AAV12P2ii (see Mol. Ther. 2020 April 8; 28(4):1016-1032 and SEQ ID NOS:3 and 9 in US20200370039 with corresponding description) | AAVDJ P2i (see Mol. Ther. 2020 April 8; 28(4):1016-1032 and SEQ ID NOS:3 and 9 in US20200370039 with corresponding description) |
| AAV2i8g9 (See J Biol Chem. 2013 Oct 4; 288(40): 28814-28823) | AAV 2i8 (see SEQ ID NO: 29 in US10548947) |
| AAV2g9 (See descriptions in WO2014144229 and J Biol Chem. 2013 Oct 4; 288(40): 28814-28823) | AAV2.5 (See SEQ ID NO: 2 in WO/2022/159871, SEQ ID NO: 16 in US9012224, and SEQ ID NO: 1 in US11639509) |
| AAV2.5g9 (see SEQ ID NO: 2 in US11639509 and SEQ ID NO: 2 in WO/2020/219656) | AAV 2i10 (see SEQ ID NO: 11 and its corresponding description in US9475845) |
| AAV2i9 (see SEQ ID NO: 10 and its corresponding description in US9475845) | MyoAAV3A (see SEQ ID NO:22 and corresponding description in WO/2022/020616) |
| MyoAAV1A (see SEQ ID NO:12 and corresponding description in WO/2022/020616) | MyoAAV3C (see SEQ ID NO:24 and corresponding description in WO/2022/020616) |
| MyoAAV3B (see SEQ ID NO:23 and corresponding description in WO/2022/020616) | MyoAAV3E (see SEQ ID NO:26 and corresponding description in WO/2022/020616) |
| MyoAAV3D (see SEQ ID NO:25 and corresponding description in WO/2022/020616) | MyoAAV4A (see SEQ ID NO:28 and corresponding description in WO/2022/020616) |
| MyoAAV3F (see SEQ ID NO:27 and corresponding description in WO/2022/020616) | MyoAAV4C (see SEQ ID NO:30 and corresponding description in WO/2022/020616) |
| MyoAAV4B (see SEQ ID NO:29 and corresponding description in WO/2022/020616) | MyoAAV4E (see SEQ ID NO:32 and corresponding description in WO/2022/020616) |
| MyoAAV4D (see SEQ ID NO:31 and corresponding description in WO/2022/020616) | AAV9-P1 (589) (AAVMYO) (see SEQ ID NO:16 in US20210363193) |
| AAV9-P1(588) (AAVMYO) (see SEQ ID NO:12 in US20210363193) | AAV9-P3(589) (see SEQ ID NO:20 in US20210363193) |
| AAV9-P3(588) (see SEQ ID NO:18 in US20210363193) | AAVS1-P1 (see SEQ ID NO:30 in 20210363193) |
| AAVS1 (see SEQ ID NO:30 without P1 peptide in 20210363193) | AAVS10-P1 (see SEQ ID NO:28 in 20210363193) |
| AAVS10 (see SEQ ID NO:28 without P1 peptide in 20210363193) | AAV.cc44 (see SEQ ID NO:5 in WO2021226267) |
| AAV.cc47 (see SEQ ID NO:8 in WO2021226267) | AAV.cc84 (see SEQ ID NO:14 in WO2021226267) |
| AAV.cc81 (see SEQ ID NO:11 in WO2021226267) | AAVRec3 (see SEQ ID NO:3 in US20210371880) |
| AAVRec2 (see SEQ ID NO:2 in US20210371880) | AAV-Qlig001 (see SEQ ID NO:2 in US9636370) |
| AAVLC.V1 (see SEQ ID NO:6 in WO2022051633) | AAV-Olig003 (see SEQ ID NO:4 in US9636370) |
| AAV-Olig002 (see SEQ ID NO:3 in US9636370) | AAV.PHP.B (see SEQ ID NO:3 in US11149256 and SEQ ID NO: 7 in WO2019200016) |
| AAV.PHP.S (see SEQ IDNO:4 in US11149256) | AAV9.24 (AAV9 with W503R; see Table 6 in US9409953) |
| AAV9-PHP.eB (See SEQ ID NOS:4 and 46 in US20170166926 and SEQ ID NO:5 in US11149256) | AAV9.47 (AAV9 with S414N, G453D, K557E, and T582I; see Table 6 in US9409953) |
| AAV9.45 (AAV9 with N498Y and L602F; see Table 6 in US9409953) | AAV9.68 (AAV9 with P504T; see Table 6 in US9409953) |
| AAV9.61 (AAV9 with N498Y; see Table 6 in US9409953) | AAVXL12 (see SEQ ID NOS:1 and 4 in US20210246467) |
| AAV9.84 (AAV9 with P468T and E500D; see Table 6 in US9409953) | AAVXL32.1 (see SEQ ID NOS:3 and 6 in US20210246467) |
| AAVXL32 (see SEQ ID NOS:2 and 5 in US20210246467) | |
| | |
| AAVLK03 (see SEQ ID NO: 20 in US20220354969) | AAVrh20 (see SEQ ID NO: 17 in US20220354969) |
| AAVhu37 (see SEQ ID NO: 16 in US20220354969) | AAVKP3 (see SEQ ID NO: 41 in US20220354969) |
| AAVKP1 (see SEQ ID NO: 39 in US20220354969) | AAVrh10 (see SEQ ID NO: 13 in US20220354969) |
| AAV2.7m8 (see SEQ ID NO: 34 in US20220354969) | AAVNP40 (see SEQ ID NO: 25 in US20220354969) |
| AAVsh10 (see SEQ ID NO: 35 US20220354969) | AAVrh8 (see SEQ ID NO: 12 in US20220354969) |
| AAV2i8 (see SEQ ID NO: 22 US20220354969) | AAVhu13 (see SEQ ID NO: 15 in US20220354969) |
| AAV2G9 (see SEQ ID NO: 24 US20220354969) | AAVAnc80 (see SEQ ID NO: 33 in US20220354969) |
| AAVPHPB (see SEQ ID NO: 27 in US20220354969) | AAVPHPeB (see SEQ ID NO: 28 in US20220354969) |
| AAVbovine (see SEQ ID NO. 12 in US20220184227 | AAV2R585E (see SEQ ID NO: in US11866462) |

In some embodiments of any of the aspects, the rAAV particle produced using a polynucleotide described herein comprises a capsid protein, and a rAAV genome in the capsid protein. A rAAV capsid of the rAAV particle can be any of those listed in **Table 2** herein, or as disclosed in Table 1 of PCT applications WO2020/102645, and WO2020/102667, contents of each of which is incorporated herein by reference in their entireties. In some embodiments of any of the aspects, the rAAV particle comprises AAV capsid proteins that can be polyploid (also referred to as haploid, or rational haploid, or rational polyploid), e.g., they can comprise VP1, VP2, and VP3 capsid proteins from more than one AAV serotypes in a single AAV virion as described in PCT application Nos PCT/US2018/022725, PCT/US2018/044632, and US Patent No. 10,550,405, contents of all of which are incorporated here by reference in their entireties. In some embodiments of any of the aspects, the rAAV particle comprising AAV capsid comprises at least one of VP1, VP2, and VP3 capsid selected from any of the AAV capsids listed in Table 2.

In some embodiments of any of the aspects, rAAV comprises a capsid protein from any of those disclosed in WO2019/241324, content of which is incorporated herein in its entirety by reference. In some embodiments of any of the aspects, the rAAV vector comprises a liver specific capsid, e.g., a liver specific capsid selected from XL32 and XL32.1, as disclosed in WO2019/241324, content of which is incorporated herein in its entirety by reference. In some embodiments of any of the aspects, the rAAV vector is a AAVXL32 or AAVXL32.1 as disclosed in WO2019/241324, content of which is incorporated herein in its entirety by reference.

Exemplary chimeric or variant capsid proteins that can be used as the AAV capsid in the rAAV vector described herein can be selected from Table 2 from WO 2021/102107, content of which is incorporated herein by reference herein in its entirety or can be used with any combination with wild type capsid proteins and/or other chimeric or variant capsid proteins now known or later identified and each is incorporated herein. In some embodiments of any of the aspects, the rAAV vector is a chimeric vector, e.g., as disclosed in US Patent No. 9,012,224 and US Patent No. 7,892,809, contents of each of which are incorporated herein in their entireties by reference.

In some embodiments of any of the aspects, the rAAV vector is a haploid rAAV vector, as disclosed in US patent publication US2018/0371496 and PCT application no. PCT/US18/22725, or polyploid rAAV vector, e.g., as disclosed in PCT application no. PCT/US2018/044632 and in US application no. 16/151,110, contents of each of which are incorporated herein in their entireties by reference. In some embodiments of any of the aspects, the rAAV vector is a rAAV3 vector, as disclosed in US patent no. 9,012,224 and PCT publication WO 2017/106236, contents of all of which are incorporated herein in their entireties by reference.

In some embodiments of any of the aspects, the rAAV is a AAVXL32 or AAVXL32.1 AAV vector as disclosed in WO2019/241324, content of which is incorporated herein in its entirety by reference. In some embodiments of any of the aspects, the rAAV vector comprises a capsid disclosed in WO2019241324A1, or International Patent application PCT/US2019/036676, contents of each of which are incorporated herein in their entireties by reference. In some embodiments of any of the aspects, the AAV vector is a AAV8 vector or a rational haploid comprising an AAV8 capsid protein. In some embodiments of any of the aspects, the recombinant AAV vector is a chimeric AAV vector, haploid AAV vector, a hybrid AAV vector or polyploid AAV vector. In some embodiments of any of the aspects, the recombinant AAV vector is a rational haploid vector, a mosaic AAV vector, a chemically modified AAV vector, or a AAV vector from any AAV serotypes, for example, from any AAV serotype disclosed in Table 1 of PCT applications WO2020/102645, and WO2020/102667, contents of each of which are incorporated by reference herein in their entirety.

In some embodiments of any of the aspects, an rAAV vector is an AAV3b capsid. AAV3b capsids encompassed for use are described in US Patent No. 9,012,224 and 7,892,809, US patent publication 2017/106236, and PCT application PCT/US19/61653, and PCT publication WO2020/102645 and WO2020/102667, contents of each of which are incorporated herein by reference in their entireties.

In some embodiments of any of the aspects, the AAV3b capsid comprises SEQ ID NO: 44 as disclosed in PCT publication no. WO 2020/102645 and WO2021102107, contents of each of which are incorporated by reference herein it their entireties. In some embodiments of any of the aspects, the AAV capsid can be a modified AAV capsid that is derived in whole or in part from the AAV capsid set forth in SEQ ID NO: 44 as disclosed in PCT publication no. WO 2020/102645 and WO2021102107, contents of all of which are incorporated herein by reference in their entireties. In some embodiments of any of the aspects, the amino acids from an AAV3b capsid as set forth in SEQ ID NO: 44 as disclosed in PCT publication no. WO 2020/102645 and WO2021102107 can be, or are substituted with amino acids from another capsid of a different AAV serotype, wherein the substituted and/or inserted amino acids can be from any AAV serotype, and can include either naturally occurring or partially or completely synthetic amino acids.

In some embodiments of any of the aspects, an AAV capsid can be an AAV3b265D capsid. In this particular embodiment, an AAV3b265D capsid comprises a modification in the amino acid sequence of the two-fold axis loop of an AAV3b capsid via replacement of amino acid G265 of the AAV3b capsid with D265. In some embodiments of any of the aspects, an AAV3b265D capsid comprises SEQ ID NO: 46 as set forth in PCT publication no. WO 2020/102645 and WO2021102107, each of which is incorporated by reference herein in its entirety. However, the modified virus capsids are not limited to AAV capsids set forth in SEQ ID NO: 46 disclosed in PCT publications WO 2020/102645 and WO2021102107. In some embodiments of any of the aspects, the amino acids from AAV3b265D as set forth in SEQ ID NO. 46 disclosed in PCT publications WO 2020/102645 and WO2021102107 can be, or are substituted with amino acids from a capsid from an AAV of a different serotype, wherein the substituted and/or inserted amino acids can be from any AAV serotype, and can include either naturally occurring or partially or completely synthetic amino acids.

In some embodiments of any of the aspects, an rAAV vector is an AAV3b265D549A capsid. In this particular embodiment, an AAV3b265D549A capsid comprises a modification in the amino acid sequence of the two-fold axis loop of an AAV3b capsid via replacement of amino acid G265 of the AAV3b capsid with D265 and replacement of amino acid T549 of the AAV3b capsid with A549. In some embodiments of any of the aspects, an AAV3b265D549A capsid comprises SEQ ID NO: 50 as disclosed in PCT publication WO 2020/102645 and WO2021102107. However, the modified virus capsids are not limited toAAV capsids set forth in SEQ ID NO: 50 disclosed in PCT publications WO 2020/102645 and WO2021102107. In some embodiments of any of the aspects, the amino acids from AAV3b265D549A as set forth in SEQ ID NO: 50 disclosed in PCT publications WO 2020/102645 and WO2021102107 can be, or are substituted with amino acids from a capsid from an AAV of a different serotype, wherein the substituted and/or inserted amino acids can be from any AAV serotype, and can include either naturally occurring or partially or completely synthetic amino acids. In some embodiments of any of the aspects, the amino acids from AAV3bSASTG (i.e., a AAV3b capsid comprising Q263A/T265 mutations) can be, or are substituted with amino acids from a capsid from an AAV of a different serotype, wherein the substituted and/or inserted amino acids can be from any AAV serotype, and can include either naturally occurring or partially or completely synthetic amino acids.

In another embodiment, an rAAV vector is an AAV3b549A capsid. In this particular embodiment, an AAV3b549A capsid comprises a modification in the amino acid sequence of the two-fold axis loop of an AAV3b capsid via replacement of amino acid T549 of the AAV3b capsid with A549. In some embodiments, an AAV3b549A capsid comprises SEQ ID NO: 52 as disclosed in PCT publications WO 2020/102645 and WO2021102107. However, the modified virus capsids are not limited to AAV capsids set forth in SEQ ID NO: 52 disclosed in PCT publications WO 2020/102645 and WO2021102107. In some embodiments of any of the aspects, the amino acids from AAV3b549A as set forth in SEQ ID NO: 52 disclosed in PCT publications WO 2020/102645 and WO2021102107 can be, or are substituted with amino acids from a capsid from an AAV of a different serotype, wherein the substituted and/or inserted amino acids can be from any AAV serotype, and can include either naturally occurring or partially or completely synthetic amino acids.

In another embodiment, an rAAV vector is an AAV3bQ263Y capsid. In this particular embodiment, an AAV3bQ263Y capsid comprises a modification in the amino acid sequence of the two-fold axis loop of an AAV3b capsid via replacement of amino acid Q263 of the AAV3b capsid with Y263. In some embodiments of any of the aspects, an AAV3b549A capsid comprises SEQ ID NO: 54 as disclosed in PCT publications WO 2020/102645 and WO2021102107. However, the modified virus capsids are not limited to AAV capsids set forth in SEQ ID NO: 54 disclosed in PCT publications WO 2020/102645 and WO2021102107. In some embodiments of any of the aspects, the amino acids from AAV3bQ263Y as set forth in SEQ ID NO: 54 disclosed in PCT publications WO 2020/102645 and WO2021102107 can be, or are substituted with amino acids from a capsid from an AAV of a different serotype, wherein the substituted and/or inserted amino acids can be from any AAV serotype, and can include either naturally occurring or partially or completely synthetic amino acids.

In another embodiment, an rAAV vector is AAV3bSASTG serotype or comprises a AAV3bSASTG capsid. In this particular embodiment, an AAV3bSASTG capsid comprises a modification in the amino acid sequence to comprise a SASTG mutation, in particular, the AAV3b capsid was modified to resemble AAV2 Q263A/T265 subvariant by introducing these modifications at similar positions in the AAV3b capsid (as disclosed in Messina EL, et al., "Adeno-associated viral vectors based on serotype 3b use components of the fibroblast growth factor receptor signaling complex for efficient transduction." Hum. Gene Ther. 2012 Oct: 23(10):1031-4, Piacentino III, Valentino, et al. "X-linked inhibitor of apoptosis protein-mediated attenuation of apoptosis, using a novel cardiac-enhanced adeno-associated viral vector." Human gene therapy 23.6 (2012): 635-646, contents of all of which are incorporated herein in their entireties by reference). Accordingly, in some embodiments of any of the aspects, an rAAV vector is AAV3bSASTG serotype or comprises a AAV3bSASTG capsid comprising a AAV3b Q263A/T265 capsid. In some embodiments of any of the aspects, the amino acids from AAV3bSASTG can be, or are substituted with amino acids from a capsid from an AAV of a different serotype, wherein the substituted and/or inserted amino acids can be from any AAV serotype, and can include either naturally occurring or partially or completely synthetic amino acids.

Various aspects of the disclosure can be embodied in any of the embodiments described herein, including, but not limited to, the following numbered embodiments:
Embodiment 1: A polynucleotide comprising a nucleotide sequence encoding:
   an adenoviral E2a region;
   an adenoviral E4 region; and
   an adenoviral virus associated (VA) RNA region, and
      wherein the polynucleotide does not comprise a nucleotide sequence encoding
   at least 1 (e.g., 2, 3, 4, or all 5) of:
   a full length adenoviral L4-100K protein or a portion thereof;
   a full length adenoviral L5 fiber protein or a portion thereof;
   a full length adenoviral pVIII protein or a portion thereof;
   a full length adenoviral L1-52K/55K protein or a portion thereof; and
   a full length adenoviral precursor terminal protein (pTP) or a portion thereof; and
      wherein the polynucleotide comprises at least one (e.g., 1, 2, 3, 4, 5, 6 or all 7) of:
   a deletion of G₃ and G₄ in a nucleotide sequence encoding an adenoviral L-22K protein (e.g., **SEQ ID NO: 103)** and/or a L-33K protein (e.g., **SEQ ID NO: 104);**
   a deletion of G₃ and G₄ in a nucleotide sequence encoding an adenoviral L4-100K protein (e.g., **SEQ ID NO: 105);**
   a deletion of Gain a nucleotide sequence encoding adenoviral pVIII protein (e.g., **SEQ ID NO: 106);**
   a AC → TT mutation at positions 149-150 of a nucleotide sequence encoding adenoviral pVIII protein (e.g., **SEQ ID NO: 106);**
   a deletion of G₃ in a nucleotide sequence encoding adenoviral fiber protein (e.g., **SEQ ID NO: 107);**
   a C → T mutation at position 503 of a nucleotide encoding adenoviral L5 fiber protein (e.g., **SEQ ID NO: 103);** and
   a TA → CG mutation at positions 1692-1693 of a nucleotide encoding adenoviral L5 fiber protein (e.g., **SEQ ID NO: 107).**
Embodiment 2: The polynucleotide of Embodiment 1, wherein the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 5.**
Embodiment 3: The polynucleotide of Embodiment 1, wherein the polynucleotide comprises nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 6.**
Embodiment 4: The polynucleotide of Embodiment 1, wherein the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 4.**
Embodiment 5: The polynucleotide of Embodiment 1, wherein the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 3.**
Embodiment 6: The polynucleotide of Embodiment 1, wherein the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 2.**
Embodiment 7: The polynucleotide of Embodiment 1, wherein the polynucleotide comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 1.**
Embodiment 8: The polynucleotide of any one of Embodiments 1-7, wherein the polynucleotide does not comprise nucleotides 26197-26198 relative to Genbank Accession Number AC_000008.
Embodiment 9: The polynucleotide of any one of Embodiments 1-8, wherein the polynucleotide does not comprise nucleotides 24063-24064 relative to Genbank Accession Number AC_000008.
Embodiment 10: The polynucleotide of any one of Embodiments 1-9, wherein the polynucleotide does not comprise nucleotide 27176 relative to Genbank Accession Number AC_000008.
Embodiment 11: The polynucleotide of any one of Embodiments 1-10, wherein the polynucleotide comprises AC → TT mutation at positions 27322-27324 relative to Genbank Accession Number AC_000008.
Embodiment 12: The polynucleotide of any one of Embodiments 1-11, wherein the polynucleotide does not comprise nucleotide 31024 relative to Genbank Accession Number AC_000008.
Embodiment 13: The polynucleotide of any one of Embodiments 1-12, wherein the polynucleotide comprises C → T mutation at positions 31544 relative to Genbank Accession Number AC_000008.
Embodiment 14: The polynucleotide of any one of Embodiments 1-13, wherein the polynucleotide comprises TA → CG mutation at positions 32733-32734 relative to Genbank Accession Number AC_000008.
Embodiment 15: The polynucleotide of any one of Embodiments 1-14, wherein the polynucleotide does not comprise a nucleotide sequence encoding a protein comprising, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95.**
Embodiment 16: The polynucleotide of Embodiment 1, wherein the polynucleotide does not comprise a nucleotide sequence encoding a full length adenoviral L4-22K protein or a portion thereof.
Embodiment 17: The polynucleotide of Embodiment 16, wherein the polynucleotide comprises a nucleotide sequence encoding a less than full length fragment of adenoviral L4-22K protein, i.e., polynucleotide comprises a nucleotide sequence encoding a truncated adenoviral L4-22K protein.
Embodiment 18: The polynucleotide of Embodiment 16 or 17, wherein the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the protein does not comprise, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95.**
Embodiment 19: The polynucleotide of Embodiment 16 or 17, wherein the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a full length adenoviral L4-22K protein having the amino acid sequence **SEQ ID NO: 7.**
Embodiment 20: The polynucleotide of Embodiment 1, wherein the polynucleotide does not comprise a nucleotide sequence encoding: a full length adenoviral L4-33K protein or a portion thereof.
Embodiment 21: The polynucleotide of Embodiment 20, wherein the polynucleotide comprises a nucleotide sequence encoding a less than full length fragment of adenoviral L4-33K protein, i.e., polynucleotide comprises a nucleotide sequence encoding a truncated adenoviral L4-33K protein.
Embodiment 22: The polynucleotide of 20 or 21, wherein the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the protein does not comprise at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95**
Embodiment 23: The polynucleotide of 20 or 21, wherein the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the polynucleotide does not comprise a nucleotide sequence encoding a full-length adenoviral L4-33K protein having the amino acid sequence **SEQ ID NO: 8.**
Embodiment 24: The polynucleotide of Embodiment 1, wherein the polynucleotide does not comprise a nucleotide sequence encoding the L4-100K protein or a portion thereof, and wherein the L4-100K protein has the amino acid sequence **SEQ ID NO: 9.**
Embodiment 25: The polynucleotide of Embodiment 1, wherein the polynucleotide does not comprise a nucleotide sequence encoding the L5 fiber protein or a portion thereof, and wherein the L5 fiber protein has the amino acid sequence **SEQ ID NO: 10.**
Embodiment 26: The polynucleotide of Embodiment 1, wherein the polynucleotide does not comprise a nucleotide sequence encoding the pVIII protein or a portion thereof, and wherein the pVIII protein has the amino acid sequence **SEQ ID NO: 11.**
Embodiment 27: The polynucleotide of Embodiment 1, wherein the polynucleotide does not comprise a nucleotide sequence encoding the L1-52K/55K protein or a portion thereof, and wherein the L1-52K/55K protein has the amino acid sequence **SEQ ID NO: 12.**
Embodiment 28: The polynucleotide of Embodiment 1, wherein the polynucleotide does not comprise a nucleotide sequence encoding the pTP or a portion thereof, and wherein the pTP has the amino acid sequence **SEQ ID NO: 13.**
Embodiment 29: The polynucleotide of Embodiment 1, wherein the polynucleotide does not comprise a nucleotide sequence encoding:
   a full length L4-100K protein or a portion thereof;
   a full length L1-52K/55K protein or a portion thereof;
   a full length L4-33K protein or a portion thereof;
   a full length L4-22K protein or a portion thereof;
   a full length L5 fiber protein or a portion thereof;
   a full length pVIII protein or a portion thereof; and
   a full length pTP or a portion thereof.
Embodiment 30: The polynucleotide of Embodiment 29, wherein the polynucleotide does not comprise a nucleotide sequence encoding:
   a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
   a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
   a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
   a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
   a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ** ID **NO: 10,** or a portion thereof;
   a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
   a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof.
Embodiment 31: The polynucleotide of Embodiment 29, wherein the polynucleotide does not comprise a nucleotide sequence encoding:
   a full length adenoviral E3 region or a portion thereof;
   a full length adenoviral U exon protein (UXP) exon 1 or a portion thereof;
   a full length L4-100K protein or a portion thereof;
   a full length L1-55K protein or a portion thereof;
   a full length L4-33K protein or a portion thereof;
   a full length L4-22K protein or a portion thereof;
   a full length L5 fiber protein or a portion thereof;
   a full length pVIII protein or a portion thereof; and
   a full length pTP or a portion thereof.
Embodiment 32: The polynucleotide of Embodiment 31, wherein the polynucleotide does not comprise a nucleotide sequence encoding:
   a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
   a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
   a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
   a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
   a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
   a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
   a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
   a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
   a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof.
Embodiment 33: The polynucleotide of Embodiment 30, wherein the wherein the polynucleotide does not comprise a nucleotide sequence encoding:
   a full length adenoviral E3 region or a portion thereof;
   a full length adenoviral U exon protein promoter or a portion thereof;
   a full length adenoviral U exon protein exon 1 or a portion thereof;
   a full length L4-100K protein or a portion thereof;
   a full length L1-52K/55K protein or a portion thereof;
   a full length L4-33K protein or a portion thereof;
   a full length L4-22K protein or a portion thereof;
   a full length L5 fiber protein or a portion thereof;
   a full length pVIII protein or a portion thereof; and
   a full length pTP or a portion thereof
Embodiment 34: The polynucleotide of Embodiment 31, wherein the polynucleotide does not comprise a nucleotide sequence encoding:
   a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
   a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion thereof;
   a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
   a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
   a full length L1 -52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
   a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
   a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
   a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
   a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
   a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof.
Embodiment 35: The polynucleotide of Embodiment 1, wherein the polynucleotide comprises a nucleotide sequence encoding:
   a full-length L4-33K protein; and
   a full-length L4-22K protein, and
   wherein the wherein the polynucleotide does not comprise a nucleotide sequence encoding:
      a full length adenoviral E3 region or a portion thereof;
      a full length adenoviral U exon protein promoter or a portion thereof;
      a full length adenoviral U exon protein exon 1 or a portion thereof;
      a full length L4-100K protein or a portion thereof;
      a full length L1-55K protein or a portion thereof;
      a full length L5 fiber protein or a portion thereof;
      a full length pVIII protein or a portion thereof; and
      a full length pTP or a portion thereof.
Embodiment 36: The polynucleotide of Embodiment 31, wherein the polynucleotide comprises a nucleotide sequence encoding:
   a L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof; and
   a L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof; and
   wherein the polynucleotide does not comprise a nucleotide sequence encoding:
   a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
   a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion thereof;
   a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
   a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
   a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
   a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ** ID **NO:** 10, or a portion thereof;
   a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
   a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof.
Embodiment 37: The polynucleotide of any one of Embodiments 1-36, wherein the polynucleotide does not comprise a nucleotide sequence encoding: an adenoviral E3 region or a portion thereof.
Embodiment 38: The polynucleotide of any one of Embodiments 1-37, wherein the polynucleotide does not comprise a nucleotide sequence encoding an adenoviral E3 12.5k protein or a portion thereof.
Embodiment 39: The polynucleotide of any one of Embodiments 1-38, wherein the polynucleotide does not comprise a nucleotide sequence encoding an adenoviral E3 12.5k protein or a portion thereof, and wherein the E3 12.5k protein has the amino acid sequence **SEQ ID NO: 14.**
Embodiment 40: The polynucleotide of any one of Embodiments 1-39, wherein the polynucleotide does not comprise a nucleotide sequence encoding an adenoviral E3 CR1-alpha protein or a portion thereof
Embodiment 41: The polynucleotide of any one of Embodiments 1-40, wherein the polynucleotide does not comprise a nucleotide sequence encoding an adenoviral E3 CR1-alpha or a portion thereof, and wherein the E3 CR1-alpha protein has the amino acid sequence **SEQ ID NO: 15.**
Embodiment 42: The polynucleotide of any one of Embodiments 1-41, wherein the polynucleotide does not comprise a nucleotide sequence encoding an adenoviral E3 gp19k protein or a portion thereof.
Embodiment 43: The polynucleotide of any one of Embodiments 1-42, wherein the polynucleotide does not comprise a nucleotide sequence encoding an adenoviral E3 gp19k protein or a portion thereof, and wherein the E3 gp19k protein has the amino acid sequence **SEQ ID NO: 16.**
Embodiment 44: The polynucleotide of any one of Embodiments 1-43, an adenoviral wherein the polynucleotide does not comprise a nucleotide sequence encoding E3 10.5kd protein.
Embodiment 45: The polynucleotide of any one of Embodiments 1-44, an adenoviral wherein the polynucleotide does not comprise a nucleotide sequence encoding E3 10.5kd protein or a portion thereof, and wherein the E3 10.5kd protein has the amino acid sequence **SEQ ID NO: 17.**
Embodiment 46: The polynucleotide of any one of Embodiments 1-45, wherein the polynucleotide does not comprise a nucleotide sequence encoding an adenoviral E3 RID-alpha protein.
Embodiment 47: The polynucleotide of any one of Embodiments 1-46, wherein the polynucleotide does not comprise a nucleotide sequence encoding an adenoviral E3 RID-alpha protein, and wherein the E3 RID-alpha protein has the amino acid sequence **SEQ ID NO: 18.**
Embodiment 48: The polynucleotide of any one of Embodiments 1-47, wherein the polynucleotide does not comprise a nucleotide sequence encoding an adenoviral E3 RID-beta protein.
Embodiment 49: The polynucleotide of any one of Embodiments 1-48, wherein the polynucleotide does not comprise a nucleotide sequence encoding an adenoviral E3 RID-beta protein or a portion thereof, and wherein the E3 RID-beta protein has the amino acid sequence **SEQ ID NO: 19.**
Embodiment 50: The polynucleotide of any one of Embodiments 1-49, wherein the polynucleotide does not comprise a nucleotide sequence encoding an adenoviral E3 14.7k protein.
Embodiment 51: The polynucleotide of any one of Embodiments 1-50, wherein the polynucleotide does not comprise a nucleotide sequence encoding an adenoviral E3 14.7k protein or a portion thereof, and wherein the E3 14.7k protein has the amino acid sequence **SEQ ID NO: 20.**
Embodiment 52: The polynucleotide of any one of Embodiments 1-30, wherein the polynucleotide comprises a nucleotide sequence encoding: an adenoviral E3 region.
Embodiment 53: The polynucleotide of Embodiment 52, wherein the E3 region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21.**
Embodiment 54: The polynucleotide of Embodiment 52 or 53, wherein the E3 region is between the E2a region and the E4 region.
Embodiment 55: The polynucleotide of any one of Embodiments 52-54, wherein the E3 region is downstream of the E2a region.
Embodiment56 : The polynucleotide of any one of Embodiments 52-55, wherein the E3 region is upstream of the E4 region.
Embodiment 57: The polynucleotide of any one of Embodiments 1-56, wherein the VA region is oriented in a 5'->3' direction.
Embodiment 58: The polynucleotide of any one of Embodiments 1-57, wherein the E2a region is oriented in a 3'->5' direction.
Embodiment 59: The polynucleotide of any one of Embodiments 1-58, wherein the E4 region is oriented in a 3'->5' direction.
Embodiment 60: The polynucleotide of any one of Embodiments 1-59, wherein the polynucleotide comprises in series: the poly A site, the VA RNA region, the E2a region, and the E4 region.
Embodiment 61: The polynucleotide of any one of Embodiments 1-60, wherein the VA RNA region is flanked on each side by at least one restriction site.
Embodiment 62: The polynucleotide of any one of Embodiments 1-61, wherein the polynucleotide comprises at least 2, e.g., 3, 4, 5 or more restriction sites upstream of the VA RNA region.
Embodiment 63: The polynucleotide of any one of Embodiments 1-62, wherein the polynucleotide comprises at least one restriction site downstream of the VA RNA region, and wherein the restriction site is between the VA region and the E2a region.
Embodiment 64: The polynucleotide of any one of Embodiments 1-63, wherein the polynucleotide comprises at least one restriction site downstream of the E4 region.
Embodiment 65: The polynucleotide of any one of Embodiments 1-64, wherein the polynucleotide comprises a nucleotide sequence encoding: a polyadenylation (poly A) site, and wherein the poly A site is upstream of the VA RNA region.
Embodiment 66: The polynucleotide of Embodiment 65, wherein the poly A site comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 22** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 22.**
Embodiment 67: The polynucleotide of Embodiments 65 or 66, wherein the poly A site is flanked by at least one restriction site on each side.
Embodiment 68: The polynucleotide of any one of Embodiments 65-67, wherein the polynucleotide comprises at least three restriction sites upstream of the poly A site.
Embodiment 69: The polynucleotide of any one of Embodiments 65-68, wherein the polynucleotide comprises at least one restriction site downstream of the poly A site, and wherein the at least one restriction site is between the poly A site and the VA region.
Embodiment 70: The polynucleotide of any one of Embodiments 1-69, wherein the polynucleotide comprises at least one inverted terminal repeat (ITR) sequence
Embodiment 71: The polynucleotide of any one of Embodiments 1-70, wherein the polynucleotide comprises at least one inverted terminal repeat (ITR) sequence upstream of the E4 region.
Embodiment 72: The polynucleotide of Embodiment 69 or 71, wherein the ITR comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 23** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 23.**
Embodiment 73: The polynucleotide of any one of Embodiments 1-72, wherein the VA RNA region comprises a VA RNA I region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 24** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 24.**
Embodiment 74: The polynucleotide of any one of Embodiments 1-73, wherein the VA RNA region comprises a VA RNA II region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 25** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 25.**
Embodiment 75: The polynucleotide of any one of Embodiments 1-74, wherein the VA region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 26** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 26.**
Embodiment 76: The polynucleotide of any one of Embodiments 1-75, wherein the E2a region comprises a nucleotide sequence encoding an adenoviral DNA binding protein (DBP).
Embodiment 77: The polynucleotide of any one of Embodiments 1-76, wherein the E2a region comprises a nucleotide sequence encoding an adenoviral DNA binding protein (DBP) and having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 27**or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 27.**
Embodiment 78: The polynucleotide of any one of Embodiments 1-77, wherein the E2a region comprises a nucleotide sequence encoding a DNA binding protein (DBP) having the amino acid sequence **SEQ ID NO: 28.**
Embodiment 79: The polynucleotide of any one of Embodiments 1-78, wherein the E2a region comprises an adenoviral U exon protein (UXP) exon 3 sequence.
Embodiment 80: The polynucleotide of any one of Embodiments 1-79, wherein the E2a region comprises an adenoviral UXP exon 3 sequence having at least 8% identity to **SEQ ID NO: 29** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 29.**
Embodiment 81: The polynucleotide of any one of Embodiments 1-80, wherein the E2a region comprises an adenoviral E2a exon2/leader sequence.
Embodiment 82: The polynucleotide of any one of Embodiments 1-81, wherein the E2a region comprises an adenoviral E2a exon2/leader sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30.**
Embodiment 83: The polynucleotide of any one of Embodiments 1-82, wherein the E2a region comprises an adenoviral UXP exon 2 sequence.
Embodiment 84: The polynucleotide of any one of Embodiments 1-83, wherein the E2a region comprises an adenoviral UXP exon 2 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30.**
Embodiment 85: The polynucleotide of any one of Embodiments 1-84, wherein the E2a region comprises an adenoviral E2a late promoter sequence.
Embodiment 86: The polynucleotide of any one of Embodiments 1-85, wherein the E2a region comprises an adenoviral E2a late promoter sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 31** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 31.**
Embodiment 87: The polynucleotide of any one of Embodiments 1-86, wherein the E2a region comprises an adenoviral E2a exon 1 sequence.
Embodiment 88: The polynucleotide of any one of Embodiments 1-87, wherein the E2a region comprises an adenoviral E2a exon 1 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 32** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 32.**
Embodiment 89: The polynucleotide of any one of Embodiments 1-88, wherein the E2a is under the control of one or more of promoters, optionally the promoter is not a chicken β-actin promoter or a SV40 promoter.
Embodiment 90: The polynucleotide of any one of Embodiments 1-89, wherein the E2a region comprises an adenoviral E2a early promoter sequence.
Embodiment 91: The polynucleotide of any one of Embodiments 1-90, wherein the E2a region comprises an adenoviral E2a early promoter sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 33** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 33.**
Embodiment 92: The polynucleotide of any one of Embodiments 1-91, wherein the E2a region comprises an adenoviral E2a late primary transcript sequence.
Embodiment 93: The polynucleotide of any one of Embodiments 1-92, wherein the E2a region comprises an adenoviral E2a late primary transcript sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 34** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 34.**
Embodiment 94: The polynucleotide of any one of Embodiments 1-93, wherein the E2a region comprises an adenoviral E2a early primary transcript sequence.
Embodiment 95: The polynucleotide of any one of Embodiments 1-94, wherein the E2a region comprises an adenoviral E2a early primary transcript sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 35** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 35.**
Embodiment 96: The polynucleotide of any one of Embodiments 1-95, wherein the E2a region comprises a nucleotide having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 36** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 36.**
Embodiment 97: The polynucleotide of any one of Embodiments 1-96, wherein the E4 region comprises an adenoviral E4 orf6/7 sequence.
Embodiment 98: The polynucleotide of any one of Embodiments 1-97, wherein the E4 region comprises an adenoviral E4 orf6/7 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 37** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 37.**
Embodiment 99: The polynucleotide of any one of Embodiments 1-98, wherein the E4 region comprises a nucleotide sequence encoding an adenoviral E4 orf6/7 protein having the amino acid sequence **SEQ ID NO: 38.**
Embodiment 100: The polynucleotide of any one of Embodiments 1-99, wherein the E4 region comprises a nucleotide sequence encoding an adenoviral E4 34K protein having the amino acid sequence **SEQ ID NO: 39.**
Embodiment 101: The polynucleotide of any one of Embodiments 1-100, wherein the E4 region comprises an adenoviral E4 orf4 sequence.
Embodiment 102: The polynucleotide of any one of Embodiments 1-101, wherein the E4 region comprises an adenoviral E4 orf4 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 40** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 40.**
Embodiment 103: The polynucleotide of any one of Embodiments 1-102, wherein the E4 region comprises a nucleotide sequence encoding an adenoviral E4 orf4 protein having the amino acid sequence **SEQ ID NO: 41.**
Embodiment 104: The polynucleotide of any one of Embodiments 1-103, wherein the E4 region comprises an adenoviral E4 orf3 sequence.
Embodiment 105: The polynucleotide of any one of Embodiments 1-104, wherein the E4 region comprises an adenoviral E4 orf3 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 42** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 42.**
Embodiment 106: The polynucleotide of any one of Embodiments 1-105, wherein the E4 region comprises a nucleotide sequence encoding an adenoviral E4 orf3 protein having the amino acid sequence **SEQ ID NO: 43.**
Embodiment 107: The polynucleotide of any one of Embodiments 1-106, wherein the E4 region comprises an adenoviral E4 orf8 sequence.
Embodiment 108: The polynucleotide of any one of Embodiments 1-107, wherein the E4 region comprises an adenoviral E4 orf4 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 44** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 44.**
Embodiment 109: The polynucleotide of any one of Embodiments 1-108, wherein the E4 region comprises a nucleotide sequence encoding an adenoviral E4 orf4 protein having the amino acid sequence **SEQ ID NO: 45.**
Embodiment 110: The polynucleotide of any one of Embodiments 1-109, wherein the E4 region comprises an adenoviral E4 orf1 sequence.
Embodiment 111: The polynucleotide of any one of Embodiments 1-110, wherein the E4 region comprises an adenoviral E4 orf1 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 46** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 46.**
Embodiment 112: The polynucleotide of any one of Embodiments 1-111, wherein the E4 region comprises a nucleotide sequence encoding an adenoviral E4 orf1 protein having the amino acid sequence **SEQ ID NO: 47.**
Embodiment 113: The polynucleotide of any one of Embodiments 1-112, wherein the E4 region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 48** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 48.**
Embodiment 114: The polynucleotide of any one of Embodiments 1-113, wherein the E4 region is operably linked to a promoter, optionally the promoter is not a chicken β-actin promoter or a SV40 promoter.
Embodiment 115: The polynucleotide of any one of Embodiments 1-114, wherein the polynucleotide does not comprise a nucleotide sequence encoding:
   adenoviral E1 region or a portion thereof.
Embodiment 116: The polynucleotide of any one of Embodiments 1-115, wherein the polynucleotide does not comprise a nucleotide sequence encoding an adenoviral protease or a portion thereof.
Embodiment 117: The polynucleotide of any one of Embodiments 1-116, wherein the polynucleotide does not comprise a nucleotide sequence encoding an adenoviral protease or a portion thereof, and wherein the protease has the amino acid sequence **SEQ ID NO: 49.**
Embodiment 118: The polynucleotide of any one of Embodiments 1-117, wherein the polynucleotide does not comprise a nucleotide sequence encoding an adenoviral E1a protein 13S or a portion thereof.
Embodiment 119: The polynucleotide of any one of Embodiments 1-118, wherein the polynucleotide does not comprise a nucleotide sequence encoding an adenoviral E1a protein 13S or a portion thereof, and wherein the E1a protein 13S has the amino acid sequence **SEQ ID NO: 50.**
Embodiment 120: The polynucleotide of any one of Embodiments 1-119, wherein the polynucleotide does not comprise a nucleotide sequence encoding an adenoviral E1a protein 12S or a portion thereof.
Embodiment 121: The polynucleotide of any one of Embodiments 1-120, wherein the polynucleotide does not comprise a nucleotide sequence encoding an adenoviral E1a protein 12S or a portion thereof, and wherein the E1a protein 12S has the amino acid sequence **SEQ ID NO: 51.**
Embodiment 122: The polynucleotide of any one of Embodiments 1-121, wherein the polynucleotide does not comprise a nucleotide sequence encoding an adenoviral E1a protein 11S or a portion thereof.
Embodiment 123: The polynucleotide of any one of Embodiments 1-122, wherein the polynucleotide does not comprise a nucleotide sequence encoding an adenoviral E1a protein 11S or a portion thereof, and wherein the E1a protein 11S has the amino acid sequence **SEQ ID NO: 52.**
Embodiment 124: The polynucleotide of any one of Embodiments 1-123, wherein the polynucleotide does not comprise a nucleotide sequence encoding an adenoviral E1a protein 10S or a portion thereof.
Embodiment 512: The polynucleotide of any one of Embodiments 1-124, wherein the polynucleotide does not comprise a nucleotide sequence encoding an adenoviral E1a protein 10S or a portion thereof, and wherein the E1a protein 10S has the amino acid sequence **SEQ ID NO: 53.**
Embodiment 126: The polynucleotide of any one of Embodiments 1-125, wherein the polynucleotide does not comprise a nucleotide sequence encoding an adenoviral E1a protein 9S or a portion thereof.
Embodiment 127: The polynucleotide of any one of Embodiments 1-126, wherein the polynucleotide does not comprise a nucleotide sequence encoding an adenoviral E1a protein 9S or a portion thereof, and wherein the E1a protein 9S has the amino acid sequence **SEQ ID NO: 54.**
Embodiment 128: The polynucleotide of any one of Embodiments 1-127, wherein the polynucleotide does not comprise a nucleotide sequence encoding an adenoviral E1b protein 19K or a portion thereof.
Embodiment 129: The polynucleotide of any one of Embodiments 1-128, wherein the polynucleotide does not comprise a nucleotide sequence encoding an adenoviral E1b protein 19K or a portion thereof, and wherein the E1b protein 19K has the amino acid sequence **SEQ ID NO: 55.**
Embodiment 130: The polynucleotide of any one of Embodiments 1-129, wherein the polynucleotide does not comprise a nucleotide sequence encoding an adenoviral E1b protein 55K or a portion thereof.
Embodiment 131: The polynucleotide of any one of Embodiments 1-130, wherein the polynucleotide does not comprise a nucleotide sequence encoding an adenoviral E1b protein 55K or a portion thereof, and wherein the E1b protein 55K has the amino acid sequence **SEQ ID NO: 56.**
Embodiment 132: The polynucleotide of any one of Embodiments 1-131, wherein the polynucleotide does not comprise a nucleotide sequence encoding an adenoviral hexon protein or a portion thereof.
Embodiment 133: The polynucleotide of any one of Embodiments 1-132, wherein the polynucleotide does not comprise a nucleotide sequence encoding an adenoviral hexon protein or a portion thereof, and wherein the hexon protein has the amino acid sequence **SEQ ID NO: 57.**
Embodiment 134: The polynucleotide of any one of Embodiments 1-133, wherein the polynucleotide does not comprise a nucleotide sequence encoding an adenoviral peripentonal hexon-associated protein or a portion thereof.
Embodiment 135: The polynucleotide of any one of Embodiments 1-134, wherein the polynucleotide does not comprise a nucleotide sequence encoding an adenoviral peripentonal hexon-associated protein or a portion thereof, and wherein the peripentonal hexon-associated protein has the amino acid sequence **SEQ ID NO: 58.**
Embodiment 136: The polynucleotide of any one of Embodiments 1-135, wherein the polynucleotide is about 16,500 nucleotides or less in size.
Embodiment 137: The polynucleotide of any one of Embodiments 1-136, wherein the polynucleotide is about 15,500 nucleotides or less in size.
Embodiment 138: The polynucleotide of any one of Embodiments 1-137, wherein the polynucleotide is about 14,000 nucleotide or less in size.
Embodiment 139: The polynucleotide of any one of Embodiments 1-138, wherein the polynucleotide is about 12,500 nucleotides or less in size.
Embodiment 140: The polynucleotide of any one of Embodiments 1-139, wherein the polynucleotide is about 12,000 nucleotides or less in size.
Embodiment 141: The polynucleotide of any one of Embodiments 1-140, wherein the polynucleotide is about 11,000 nucleotides or less in size.
Embodiment 142: The polynucleotide of any one of Embodiments 1-141, wherein the polynucleotide is double-stranded.
Embodiment 143: The polynucleotide of any one of Embodiments 1-142, wherein the polynucleotide is single-stranded.
Embodiment 144: The polynucleotide of any one of Embodiments 1-143, wherein the polynucleotide is linear.
Embodiment 145: The polynucleotide of any one of Embodiments 1-144, wherein the polynucleotide is circular.
Embodiment 146: The polynucleotide of any one of Embodiments 1-145, wherein the polynucleotide is a close ended linear duplexed DNA (cIDNA).
Embodiment 147: The polynucleotide of any one of Embodiments 1-146, wherein the polynucleotide does not comprise a chicken β-actin promoter and/or an SV40 promoter.
Embodiment 148: The polynucleotide of any one of Embodiments 1-147, wherein the expression of E2a region is not under the control of a chicken β-actin promoter and/or an SV40 promoter.
Embodiment 149: The polynucleotide of any one of Embodiments 1-148, wherein the expression of E4 open reading frames (ORFs) is not under the control of a chicken β-actin promoter and/or an SV40 promoter.
Embodiment 150: A vector comprising a polynucleotide of any one of Embodiments 1-149.
Embodiment 151: The vector of Embodiment 150, wherein the vector is a viral vector.
Embodiment 152: The vector of Embodiment 151, wherein the viral vector is a retroviral vector, a lentiviral vector, an adenoviral vector, an adeno-associated viral vector, or a herpes simplex viral vector.
Embodiment 153: The vector of Embodiment 152, wherein the viral vector is an adenoviral vector.
Embodiment 154: The vector of any one of Embodiments 150-153, wherein the vector does not comprise a nucleotide sequence encoding full length L-22K and/or a full length L-33K protein.
Embodiment 155: The vector of any one of Embodiments 150-154, wherein the vector does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7.**
Embodiment 156: The vector of any one of Embodiments 150-155, wherein the vector does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8.**
Embodiment 157: A plasmid comprising a polynucleotide of any one of Embodiments 1-149.
Embodiment 158: The plasmid of Embodiment 157, wherein the plasmid does not comprise a nucleotide sequence encoding full length L-22K and/or a full length L-33K protein.
Embodiment 159: The plasmid of Embodiments 157 or 158, wherein the plasmid does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7.**
Embodiment 160: The plasmid of any one of Embodiments 157-159, wherein the plasmid does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8.**
Embodiment 161: A cell comprising a polynucleotide of any one of Embodiments 1-149, a vector of any one of Embodiments 150-156, or a plasmid of any one of Embodiments 157-160.
Embodiment 162: The cell of Embodiment 161, wherein the cell further comprises a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR), and wherein the L-ITR and R-ITR are selected independently from AAV ITRs and adenoviral ITRs.
Embodiment 163: The cell of Embodiment 161 or 168, wherein the cell further comprises a polynucleotide encoding viral capsid (e.g., AAV capsid) and/or non-structural replication (e.g., AAV Rep) genes.
Embodiment 164: The cell of any one of Embodiments 161-163, wherein the cell comprises: (i) a polynucleotide of any one of Embodiments 1-149, a vector of any one of Embodiments 150-156, or a plasmid of any one of Embodiments 157-160; (ii) a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR); and (iii) a polynucleotide encoding viral capsid (e.g., AAV capsid) and/or non-structural replication (e.g., AAV Rep) genes.
Embodiment 165: The cell of Embodiment 161 or 162, wherein the polynucleotide of any one of Embodiments 1-149, a vector of any one of Embodiments 150-156, or a plasmid of any one of Embodiments 157-160 and the polynucleotide encoding the viral capsid (e.g., AAV capsid) and/or the non-structural replication (e.g., AAV Rep) genes are the same.
Embodiment 166: The cell of any one of Embodiments 161-165, wherein cell is an insect cell or a mammalian cell, preferably the cell is a mammalian cell.
Embodiment 167: The cell of any one of Embodiments 161-166, wherein the cell is a HeLa cell, COS cell, COS-1 cell, COS-7 cell, HEK293 cell, A549 cell, BHK cell, BSC-1 cell, BSC-40 cell, Vero cell, Sf"c9 cell, Sf -21 cell, Tn-368 cell, BTI-Tn-5B1-4 (High-Five) cell, Saos cell, C2C12 cell, L cell, HT1080 cell, HepG2 cell, WEHI cell, 3T3 cell, 10T1/2 cell, MDCK cell, BMT-10 cell, WI38 cell, or a primary fibroblast, hepatocyte or myoblast cells derived from a mammal.
Embodiment 168: The cell of any one of Embodiments 161-167, wherein the cell is a HEK293 cell or HeLa cell.
Embodiment 169: The cell of any one of Embodiments 161-168, wherein the cell is a suspension adapted cell.
Embodiment 170: The cell of any one of Embodiments 161-169, wherein the cell is a producer cell.
Embodiment 171: The cell of any one of Embodiments 161-170, wherein the cell does not comprise a polynucleotide encoding a full length L-22K and/or a full length L-33K protein.
Embodiment 172: The cell of any one of Embodiments 161-171, wherein the cell does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7.**
Embodiment 173: The cell of any one of Embodiments 157-168, wherein the cell does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8.**
Embodiment 174: of the cell of any one of Embodiments 161-173 for producing recombinant adeno-associated viral (rAAV) particles.
Embodiment 175: Use of a polynucleotide of any one of Embodiments 1-149, a vector of any one of Embodiments 150-156, or a plasmid of any one of Embodiments 157-160 for producing recombinant adeno-associated viral (rAAV) particles.
Embodiment 176: A recombinant adeno-associated virus (rAAV) in combination with a polynucleotide of any one of Embodiments 1-149, a vector of any one of Embodiments 150-156, or a plasmid of Embodiment 157-160.
Embodiment 177: A method for producing recombinant adeno-associated viral (rAAV) particles, the method comprising: culturing a cell of any one of Embodiments 161-73 in a culture medium under conditions in which rAAV particles are produced.
Embodiment 178: A method for producing recombinant adeno-associated viral (rAAV) particles, the method comprising: culturing a host cell in a culture medium under conditions in which rAAV particles are produced, where the host cell comprises: (i) a polynucleotide of any one of Embodiments 1-149, a vector of any one of Embodiments 150-156, or a plasmid of any one of Embodiments 157-160; (ii) a polynucleotide encoding a virus genome (e.g., an AAV endogenous genome, optionally flanked by left inverted terminal repeat (L-AAV ITR) and/or right ITR (R-AAV ITR), or a recombinant AAV genome comprising polynucleotide encoding a transgene, optionally flanked by L-AAVITR and/or R-AAV ITR); and (iii) a polynucleotide encoding viral capsid (e.g., AAV capsid) and/or non-structural replication (e.g., AAV Rep) genes.
Embodiment 179: The method of Embodiment 178, wherein the polypeptide of (i) and the polypeptide of (iii) are the same.
Embodiment 180: The method of any one of Embodiments 177-179, wherein the cell culture is substantially free of a polynucleotide encoding a full-length L-22K protein and/or a full-length L-33K protein.
Embodiment 181: The method of any one of Embodiments 177-180, wherein the cell culture is substantially free of a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7.**
Embodiment 182: The method of any one of Embodiments 177-181, wherein the cell culture is substantially free of a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8.**
Embodiment 183: The method of any one of Embodiments 177-182, wherein a full-length L-22K protein and/or a full-length L-33K protein is not expressed during the culturing of the cell.
Embodiment 184: The method of any one of Embodiments 177-183, wherein a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7** is not expressed during the culturing of the cell.
Embodiment 185: The method of any one of Embodiments 177-184, wherein a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8** is not expressed during the culturing of the cell.
Embodiment 186: The method of any one of Embodiments 177-185, wherein the method comprises a step of transfecting the cell with a polynucleotide of any one of Embodiments 1-149, a vector of any one of Embodiments 150-156, or a plasmid of any one of Embodiments 157-160.
Embodiment 187: The method of any one of Embodiment 177-186, wherein the method comprises a step of transfecting the cell with a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR).
Embodiment 188: The method of any one of Embodiments 177-187, wherein the method comprises a step of transfecting the cell with a polynucleotide encoding viral capsid (e.g., AAV capsid) and/or non-structural replication (e.g., AAV Rep) genes.
Embodiment 189: The method of any one of Embodiment 177-188, wherein the cell stably expresses viral capsid (e.g., AAV capsid) and/or non-structural replication (e.g., AAV Rep) genes.
Embodiment 190: The method of any one of Embodiments 177-189, wherein the cells are cultured for at least 10 hours (e.g., at least 11 hours, at least 12 hours, at least 13 hours, at least 14 hours, at least 15 hours, at least 16 hours, at least 17 hours, at least 18 hours, at least 19 hours, at least 20 hours, at least 21 hours, at least 22 hours, at leas1t 23 hours, at least 24 hours, at least 25 hours, at least 26 hours, at least 27 hours, at least 28 hours, at least 29 hours, at least 30 hours, at least 31 hours, at least 32 hours, at least 33 hours, at least 34 hours, at least 35 hours, at least 36 hours, at least 37 hours, at least 38 hours, at least 39 hours, at least 40 hours, at least 41 hours, at least 42 hours, at least 43 hours, at least 44 hours, at least 45 hours, at least 46 hours, at least 47 hours, at least 48 hours, at least 49 hours, at least 50 hours, at least 51 hours, at least 52 hours, at least 53 hours, at least 54 hours, at least 55 hours, at least 56 hours, at least 57 hours, at least 58 hours, at least 59 hours, at least 60 hours, at least 61 hours, at least 62 hours, at least 63 hours, at least 64 hours, at least 65 hours, at least 66 hours, at least 67 hours, at least 68 hours, at least 69 hours, at least 70 hours, at least 71 hours, at least 72 hours, at least 73 hours, at least 74 hours, at least 75 hours, at least 76 hours, at least 77 hours, at least 78 hours, at least 79 hours, at least 80 hours, at least 81 hours, at least 82 hours, at least 83 hours, at least 84 hours, at least 85 hours, at least 86 hours, at least 87 hours, at least 88 hours, at least 89 hours, at least 90 hours, at least 91 hours, at least 92 hours, at least 93 hours, at least 94 hours, at least 95 hours, at least 96 hours, at least 97 hours, at least 98 hours, at least 99 hours, at least 100 hours or more).
Embodiment 191: The method of any one of Embodiments 177-190, wherein the method further comprises a step, e.g., a post-lysis step of removing or reducing an amount of impurities, e.g., hcDNA from the cell culture or cell culture supernatant.
Embodiment 192: The method of Embodiment 191, wherein the step of removing or reducing an amount of impurities comprises adding a cationic amine or nuclease to the cell culture or cell culture supernatant.
Embodiment 193: The method of Embodiment 191 or 192, wherein the step of removing or reducing an amount of impurities comprises adding a selective precipitation agent to the cell culture or cell culture supernatant.
Embodiment 194: The method of any one of Embodiments 177-193, wherein the method further comprises a step of clarifying the cell culture or cell culture supernatant.
Embodiment 195: The method of Embodiment 194, wherein the step of clarifying the cell culture or cell culture supernatant comprises depth filtration.
Embodiment 196: The method of Embodiment 194 or 195, wherein the method further comprises a step of concentrating the clarified cell culture or cell culture supernatant.
Embodiment 197: The method of Embodiment 196, wherein the step of concentrating the clarified cell culture or cell culture supernatant comprises tangential flow filtration.
Embodiment 198: The method of any one of Embodiments 177-197, wherein the method further comprises a step of enriching or purifying rAAV particles.
Embodiment 199: The method of Embodiment 198, wherein the step of enriching or purifying rAAV particles comprises chromatography.
Embodiment 200: The method of Embodiment 199, wherein the step of enriching or purifying rAAV particles comprises affinity chromatography and/or anion exchange chromatography.
Embodiment 201: The method of any one of Embodiments 177-200, wherein at least about 50%, (e.g., at least about 55%, at least about 60%, at least about 65%, at least about 70%, at least about 75%, at least about 80%, at least about 85%, at last about 95%, at least about 95% or a higher percentage) of the rAAV particles produced are full capsid particles.
Embodiment 202: The method of any one of Embodiments 177-201, wherein the method produces at least about 1 x 10⁴ (e.g., e.g., at least about 2 x 10⁴, 3 x 10⁴, 4 x 10⁴, 5 x 10⁴, 6 x 10⁴, 7 x 10⁴, 8 x 10⁴, 9 x 10⁴, or 1 x 10⁵ or more) vector genome-containing particles per cell prior to purification.
Embodiment 203: The method of any one of Embodiments 177-202, wherein the method provides at least about 1e¹² vector genome per ml (vg/ml) (e.g., at least about 1.5e¹² vg/ml, at least about 2e¹² vg/ml, at least about 2.5e¹² vg/ml, at least about 3e¹² vg/ml, at least about 3.5e¹² vg/ml, at least about 4e¹² vg/ml, at least about 4.5e¹² vg/ml, at least about 5e¹² vg/ml, at least about 5.5e¹² vg/ml, at least about 6e¹² vg/ml, at least about 6.5e¹² vg/ml, at least about 7e¹² vg/ml, at least about 7.5e¹² vg/ml, at least about 8e¹² vg/ml, at least about 8.5e¹² vg/ml, at least about 9e¹² vg/ml, at least about 9.5e¹³ vg/ml, at least about 1e¹³vg/ml, at least about 1.5e¹³vg/ml, at least about 2e¹³vg/ml, at least about 2.5e¹³vg/ml, at least about 3e¹³vg/ml, at least about 3.5e¹³vg/ml, at least about 4e¹³ vg/ml, at least about 4.5e¹³ vg/ml, at least about 5e¹³ vg/ml, at least about 5.5e¹³ vg/ml, at least about 6e¹³ vg/ml, at least about 6.5e¹³ vg/ml, at least about 7e¹³vg/ml, at least about 7.5e¹³vg/ml, at least about 8e¹³vg/ml, at least about 8.5e¹³vg/ml, at least about 9e¹³vg/ml, at least about 9.5e¹³ vg/ml, at least about 1e¹⁴vg/ml or more), e.g., after purification, such as after affinity purification.
Embodiment 204: The method of any one of Embodiments 177-203, wherein the method provides at least about 1e¹² viral particles per ml (vp/ml) at least about 1e¹²viral particles per ml (vp/ml) (e.g., at least about 1.5e¹²vp/ml, at least about 2e¹² vp/ml, at least about 2.5e¹² vp/ml, at least about 3e¹² vp/ml, at least about 3.5e¹² vp/ml, at least about 4e¹² vp/ml, at least about 4.5e¹² vp/ml, at least about 5e¹² vp/ml, at least about 5.5e¹² vp/ml, at least about 6e¹² vp/ml, at least about 6.5e¹² vp/ml, at least about 7e¹² vp/ml, at least about 7.5e¹² vp/ml, at least about 8e¹² vp/ml, at least about 8.5e¹²vp/ml, at least about 9e¹²vp/ml, at least about 9.5e¹³ vp/ml, at least about 1e¹³vp/ml, at least about 1.5e¹³vp/ml, at least about 2e¹³vp/ml, at least about 2.5e¹³vp/ml, at least about 3e¹³vp/ml, at least about 3.5e¹³vp/ml, at least about 4e¹³ vp/ml, at least about 4.5e¹³ vp/ml, at least about 5e¹³ vp/ml, at least about 5.5e¹³ vp/ml, at least about 6e¹³ vp/ml, at least about 6.5e¹³ vp/ml, at least about 7e¹³vp/ml, at least about 7.5e¹³vp/ml, at least about 8e¹³vp/ml, at least about 8.5e¹³vp/ml, at least about 9e¹³vp/ml, at least about 9.5e¹³ vp/ml, at least about 1e¹⁴ vp/ml or more), e.g., after purification, such as after affinity purification.
Embodiment 205: The method of any one of Embodiments 177-204, wherein the rAAV particles are AAV-1, AAV-2, AAV-2i8, AAV-3, AAV-4, AAV-5, AAV-6, AAV-7, AAV-8, AAV-9, AAV-10, AAVrh10, AAV-11, AAV-12, AAV-13, AAV-14, AAV-15, AAV-16 or a chimera, derivative, modification, or pseudotype thereof.
Embodiment 206: The method of any one of Embodiments 177-205, wherein the rAAV particle comprises at least one capsid protein (e.g., VP1, VP2, or VP3) from the AAV serotypes listed in **Table 2** in the description.
Embodiment 207: The polynucleotide of any one of Embodiments 1-149, wherein the polynucleotide is capable of producing recombinant adeno-associated viral (rAAV) particles in an amount that is at least 80% of an amount produced under similar conditions with a similar polynucleotide that further comprises a nucleotide sequence encoding a full-length L-22K protein and/or a full-length L-33K protein.
Embodiment 208: The polynucleotide of any one of Embodiments 1-149 or 207, wherein a packaging efficiency of the polynucleotide in a method of producing rAAV is at least 80% of a packaging efficiency of a similar polypeptide that further comprises a nucleotide sequence encoding a full-length L-22K protein and/or a full-length L-33K protein.

### Sequences

The below Table of Sequences lists and describes the various sequences discussed herein. Unless stated otherwise, all sequences are recited with 5' to 3' directionality of the positive strand of a plasmid. This directionality is preserved irrespective of the orientation of a gene or element described to be associated with a sequence.

| **Table 3: Exemplary sequences** | | |
|---|---|---|
| SEQ ID NO: | | SEQUENCE* |
| 1 | HAD1 | atcgaagtactcggggaaatgtgcgcggaacccctatttgtttatttttctaaatacattcaa atatgtatccgctcatgagacaataaccctgataaatgcttcaataatattgaaaaagga agagtatgagccatattcaacgggaaacgtcgaggccgcgattaaattccaacatgga tgctgatttatatgggtataaatgggctcgcgataatgtcgggcaatcaggtgcgacaatc tatcgcttgtatgggaagcccgatgcgccagagttgtttctgaaacatggcaaaggtagc gttgccaatgatgttacagatgagatggtcagactaaactggctgacggaatttatgcctc ttccgaccatcaagcattttatccgtactcctgatgatgcatggttactcaccactgcgatcc ccggaaaaacagcattccaggtattagaagaatatcctgattcaggtgaaaatattgttg |
| | | atgcgctggcagtgttcctgcgccggttgcattcgattcctgtttgtaattgtccttttaacagc gatcgcgtatttcgtctcgctcaggcgcaatcacgaatgaataacggtttggttgatgcga gtgattttgatgacgagcgtaatggctggcctgttgaacaagtctggaaagaaatgcata aacttttgccattctcaccggattcagtcgtcactcatggtgatttctcacttgataaccttattt ttgacgaggggaaattaataggttgtattgatgttggacgagtcggaatcgcagaccgat accaggatcttgccatcctatggaactgcctcggtgagttttctccttcattacagaaacgg ctttttcaaaaatatggtattgataatcctgatatgaataaattgcagtttcatttgatgctcga tgagtttttctaactgtcagaccaagtttactcatatatactttagattgatttaaaacttcatttt aatttaaaaggatctaggtgaagatcctttttgataatctcatgaccaaaatcccttaacgt gagttttcgttccactgagcgtcagaccccgtagaaaagatcaaaggatcttcttgagatc ctttttttctgcgcgtaatctgctgcttgcaaacaaaaaaaccaccgctaccagcggtggtt tgtttgccggatcaagagctaccaactctttttccgaaggtaactggcttcagcagagcgc agataccaaatactgttcttctagtgtagccgtagttaggccaccacttcaagaactctgta gcaccgcctacatacctcgctctgctaatcctgttaccagtggctgctgccagtggcgata agtcgtgtcttaccgggttggactcaagacgatagttaccggataaggcgcagcggtcg ggctgaacggggggttcgtgcacacagcccagcttggagcgaacgacctacaccga actgagatacctacagcgtgagctatgagaaagcgccacgcttcccgaagggagaaa ggcggacaggtatccggtaagcggcagggtcggaacaggagagcgcacgaggga gcttccagggggaaacgcctggtatctttatagtcctgtcgggtttcgccacctctgacttg agcgtcgatttttgtgatgctcgtcaggggggcggagcctatggaaaaacgccagcaa cgcggcctttttacggttcctggccttttttcctgcagcccggggggatccgattaattaaga cgtttaaacaataaaatatctttattttcattacatctgtgtgttggttttttgtgtgtcgac gagatcttccaacataaggcgatgatatccgtagatgtacctggacatcaggtgatgcc ggcggcggtggtggaggcgcgcggaaagtcgcggacgcggttccagatgttgcgca gcggcaaaaagtgctccatggtcgggacgctctggccggtcaggcgcgcgca atcgttgacgctctagaccgtgcaaaaggagagcctgtaagcgggcactcttcc gtggtctggtggataaattcgcaagggtatcatggcggacgaccggggttcgag ccccgtatccggccgtccgccgtgatccatgcggttaccgcccgcgtgtcgaac ccaggtgtgcgacgtcagacaacgggggagtgctccttttggcttccttccaggc gcggcggctgctgcgctagcttttttggccactggccgcgcgcagcgtaagcg gttaggctggaaagcgaaagcattaagtggctcgctccctgtagccggagggtt attttccaagggttgagtcgcgggacccccggttcgagtctcggaccggccgga ctgcggcgaacgggggtttgcctccccgtcatgcaagaccccgcttgcaaattc ctccggaaacagggacgagcccctttttttgcttttcccagatcatccggtgctgcgg cagatgcgcccccctcctcagcagcggcaagagcaagagcagcggcagacatgca gggcaccctccatgcatccactttattttgtcacttgaaaaacatgtaaaaataaatgtact agagacactttcaataaaggcaaatgcttttatttgtacactctcgggtgattatttac cccccacccttgccgtctgcgccgtttaaaaatcaaaggggttctgccgcgcatcg ctatgcgccactggcagggacacgttgcgatactggtgtttagtgctccacttaaa ctcaggcacaaccatccgcggcagctcggtgaagttttcactccacaggctgcg caccatcaccaacgcgtttagcaggtcgggcgccgatatcttgaagtcgcagtt ggggcctccgccctgcgcgcgcgagttgcgatacacagggttgcagcactgg aacactatcagcgccgggtggtgcacgctggccagcacgctcttgtcggagat cagatccgcgtccaggtcctccgcgttgctcagggcgaacggagtcaactttgg tagctgccttcccaaaaagggcgcgtgcccaggctttgagttgcactcgcaccg tagtggcatcaaaaggtgaccgtgcccggtctgggcgttaggatacagcgcctg cataaaagccttgatctgcttaaaagccacctgagcctttgcgccttcagagaag aacatgccgcaagacttgccggaaaactgattggccggacaggccgcgtcgtg |
| | | cacgcagcaccttgcgtcggtgttggagatctgcaccacatttcggccccaccg gttcttcacgatcttggccttgctagactgctccttcagcgcgcgctgcccgttttc gctcgtcacatccatttcaatcacgtgctccttatttatcataatgcttccgtgtagac acttaagctcgccttcgatctcagcgcagcggtgcagccacaacgcgcagccc gtgggctcgtgatgcttgtaggtcacctctgcaaacgactgcaggtacgcctgca ggaatcgccccatcatcgtcacaaaggtcttgttgctggtgaaggtcagctgcaa cccgcggtgctcctcgttcagccaggtcttgcatacggccgccagagcttccact tggtcaggcagtagtttgaagttcgcctttagatcgttatccacgtggtacttgtcca tcagcgcgcgcgcagcctccatgcccttctcccacgcagacacgatcggcaca ctcagcgggttcatcaccgtaatttcactttccgcttcgctgggctcttcctcttcctc ttgcgtccgcataccacgcgccactgggtcgtcttcattcagccgccgcactgtg cgcttacctcctttgccatgcttgattagcaccggtgggttgctgaaacccaccatt tgtagcgccacatcttctcttcttcctcgctgtccacgattacctctggtgatggcg ggcgctcgggcttgggagaagggcgcttcttttcttcttgggcgcaatggccaa atccgccgccgaggtcgatggccgcgggctgggtgtgcgcggcaccagcgc gtcttgtgatgagtcttcctcgtcctcggactcgatacgccgcctcatccgcttttt gggggcgcccggggaggcggcggcgacggggacggggacgacacgtcct ccatggttgggggacgtcgcgccgcaccgcgtccgcgctcgggggtggtttcg cgctgctcctcttcccgactggccatttccttctcctataggcagaaaaagatcata gtcagtcgagaagaaggacagcctaaccgcccctctgagttcgccaccaccg cctccaccgatgccgccaacgcgcctaccaccttccccgtcgaggcacccccg cttgaggaggaggaagtgattatcgagcaggacccaggttttgtaagcgaagac gacgaggaccgctcagtaccaacagaggataaaaagcaagaccaggacaac gcagaggcaaacgaggaacaagtcgggcgggggggacgaaaggcatggcga ctacctagatgtgggagacgacgtgctgttgaagcatctgcagcgccagtgcgc cattatctgcgacgcgttgcaagagcgcagcgatgtgcccctcgccatagcgga tgtcagccttgcctacgaacgccacctattctcaccgcgcgtaccccccaaacg ccaagaaaacggcacatgcgagcccaacccgcgcctcaacttctaccccgtat ttgccgtgccagaggtgcttgccacctatcacatctttttccaaaactgcaagatac ccctatcctgccgtgccaaccgcagccgagcggacaagcagctggccttgcg gcagggcgctgtcatacctgatatcgcctcgctcaacgaagtgccaaaaatcttt gagggtcttggacgcgacgagaagcgcgcggcaaacgctctgcaacaggaa aacagcgaaaatgaaagtcactctggagtgttggtggaactcgagggtgacaa cgcgcgcctagccgtactaaaacgcagcatcgaggtcacccactttgcctaccc ggcacttaacctacccccccaaggtcatgagcacagtcatgagtgagctgatcgt gcgccgtgcgcagcccctggagagggatgcaaatttgcaagaacaaacagag gagggcctacccgcagttggcgacgagcagctagcgcgctggcttcaaacgc gcgagcctgccgacttggaggagcgacgcaaactaatgatggccgcagtgctc gttaccgtggagcttgagtgcatgcagcggttctttgctgacccggagatgcagc gcaagctagaggaaacattgcactacacctttcgacagggctacgtacgccag gcctgcaagatctccaacgtggagctctgcaacctggtctcctaccttggaatttt gcacgaaaaccgccttgggcaaaacgtgcttcattccacgctcaagggcgagg cgcgccgcgactacgtccgcgactgcgtttacttatttctatgctacacctggcag acggccatgggcgtttggcagcagtgcttggaggagtgcaacctcaaggagct gcagaaactgctaaagcaaaacttgaaggacctatggacggccttcaacgagc gctccgtggccgcgcacctggcggacatcattttccccgaacgcctgcttaaaa ccctgcaacagggtctgccagacttcaccagtcaaagcatgttgcagaactttag gaactttatcctagagcgctcaggaatcttgcccgccacctgctgtgcacttccta |
| | | gcgactttgtgcccattaagtaccgcgaatgccctccgccgctttggggccactg ctaccttctgcagctagccaactaccttgcctaccactctgacataatggaagac gtgagcggtgacggtctactggagtgtcactgtcgctgcaacctatgcaccccg caccgctccctggtttgcaattcgcagctgcttaacgaaagtcaaattatcggtac ctttgagctgcagggtccctcgcctgacgaaaagtccgcggctccggggttgaa actcactccggggctgtggacgtcggcttaccttcgcaaatttgtacctgaggact accacgcccacgagattaggttctacgaagaccaatcccgcccgccaaatgcg gagcttaccgcctgcgtcattacccagggccacattcttggccaattgcaagcca tcaacaaagcccgccaagagtttctgctacgaaagggacgggggtttacttgg acccccagtccggcgaggagctcaacccaatcccccgccgccgcagcccta tcagcagcagccgcgggcccttgcttcccaggatcacccaaaagaagctgca gctgccgccgccacccacggacgaggaggaatactgggacagtcaggcaga ggaggttttggacgaggaggaggaggacatgatggaagactgggagagccta gacgaggaagcttccgaggtcgaagaggtgtcagacgaaacaccgtcaccct cggtcgcattcccctcgccggcgccccagaaatcggcaaccggttccagcatg gctacaacctccgctcctcaggcgccgccggcactgcccgttcgccgacccaa ccgtagatgggacaccactggaaccagggccggtaagtccaagcagccgcc gccgttagcccaagagcaacaacagcgccaaggctaccgctcatggcgcggg cacaagaacgccatagttgcttgcttgcaagactgtgggggcaacatctccttcg cccgccgctttcttctctaccatcacggcgtggccttcccccgtaacatcctgcatt actaccgtcatctctacagcccatactgcaccggcggcagcggcagcggcagc aacagcagcggccacacagaagcaaaggcgaccggatagcaagactctgac aaagcccaagaaatccacagcggcggcagcagcaggaggaggagcgctgc gtctggcgcccaacgaacccgtatcgacccgcgagcttagaaacaggatttttc ccactctgtatgctatatttcaacagagcaggggccaagaacaagagctgaaaa taaaaaacaggtctctgcgatccctcacccgcagctgcctgtatcacaaaagcg aagatcagcttcggcgcacgctggaagacgcggaggctctcttcagtaaatact gcgcgctgactcttaaggactagtttcgcgccctttctcaaatttaagcgcgaaaa ctacgtcatctccagcggccacacccggcgccagcacctgtcgtcagcgccattata gcaaggaaattcccacgccctacatgtggagttaccagccacaaatgggacttgcggc tggagctgcccaagactactcaacccgaataaactacatgagcgcgggaccccacat gatatcccgggtcaacggaatccgcgcccttcgaaaccgaattctcttggaacaggcg gctattaccaccacacctcgtaataaccttaatccccgtagttggcccgctgccctggtgt accaggaaagtcccgctcccaccactgtggtacttcccagagacgcccaggccgaag ttcagatgactaactcaggggcgcagcttgcgggcggctttcgtcacagggtgcggtcg cccgggcagggtataactcacctgacaatcagagggcgaggtattcagctcaacgac gagtcggtgagctcctcgcttggtctccgtccggacgggacatttcagatcggcggcgcc ggccgtccttcattcacgcctcgtcaggcaatcctaactctgcagacctcgtcctctgagc cgcgctctggaggcattggaactctgcaatttattgaggagtttgtgccatcggtctacttta acccctttctcgggacctcccggccactatccggatcaatttattcctaactttgacgcggta aaggactcggcggacggctacgactgaatgttaagtggagaggcagagcaactgcg cctgaaacacctggtccactgtcgccgccacaagtgctttgcccgcgactccggtgagtt ttgctactttgaattgcccgaggatcatatcgagggcccggcgcacggcgtccggcttac cgcccagggagagcttgcccgtagcctgattcgggagtttacccagcgcccccctgctag ttgagcgggacaggggaccctgtgttctcactgtgatttgcaactgtcctaaccttggatta catcaagatctttgttgccatctctgtgctgagtataataaatacagaaattaaaatatactg gggctcctatcgccatcctgtaaacgccaccgtcttcacccgcccaagcaaaccaagg cgaaccttacctggtacttttaacatctctccctctgtgatttacaacagtttcaacccagac |
| | | ggagtgagtctacgagagaacctctccgagctcagctactccatcagaaaaacacc accctccttacctgccgggaacgtacgagtgcgtcaccggccgctgcaccacacctac cgcctgaccgtaaaccagacttttccggacagacctcaataactctgtttaccagaaca ggaggtgagcttagaaaacccttagggtattaggccaaaggcgcagctactgtggggtt tatgaacaattcaagcaactctacggctattctaattcaggtttctctagaatcggggttg gggttattctctgtcttgtgattctctttattcttatactaacgcttctctgcctaaggctcgccgc ctgctgtgtgcacatttgcatttattgtcagctttttaaacgctggggtcgccacccaagatg attaggtacataatcctaggtttactcacccttgcgtcagcccacggtaccacccaaaag gtggattttaaggagccagcctgtaatgttacattcgcagctgaagctaatgagtgcacc actcttataaaatgcaccacagaacatgaaaagctgcttattcgccacaaaaacaaaat tggcaagtatgctgtttatgctatttggcagccaggtgacactacagagtataatgttacag ttttccagggtaaaagtcataaaacttttatgtatacttttccattttatgaaatgtgcgacatta ccatgtacatgagcaaacagtataagttgtggcccccacaaaattgtgtggaaaacact ggcactttctgctgcactgctatgctaattacagtgctcgctttggtctgtaccctactctatat taaatacaaaagcagacgcagctttattgaggaaaagaaaatgccttaatttactaagtt acaaagctaatgtcaccactaactgctttactcgctgcttgcaaaacaaattcaaaaagtt agcattataattagaataggatttaaaccccccggtcatttcctgctcaataccattcccct gaacaattgactctatgtgggatatgctccagcgctacaaccttgaagtcaggcttcctgg atgtcagcatctgactttggccagcacctgtcccgcggatttgttccagtccaactacagc gacccaccctaacagagatgaccaacacaaccaacgcggccgccgctaccggactt acatctaccacaaatacaccccaagtttctgcctttgtcaataactgggataacttgggca tgtggtggttctccatagcgcttatgtttgtatgccttattattatgtggctcatctgctgcctaa agcgcaaacgcgcccgaccacccatctatagtcccatcattgtgctacacccaaacaa tgatggaatccatagattggacggactgaaacacatgttcttttctcttacagtatgattaaa tgagacatgattcctcgagtttttatattactgacccttgttgcgctttttgtgcgtgctccacat tggctgcggtttctcacatcgaagtagactgcattccagccttcacagtctatttgctttacg gatttgtcaccctcacgctcatctgcagcctcatcactgtggtcatcgcctttatccagtgca ttgactgggtctgtgtgcgctttgcatatctcagacaccatccccagtacagggacagga ctatagctgagcttcttagaattctttaattatgaaatttactgtgacttttctgctgattatttgca ccctatctgcgttttgttccccgacctccaagcctcaaagacatatatcatgcagattcact cgtatatggaatattccaagttgctacaatgaaaaagcgatctttccgaagcctggttat atgcaatcatctctgttatggtgttctgcagtaccatcttagccctagctatatatccctacctt gacattggctggaaacgaatagatgccatgaaccacccaactttccccgcgcccgctat gcttccactgcaacaagttgttgccggcggctttgtcccagccaatcagcctcgcccact tctcccacccccactgaaatcagctactttaatctaacaggaggagatgactgacaccct agatctagaaatggacggaattattacagagcagcgcctgctagaaagacgcagggc agcggccgagcaacagcgcatgaatcaagagctccaagacatggttaacttgcacca gtgcaaaaggggtatcttttgtctggtaaagcaggccaaagtcacctacgacagtaata ccaccggacaccgccttagctacaagttgccaaccaagcgtcagaaattggtggtcat ggtgggagaaaagcccattaccataactcagcactcggtagaaaccgaaggctgcatt cactcaccttgtcaaggacctgaggatctctgcacccttattaagaccctgtgcggtctca aagatcttattccctttaactaataaaaaaaaataataaagcatcacttacttaaaatcagt tagcaaatttctgtccagtttattcagcagcacctccttgccctcctcccagctctggtattgc agcttcctcctggctgcaaactttctccacaatctaaatggaatgtcagtttcctcctgttcct gtccatccgcacccactatcttcatgttgttgcagataagcgcgcaagaccgtctgaaga taccttcaaccccgtgtatccatatgacacggaaaccggtcctccaactgtgccttttctta ctcctccctttgtatcccccaatgggtttcaagagagtcccccctggggtactctctttgcgcc tatccgaacctctagttacctccaatggcatgcttgcgctcaaaatgggcaacggcctctc |
| | | tctggacgaggccggcaaccttacctcccaaaatgtaaccactgtgagcccacctctca aaaaaaccaagtcaaacataaacctggaaatatctgcacccctcacagttacctcaga agccctaactgtggctgccgccgcacctctaatggtcgcgggcaacacactcaccatg caatcacaggccccgctaaccgtgcacgactccaaacttagcattgccacccaagga cccctcacagtgtcagaaggaaagctagccctgcaaacatcaggcccccctcaccacc atcgatagcagtacccttactatcactgcctcacccccctctaactactgccactggtagctt gggcattgacttgaaagagcccatttatacacaaaatggaaaactaggactaaagtac ggggctcctttgcatgtaacagacgacctaaacactttgaccgtagcaactggtccaggt gtgactattaataatacttccttgcaaactaaagttactggagccttgggttttgattcacaa ggcaatatgcaacttaatgtagcaggaggactaaggattgattctcaaaacagacgcct tatacttgatgttagttatccgtttgatgctcaaaaccaactaaatctaagactaggacagg gccctctttttataaactcagcccacaacttggatattaactacaacaaaggcctttacttgt ttacagcttcaaacaattccaaaaagcttgaggttaacctaagcactgccaaggggttg atgtttgacgctacagccatagccattaatgcaggagatgggcttgaatttggttcaccta atgcaccaaacacaaatcccctcaaaacaaaaattggccatggcctagaatttgattca aacaaggctatggttcctaaactaggaactggccttagttttgacagcacaggtgccatt acagtaggaaacaaaaataatgataagctaactttgtggaccacaccagctccatctcc taactgtagactaaatgcagagaaagatgctaaactcactttggtcttaacaaaatgtgg cagtcaaatacttgctacagtttcagttttggctgttaaaggcagtttggctccaatatctgg aacagttcaaagtgctcatcttattataagatttgacgaaaatggagtgctactaaacaatt ccttcctggacccagaatattggaactttagaaatggagatcttattgaaggcacagcct atacaaacgctgttggatttatgcctaacctatcagcttatccaaaatctcacggtaaaact gccaaaagtaacattgtcagtcaagtttacttaaacggagacaaaactaaacctgtaac actaaccattacactaaacggtacacaggaaacaggagacacaactccaagtgcata ctctatgtcattttcatgggactggtctggccacaactacatcgatgaaatatttgccacatc ctcttacactttttcatacattgcccaagaataaagaatcgtttgtgttatgtttcaacgtgttta tttttcaattgcagaaaatttcaagtcatttttcattcagtagtatagccccaccaccacatag cttatacagatcaccgtaccttaatcaaactcacagaaccctagtattcaacctgcca cctccctcccaacacacagagtacacagtcctttctccccggctggccttaaaaa gcatcatatcatgggtaacagacatattcttaggtgttatattccacacggtttcctg tcgagccaaacgctcatcagtgatattaataaactccccgggcagctcacttaag ttcatgtcgctgtccagctgctgagccacaggctgctgtccaacttgcggttgctt aacgggcggcgaaggagaagtccacgcctacatgggggtagagtcataatcg tgcatcaggatagggcggtggtgctgcagcagcgcgcgaataaactgctgccg ccgccgctccgtcctgcaggaatacaacatggcagtggtctcctcagcgatgatt cgcaccgcccgcagcataaggcgccttgtcctccgggcacagcagcgcaccc tgatctcacttaaatcagcacagtaactgcagcacagcaccacaatattgttcaaa atcccacagtgcaaggcgctgtatccaaagctcatggcggggaccacagaacc cacgtggccatcataccacaagcgcaggtagattaagtggcgacccctcataaa cacgctggacataaacattacctcttttggcatgttgtaattcaccacctcccggta ccatataaacctctgattaaacatggcgccatccaccaccatcctaaaccagctg gccaaaacctgcccgccggctatacactgcagggaaccgggactggaacaat gacagtggagagcccaggactcgtaaccatggatcatcatgctcgtcatgatatc aatgttggcacaacacaggcacacgtgcatacacttcctcaggattacaagctcc tcccgcgttagaaccatatcccagggaacaacccattcctgaatcagcgtaaatc ccacactgcagggaagacctcgcacgtaactcacgttgtgcattgtcaaagtgtt acattcgggcagcagcggatgatcctccagtatggtagcgcgggtttctgtctca aaaggaggtagacgatccctactgtacggagtgcgccgagacaaccgagatc |
| | | gtgttggtcgtagtgtcatgccaaatggaacgccggacgtagtcatatttcctgaa gcaaaaccaggtgcgggcgtgacaaacagatctgcgtctccggtctcgccgctt agatcgctctgtgtagtagttgtagtatatccactctctcaaagcatccaggcgcc ccctggcttcgggttctatgtaaactccttcatgcgccgctgccctgataacatcc accaccgcagaataagccacacccagccaacctacacattcgttctgcgagtca cacacgggaggagcgggaagagctggaagaaccatgtttttttttattccaaaa gattatccaaaacctcaaaatgaagatctattaagtgaacgcgctcccctccggt ggcgtggtcaaactctacagccaaagaacagataatggcatttgtaagatgttgc acaatggcttccaaaaggcaaacggccctcacgtccaagtggacgtaaaggct aaacccttcagggtgaatctcctctataaacattccagcaccttcaaccatgccca aataattctcatctcgccaccttctcaatatatctctaagcaaatcccgaatattaag tccggccattgtaaaaatctgctccagagcgccctccaccttcagcctcaagcag cgaatcatgattgcaaaaattcaggttcctcacagacctgtataagattcaaaagc ggaacattaacaaaaataccgcgatcccgtaggtcccttcgcagggccagctg aacataatcgtgcaggtctgcacggaccagcgcggccacttccccgccaggaa ccatgacaaaagaacccacactgattatgacacgcatactcggagctatgctaa ccagcgtagccccgatgtaagcttgttgcatgggcggcgatataaaatgcaagg tgctgctcaaaaaatcaggcaaagcctcgcgcaaaaaagaaagcacatcgtag tcatgctcatgcagataaaggcaggtaagctccggaaccaccacagaaaaaga caccatttttctctcaaacatgtctgcgggtttctgcataaacacaaaataaaataa caaaaaaacatttaaacattagaagcctgtcttacaacaggaaaaacaaccctta taagcataagacggactacggccatgccggcgtgaccgtaaaaaaactggtca ccgtgattaaaaagcaccaccgacagctcctcggtcatgtccggagtcataatgt aagactcggtaaacacatcaggttgattcacatcggtcagtgctaaaaagcgac cgaaatagcccgggggaatacatacccgcaggcgtagagacaacattacagc cccataggaggtataacaaaattaataggagagaaaaacacataaacacctg aaaaaccctcctgcctaggcaaaatagcaccctcccgctccagaacaacatac agcgcttccacagcggcagccataacagtcagccttaccagtaaaaaagaaaacc tattaaaaaaacaccactcgacacggcaccagctcaatcagtcacagtgtaaaaag ggccaagtgcagagcgagtatatataggactaaaaaatgacgtaacggttaaagtcca caaaaaacacccagaaaaccgcacgcgaacctacgcccagaaacgaaagccaaa aaacccacaacttcctcaaatcgtcacttccgttttcccacgttacgtaacttcccattttaa gaaaactacaattcccaacacatacaagttactccgccctaaaacctacgtcacccg ccccgttcccacgccccgcgccacgtcacaaactccacccctcattatcatatt ggcttcaatccaaaataaggtatattattgatgatgatttaaatcagtcgcgatcg |
| 2 | HAD2 | atcgaagtactcggggaaatgtgcgcggaacccctatttgtttatttttctaaatacattcaa atatgtatccgctcatgagacaataaccctgataaatgcttcaataatattgaaaaagga agagtatgagccatattcaacgggaaacgtcgaggccgcgattaaattccaacatgga tgctgatttatatgggtataaatgggctcgcgataatgtcgggcaatcaggtgcgacaatc tatcgcttgtatgggaagcccgatgcgccagagttgtttctgaaacatggcaaaggtagc gttgccaatgatgttacagatgagatggtcagactaaactggctgacggaatttatgcctc ttccgaccatcaagcattttatccgtactcctgatgatgcatggttactcaccactgcgatcc ccggaaaaacagcattccaggtattagaagaatatcctgattcaggtgaaaatattgttg atgcgctggcagtgttcctgcgccggttgcattcgattcctgtttgtaattgtccttttaacagc gatcgcgtatttcgtctcgctcaggcgcaatcacgaatgaataacggtttggttgatgcga gtgattttgatgacgagcgtaatggctggcctgttgaacaagtctggaaagaaatgcata aacttttgccattctcaccggattcagtcgtcactcatggtgatttctcacttgataaccttattt ttgacgaggggaaattaataggttgtattgatgttggacgagtcggaatcgcagaccgat |
| | | accaggatcttgccatcctatggaactgcctcggtgagttttctccttcattacagaaacgg ctttttcaaaaatatggtattgataatcctgatatgaataaattgcagtttcatttgatgctcga tgagtttttctaactgtcagaccaagtttactcatatatactttagattgatttaaaacttcattttt aatttaaaaggatctaggtgaagatcctttttgataatctcatgaccaaaatcccttaacgt gagttttcgttccactgagcgtcagaccccgtagaaaagatcaaaggatcttcttgagatc ctttttttctgcgcgtaatctgctgcttgcaaacaaaaaaaccaccgctaccagcggtggtt tgtttgccggatcaagagctaccaactctttttccgaaggtaactggcttcagcagagcgc agataccaaatactgttcttctagtgtagccgtagttaggccaccacttcaagaactctgta gcaccgcctacatacctcgctctgctaatcctgttaccagtggctgctgccagtggcgata agtcgtgtcttaccgggttggactcaagacgatagttaccggataaggcgcagcggtcg ggctgaacggggggttcgtgcacacagcccagcttggagcgaacgacctacaccga actgagatacctacagcgtgagctatgagaaagcgccacgcttcccgaagggagaaa ggcggacaggtatccggtaagcggcagggtcggaacaggagagcgcacgaggga gcttccagggggaaacgcctggtatctttatagtcctgtcgggtttcgccacctctgacttg agcgtcgatttttgtgatgctcgtcaggggggcggagcctatggaaaaacgccagcaa cgcggcctttttacggttcctggccttttttcctgcagcccgggggatccgattaattaaga cgtttaaacaataaaatatctttattttcattacatctgtgtgttggttttttgtgtgtcgac gagatcttccaacataaggcgatgatatccgtagatgtacctggacatcaggtgatgcc ggcggcggtggtggaggcgcgcggaaagtcgcggacgcggttccagatgttgcgca gcggcaaaaagtgctccatggtcgggacgctctggccggtcaggcgcgcgca atcgttgacgctctagaccgtgcaaaaggagagcctgtaagcgggcactcttcc gtggtctggtggataaattcgcaagggtatcatggcggacgaccggggttcgag ccccgtatccggccgtccgccgtgatccatgcggttaccgcccgcgtgtcgaac ccaggtgtgcgacgtcagacaacgggggagtgctccttttggcttccttccaggc gcggcggctgctgcgctagcttttttggccactggccgcgcgcagcgtaagcg gttaggctggaaagcgaaagcattaagtggctcgctccctgtagccggagggtt attttccaagggttgagtcgcgggacccccggttcgagtctcggaccggccgga ctgcggcgaacgggggtttgcctccccgtcatgcaagaccccgcttgcaaattc ctccggaaacagggacgagcccctttttttgcttttcccagatcatccggtgctgcgg cagatgcgcccccctcctcagcagcggcaagagcaagagcagcggcagacatgca gggcaccctccatgcatccactttatttgtcacttgaaaaacatgtaaaaataaatgtact agagacactttcaataaaggcaaatgcttttatttgtacactctcgggtgattatttac cccccacccttgccgtctgcgccgtttaaaaatcaaaggggttctgccgcgcatcg ctatgcgccactggcagggacacgttgcgatactggtgtttagtgctccacttaaa ctcaggcacaaccatccgcggcagctcggtgaagttttcactccacaggctgcg caccatcaccaacgcgtttagcaggtcgggcgccgatatcttgaagtcgcagtt ggggcctccgccctgcgcgcgcgagttgcgatacacagggttgcagcactgg aacactatcagcgccgggtggtgcacgctggccagcacgctcttgtcggagat cagatccgcgtccaggtcctccgcgttgctcagggcgaacggagtcaactttgg tagctgccttcccaaaaagggcgcgtgcccaggctttgagttgcactcgcaccg tagtggcatcaaaaggtgaccgtgcccggtctgggcgttaggatacagcgcctg cataaaagccttgatctgcttaaaagccacctgagcctttgcgccttcagagaag aacatgccgcaagacttgccggaaaactgattggccggacaggccgcgtcgtg cacgcagcaccttgcgtcggtgttggagatctgcaccacatttcggccccaccg gttcttcacgatcttggccttgctagactgctccttcagcgcgcgctgcccgttttc gctcgtcacatccatttcaatcacgtgctccttatttatcataatgcttccgtgtagac acttaagctcgccttcgatctcagcgcagcggtgcagccacaacgcgcagccc gtgggctcgtgatgcttgtaggtcacctctgcaaacgactgcaggtacgcctgca |
| | | ggaatcgccccatcatcgtcacaaaggtcttgttgctggtgaaggtcagctgcaa cccgcggtgctcctcgttcagccaggtcttgcatacggccgccagagcttccact tggtcaggcagtagtttgaagttcgcctttagatcgttatccacgtggtacttgtcca tcagcgcgcgcgcagcctccatgcccttctcccacgcagacacgatcggcaca ctcagcgggttcatcaccgtaatttcactttccgcttcgctgggctcttcctcttcctc ttgcgtccgcataccacgcgccactgggtcgtcttcattcagccgccgcactgtg cgcttacctcctttgccatgcttgattagcaccggtgggttgctgaaacccaccatt tgtagcgccacatcttctctttcttcctcgctgtccacgattacctctggtgatggcg ggcgctcgggcttgggagaagggcgcttctttttcttcttgggcgcaatggccaa atccgccgccgaggtcgatggccgcgggctgggtgtgcgcggcaccagcgc gtcttgtgatgagtcttcctcgtcctcggactcgatacgccgcctcatccgcttttt gggggcgcccggggaggcggcggcgacggggacggggacgacacgtcct ccatggttgggggacgtcgcgccgcaccgcgtccgcgctcgggggtggtttcg cgctgctcctcttcccgactggccatttccttctcctataggcagaaaaagatcata gtcagtcgagaagaaggacagcctaaccgcccctctgagttcgccaccaccg cctccaccgatgccgccaacgcgcctaccaccttccccgtcgaggcacccccg cttgaggaggaggaagtgattatcgagcaggacccaggttttgtaagcgaagac gacgaggaccgctcagtaccaacagaggataaaaagcaagaccaggacaac gcagaggcaaacgaggaacaagtcgggcgggggggacgaaaggcatggcga ctacctagatgtgggagacgacgtgctgttgaagcatctgcagcgccagtgcgc cattatctgcgacgcgttgcaagagcgcagcgatgtgcccctcgccatagcgga tgtcagccttgcctacgaacgccacctattctcaccgcgcgtaccccccaaacg ccaagaaaacggcacatgcgagcccaacccgcgcctcaacttctaccccgtat ttgccgtgccagaggtgcttgccacctatcacatctttttccaaaactgcaagatac ccctatcctgccgtgccaaccgcagccgagcggacaagcagctggccttgcg gcagggcgctgtcatacctgatatcgcctcgctcaacgaagtgccaaaaatcttt gagggtcttggacgcgacgagaagcgcgcggcaaacgctctgcaacaggaa aacagcgaaaatgaaagtcactctggagtgttggtggaactcgagggtgacaa cgcgcgcctagccgtactaaaacgcagcatcgaggtcacccactttgcctaccc ggcacttaacctacccccccaaggtcatgagcacagtcatgagtgagctgatcgt gcgccgtgcgcagcccctggagagggatgcaaatttgcaagaacaaacagag gagggcctacccgcagttggcgacgagcagctagcgcgctggcttcaaacgc gcgagcctgccgacttggaggagcgacgcaaactaatgatggccgcagtgctc gttaccgtggagcttgagtgcatgcagcggttctttgctgacccggagatgcagc gcaagctagaggaaacattgcactacacctttcgacagggctacgtacgccag gcctgcaagatctccaacgtggagctctgcaacctggtctcctaccttggaatttt gcacgaaaaccgccttgggcaaaacgtgcttcattccacgctcaagggcgagg cgcgccgcgactacgtccgcgactgcgtttacttatttctatgctacacctggcag acggccatgggcgtttggcagcagtgcttggaggagtgcaacctcaaggagct gcagaaactgctaaagcaaaacttgaaggacctatggacggccttcaacgagc gctccgtggccgcgcacctggcggacatcattttccccgaacgcctgcttaaaa ccctgcaacagggtctgccagacttcaccagtcaaagcatgttgcagaactttag gaactttatcctagagcgctcaggaatcttgcccgccacctgctgtgcacttccta gcgactttgtgcccattaagtaccgcgaatgccctccgccgctttggggccactg ctaccttctgcagctagccaactaccttgcctaccactctgacataatggaagac gtgagcggtgacggtctactggagtgtcactgtcgctgcaacctatgcaccccg caccgctccctggtttgcaattcgcagctgcttaacgaaagtcaaattatcggtac ctttgagctgcagggtccctcgcctgacgaaaagtccgcggctccggggttgaa |
| | | actcactccggggctgtggacgtcggcttaccttcgcaaatttgtacctgaggact accacgcccacgagattaggttctacgaagaccaatcccgcccgccaaatgcg gagcttaccgcctgcgtcattacccagggccacattcttggccaattgcaagcca tcaacaaagcccgccaagagtttctgctacgaaagggacgggggtttacttgg acccccagtccggcgaggagctcaacccaatccccccgccgccgcagcccta tcagcagcagccgcgggcccttgcttcccaggatcacccaaaaagaagctgca gctgccgccgccacccacggacgaggaggaatactgggacagtcaggcaga ggaggttttggacgaggaggaggaggacatgatggaagactgggagagccta gacgaggaagcttccgaggtcgaagaggtgtcagacgaaacaccgtcaccct cggtcgcattcccctcgccggcgccccagaaatcggcaaccggttccagcatg gctacaacctccgctcctcaggcgccgccggcactgcccgttcgccgacccaa ccgtagatgggacaccactggaaccagggccggtaagtccaagcagccgcc gccgttagcccaagagcaacaacagcgccaaggctaccgctcatggcgcggg cacaagaacgccatagttgcttgcttgcaagactgtgggggcaacatctccttcg cccgccgctttcttctctaccatcacggcgtggccttcccccgtaacatcctgcatt actaccgtcatctctacagcccatactgcaccggcggcagcggcagcggcagc aacagcagcggccacacagaagcaaaggcgaccggatagcaagactctgac aaagcccaagaaatccacagcggcggcagcagcaggaggaggagcgctgc gtctggcgcccaacgaacccgtatcgacccgcgagcttagaaacaggatttttc ccactctgtatgctatatttcaacagagcaggggccaagaacaagagctgaaaa taaaaaacaggtctctgcgatccctcacccgcagctgcctgtatcacaaaagcg aagatcagcttcggcgcacgctggaagacgcggaggctctcttcagtaaatact gcgcgctgactcttaaggactagtttcgcgccctttctcaaatttaagcgcgaaaa ctacgtcatctccagcggccacacccggcgccagcacctgtcgtcagcgccattata gcaaggaaattcccacgccctacatgtggagttaccagccacaaatgggacttgcggc tggagctgcccaagactactcaacccgaataaactacatgagcgcgggaccccacat gatatcccgggtcaacggaatccgcgcccttcgaaaccgaattctcttggaacaggcg gctattaccaccacacctcgtaataaccttaatccccgtagttggcccgctgccctggtgt accaggaaagtcccgctcccaccactgtggtacttcccagagacgcccaggccgaag ttcagatgactaactcaggggcgcagcttgcgggcggctttcgtcacagggtgcggtcg cccgggcagggtataactcacctgacaatcagagggcgaggtattcagctcaacgac gagtcggtgagctcctcgcttggtctccgtccggacgggacatttcagatcggcggcgcc ggccgtccttcattcacgcctcgtcaggcaatcctaactctgcagacctcgtcctctgagc cgcgctctggaggcattggaactctgcaatttattgaggagtttgtgccatcggtctacttta acccccttctcgggacctcccggccactatccggatcaatttattcctaactttgacgcggta aaggactcggcggacggctacgactgaatgttaagtggagaggcagagcaactgcg cctgaaacacctggtccactgtcgccgccacaagtgctttgcccgcgactccggtgagtt ttgctactttgaattgcccgaggatcatatcgagggcccggcgcacggcgtccggcttac cgcccagggagagcttgcccgtagcctgattcgggagtttacccagcgcccccctgctag ttgagcgggacaggggaccctgtgttctcactgtgatttgcaactgtcctaaccttggatta catcaagatctttgttgccatctctgtgctgagtataataaatacagaaattaaaatatactg gggctcctatcgccatcctgtaaacgccaccgtcttcacccgcccaagcaaaccaagg cgaaccttacctggtacttttaacatctctccctctgtgatttacaacagtttcaacccagac ggagtgagtctacgagagaacctctccgagctcagctactccatcagaaaaaacacc accctccttacctgccgggaacgtacgagtgcgtcaccggccgctgcaccacacctac cgcctgaccgtaaaccagactttttccggacagacctcaataactctgtttaccagaaca ggaggtgagcttagaaaacccttagggtattaggccaaaggcgcagctactgtggggtt tatgaacaattcaagcaactctacgggctattctaattcaggtttctctagaatcggggttg |
| | | gggttattctctgtcttgtgattctctttattcttatactaacgcttctctgcctaaggctcgccgc ctgctgtgtgcacatttgcatttattgtcagctttttaaacgctggggtcgccacccaagatg attaggtacataatcctaggtttactcacccttgcgtcagcccacggtaccacccaaaag gtggattttaaggagccagcctgtaatgttacattcgcagctgaagctaatgagtgcacc actcttataaaatgcaccacagaacatgaaaagctgcttattcgccacaaaaacaaaat tggcaagtatgctgtttatgctatttggcagccaggtgacactacagagtataatgttacag ttttccagggtaaaagtcataaaacttttatgtatacttttccattttatgaaatgtgcgacatta ccatgtacatgagcaaacagtataagttgtggcccccacaaaattgtgtggaaaacact ggcactttctgctgcactgctatgctaattacagtgctcgctttggtctgtaccctactctatat taaatacaaaagcagacgcagctttattgaggaaaagaaaatgccttaatttactaagtt acaaagctaatgtcaccactaactgctttactcgctgcttgcaaaacaaattcaaaaagtt agcattataattagaataggatttaaacccccggtcatttcctgctcaataccattcccct gaacaattgactctatgtgggatatgctccagcgctacaaccttgaagtcaggcttcctgg atgtcagcatctgactttggccagcacctgtcccgcggatttgttccagtccaactacagc gacccaccctaacagagatgaccaacacaaccaacgcggccgccgctaccggactt acatctaccacaaatacaccccaagtttctgcctttgtcaataactgggataacttgggca tgtggtggttctccatagcgcttatgtttgtatgccttattattatgtggctcatctgctgcctaa agcgcaaacgcgcccgaccacccatctatagtcccatcattgtgctacacccaaacaa tgatggaatccatagattggacggactgaaacacatgttcttttctcttacagtatgattaaa tgagacatgattcctcgagtttttatattactgacccttgttgcgctttttgtgcgtgctccacat tggctgcggtttctcacatcgaagtagactgcattccagccttcacagtctatttgctttacg gatttgtcaccctcacgctcatctgcagcctcatcactgtggtcatcgcctttatccagtgca ttgactgggtctgtgtgcgctttgcatatctcagacaccatccccagtacagggacagga ctatagctgagcttcttagaattctttaattatgaaatttactgtgacttttctgctgattatttgca ccctatctgcgttttgttccccgacctccaagcctcaaagacatatatcatgcagattcact cgtatatggaatattccaagttgctacaatgaaaaaagcgatctttccgaagcctggttat atgcaatcatctctgttatggtgttctgcagtaccatcttagccctagctatatatccctacctt gacattggctggaaacgaatagatgccatgaaccacccaactttccccgcgcccgctat gcttccactgcaacaagttgttgccggcggctttgtcccagccaatcagcctcgcccact tctcccacccccactgaaatcagctactttaatctaacaggaggagatgactgacaccct agatctagaaatggacggaattattacagagcagcgcctgctagaaagacgcagggc agcggccgagcaacagcgcatgaatcaagagctccaagacatggttaacttgcacca gtgcaaaaggggtatcttttgtctggtaaagcaggccaaagtcacctacgacagtaata ccaccggacaccgccttagctacaagttgccaaccaagcgtcagaaattggtggtcat ggtgggagaaaagcccattaccataactcagcactcggtagaaaccgaaggctgcatt cactcaccttgtcaaggacctgaggatctctgcacccttattaagaccctgtgcggtctca aagatcttattccctttaactaataaaaaaaaataataaagcatcacttacttaaaatcagt tagcaaatttctgtccagtttattcagcagcacctccttgccctcctcccagctctggtattgc agcttcctcctggctgcaaactttctccacaatctaaatggaatgtcagtttcctcctgttcct gtccatccgcacccactatcttcatgttgttgcagataagcgcgcaagaccgtctgaaga taccttcaaccccgtgtatccatatgacacggaaaccggtcctccaactgtgccttttctta ctcctccctttgtatcccccaatgggtttcaagagagtcccccctggggtactctctttgcgcc tatccgaacctctagttacctccaatggcatgcttgcgctcaaaatgggcaacggcctctc tctggacgaggccggcaaccttacctcccaaaatgtaaccactgtgagcccacctctca aaaaaaccaagtcaaacataaacctggaaatatctgcacccctcacagttacctcaga agccctaactgtggctgccgccgcacctctaatggtcgcgggcaacacactcaccatg caatcacaggccccgctaaccgtgcacgactccaaacttagcattgccacccaagga cccctcacagtgtcagaaggaaagctagccctgcaaacatcaggcccccctcaccacc |
| | | atcgatgaaatatttgccacatcctcttacactttttcatacattgcccaagaataaagaatc gtttgtgttatgtttcaacgtgtttatttttcaattgcagaaaatttcaagtcatttttcattcagta gtatagccccaccaccacatagcttatacagatcaccgtaccttaatcaaactcacaga accctagtattcaacctgccacctccctcccaacacacagagtacacagtcctttt ctccccggctggccttaaaaagcatcatatcatgggtaacagacatattcttaggt gttatattccacacggtttcctgtcgagccaaacgctcatcagtgatattaataaac tccccgggcagctcacttaagttcatgtcgctgtccagctgctgagccacaggct gctgtccaacttgcggttgcttaacgggcggcgaaggagaagtccacgcctac atgggggtagagtcataatcgtgcatcaggatagggcggtggtgctgcagcag cgcgcgaataaactgctgccgccgctccgtcctgcaggaatacaacatgg cagtggtctcctcagcgatgattcgcaccgcccgcagcataaggcgccttgtcct ccgggcacagcagcgcaccctgatctcacttaaatcagcacagtaactgcagc acagcaccacaatattgttcaaaatcccacagtgcaaggcgctgtatccaaagct catggcggggaccacagaacccacgtggccatcataccacaagcgcaggtag attaagtggcgacccctcataaacacgctggacataaacattacctcttttggcat gttgtaattcaccacctcccggtaccatataaacctctgattaaacatggcgccat ccaccaccatcctaaaccagctggccaaaacctgcccgccggctatacactgc agggaaccgggactggaacaatgacagtggagagcccaggactcgtaaccat ggatcatcatgctcgtcatgatatcaatgttggcacaacacaggcacacgtgcat acacttcctcaggattacaagctcctcccgcgttagaaccatatcccagggaaca acccattcctgaatcagcgtaaatcccacactgcagggaagacctcgcacgtaa ctcacgttgtgcattgtcaaagtgttacattcgggcagcagcggatgatcctccag tatggtagcgcgggtttctgtctcaaaaggaggtagacgatccctactgtacgga gtgcgccgagacaaccgagatcgtgttggtcgtagtgtcatgccaaatggaacg ccggacgtagtcatatttcctgaagcaaaaccaggtgcgggcgtgacaaacag atctgcgtctccggtctcgccgcttagatcgctctgtgtagtagttgtagtatatcca ctctctcaaagcatccaggcgccccctggcttcgggttctatgtaaactccttcat gcgccgctgccctgataacatccaccaccgcagaataagccacacccagcca acctacacattcgttctgcgagtcacacacgggaggagcgggaagagctggaa gaaccatgtttttttttattccaaagattatccaaaacctcaaaatgaagatctatt aagtgaacgcgctcccctccggtggcgtggtcaaactctacagccaaagaaca gataatggcatttgtaagatgttgcacaatggcttccaaaaggcaaacggccctc acgtccaagtggacgtaaaggctaaacccttcagggtgaatctcctctataaaca ttccagcaccttcaaccatgcccaaataattctcatctcgccaccttctcaatatatc tctaagcaaatcccgaatattaagtccggccattgtaaaaatctgctccagagcg ccctccaccttcagcctcaagcagcgaatcatgattgcaaaaattcaggttcctca cagacctgtataagattcaaaagcggaacattaacaaaaataccgcgatcccgt aggtcccttcgcagggccagctgaacataatcgtgcaggtctgcacggaccag cgcggccacttccccgccaggaaccatgacaaaagaacccacactgattatga cacgcatactcggagctatgctaaccagcgtagccccgatgtaagcttgttgcat gggcggcgatataaaatgcaaggtgctgctcaaaaaatcaggcaaagcctcgc gcaaaaaagaaagcacatcgtagtcatgctcatgcagataaaggcaggtaagc tccggaaccaccacagaaaaagacaccatttttctctcaaacatgtctgcgggttt ctgcataaacacaaaataaaataacaaaaaaacatttaaacattagaagcctgtct tacaacaggaaaaacaacccttataagcataagacggactacggccatgccgg cgtgaccgtaaaaaaactggtcaccgtgattaaaaagcaccaccgacagctcct cggtcatgtccggagtcataatgtaagactcggtaaacacatcaggttgattcac atcggtcagtgctaaaaagcgaccgaaatagcccgggggaatacatacccgca |
| | | ggcgtagagacaacattacagcccccataggaggtataacaaattaatagga gagaaaaacacataaacacctgaaaaaccctcctgcctaggcaaaatagcacc ctcccgctccagaacaacatacagcgcttccacagcggcagccataacagtca gccttaccagtaaaaaagaaaacctattaaaaaaacaccactcgacacggcaccag ctcaatcagtcacagtgtaaaaaagggccaagtgcagagcgagtatatataggactaa aaaatgacgtaacggttaaagtccacaaaaaacacccagaaaaccgcacgcgaac ctacgcccagaaacgaaagccaaaaaacccacaacttcctcaaatcgtcacttccgttt tcccacgttacgtaacttcccattttaagaaaactacaattcccaacacatacaagttactc cgccctaaaacctacgtcacccgccccgttcccacgccccgcgccacgtcaca aactccacccccctcattatcatattggcttcaatccaaaataaggtatattattgatg atgatttaaatcagtcgcgatcg |
| 3 | HAD3 | atcgaagtactcggggaaatgtgcgcggaacccctatttgtttatttttctaaatacattcaa atatgtatccgctcatgagacaataaccctgataaatgcttcaataatattgaaaaagga agagtatgagccatattcaacgggaaacgtcgaggccgcgattaaattccaacatgga tgctgatttatatgggtataaatgggctcgcgataatgtcgggcaatcaggtgcgacaatc tatcgcttgtatgggaagcccgatgcgccagagttgtttctgaaacatggcaaaggtagc gttgccaatgatgttacagatgagatggtcagactaaactggctgacggaatttatgcctc ttccgaccatcaagcattttatccgtactcctgatgatgcatggttactcaccactgcgatcc ccggaaaaacagcattccaggtattagaagaatatcctgattcaggtgaaaatattgttg atgcgctggcagtgttcctgcgccggttgcattcgattcctgtttgtaattgtccttttaacagc gatcgcgtatttcgtctcgctcaggcgcaatcacgaatgaataacggtttggttgatgcga gtgattttgatgacgagcgtaatggctggcctgttgaacaagtctggaaagaaatgcata aacttttgccattctcaccggattcagtcgtcactcatggtgatttctcacttgataaccttattt ttgacgaggggaaattaataggttgtattgatgttggacgagtcggaatcgcagaccgat accaggatcttgccatcctatggaactgcctcggtgagttttctccttcattacagaaacgg ctttttcaaaaatatggtattgataatcctgatatgaataaattgcagtttcatttgatgctcga tgagtttttctaactgtcagaccaagtttactcatatatactttagattgatttaaaacttcattttt aatttaaaaggatctaggtgaagatcctttttgataatctcatgaccaaaatcccttaacgt gagttttcgttccactgagcgtcagaccccgtagaaaagatcaaaggatcttcttgagatc ctttttttctgcgcgtaatctgctgcttgcaaacaaaaaaaccaccgctaccagcggtggtt tgtttgccggatcaagagctaccaactctttttccgaaggtaactggcttcagcagagcgc agataccaaatactgttcttctagtgtagccgtagttaggccaccacttcaagaactctgta gcaccgcctacatacctcgctctgctaatcctgttaccagtggctgctgccagtggcgata agtcgtgtcttaccgggttggactcaagacgatagttaccggataaggcgcagcggtcg ggctgaacggggggttcgtgcacacagcccagcttggagcgaacgacctacaccga actgagatacctacagcgtgagctatgagaaagcgccacgcttcccgaagggagaaa ggcggacaggtatccggtaagcggcagggtcggaacaggagagcgcacgaggga gcttccagggggaaacgcctggtatctttatagtcctgtcgggtttcgccacctctgacttg agcgtcgatttttgtgatgctcgtcaggggggcggagcctatggaaaaacgccagcaa cgcggcctttttacggttcctggccttttttcctgcagcccgggggatccgattaattaaga cgtttaaacaataaaatatctttattttcattacatctgtgtgttggttttttgtgtgtcgac gagatcttccaacataaggcgatgatatccgtagatgtacctggacatcaggtgatgcc ggcggcggtggtggaggcgcgcggaaagtcgcggacgcggttccagatgttgcgca gcggcaaaaagtgctccatggtcggacgctctggccggtcaggcgcgcgca atcgttgacgctctagaccgtgcaaaaggagagcctgtaagcgggcactcttcc gtggtctggtggataaattcgcaagggtatcatggcggacgaccggggttcgag ccccgtatccggccgtccgccgtgatccatgcggttaccgcccgcgtgtcgaac ccaggtgtgcgacgtcagacaacgggggagtgctccttttggcttccttccaggc |
| | | gcggcggctgctgcgctagctttttggccactggccgcgcagcgtaagcg gttaggctggaaagcgaaagcattaagtggctcgctccctgtagccggagggtt attttccaaggttgagtcgcgggacccccggttcgagtctcggaccggccgga ctgcggcgaacgggggtttgcctccccgtcatgcaagaccccgcttgcaaattc ctccggaaacagggacgagcccctttttgcttttcccagatcatccggtgctgcgg cagatgcgcccccctcctcagcagcggcaagagcaagagcagcggcagacatgca gggcaccctccatgcatccactttattttgtcacttgaaaaacatgtaaaataaatgtact agagacactttcaataaaggcaaatgcttttatttgtacactctcgggtgattatttac cccccacccttgccgtctgcgccgtttaaaaatcaaaggggttctgccgcgcatcg ctatgcgccactggcagggacacgttgcgatactggtgtttagtgctccacttaaa ctcaggcacaaccatccgcggcagctcggtgaagttttcactccacaggctgcg caccatcaccaacgcgtttagcaggtcgggcgccgatatcttgaagtcgcagtt ggggcctccgccctgcgcgcgcgagttgcgatacacagggttgcagcactgg aacactatcagcgccgggtggtgcacgctggccagcacgctcttgtcggagat cagatccgcgtccaggtcctccgcgttgctcagggcgaacggagtcaactttgg tagctgccttcccaaaaagggcgcgtgcccaggctttgagttgcactcgcaccg tagtggcatcaaaaggtgaccgtgcccggtctgggcgttaggatacagcgcctg cataaaagccttgatctgcttaaaagccacctgagcctttgcgccttcagagaag aacatgccgcaagacttgccggaaaactgattggccggacaggccgcgtcgtg cacgcagcaccttgcgtcggtgttggagatctgcaccacatttcggccccaccg gttcttcacgatcttggccttgctagactgctccttcagcgcgcgctgcccgttttc gctcgtcacatccatttcaatcacgtgctccttatttatcataatgcttccgtgtagac acttaagctcgccttcgatctcagcgcagcggtgcagccacaacgcgcagccc gtgggctcgtgatgcttgtaggtcacctctgcaaacgactgcaggtacgcctgca ggaatcgccccatcatcgtcacaaaggtcttgttgctggtgaaggtcagctgcaa cccgcggtgctcctcgttcagccaggtcttgcatacggccgccagagcttccact tggtcaggcagtagtttgaagttcgcctttagatcgttatccacgtggtacttgtcca tcagcgcgcgcgcagcctccatgcccttctcccacgcagacacgatcggcaca ctcagcgggttcatcaccgtaatttcactttccgcttcgctgggctcttcctcttcctc ttgcgtccgcataccacgcgccactgggtcgtcttcattcagccgccgcactgtg cgcttacctcctttgccatgcttgattagcaccggtgggttgctgaaacccaccatt tgtagcgccacatcttctctttcttcctcgctgtccacgattacctctggtgatggcg ggcgctcgggcttgggagaagggcgcttctttttcttcttgggcgcaatggccaa atccgccgccgaggtcgatggccgcgggctgggtgtgcgcggcaccagcgc gtcttgtgatgagtcttcctcgtcctcggactcgatacgccgcctcatccgcttttt gggggcgcccggggaggcggcggcgacggggacggggacgacacgtcct ccatggttgggggacgtcgcgccgcaccgcgtccgcgctcgggggtggtttcg cgctgctcctcttcccgactggccatttccttctcctataggcagaaaaagatcata gtcagtcgagaagaaggacagcctaaccgccccctctgagttcgccaccaccg cctccaccgatgccgccaacgcgcctaccaccttccccgtcgaggcacccccg cttgaggaggaggaagtgattatcgagcaggacccaggttttgtaagcgaagac gacgaggaccgctcagtaccaacagaggataaaaagcaagaccaggacaac gcagaggcaaacgaggaacaagtcgggcgggggggacgaaaggcatggcga ctacctagatgtgggagacgacgtgctgttgaagcatctgcagcgccagtgcgc cattatctgcgacgcgttgcaagagcgcagcgatgtgcccctcgccatagcgga tgtcagccttgcctacgaacgccacctattctcaccgcgcgtaccccccaaacg ccaagaaaacggcacatgcgagcccaacccgcgcctcaacttctaccccgtat ttgccgtgccagaggtgcttgccacctatcacatctttttccaaaactgcaagatac |
| | | ccctatcctgccgtgccaaccgcagccgagcggacaagcagctggccttgcg gcagggcgctgtcatacctgatatcgcctcgctcaacgaagtgccaaaaatcttt gagggtcttggacgcgacgagaagcgcgcggcaaacgctctgcaacaggaa aacagcgaaaatgaaagtcactctggagtgttggtggaactcgagggtgacaa cgcgcgcctagccgtactaaaacgcagcatcgaggtcacccactttgcctaccc ggcacttaacctacccccaaggtcatgagcacagtcatgagtgagctgatcgt gcgccgtgcgcagcccctggagagggatgcaaatttgcaagaacaaacagag gagggcctacccgcagttggcgacgagcagctagcgcgctggcttcaaacgc gcgagcctgccgacttggaggagcgacgcaaactaatgatggccgcagtgctc gttaccgtggagcttgagtgcatgcagcggttctttgctgacccggagatgcagc gcaagctagaggaaacattgcactacacctttcgacagggctacgtacgccag gcctgcaagatctccaacgtggagctctgcaacctggtctcctaccttggaatttt gcacgaaaaccgccttgggcaaaacgtgcttcattccacgctcaagggcgagg cgcgccgcgactacgtccgcgactgcgtttacttatttctatgctacacctggcag acggccatgggcgtttggcagcagtgcttggaggagtgcaacctcaaggagct gcagaaactgctaaagcaaaacttgaaggacctatggacggccttcaacgagc gctccgtggccgcgcacctggcggacatcattttccccgaacgcctgcttaaaa ccctgcaacagggtctgccagacttcaccagtcaaagcatgttgcagaactttag gaactttatcctagagcgctcaggaatcttgcccgccacctgctgtgcacttccta gcgactttgtgcccattaagtaccgcgaatgccctccgccgctttggggccactg ctaccttctgcagctagccaactaccttgcctaccactctgacataatggaagac gtgagcggtgacggtctactggagtgtcactgtcgctgcaacctatgcaccccg caccgctccctggtttgcaattcgcagctgcttaacgaaagtcaaattatcggtac ctttgagctgcagggtccctcgcctgacgaaaagtccgcggctccggggttgaa actcactccggggctgtggacgtcggcttaccttcgcaaatttgtacctgaggact accacgcccacgagattaggttctacgaagaccaatcccgcccgccaaatgcg gagcttaccgcctgcgtcattacccagggccacattcttggccaattgcaagcca tcaacaaagcccgccaagagtttctgctacgaaagggacggggggtttacttgg acccccagtccggcgaggagctcaacccaatcccccgccgccgcagcccta tcagcagcagccgcgggcccttgcttcccaggatcacccaaaaagaagctgca gctgccgccgccacccacggacgaggaggaatactgggacagtcaggcaga ggaggttttggacgaggaggaggaggacatgatggaagactgggagagccta gacgaggaagcttccgaggtcgaagaggtgtcagacgaaacaccgtcaccct cggtcgcattcccctcgccggcgccccagaaatcggcaaccggttccagcatg gctacaacctccgctcctcaggcgccgccggcactgcccgttcgccgacccaa ccgtagatgggacaccactggaaccagggccggtaagtccaagcagccgcc gccgttagcccaagagcaacaacagcgccaaggctaccgctcatggcgcggg cacaagaacgccatagttgcttgcttgcaagactgtgggggcaacatctccttcg cccgccgctttcttctctaccatcacggcgtggccttcccccgtaacatcctgcatt actaccgtcatctctacagcccatactgcaccggcggcagcggcagcggcagc aacagcagcggccacacagaagcaaaggcgaccggatagcaagactctgac aaagcccaagaaatccacagcggcggcagcagcaggaggaggagcgctgc gtctggcgcccaacgaacccgtatcgacccgcgagcttagaaacaggatttttc ccactctgtatgctatatttcaacagagcaggggccaagaacaagagctgaaaa taaaaaacaggtctctgcgatccctcacccgcagctgcctgtatcacaaaagcg aagatcagcttcggcgcacgctggaagacgcggaggctctcttcagtaaatact gcgcgctgactcttaaggactagtttcgcgccctttctcaaatttaagcgcgaaaa ctacgtcatctccagcggccacacccggcgccagcacctgtcgtcagcgccattata |
| | | gcaaggaaattcccacgccctacatgtggagttaccagccacaaatgggacttgcggc tggagctgcccaagactactcaacccgaataaactacatgagcgcggaccccacat gatatcccgggtcaacggaatccgcgcccttcgaaaccgaattctttaattatgaaattta ctgtgacttttctgctgattatttgcaccctatctgcgttttgttccccgacctccaagcctcaa agacatatatcatgcagattcactcgtatatggaatattccaagttgctacaatgaaaaaa gcgatctttccgaagcctggttatatgcaatcatctctgttatggtgttctgcagtaccatctta gccctagctatatatccctaccttgacattggctggaaacgaatagatgccatgaaccac ccaactttccccgcgcccgctatgcttccactgcaacaagttgttgccggcggctttgtccc agccaatcagcctcgccccacttctcccacccccactgaaatcagctactttaatctaac aggaggagatgactgacaccctagatctagaaatggacggaattattacagagcagc gcctgctagaaagacgcagggcagcggccgagcaacagcgcatgaatcaagagct ccaagacatggttaacttgcaccagtgcaaaaggggtatcttttgtctggtaaagcaggc caaagtcacctacgacagtaataccaccggacaccgccttagctacaagttgccaacc aagcgtcagaaattggtggtcatggtgggagaaaagcccattaccataactcagcact cggtagaaaccgaaggctgcattcactcaccttgtcaaggacctgaggatctctgcacc cttattaagaccctgtgcggtctcaaagatcttattccctttaactaataaaaaaaaataat aaagcatcacttacttaaaatcagttagcaaatttctgtccagtttattcagcagcacctcct tgccctcctcccagctctggtattgcagcttcctcctggctgcaaactttctccacaatctaa atggaatgtcagtttcctcctgttcctgtccatccgcacccactatcttcatgttgttgcagata agcgcgcaagaccgtctgaagataccttcaaccccgtgtatccatatgacacggaaac cggtcctccaactgtgccttttcttactcctccctttgtatcccccaatgggtttcaagagagt ccccctggggtactctctttgcgcctatccgaacctctagttacctccaatggcatgcttgc gctcaaaatgggcaacggcctctctctggacgaggccggcaaccttacctcccaaaat gtaaccactgtgagcccacctctcaaaaaaaccaagtcaaacataaacctggaaatat ctgcacccctcacagttacctcagaagccctaactgtggctgccgccgcacctctaatgg tcgcgggcaacacactcaccatgcaatcacaggccccgctaaccgtgcacgactcca aacttagcattgccacccaaggacccctcacagtgtcagaaggaaagctagccctgca aacatcaggcccccctcaccaccatcgatagcagtacccttactatcactgcctcacccc ctctaactactgccactggtagcttgggcattgacttgaaagagcccatttatacacaaaa tggaaaactaggactaaagtacggggctcctttgcatgtaacagacgacctaaacactt tgaccgtagcaactggtccaggtgtgactattaataatacttccttgcaaactaaagttact ggagccttgggttttgattcacaaggcaatatgcaacttaatgtagcaggaggactaagg attgattctcaaaacagacgccttatacttgatgttagttatccgtttgatgctcaaaaccaa ctaaatctaagactaggacagggccctctttttataaactcagcccacaacttggatatta actacaacaaaggcctttacttgtttacagcttcaaacaattccaaaaagcttgaggttaa cctaagcactgccaaggggttgatgtttgacgctacagccatagccattaatgcaggag atgggcttgaatttggttcacctaatgcaccaaacacaaatcccctcaaaacaaaaattg gccatggcctagaatttgattcaaacaaggctatggttcctaaactaggaactggcctta gttttgacagcacaggtgccattacagtaggaaacaaaaataatgataagctaactttgt ggaccacaccagctccatctcctaactgtagactaaatgcagagaaagatgctaaact cactttggtcttaacaaaatgtggcagtcaaatacttgctacagtttcagttttggctgttaaa ggcagtttggctccaatatctggaacagttcaaagtgctcatcttattataagatttgacga aaatggagtgctactaaacaattccttcctggacccagaatattggaactttagaaatgg agatcttattgaaggcacagcctatacaaacgctgttggatttatgcctaacctatcagctt atccaaaatctcacggtaaaactgccaaaagtaacattgtcagtcaagtttacttaaacg gagacaaaactaaacctgtaacactaaccattacactaaacggtacacaggaaacag gagacacaactccaagtgcatactctatgtcattttcatgggactggtctggccacaacta catcgatgaaatatttgccacatcctcttacactttttcatacattgcccaagaataaagaat |
| | | cgtttgtgttatgtttcaacgtgtttatttttcaattgcagaaaatttcaagtcatttttcattcagta gtatagccccaccaccacatagcttatacagatcaccgtaccttaatcaaactcacaga accctagtattcaacctgccacctccctcccaacacacagagtacacagtccttt ctccccggctggccttaaaagcatcatatcatgggtaacagacatattcttaggt gttatattccacacggtttcctgtcgagccaaacgctcatcagtgatattaataaac tccccgggcagctcacttaagttcatgtcgctgtccagctgctgagccacaggct gctgtccaacttgcggttgcttaacgggcggcgaaggagaagtccacgcctac atgggggtagagtcataatcgtgcatcaggatagggcggtggtgctgcagcag cgcgcgaataaactgctgccgccgctccgtcctgcaggaatacaacatgg cagtggtctcctcagcgatgattcgcaccgcccgcagcataaggcgccttgtcct ccgggcacagcagcgcaccctgatctcacttaaatcagcacagtaactgcagc acagcaccacaatattgttcaaaatcccacagtgcaaggcgctgtatccaaagct catggcggggaccacagaacccacgtggccatcataccacaagcgcaggtag attaagtggcgacccctcataaacacgctggacataaacattacctcttttggcat gttgtaattcaccacctcccggtaccatataaacctctgattaaacatggcgccat ccaccaccatcctaaaccagctggccaaaacctgcccgccggctatacactgc agggaaccgggactggaacaatgacagtggagagcccaggactcgtaaccat ggatcatcatgctcgtcatgatatcaatgttggcacaacacaggcacacgtgcat acacttcctcaggattacaagctcctcccgcgttagaaccatatcccagggaaca acccattcctgaatcagcgtaaatcccacactgcagggaagacctcgcacgtaa ctcacgttgtgcattgtcaaagtgttacattcgggcagcagcggatgatcctccag tatggtagcgcgggtttctgtctcaaaaggaggtagacgatccctactgtacgga gtgcgccgagacaaccgagatcgtgttggtcgtagtgtcatgccaaatggaacg ccggacgtagtcatatttcctgaagcaaaaccaggtgcgggcgtgacaaacag atctgcgtctccggtctcgccgcttagatcgctctgtgtagtagttgtagtatatcca ctctctcaaagcatccaggcgccccctggcttcgggttctatgtaaactccttcat gcgccgctgccctgataacatccaccaccgcagaataagccacacccagcca acctacacattcgttctgcgagtcacacacgggaggagcgggaagagctggaa gaaccatgtttttttttttattccaaaagattatccaaaacctcaaaatgaagatctatt aagtgaacgcgctcccctccggtggcgtggtcaaactctacagccaaagaaca gataatggcatttgtaagatgttgcacaatggcttccaaaaggcaaacggccctc acgtccaagtggacgtaaaggctaaacccttcagggtgaatctcctctataaaca ttccagcaccttcaaccatgcccaaataattctcatctcgccaccttctcaatatatc tctaagcaaatcccgaatattaagtccggccattgtaaaaatctgctccagagcg ccctccaccttcagcctcaagcagcgaatcatgattgcaaaaattcaggttcctca cagacctgtataagattcaaaagcggaacattaacaaaaataccgcgatcccgt aggtcccttcgcagggccagctgaacataatcgtgcaggtctgcacggaccag cgcggccacttccccgccaggaaccatgacaaaagaacccacactgattatga cacgcatactcggagctatgctaaccagcgtagccccgatgtaagcttgttgcat gggcggcgatataaaatgcaaggtgctgctcaaaaaatcaggcaaagcctcgc gcaaaaaagaaagcacatcgtagtcatgctcatgcagataaaggcaggtaagc tccggaaccaccacagaaaaagacaccatttttctctcaaacatgtctgcgggttt ctgcataaacacaaaataaaataacaaaaaaacatttaaacattagaagcctgtct tacaacaggaaaaacaacccttataagcataagacggactacggccatgccgg cgtgaccgtaaaaaaactggtcaccgtgattaaaaagcaccaccgacagctcct cggtcatgtccggagtcataatgtaagactcggtaaacacatcaggttgattcac atcggtcagtgctaaaaagcgaccgaaatagcccgggggaatacatacccgca ggcgtagagacaacattacagcccccataggaggtataacaaaattaatagga |
| | | gagaaaaacacataaacacctgaaaaaccctcctgcctaggcaaaatagcacc ctcccgctccagaacaacatacagcgcttccacagcggcagccataacagtca gccttaccagtaaaaaagaaaacctattaaaaaacaccactcgacacggcaccag ctcaatcagtcacagtgtaaaaaagggccaagtgcagagcgagtatatataggactaa aaaatgacgtaacggttaaagtccacaaaaaacacccagaaaaccgcacgcgaac ctacgcccagaaacgaaagccaaaaaacccacaacttcctcaaatcgtcacttccgttt tcccacgttacgtaacttcccattttaagaaaactacaattcccaacacatacaagttactc cgccctaaaacctacgtcacccgccccgttcccacgccccgcgccacgtcaca aactccacccccctcattatcatattggcttcaatccaaaataaggtatattattgatg atgatttaaatcagtcgcgatcg |
| 4 | HAD4 | atcgaagtactcggggaaatgtgcgcggaacccctatttgtttatttttctaaatacattcaa atatgtatccgctcatgagacaataaccctgataaatgcttcaataatattgaaaaagga agagtatgagccatattcaacgggaaacgtcgaggccgcgattaaattccaacatgga tgctgatttatatgggtataaatgggctcgcgataatgtcgggcaatcaggtgcgacaatc tatcgcttgtatgggaagcccgatgcgccagagttgtttctgaaacatggcaaaggtagc gttgccaatgatgttacagatgagatggtcagactaaactggctgacggaatttatgcctc ttccgaccatcaagcattttatccgtactcctgatgatgcatggttactcaccactgcgatcc ccggaaaaacagcattccaggtattagaagaatatcctgattcaggtgaaaatattgttg atgcgctggcagtgttcctgcgccggttgcattcgattcctgtttgtaattgtccttttaacagc gatcgcgtatttcgtctcgctcaggcgcaatcacgaatgaataacggtttggttgatgcga gtgattttgatgacgagcgtaatggctggcctgttgaacaagtctggaaagaaatgcata aacttttgccattctcaccggattcagtcgtcactcatggtgatttctcacttgataaccttattt ttgacgaggggaaattaataggttgtattgatgttggacgagtcggaatcgcagaccgat accaggatcttgccatcctatggaactgcctcggtgagttttctccttcattacagaaacgg ctttttcaaaaatatggtattgataatcctgatatgaataaattgcagtttcatttgatgctcga tgagtttttctaactgtcagaccaagtttactcatatatactttagattgatttaaaacttcatttt aatttaaaaggatctaggtgaagatcctttttgataatctcatgaccaaaatcccttaacgt gagttttcgttccactgagcgtcagaccccgtagaaaagatcaaaggatcttcttgagatc ctttttttctgcgcgtaatctgctgcttgcaaacaaaaaaaccaccgctaccagcggtggtt tgtttgccggatcaagagctaccaactctttttccgaaggtaactggcttcagcagagcgc agataccaaatactgttcttctagtgtagccgtagttaggccaccacttcaagaactctgta gcaccgcctacatacctcgctctgctaatcctgttaccagtggctgctgccagtggcgata agtcgtgtcttaccgggttggactcaagacgatagttaccggataaggcgcagcggtcg ggctgaacggggggttcgtgcacacagcccagcttggagcgaacgacctacaccga actgagatacctacagcgtgagctatgagaaagcgccacgcttcccgaagggagaaa ggcggacaggtatccggtaagcggcagggtcggaacaggagagcgcacgaggga gcttccagggggaaacgcctggtatctttatagtcctgtcgggtttcgccacctctgacttg agcgtcgatttttgtgatgctcgtcaggggggcggagcctatggaaaaacgccagcaa cgcggcctttttacggttcctggcctttttcctgcagcccgggggatccgattaattaaga cgtttaaacaataaaatatctttattttcattacatctgtgtgttggttttttgtgtgtcgac gagatcttccaacataaggcgatgatatccgtagatgtacctggacatcaggtgatgcc ggcggcggtggtggaggcgcgcggaaagtcgcggacgcggttccagatgttgcgca gcggcaaaaagtgctccatggtcggacgctctggccggtcaggcgcgcgca atcgttgacgctctagaccgtgcaaaaggagagcctgtaagcgggcactcttcc gtggtctggtggataaattcgcaagggtatcatggcggacgaccggggttcgag ccccgtatccggccgtccgccgtgatccatgcggttaccgcccgcgtgtcgaac ccaggtgtgcgacgtcagacaacgggggagtgctccttttggcttccttccaggc gcggcggctgctgcgctagcttttttggccactggccgcgcgcagcgtaagcg |
| | | gttaggctggaaagcgaaagcattaagtggctcgctccctgtagccggagggtt attttccaaggttgagtcgcggaccccggttcgagtctcggaccggccgga ctgcggcgaacgggggtttgcctccccgtcatgcaagaccccgcttgcaaattc ctccggaaacagggacgagcccctttttgcttttcccagatcatccggtgctgcgg cagatgcgccccctcctcagcagcggcaagagcaagagcagcggcagacatgca gggcaccctccatgcatccactttattttgtcacttgaaaaacatgtaaaataaatgtact agagacactttcaataaaggcaaatgcttttatttgtacactctcgggtgattatttac cccccacccttgccgtctgcgccgtttaaaaatcaaaggggttctgccgcgcatcg ctatgcgccactggcagggacacgttgcgatactggtgtttagtgctccacttaaa ctcaggcacaaccatccgcggcagctcggtgaagttttcactccacaggctgcg caccatcaccaacgcgtttagcaggtcgggcgccgatatcttgaagtcgcagtt ggggcctccgccctgcgcgcgcgagttgcgatacacagggttgcagcactgg aacactatcagcgccgggtggtgcacgctggccagcacgctcttgtcggagat cagatccgcgtccaggtcctccgcgttgctcagggcgaacggagtcaactttgg tagctgccttcccaaaaagggcgcgtgcccaggctttgagttgcactcgcaccg tagtggcatcaaaaggtgaccgtgcccggtctgggcgttaggatacagcgcctg cataaaagccttgatctgcttaaaagccacctgagcctttgcgccttcagagaag aacatgccgcaagacttgccggaaaactgattggccggacaggccgcgtcgtg cacgcagcaccttgcgtcggtgttggagatctgcaccacatttcggccccaccg gttcttcacgatcttggccttgctagactgctccttcagcgcgcgctgcccgttttc gctcgtcacatccatttcaatcacgtgctccttatttatcataatgcttccgtgtagac acttaagctcgccttcgatctcagcgcagcggtgcagccacaacgcgcagccc gtgggctcgtgatgcttgtaggtcacctctgcaaacgactgcaggtacgcctgca ggaatcgccccatcatcgtcacaaaggtcttgttgctggtgaaggtcagctgcaa cccgcggtgctcctcgttcagccaggtcttgcatacggccgccagagcttccact tggtcaggcagtagtttgaagttcgcctttagatcgttatccacgtggtacttgtcca tcagcgcgcgcgcagcctccatgcccttctcccacgcagacacgatcggcaca ctcagcgggttcatcaccgtaatttcactttccgcttcgctgggctcttcctcttcctc ttgcgtccgcataccacgcgccactgggtcgtcttcattcagccgccgcactgtg cgcttacctcctttgccatgcttgattagcaccggtgggttgctgaaacccaccatt tgtagcgccacatcttctctttcttcctcgctgtccacgattacctctggtgatggcg ggcgctcgggcttgggagaagggcgcttctttttcttcttgggcgcaatggccaa atccgccgccgaggtcgatggccgcgggctgggtgtgcgcggcaccagcgc gtcttgtgatgagtcttcctcgtcctcggactcgatacgccgcctcatccgcttttt gggggcgcccggggaggcggcggcgacggggacggggacgacacgtcct ccatggttgggggacgtcgcgccgcaccgcgtccgcgctcgggggtggtttcg cgctgctcctcttcccgactggccatttccttctcctataggcagaaaaagatcata gtcagtcgagaagaaggacagcctaaccgcccctctgagttcgccaccaccg cctccaccgatgccgccaacgcgcctaccaccttccccgtcgaggcacccccg cttgaggaggaggaagtgattatcgagcaggacccaggttttgtaagcgaagac gacgaggaccgctcagtaccaacagaggataaaaagcaagaccaggacaac gcagaggcaaacgaggaacaagtcgggcgggggggacgaaaggcatggcga ctacctagatgtgggagacgacgtgctgttgaagcatctgcagcgccagtgcgc cattatctgcgacgcgttgcaagagcgcagcgatgtgcccctcgccatagcgga tgtcagccttgcctacgaacgccacctattctcaccgcgcgtaccccccaaacg ccaagaaaacggcacatgcgagcccaacccgcgcctcaacttctaccccgtat ttgccgtgccagaggtgcttgccacctatcacatctttttccaaaactgcaagatac ccctatcctgccgtgccaaccgcagccgagcggacaagcagctggccttgcg |
| | | gcagggcgctgtcatacctgatatcgcctcgctcaacgaagtgccaaaaatcttt gagggtcttggacgcgacgagaagcgcgcggcaaacgctctgcaacaggaa aacagcgaaaatgaaagtcactctggagtgttggtggaactcgagggtgacaa cgcgcgcctagccgtactaaaacgcagcatcgaggtcacccactttgcctaccc ggcacttaacctacccccaaggtcatgagcacagtcatgagtgagctgatcgt gcgccgtgcgcagcccctggagagggatgcaaatttgcaagaacaaacagag gagggcctacccgcagttggcgacgagcagctagcgcgctggcttcaaacgc gcgagcctgccgacttggaggagcgacgcaaactaatgatggccgcagtgctc gttaccgtggagcttgagtgcatgcagcggttctttgctgacccggagatgcagc gcaagctagaggaaacattgcactacacctttcgacagggctacgtacgccag gcctgcaagatctccaacgtggagctctgcaacctggtctcctaccttggaatttt gcacgaaaaccgccttgggcaaaacgtgcttcattccacgctcaagggcgagg cgcgccgcgactacgtccgcgactgcgtttacttatttctatgctacacctggcag acggccatgggcgtttggcagcagtgcttggaggagtgcaacctcaaggagct gcagaaactgctaaagcaaaacttgaaggacctatggacggccttcaacgagc gctccgtggccgcgcacctggcggacatcattttccccgaacgcctgcttaaaa ccctgcaacagggtctgccagacttcaccagtcaaagcatgttgcagaactttag gaactttatcctagagcgctcaggaatcttgcccgccacctgctgtgcacttccta gcgactttgtgcccattaagtaccgcgaatgccctccgccgctttggggccactg ctaccttctgcagctagccaactaccttgcctaccactctgacataatggaagac gtgagcggtgacggtctactggagtgtcactgtcgctgcaacctatgcaccccg caccgctccctggtttgcaattcgcagctgcttaacgaaagtcaaattatcggtac ctttgagctgcagggtccctcgcctgacgaaaagtccgcggctccggggttgaa actcactccggggctgtggacgtcggcttaccttcgcaaatttgtacctgaggact accacgcccacgagattaggttctacgaagaccaatcccgcccgccaaatgcg gagcttaccgcctgcgtcattacccagggccacattcttggccaattgcaagcca tcaacaaagcccgccaagagtttctgctacgaaagggacggggggtttacttgg acccccagtccggcgaggagctcaacccaatcccccgccgccgcagcccta tcagcagcagccgcgggcccttgcttcccaggatcacccaaaaagaagctgca gctgccgccgccacccacggacgaggaggaatactgggacagtcaggcaga ggaggttttggacgaggaggaggaggacatgatggaagactgggagagccta gacgaggaagcttccgaggtcgaagaggtgtcagacgaaacaccgtcaccct cggtcgcattcccctcgccggcgccccagaaatcggcaaccggttccagcatg gctacaacctccgctcctcaggcgccgccggcactgcccgttcgccgacccaa ccgtagatgggacaccactggaaccagggccggtaagtccaagcagccgcc gccgttagcccaagagcaacaacagcgccaaggctaccgctcatggcgcgg cacaagaacgccatagttgcttgcttgcaagactgtgggggcaacatctccttcg cccgccgctttcttctctaccatcacggcgtggccttcccccgtaacatcctgcatt actaccgtcatctctacagcccatactgcaccggcggcagcggcagcggcagc aacagcagcggccacacagaagcaaaggcgaccggatagcaagactctgac aaagcccaagaaatccacagcggcggcagcagcaggaggaggagcgctgc gtctggcgcccaacgaacccgtatcgacccgcgagcttagaaacaggatttttc ccactctgtatgctatatttcaacagagcaggggccaagaacaagagctgaaaa taaaaaacaggtctctgcgatccctcacccgcagctgcctgtatcacaaaagcg aagatcagcttcggcgcacgctggaagacgcggaggctctcttcagtaaatact gcgcgctgactcttaaggactagtttcgcgccctttctcaaatttaagcgcgaaaa ctacgtcatctccagcggccacacccggcgccagcacctgtcgtcagcgccattata gcaaggaaattcccacgccctacatgtggagttaccagccacaaatgggacttgcggc |
| | | tggagctgcccaagactactcaacccgaataaactacatgagcgcggaccccacat gatatcccgggtcaacggaatccgcgcccttcgaaaccgaattctttaattatgaaattta ctgtgacttttctgctgattatttgcaccctatctgcgttttgttccccgacctccaagcctcaa agacatatatcatgcagattcactcgtatatggaatattccaagttgctacaatgaaaaaa gcgatctttccgaagcctggttatatgcaatcatctctgttatggtgttctgcagtaccatctta gccctagctatatatccctaccttgacattggctggaaacgaatagatgccatgaaccac ccaactttccccgcgcccgctatgcttccactgcaacaagttgttgccggcggctttgtccc agccaatcagcctcgccccacttctcccacccccactgaaatcagctactttaatctaac aggaggagatgactgacaccctagatctagaaatggacggaattattacagagcagc gcctgctagaaagacgcagggcagcggccgagcaacagcgcatgaatcaagagct ccaagacatggttaacttgcaccagtgcaaaaggggtatcttttgtctggtaaagcaggc caaagtcacctacgacagtaataccaccggacaccgccttagctacaagttgccaacc aagcgtcagaaattggtggtcatggtgggagaaaagcccattaccataactcagcact cggtagaaaccgaaggctgcattcactcaccttgtcaaggacctgaggatctctgcacc cttattaagaccctgtgcggtctcaaagatcttattccctttaactaataaaaaaaaataat aaagcatcacttacttaaaatcagttagcaaatttctgtccagtttattcagcagcacctcct tgccctcctcccagctctggtattgcagcttcctcctggctgcaaactttctccacaatctaa atggaatgtcagtttcctcctgttcctgtccatccgcacccactatcttcatgttgttgcagata agcgcgcaagaccgtctgaagataccttcaaccccgtgtatccatatgacacggaaac cggtcctccaactgtgccttttcttactcctccctttgtatcccccaatgggtttcaagagagt ccccctggggtactctctttgcgcctatccgaacctctagttacctccaatggcatgcttgc gctcaaaatgggcaacggcctctctctggacgaggccggcaaccttacctcccaaaat gtaaccactgtgagcccacctctcaaaaaaaccaagtcaaacataaacctggaaatat ctgcacccctcacagttacctcagaagccctaactgtggctgccgccgcacctctaatgg tcgcgggcaacacactcaccatgcaatcacaggccccgctaaccgtgcacgactcca aacttagcattgccacccaaggacccctcacagtgtcagaaggaaagctagccctgca aacatcaggcccccctcaccaccatcgatgaaatatttgccacatcctcttacactttttcat acattgcccaagaataaagaatcgtttgtgttatgtttcaacgtgtttatttttcaattgcagaa aatttcaagtcatttttcattcagtagtatagccccaccaccacatagcttatacagatcac cgtaccttaatcaaactcacagaaccctagtattcaacctgccacctccctcccaac acacagagtacacagtcctttctccccggctggccttaaaaagcatcatatcatg ggtaacagacatattcttaggtgttatattccacacggtttcctgtcgagccaaacg ctcatcagtgatattaataaactccccgggcagctcacttaagttcatgtcgctgtc cagctgctgagccacaggctgctgtccaacttgcggttgcttaacgggcggcga aggagaagtccacgcctacatgggggtagagtcataatcgtgcatcaggatag ggcggtggtgctgcagcagcgcgcgaataaactgctgccgccgccgctccgt cctgcaggaatacaacatggcagtggtctcctcagcgatgattcgcaccgcccg cagcataaggcgccttgtcctccgggcacagcagcgcaccctgatctcacttaa atcagcacagtaactgcagcacagcaccacaatattgttcaaaatcccacagtg caaggcgctgtatccaaagctcatggcggggaccacagaacccacgtggcca tcataccacaagcgcaggtagattaagtggcgacccctcataaacacgctggac ataaacattacctcttttggcatgttgtaattcaccacctcccggtaccatataaacc tctgattaaacatggcgccatccaccaccatcctaaaccagctggccaaaacct gcccgccggctatacactgcagggaaccgggactggaacaatgacagtggag agcccaggactcgtaaccatggatcatcatgctcgtcatgatatcaatgttggcac aacacaggcacacgtgcatacacttcctcaggattacaagctcctcccgcgttag aaccatatcccagggaacaacccattcctgaatcagcgtaaatcccacactgca gggaagacctcgcacgtaactcacgttgtgcattgtcaaagtgttacattcgggc |
| | | agcagcggatgatcctccagtatggtagcgcgggtttctgtctcaaaaggaggta gacgatccctactgtacggagtgcgccgagacaaccgagatcgtgttggtcgta gtgtcatgccaaatggaacgccggacgtagtcatatttcctgaagcaaaaccag gtgcgggcgtgacaaacagatctgcgtctccggtctcgccgcttagatcgctctg tgtagtagttgtagtatatccactctctcaaagcatccaggcgccccctggcttcg ggttctatgtaaactccttcatgcgccgctgccctgataacatccaccaccgcag aataagccacacccagccaacctacacattcgttctgcgagtcacacacggga ggagcgggaagagctggaagaaccatgtttttttttattccaaaagattatccaaa acctcaaaatgaagatctattaagtgaacgcgctcccctccggtggcgtggtcaa actctacagccaaagaacagataatggcatttgtaagatgttgcacaatggcttcc aaaaggcaaacggccctcacgtccaagtggacgtaaaggctaaacccttcagg gtgaatctcctctataaacattccagcaccttcaaccatgcccaaataattctcatct cgccaccttctcaatatatctctaagcaaatcccgaatattaagtccggccattgta aaaatctgctccagagcgccctccaccttcagcctcaagcagcgaatcatgattg caaaaattcaggttcctcacagacctgtataagattcaaaagcggaacattaaca aaaataccgcgatcccgtaggtcccttcgcagggccagctgaacataatcgtgc aggtctgcacggaccagcgcggccacttccccgccaggaaccatgacaaaag aacccacactgattatgacacgcatactcggagctatgctaaccagcgtagccc cgatgtaagcttgttgcatgggcggcgatataaaatgcaaggtgctgctcaaaaa atcaggcaaagcctcgcgcaaaaaagaaagcacatcgtagtcatgctcatgca gataaaggcaggtaagctccggaaccaccacagaaaaagacaccatttttctct caaacatgtctgcgggtttctgcataaacacaaaataaaataacaaaaaaacattt aaacattagaagcctgtcttacaacaggaaaaacaacccttataagcataagac ggactacggccatgccggcgtgaccgtaaaaaaactggtcaccgtgattaaaa agcaccaccgacagctcctcggtcatgtccggagtcataatgtaagactcggta aacacatcaggttgattcacatcggtcagtgctaaaaagcgaccgaaatagccc ggggggaatacatacccgcaggcgtagagacaacattacagccccccataggag gtataacaaaattaataggagagaaaaacacataaacacctgaaaaaccctcct gcctaggcaaaatagcaccctcccgctccagaacaacatacagcgcttccaca gcggcagccataacagtcagccttaccagtaaaaaagaaaacctattaaaaaaaca ccactcgacacggcaccagctcaatcagtcacagtgaaaaaagggccaagtgcag agcgagtatatataggactaaaaaatgacgtaacggttaaagtccacaaaaaacacc cagaaaaccgcacgcgaacctacgcccagaaacgaaagccaaaaaacccacaac ttcctcaaatcgtcacttccgttttcccacgttacgtaacttcccattttaagaaaactacaatt cccaacacatacaagttactccgccctaaaacctacgtcacccgccccgttcccac gccccgcgccacgtcacaaactccaccccctcattatcatattggcttcaatcca aaataaggtatattattgatgatgatttaaatcagtcgcgatcg |
| 5 | HAD5 | aataaaatatctttattttcattacatctgtgtgttggttttttgtgtgtcgacgagatcttc caacataaggcgatgatatccgtagatgtacctggacatcaggtgatgccggcggcgg tggtggaggcgcgcggaaagtcgcggacgcggttccagatgttgcgcagcggcaa aaagtgctccatggtcgggacgctctggccggtcaggcgcgcgcaatcgttga cgctctagaccgtgcaaaaggagagcctgtaagcgggcactcttccgtggtctg gtggataaattcgcaagggtatcatggcggacgaccggggttcgagccccgtat ccggccgtccgccgtgatccatgcggttaccgcccgcgtgtcgaacccaggtg tgcgacgtcagacaacgggggagtgctccttttggcttccttccaggcgcggcg gctgctgcgctagcttttttggccactggccgcgcgcagcgtaagcggttaggct ggaaagcgaaagcattaagtggctcgctccctgtagccggagggttattttccaa gggttgagtcgcgggacccccggttcgagtctcggaccggccggactgcggc |
| | | gaacggggtttgcctccccgtcatgcaagaccccgcttgcaattcctccgga aacagggacgagcccctttttgcttttcccagatcatccggtgctgcggcagatgcg cccccctcctcagcagcggcaagagcaagagcagcggcagacatgcagggcaccc tccatgcatccactttattttgtcacttgaaaaacatgtaaaataaatgtactagagacac tttcaataaaggcaaatgcttttatttgtacactctcgggtgattatttacccccaccct tgccgtctgcgccgtttaaaaatcaaaggggttctgccgcgcatcgctatgcgcc actggcagggacacgttgcgatactggtgtttagtgctccacttaaactcaggca caaccatccgcggcagctcggtgaagttttcactccacaggctgcgcaccatca ccaacgcgtttagcaggtcgggcgccgatatcttgaagtcgcagttggggcctc cgccctgcgcgcgcgagttgcgatacacagggttgcagcactggaacactatc agcgccgggtggtgcacgctggccagcacgctcttgtcggagatcagatccgc gtccaggtcctccgcgttgctcagggcgaacggagtcaactttggtagctgcctt cccaaaaagggcgcgtgcccaggctttgagttgcactcgcaccgtagtggcatc aaaaggtgaccgtgcccggtctgggcgttaggatacagcgcctgcataaaagc cttgatctgcttaaaagccacctgagcctttgcgccttcagagaagaacatgccg caagacttgccggaaaactgattggccggacaggccgcgtcgtgcacgcagc accttgcgtcggtgttggagatctgcaccacatttcggccccaccggttcttcacg atcttggccttgctagactgctccttcagcgcgcgctgcccgttttcgctcgtcaca tccatttcaatcacgtgctccttatttatcataatgcttccgtgtagacacttaagctc gccttcgatctcagcgcagcggtgcagccacaacgcgcagcccgtgggctcgt gatgcttgtaggtcacctctgcaaacgactgcaggtacgcctgcaggaatcgcc ccatcatcgtcacaaaggtcttgttgctggtgaaggtcagctgcaacccgcggtg ctcctcgttcagccaggtcttgcatacggccgccagagcttccacttggtcaggc agtagtttgaagttcgcctttagatcgttatccacgtggtacttgtccatcagcgcg cgcgcagcctccatgcccttctcccacgcagacacgatcggcacactcagcgg gttcatcaccgtaatttcactttccgcttcgctgggctcttcctcttcctcttgcgtcc gcataccacgcgccactgggtcgtcttcattcagccgccgcactgtgcgcttacc tcctttgccatgcttgattagcaccggtgggttgctgaaacccaccatttgtagcgc cacatcttctctttcttcctcgctgtccacgattacctctggtgatggcgggcgctc gggcttgggagaagggcgcttctttttcttcttgggcgcaatggccaaatccgcc gccgaggtcgatggccgcgggctgggtgtgcgcggcaccagcgcgtcttgtg atgagtcttcctcgtcctcggactcgatacgccgcctcatccgcttttttgggggc gcccggggaggcggcggcgacggggacggggacgacacgtcctccatggtt gggggacgtcgcgccgcaccgcgtccgcgctcgggggtggtttcgcgctgct cctcttcccgactggccatttccttctcctataggcagaaaaagatcatagtcagtc gagaagaaggacagcctaaccgccccctctgagttcgccaccaccgcctcca ccgatgccgccaacgcgcctaccaccttccccgtcgaggcacccccgcttgag gaggaggaagtgattatcgagcaggacccaggttttgtaagcgaagacgacga ggaccgctcagtaccaacagaggataaaaagcaagaccaggacaacgcaga ggcaaacgaggaacaagtcgggcgggggacgaaaggcatggcgactacct agatgtgggagacgacgtgctgttgaagcatctgcagcgccagtgcgccattat ctgcgacgcgttgcaagagcgcagcgatgtgcccctcgccatagcggatgtca gccttgcctacgaacgccacctattctcaccgcgcgtacccccaaacgccaa gaaaacggcacatgcgagcccaacccgcgcctcaacttctaccccgtatttgcc gtgccagaggtgcttgccacctatcacatctttttccaaaactgcaagatacccct atcctgccgtgccaaccgcagccgagcggacaagcagctggccttgcggcag ggcgctgtcatacctgatatcgcctcgctcaacgaagtgccaaaaatctttgagg gtcttggacgcgacgagaagcgcgcggcaaacgctctgcaacaggaaaaca |
| | | gcgaaaatgaaagtcactctggagtgttggtggaactcgagggtgacaacgcg cgcctagccgtactaaaacgcagcatcgaggtcacccactttgcctacccggca cttaacctacccccaaggtcatgagcacagtcatgagtgagctgatcgtgcgc cgtgcgcagcccctggagagggatgcaaatttgcaagaacaaacagaggagg gcctacccgcagttggcgacgagcagctagcgcgctggcttcaaacgcgcga gcctgccgacttggaggagcgacgcaaactaatgatggccgcagtgctcgtta ccgtggagcttgagtgcatgcagcggttctttgctgacccggagatgcagcgca agctagaggaaacattgcactacacctttcgacagggctacgtacgccaggcct gcaagatctccaacgtggagctctgcaacctggtctcctaccttggaattttgcac gaaaaccgccttgggcaaaacgtgcttcattccacgctcaagggcgaggcgcg ccgcgactacgtccgcgactgcgtttacttatttctatgctacacctggcagacgg ccatgggcgtttggcagcagtgcttggaggagtgcaacctcaaggagctgcag aaactgctaaagcaaaacttgaaggacctatggacggccttcaacgagcgctcc gtggccgcgcacctggcggacatcattttccccgaacgcctgcttaaaaccctg caacagggtctgccagacttcaccagtcaaagcatgttgcagaactttaggaact ttatcctagagcgctcaggaatcttgcccgccacctgctgtgcacttcctagcgac tttgtgcccattaagtaccgcgaatgccctccgccgctttggggccactgctacct tctgcagctagccaactaccttgcctaccactctgacataatggaagacgtgagc ggtgacggtctactggagtgtcactgtcgctgcaacctatgcaccccgcaccgc tccctggtttgcaattcgcagctgcttaacgaaagtcaaattatcggtacctttgag ctgcagggtccctcgcctgacgaaaagtccgcggctccggggttgaaactcact ccggggctgtggacgtcggcttaccttcgcaaatttgtacctgaggactaccacg cccacgagattaggttctacgaagaccaatcccgcccgccaaatgcggagctta ccgcctgcgtcattacccagggccacattcttggccaattgcaagccatcaacaa agcccgccaagagtttctgctacgaaagggacggggggtttacttggacccccc agtccggcgaggagctcaacccaatccccccgccgccgcagccctatcagca gcagccgcgggcccttgcttcccaggatcacccaaaaagaagctgcagctgcc gccgccacccacggacgaggaggaatactgggacagtcaggcagaggaggt tttggacgaggaggaggaggacatgatggaagactgggagagcctagacgag gaagcttccgaggtcgaagaggtgtcagacgaaacaccgtcaccctcggtcgc attcccctcgccggcgccccagaaatcggcaaccggttccagcatggctacaa cctccgctcctcaggcgccgccggcactgcccgttcgccgacccaaccgtaga tgggacaccactggaaccagggccggtaagtccaagcagccgccgccgttag cccaagagcaacaacagcgccaaggctaccgctcatggcgcgggcacaaga acgccatagttgcttgcttgcaagactgtgggggcaacatctccttcgcccgccg ctttcttctctaccatcacggcgtggccttcccccgtaacatcctgcattactaccgt catctctacagcccatactgcaccggcggcagcggcagcggcagcaacagca gcggccacacagaagcaaaggcgaccggatagcaagactctgacaaagccc aagaaatccacagcggcggcagcagcaggaggaggagcgctgcgtctggcg cccaacgaacccgtatcgacccgcgagcttagaaacaggatttttcccactctgt atgctatatttcaacagagcaggggccaagaacaagagctgaaaataaaaaac aggtctctgcgatccctcacccgcagctgcctgtatcacaaaagcgaagatcag cttcggcgcacgctggaagacgcggaggctctcttcagtaaatactgcgcgctg actcttaaggactagtttcgcgccctttctcaaatttaagcgcgaaaactacgtcat ctccagcggccacacccggcgccagcacctgtcgtcagcgccattatagcaaggaa attcccacgccctacatgtggagttaccagccacaaatgggacttgcggctggagctgc ccaagactactcaacccgaataaactacatgagcgcgggaccccacatgatatcccg ggtcaacggaatccgcgcccttcgatgaaatatttgccacatcctcttacactttttcataca |
| | | ttgcccaagaataaagaatcgtttgtgttatgtttcaacgtgtttatttttcaattgcagaaaat ttcaagtcatttttcattcagtagtatagccccaccaccacatagcttatacagatcaccgt accttaatcaaactcacagaaccctagtattcaacctgccacctccctcccaacac acagagtacacagtcctttctccccggctggccttaaaagcatcatatcatgggt aacagacatattcttaggtgttatattccacacggtttcctgtcgagccaaacgctc atcagtgatattaataaactccccgggcagctcacttaagttcatgtcgctgtcca gctgctgagccacaggctgctgtccaacttgcggttgcttaacgggcggcgaag gagaagtccacgcctacatgggggtagagtcataatcgtgcatcaggataggg cggtggtgctgcagcagcgcgaataaactgctgccgccgctccgtcct gcaggaatacaacatggcagtggtctcctcagcgatgattcgcaccgcccgca gcataaggcgccttgtcctccgggcacagcagcgcaccctgatctcacttaaat cagcacagtaactgcagcacagcaccacaatattgttcaaaatcccacagtgca aggcgctgtatccaaagctcatggcggggaccacagaacccacgtggccatc ataccacaagcgcaggtagattaagtggcgacccctcataaacacgctggacat aaacattacctcttttggcatgttgtaattcaccacctcccggtaccatataaacctc tgattaaacatggcgccatccaccaccatcctaaaccagctggccaaaacctgc ccgccggctatacactgcagggaaccgggactggaacaatgacagtggagag cccaggactcgtaaccatggatcatcatgctcgtcatgatatcaatgttggcacaa cacaggcacacgtgcatacacttcctcaggattacaagctcctcccgcgttagaa ccatatcccagggaacaacccattcctgaatcagcgtaaatcccacactgcagg gaagacctcgcacgtaactcacgttgtgcattgtcaaagtgttacattcgggcag cagcggatgatcctccagtatggtagcgcgggtttctgtctcaaaaggaggtaga cgatccctactgtacggagtgcgccgagacaaccgagatcgtgttggtcgtagt gtcatgccaaatggaacgccggacgtagtcatatttcctgaagcaaaaccaggt gcgggcgtgacaaacagatctgcgtctccggtctcgccgcttagatcgctctgtg tagtagttgtagtatatccactctctcaaagcatccaggcgccccctggcttcggg ttctatgtaaactccttcatgcgccgctgccctgataacatccaccaccgcagaat aagccacacccagccaacctacacattcgttctgcgagtcacacacgggagga gcgggaagagctggaagaaccatgtttttttttattccaaaagattatccaaaacc tcaaaatgaagatctattaagtgaacgcgctcccctccggtggcgtggtcaaact ctacagccaaagaacagataatggcatttgtaagatgttgcacaatggcttccaa aaggcaaacggccctcacgtccaagtggacgtaaaggctaaacccttcagggt gaatctcctctataaacattccagcaccttcaaccatgcccaaataattctcatctc gccaccttctcaatatatctctaagcaaatcccgaatattaagtccggccattgtaa aaatctgctccagagcgccctccaccttcagcctcaagcagcgaatcatgattgc aaaaattcaggttcctcacagacctgtataagattcaaaagcggaacattaacaa aaataccgcgatcccgtaggtcccttcgcagggccagctgaacataatcgtgca ggtctgcacggaccagcgcggccacttccccgccaggaaccatgacaaaaga acccacactgattatgacacgcatactcggagctatgctaaccagcgtagcccc gatgtaagcttgttgcatgggcggcgatataaaatgcaaggtgctgctcaaaaaa tcaggcaaagcctcgcgcaaaaaagaaagcacatcgtagtcatgctcatgcag ataaaggcaggtaagctccggaaccaccacagaaaaagacaccatttttctctc aaacatgtctgcgggtttctgcataaacacaaaataaaataacaaaaaaacattta aacattagaagcctgtcttacaacaggaaaaacaacccttataagcataagacg gactacggccatgccggcgtgaccgtaaaaaaactggtcaccgtgattaaaaa gcaccaccgacagctcctcggtcatgtccggagtcataatgtaagactcggtaa acacatcaggttgattcacatcggtcagtgctaaaaagcgaccgaaatagcccg gggggaatacatacccgcaggcgtagagacaacattacagcccccataggagg |
| | | tataacaaaattaataggagagaaaaacacataaacacctgaaaaaccctcctg cctaggcaaaatagcaccctcccgctccagaacaacatacagcgcttccacag cggcagccataacagtcagccttaccagtaaaaaagaaaacctattaaaaaacac cactcgacacggcaccagctcaatcagtcacagtgtaaaaaagggccaagtgcaga gcgagtatatataggactaaaaaatgacgtaacggttaaagtccacaaaaaacaccc agaaaaccgcacgcgaacctacgcccagaaacgaaagccaaaaaacccacaactt cctcaaatcgtcacttccgttttcccacgttacgtaacttcccattttaagaaaactacaattc ccaacacatacaagttactccgccctaaaacctacgtcacccgccccgttcccacg ccccgcgccacgtcacaaactccacccctcattatcatattggcttcaatccaa aataaggtatattattgatgatgatttaaatcagtcgcgatcg |
| 6 | HAD6 | atcgaagtactcggggaaatgtgcgcggaacccctatttgtttatttttctaaatacattcaa atatgtatccgctcatgagacaataaccctgataaatgcttcaataatattgaaaaagga agagtatgagccatattcaacgggaaacgtcgaggccgcgattaaattccaacatgga tgctgatttatatgggtataaatgggctcgcgataatgtcgggcaatcaggtgcgacaatc tatcgcttgtatgggaagcccgatgcgccagagttgtttctgaaacatggcaaaggtagc gttgccaatgatgttacagatgagatggtcagactaaactggctgacggaatttatgcctc ttccgaccatcaagcattttatccgtactcctgatgatgcatggttactcaccactgcgatcc ccggaaaaacagcattccaggtattagaagaatatcctgattcaggtgaaaatattgttg atgcgctggcagtgttcctgcgccggttgcattcgattcctgtttgtaattgtccttttaacagc gatcgcgtatttcgtctcgctcaggcgcaatcacgaatgaataacggtttggttgatgcga gtgattttgatgacgagcgtaatggctggcctgttgaacaagtctggaaagaaatgcata aacttttgccattctcaccggattcagtcgtcactcatggtgatttctcacttgataaccttattt ttgacgaggggaaattaataggttgtattgatgttggacgagtcggaatcgcagaccgat accaggatcttgccatcctatggaactgcctcggtgagttttctccttcattacagaaacgg ctttttcaaaaatatggtattgataatcctgatatgaataaattgcagtttcatttgatgctcga tgagtttttctaactgtcagaccaagtttactcatatatactttagattgatttaaaacttcattttt aatttaaaaggatctaggtgaagatcctttttgataatctcatgaccaaaatcccttaacgt gagttttcgttccactgagcgtcagaccccgtagaaaagatcaaaggatcttcttgagatc ctttttttctgcgcgtaatctgctgcttgcaaacaaaaaaaccaccgctaccagcggtggtt tgtttgccggatcaagagctaccaactctttttccgaaggtaactggcttcagcagagcgc agataccaaatactgttcttctagtgtagccgtagttaggccaccacttcaagaactctgta gcaccgcctacatacctcgctctgctaatcctgttaccagtggctgctgccagtggcgata agtcgtgtcttaccgggttggactcaagacgatagttaccggataaggcgcagcggtcg ggctgaacggggggttcgtgcacacagcccagcttggagcgaacgacctacaccga actgagatacctacagcgtgagctatgagaaagcgccacgcttcccgaagggagaaa ggcggacaggtatccggtaagcggcagggtcggaacaggagagcgcacgaggga gcttccagggggaaacgcctggtatctttatagtcctgtcgggtttcgccacctctgacttg agcgtcgatttttgtgatgctcgtcaggggggcggagcctatggaaaaacgccagcaa cgcggcctttttacggttcctggccttttttcctgcagcccgggggatccgattaattaaga cgtttaaacaataaaatatctttattttcattacatctgtgtgttggttttttgtgtgtcgac gagatcttccaacataaggcgatgatatccgtagatgtacctggacatcaggtgatgcc ggcggcggtggtggaggcgcgcggaaagtcgcggacgcggttccagatgttgcgca gcggcaaaaagtgctccatggtcgggacgctctggccggtcaggcgcgcgca atcgttgacgctctagaccgtgcaaaaggagagcctgtaagcgggcactcttcc gtggtctggtggataaattcgcaagggtatcatggcggacgaccggggttcgag ccccgtatccggccgtccgccgtgatccatgcggttaccgcccgcgtgtcgaac ccaggtgtgcgacgtcagacaacgggggagtgctccttttggcttccttccaggc gcggcggctgctgcgctagcttttttggccactggccgcgcgcagcgtaagcg |
| | | gttaggctggaaagcgaaagcattaagtggctcgctccctgtagccggagggtt attttccaaggttgagtcgcggaccccggttcgagtctcggaccggccgga ctgcggcgaacgggggtttgcctccccgtcatgcaagaccccgcttgcaaattc ctccggaaacagggacgagcccctttttgcttttcccagatcatccggtgctgcgg cagatgcgcccccctcctcagcagcggcaagagcaagagcagcggcagacatgca gggcaccctccatgcatccactttattttgtcacttgaaaaacatgtaaaataaatgtact agagacactttcaataaaggcaaatgcttttatttgtacactctcgggtgattatttac ccccacccttgccgtctgcgccgtttaaaatcaaaggggttctgccgcgcatcg ctatgcgccactggcagggacacgttgcgatactggtgtttagtgctccacttaaa ctcaggcacaaccatccgcggcagctcggtgaagttttcactccacaggctgcg caccatcaccaacgcgtttagcaggtcgggcgccgatatcttgaagtcgcagtt ggggcctccgccctgcgcgcgcgagttgcgatacacagggttgcagcactgg aacactatcagcgccgggtggtgcacgctggccagcacgctcttgtcggagat cagatccgcgtccaggtcctccgcgttgctcagggcgaacggagtcaactttgg tagctgccttcccaaaaagggcgcgtgcccaggctttgagttgcactcgcaccg tagtggcatcaaaaggtgaccgtgcccggtctgggcgttaggatacagcgcctg cataaaagccttgatctgcttaaaagccacctgagcctttgcgccttcagagaag aacatgccgcaagacttgccggaaaactgattggccggacaggccgcgtcgtg cacgcagcaccttgcgtcggtgttggagatctgcaccacatttcggccccaccg gttcttcacgatcttggccttgctagactgctccttcagcgcgcgctgcccgttttc gctcgtcacatccatttcaatcacgtgctccttatttatcataatgcttccgtgtagac acttaagctcgccttcgatctcagcgcagcggtgcagccacaacgcgcagccc gtgggctcgtgatgcttgtaggtcacctctgcaaacgactgcaggtacgcctgca ggaatcgccccatcatcgtcacaaaggtcttgttgctggtgaaggtcagctgcaa cccgcggtgctcctcgttcagccaggtcttgcatacggccgccagagcttccact tggtcaggcagtagtttgaagttcgcctttagatcgttatccacgtggtacttgtcca tcagcgcgcgcgcagcctccatgcccttctcccacgcagacacgatcggcaca ctcagcgggttcatcaccgtaatttcactttccgcttcgctgggctcttcctcttcctc ttgcgtccgcataccacgcgccactgggtcgtcttcattcagccgccgcactgtg cgcttacctcctttgccatgcttgattagcaccggtgggttgctgaaacccaccatt tgtagcgccacatcttctctttcttcctcgctgtccacgattacctctggtgatggcg ggcgctcgggcttgggagaagggcgcttctttttcttcttgggcgcaatggccaa atccgccgccgaggtcgatggccgcgggctgggtgtgcgcggcaccagcgc gtcttgtgatgagtcttcctcgtcctcggactcgatacgccgcctcatccgcttttt gggggcgcccggggaggcggcggcgacggggacggggacgacacgtcct ccatggttgggggacgtcgcgccgcaccgcgtccgcgctcgggggtggtttcg cgctgctcctcttcccgactggccatttccttctcctataggcagaaaaagatcata gtcagtcgagaagaaggacagcctaaccgcccccctctgagttcgccaccaccg cctccaccgatgccgccaacgcgcctaccaccttccccgtcgaggcacccccg cttgaggaggaggaagtgattatcgagcaggacccaggttttgtaagcgaagac gacgaggaccgctcagtaccaacagaggataaaaagcaagaccaggacaac gcagaggcaaacgaggaacaagtcgggcgggggggacgaaaggcatggcga ctacctagatgtgggagacgacgtgctgttgaagcatctgcagcgccagtgcgc cattatctgcgacgcgttgcaagagcgcagcgatgtgcccctcgccatagcgga tgtcagccttgcctacgaacgccacctattctcaccgcgcgtaccccccaaacg ccaagaaaacggcacatgcgagcccaacccgcgcctcaacttctaccccgtat ttgccgtgccagaggtgcttgccacctatcacatctttttccaaaactgcaagatac ccctatcctgccgtgccaaccgcagccgagcggacaagcagctggccttgcg |
| | | gcagggcgctgtcatacctgatatcgcctcgctcaacgaagtgccaaaaatcttt gagggtcttggacgcgacgagaagcgcgcggcaaacgctctgcaacaggaa aacagcgaaaatgaaagtcactctggagtgttggtggaactcgagggtgacaa cgcgcgcctagccgtactaaaacgcagcatcgaggtcacccactttgcctaccc ggcacttaacctacccccaaggtcatgagcacagtcatgagtgagctgatcgt gcgccgtgcgcagcccctggagagggatgcaaatttgcaagaacaaacagag gagggcctacccgcagttggcgacgagcagctagcgcgctggcttcaaacgc gcgagcctgccgacttggaggagcgacgcaaactaatgatggccgcagtgctc gttaccgtggagcttgagtgcatgcagcggttctttgctgacccggagatgcagc gcaagctagaggaaacattgcactacacctttcgacagggctacgtacgccag gcctgcaagatctccaacgtggagctctgcaacctggtctcctaccttggaattt gcacgaaaaccgccttgggcaaaacgtgcttcattccacgctcaagggcgagg cgcgccgcgactacgtccgcgactgcgtttacttatttctatgctacacctggcag acggccatgggcgtttggcagcagtgcttggaggagtgcaacctcaaggagct gcagaaactgctaaagcaaaacttgaaggacctatggacggccttcaacgagc gctccgtggccgcgcacctggcggacatcattttccccgaacgcctgcttaaaa ccctgcaacagggtctgccagacttcaccagtcaaagcatgttgcagaactttag gaactttatcctagagcgctcaggaatcttgcccgccacctgctgtgcacttccta gcgactttgtgcccattaagtaccgcgaatgccctccgccgctttggggccactg ctaccttctgcagctagccaactaccttgcctaccactctgacataatggaagac gtgagcggtgacggtctactggagtgtcactgtcgctgcaacctatgcaccccg caccgctccctggtttgcaattcgcagctgcttaacgaaagtcaaattatcggtac ctttgagctgcagggtccctcgcctgacgaaaagtccgcggctccggggttgaa actcactccggggctgtggacgtcggcttaccttcgcaaatttgtacctgaggact accacgcccacgagattaggttctacgaagaccaatcccgcccgccaaatgcg gagcttaccgcctgcgtcattacccagggccacattcttggccaattgcaagcca tcaacaaagcccgccaagagtttctgctacgaaagggacggggggtttacttgg acccccagtccggcgaggagctcaacccaatccccccgccgccgcagcccta tcagcagcagccgcgggcccttgcttcccaggatggcacccaaaaagaagct gcagctgccgccgccacccacggacgaggaggaatactgggacagtcaggc agaggaggttttggacgaggaggaggaggacatgatggaagactgggagag cctagacgaggaagcttccgaggtcgaagaggtgtcagacgaaacaccgtca ccctcggtcgcattcccctcgccggcgccccagaaatcggcaaccggttccag catggctacaacctccgctcctcaggcgccgccggcactgcccgttcgccgac ccaaccgtagatgggacaccactggaaccagggccggtaagtccaagcagcc gccgccgttagcccaagagcaacaacagcgccaaggctaccgctcatggcgc gggcacaagaacgccatagttgcttgcttgcaagactgtgggggcaacatctcc ttcgcccgccgctttcttctctaccatcacggcgtggccttcccccgtaacatcctg cattactaccgtcatctctacagcccatactgcaccggcggcagcggcagcgg cagcaacagcagcggccacacagaagcaaaggcgaccggatagcaagactc tgacaaagcccaagaaatccacagcggcggcagcagcaggaggaggagcg ctgcgtctggcgcccaacgaacccgtatcgacccgcgagcttagaaacaggat ttttcccactctgtatgctatatttcaacagagcaggggccaagaacaagagctga aaataaaaaacaggtctctgcgatccctcacccgcagctgcctgtatcacaaaa gcgaagatcagcttcggcgcacgctggaagacgcggaggctctcttcagtaaa tactgcgcgctgactcttaaggactagtttcgcgccctttctcaaatttaagcgcga aaactacgtcatctccagcggccacacccggcgccagcacctgtcgtcagcgccatt atagcaaggaaattcccacgccctacatgtggagttaccagccacaaatgggacttgc |
| | | ggctggagctgcccaagactactcaacccgaataaactacatgagcgcggacccca catgatatcccgggtcaacggaatccgcgcccttcgatgaaatatttgccacatcctctta cactttttcatacattgcccaagaataaagaatcgtttgtgttatgtttcaacgtgtttatttttca attgcagaaaatttcaagtcatttttcattcagtagtatagccccaccaccacatagcttata cagatcaccgtaccttaatcaaactcacagaaccctagtattcaacctgccacctcc ctcccaacacacagagtacacagtcctttctccccggctggccttaaaagcatc atatcatgggtaacagacatattcttaggtgttatattccacacggtttcctgtcgag ccaaacgctcatcagtgatattaataaactccccgggcagctcacttaagttcatg tcgctgtccagctgctgagccacaggctgctgtccaacttgcggttgcttaacgg gcggcgaaggagaagtccacgcctacatgggggtagagtcataatcgtgcatc aggatagggcggtggtgctgcagcagcgcgaataaactgctgccgccgcc gctccgtcctgcaggaatacaacatggcagtggtctcctcagcgatgattcgcac cgcccgcagcataaggcgccttgtcctccgggcacagcagcgcaccctgatct cacttaaatcagcacagtaactgcagcacagcaccacaatattgttcaaaatccc acagtgcaaggcgctgtatccaaagctcatggcggggaccacagaacccacg tggccatcataccacaagcgcaggtagattaagtggcgacccctcataaacacg ctggacataaacattacctcttttggcatgttgtaattcaccacctcccggtaccata taaacctctgattaaacatggcgccatccaccaccatcctaaaccagctggcca aaacctgcccgccggctatacactgcagggaaccgggactggaacaatgaca gtggagagcccaggactcgtaaccatggatcatcatgctcgtcatgatatcaatg ttggcacaacacaggcacacgtgcatacacttcctcaggattacaagctcctccc gcgttagaaccatatcccagggaacaacccattcctgaatcagcgtaaatccca cactgcagggaagacctcgcacgtaactcacgttgtgcattgtcaaagtgttaca ttcgggcagcagcggatgatcctccagtatggtagcgcgggtttctgtctcaaaa ggaggtagacgatccctactgtacggagtgcgccgagacaaccgagatcgtgt tggtcgtagtgtcatgccaaatggaacgccggacgtagtcatatttcctgaagca aaaccaggtgcgggcgtgacaaacagatctgcgtctccggtctcgccgcttaga tcgctctgtgtagtagttgtagtatatccactctctcaaagcatccaggcgcccct ggcttcgggttctatgtaaactccttcatgcgccgctgccctgataacatccacca ccgcagaataagccacacccagccaacctacacattcgttctgcgagtcacaca cgggaggagcgggaagagctggaagaaccatgtttttttttattccaaaagatta tccaaaacctcaaaatgaagatctattaagtgaacgcgctcccctccggtggcgt ggtcaaactctacagccaaagaacagataatggcatttgtaagatgttgcacaat ggcttccaaaaggcaaacggccctcacgtccaagtggacgtaaaggctaaacc cttcagggtgaatctcctctataaacattccagcaccttcaaccatgcccaaataat tctcatctcgccaccttctcaatatatctctaagcaaatcccgaatattaagtccgg ccattgtaaaaatctgctccagagcgccctccaccttcagcctcaagcagcgaat catgattgcaaaaattcaggttcctcacagacctgtataagattcaaaagcggaa cattaacaaaaataccgcgatcccgtaggtcccttcgcagggccagctgaacat aatcgtgcaggtctgcacggaccagcgcggccacttccccgccaggaaccat gacaaaagaacccacactgattatgacacgcatactcggagctatgctaaccag cgtagccccgatgtaagcttgttgcatgggcggcgatataaaatgcaaggtgctg ctcaaaaaatcaggcaaagcctcgcgcaaaaaagaaagcacatcgtagtcatg ctcatgcagataaaggcaggtaagctccggaaccaccacagaaaaagacacc atttttctctcaaacatgtctgcgggtttctgcataaacacaaaataaaataacaaa aaaacatttaaacattagaagcctgtcttacaacaggaaaaacaacccttataag cataagacggactacggccatgccggcgtgaccgtaaaaaaactggtcaccgt gattaaaaagcaccaccgacagctcctcggtcatgtccggagtcataatgtaag |
| | | actcggtaaacacatcaggttgattcacatcggtcagtgctaaaaagcgaccga aatagcccgggggaatacatacccgcaggcgtagagacaacattacagcccc cataggaggtataacaaaattaataggagagaaaaacacataaacacctgaaa aaccctcctgcctaggcaaaatagcaccctcccgctccagaacaacatacagc gcttccacagcggcagccataacagtcagccttaccagtaaaaaagaaaacctatt aaaaaaacaccactcgacacggcaccagctcaatcagtcacagtgtaaaaaagggc caagtgcagagcgagtatatataggactaaaaaatgacgtaacggttaaagtccacaa aaaacacccagaaaaccgcacgcgaacctacgcccagaaacgaaagccaaaaa acccacaacttcctcaaatcgtcacttccgttttcccacgttacgtaacttcccattttaaga aaactacaattcccaacacatacaagttactccgccctaaaacctacgtcacccgcc ccgttcccacgccccgcgccacgtcacaaactccaccccctcattatcatattgg cttcaatccaaaataaggtatattattgatgatgatttaaatcagtcgcgatcg |
| 7 | L-22K protein | MAPKKKLQLPPPPTDEEEYWDSQAEEVLDEEEEDMMEDWES LDEEASEVEEVSDETPSPSVAFPSPAPQKSATGSSMATTSAP QAPPALPVRRPNRRWDTTGTRAGKSKQPPPLAQEQQQRQG YRSWRGHKNAIVACLQDCGGNISFARRFLLYHHGVAFPRNILH YYRHLYSPYCTGGSGSGSNSSGHTEAKATG |
| 8 | L-33K protein | MAPKKKLQLPPPPTDEEEYWDSQAEEVLDEEEEDMMEDWES LDEEASEVEEVSDETPSPSVAFPSPAPQKSATGSSMATTSAP QAPPALPVRRPNRRWDTTGTRAAHTAPAAAAAAATAAATQK QRRPDSKTLTKPKKSTAAAAAGGGALRLAPNEPVSTRELRNRI FPTLYAIFQQSRGQEQELKIKNRSLRSLTRSCLYHKSEDQLRR TLEDAEALFSKYCALTLKD |
| 9 | L4-100K protein | MESVEKKDSLTAPSEFATTASTDAANAPTTFPVEAPPLEEEEVI IEQDPGFVSEDDEDRSVPTEDKKQDQDNAEANEEQVGRGDE RHGDYLDVGDDVLLKHLQRQCAIICDALQERSDVPLAIADVSL AYERHLFSPRVPPKRQENGTCEPNPRLNFYPVFAVPEVLATY HIFFQNCKIPLSCRANRSRADKQLALRQGAVIPDIASLNEVPKIF EGLGRDEKRAANALQQENSENESHSGVLVELEGDNARLAVLK RSIEVTHFAYPALNLPPKVMSTVMSELIVRRAQPLERDANLQE QTEEGLPAVGDEQLARWLQTREPADLEERRKLMMAAVLVTVE LECMQRFFADPEMQRKLEETLHYTFRQGYVRQACKISNVELC NLVSYLGILHENRLGQNVLHSTLKGEARRDYVRDCVYLFLCYT WQTAMGVWQQCLEECNLKELQKLLKQNLKDLWTAFNERSVA AHLADIIFPERLLKTLQQGLPDFTSQSMLQNFRNFILERSGILPA TCCALPSDFVPIKYRECPPPLWGHCYLLQLANYLAYHSDIMED VSGDGLLECHCRCNLCTPHRSLVCNSQLLNESQIIGTFELQGP SPDEKSAAPGLKLTPGLWTSAYLRKFVPEDYHAHEIRFYEDQS RPPNAELTACVITQGHILGQLQAINKARQEFLLRKGRGVYLDP QSGEELNPIPPPPQPYQQQPRALASQDGTQKEAAAAATHGR GGILGQSGRGGFGRGGGHDGRLGEPRRGSFRGRRGVRRN TVTLGRIPLAGAPEIGNRFQHGYNLRSSGAAGTARSPTQP |
| 10 | L5-fiber protein | MKRARPSEDTFNPVYPYDTETGPPTVPFLTPPFVSPNGFQES PPGVLSLRLSEPLVTSNGMLALKMGNGLSLDEAGNLTSQNVT TVSPPLKKTKSNINLEISAPLTVTSEALTVAAAAPLMVAGNTLT MQSQAPLTVHDSKLSIATQGPLTVSEGKLALQTSGPLTTTDSS TLTITASPPLTTATGSLGIDLKEPIYTQNGKLGLKYGAPLHVTDD LNTLTVATGPGVTINNTSLQTKVTGALGFDSQGNMQLNVAGG |
| | | LRIDSQNRRLILDVSYPFDAQNQLNLRLGQGPLFINSAHNLDIN YNKGLYLFTASNNSKKLEVNLSTAKGLMFDATAIAINAGDGLEF GSPNAPNTNPLKTKIGHGLEFDSNKAMVPKLGTGLSFDSTGAI TVGNKNNDKLTLWTTPAPSPNCRLNAEKDAKLTLVLTKCGSQI LATVSVLAVKGSLAPISGTVQSAHLIIRFDENGVLLNNSFLDPEY WNFRNGDLTEGTAYTNAVGFMPNLSAYPKSHGKTAKSNIVSQ VYLNGDKTKPVTLTITLNGTQETGDTTPSAYSMSFSWDWSGH NYINEIFATSSYTFSYIAQE |
| 11 | pVIII protein | MSKEIPTPYMWSYQPQMGLAAGAAQDYSTRINYMSAGPHMIS RVNGIRAHRNRILLEQAAITTTPRNNLNPRSWPAALVYQESPA PTTVVLPRDAQAEVQMTNSGAQLAGGFRHRVRSPGQGITHLT IRGRGIQLNDESVSSSLGLRPDGTFQIGGAGRPSFTPRQAILTL QTSSSEPRSGGIGTLQFIEEFVPSVYFNPFSGPPGHYPDQFIP NFDAVKDSADGYD |
| 12 | L1-52/55K protein | MHPVLRQMRPTPPATTATAAVAGAGASQPQPQTEMDLEEGE GLARLGAPSPERHPRVQLQKDVRPAYVPAQNLFRDRSGEEP EEMRDCRFRAGRELREGLDRQRVLRDEDFEPNEQTGISPARA HVAAANLVTAYEQTVKQERNFQKSFNNHVRTLIAREEVALGLM HLWDLAEAIVQNPDSKPLTAQLFLVVQHSRDNEAFREALLNIA EPEGRWLLELINILQSIVVQERSLSLAEKVAAINYSVLSLGKFYA RKIYKTPYVPIDKEVKIDSFYMRMALKVLTLSDDLGVYRNDRIH KAVSTSRRRELSDRELMLSLRRALVGGAAGGEESYFDMGADL HWQPSRRALEAAYGPEDLDEEEEEEEDAPAAGY |
| 13 | pTP | MALSVNDCARLTGQSVPTMEHFLPLRNIWNRVRDFPRASTTA AGITWMSRYIYGYHRLMLEDLAPGAPATLRWPLYRQPPPHFL VGYQYLVRTCNDYVFDSRAYSRLRYTELSQPGHQTVNWSVM ANCTYTINTGAYHRFVDMDDFQSTLTQVQQAILAERVVADLAL LQPMRGFGVTRMGGRGRHLRPNSAAAAAIDARDAGQEEGEE EVPVERLMQDYYKDLRRCQNEAWGMADRLRIQQAGPKDMVL LSTIRRLKTAYFNYIISSTSARNNPDRRPLPPATVLSLPCDCDW LDAFLERFSDPVDADSLRSLGGGVPTQQLLRCIVSAVSLPHGS PPPTHNRDMTGGVFQLRPRENGRAVTETMRRRRGEMIERFV DRLPVRRRRRRVPPPPPPPEEEEGEALMEEEIEEEEEAPVAF EREVRDTVAELIRLLEEELTVSARNSQFFNFAVDFYEAMERLE ALGDINESTLRRWVMYFFVAEHTATTLNYLFQRLRNYAVFARH VELNLAQVVMRARDAEGGVVYSRVWNEGGLNAFSQLMARIS NDLAATVERAGRGDLQEEEIEQFMAEIAYQDNSGDVQEILRQA AVNDTEIDSVELSFRLKLTGPVVFTQRRQIQEINRRVVAFASNL RAQHQLLPARGADVPLPPLPAGPEPPLPPGARPRHRF |
| 14 | E3 12.5k protein | MLSGEAEQLRLKHLVHCRRHKCFARDSGEFCYFELPEDHIEG PAHGVRLTAQGELARSLIREFTQRPLLVERDRGPCVLTVICNC PNLGLHQDLCCHLCAEYNKYRN |
| 15 | E3 CR1-α protein | MNNSSNSTGYSNSGFSRIGVGVILCLVILFILILTLLCLRLAACCV HICIYCQLFKRWGRHP |
| 16 | E3 gp19k protein | MIRYIILGLLTLASAHGTTQKVDFKEPACNVTFAAEANECTTLIK CTTEHEKLLIRHKNKIGKYAVYAIWQPGDTTEYNVTVFQGKSH KTFMYTFPFYEMCDITMYMSKQYKLWPPQNCVENTGTFCCTA MLITVLALVCTLLYIKYKSRRSFIEEKKMP |
| 17 | E3 10.5k protein | MTNTTNAAAATGLTSTTNTPQVSAFVNNWDNLGMWWFSIAL MFVCLIIMWLICCLKRKRARPPIYSPIIVLHPNNDGIHRLDGLKH MFFSLTV |
| 18 | E3 RID-α protein | MIPRVFILLTLVALFCACSTLAAVSHIEVDCIPAFTVYLLYGFVTL TLICSLITVVIAFIQCIDWVCVRFAYLRHHPQYRDRTIAELLRIL |
| 19 | E3-RID-β protein | MKFTVTFLLIICTLSAFCSPTSKPQRHISCRFTRIWNIPSCYNEK SDLSEAWLYAIISVMVFCSTILALAIYPYLDIGWKRIDAMNHPTF PAPAMLPLQQVVAGGFVPANQPRPTSPTPTEISYFNLTGGDD |
| 20 | E3 14.7k protein | MTDTLDLEMDGIITEQRLLERRRAAAEQQRMNQELQDMVNLH QCKRGIFCLVKQAKVTYDSNTTGHRLSYKLPTKRQKLVVMVG EKPITITQHSVETEGCIHSPCQGPEDLCTLIKTLCGLKDLIPFN |
| 21 | E3 region full-length sequence | ccccgtagttggcccgctgccctggtgtaccaggaaagtcccgctcccaccactgtggt acttcccagagacgcccaggccgaagttcagatgactaactcaggggcgcagcttgc gggcggctttcgtcacagggtgcggtcgcccgggcagggtataactcacctgacaatc agagggcgaggtattcagctcaacgacgagtcggtgagctcctcgcttggtctccgtcc ggacgggacatttcagatcggcggcgccggccgtccttcattcacgcctcgtcaggcaa tcctaactctgcagacctcgtcctctgagccgcgctctggaggcattggaactctgcaattt attgaggagtttgtgccatcggtctactttaacccccttctcgggacctcccggccactatcc ggatcaatttattcctaactttgacgcggtaaaggactcggcggacggctacgactgaat gttaagtggagaggcagagcaactgcgcctgaaacacctggtccactgtcgccgcca caagtgctttgcccgcgactccggtgagttttgctactttgaattgcccgaggatcatatcg agggcccggcgcacggcgtccggcttaccgcccagggagagcttgcccgtagcctga ttcgggagtttacccagcgccccctgctagttgagcgggacaggggaccctgtgttctca ctgtgatttgcaactgtcctaaccttggattacatcaagatctttgttgccatctctgtgctgag tataataaatacagaaattaaaatatactggggctcctatcgccatcctgtaaacgccac cgtcttcacccgcccaagcaaaccaaggcgaaccttacctggtacttttaacatctctccc tctgtgatttacaacagtttcaacccagacggagtgagtctacgagagaacctctccgag ctcagctactccatcagaaaaaacaccaccctccttacctgccgggaacgtacgagtg cgtcaccggccgctgcaccacacctaccgcctgaccgtaaaccagacttttccggaca gacctcaataactctgtttaccagaacaggaggtgagcttagaaaacccttagggtatta ggccaaaggcgcagctactgtggggtttatgaacaattcaagcaactctacggctattc taattcaggtttctctagaatcggggttggggttattctctgtcttgtgattctctttattcttatact aacgcttctctgcctaaggctcgccgcctgctgtgtgcacatttgcatttattgtcagcttttta aacgctggggtcgccacccaagatgattaggtacataatcctaggtttactcacccttgc gtcagcccacggtaccacccaaaaggtggattttaaggagccagcctgtaatgttacatt cgcagctgaagctaatgagtgcaccactcttataaaatgcaccacagaacatgaaaag ctgcttattcgccacaaaaacaaaattggcaagtatgctgtttatgctatttggcagccag gtgacactacagagtataatgttacagttttccagggtaaaagtcataaaacttttatgtat acttttccattttatgaaatgtgcgacattaccatgtacatgagcaaacagtataagttgtgg cccccacaaaattgtgtggaaaacactggcactttctgctgcactgctatgctaattacag tgctcgctttggtctgtaccctactctatattaaatacaaaagcagacgcagctttattgagg aaaagaaaatgccttaatttactaagttacaaagctaatgtcaccactaactgctttactc gctgcttgcaaaacaaattcaaaaagttagcattataattagaataggatttaaaccccc cggtcatttcctgctcaataccattcccctgaacaattgactctatgtgggatatgctccagc gctacaaccttgaagtcaggcttcctggatgtcagcatctgactttggccagcacctgtcc cgcggatttgttccagtccaactacagcgacccaccctaacagagatgaccaacacaa ccaacgcggccgccgctaccggacttacatctaccacaaatacaccccaagtttctgcc tttgtcaataactgggataacttgggcatgtggtggttctccatagcgcttatgtttgtatgcct |
| | | tattattatgtggctcatctgctgcctaaagcgcaaacgcgcccgaccacccatctatagt cccatcattgtgctacacccaaacaatgatggaatccatagattggacggactgaaaca catgttcttttctcttacagtatgattaaatgagacatgattcctcgagtttttatattactgacc cttgttgcgctttttttgtgcgtgctccacattggctgcggtttctcacatcgaagtagactgcat tccagccttcacagtctatttgctttacggatttgtcaccctcacgctcatctgcagcctcatc actgtggtcatcgcctttatccagtgcattgactgggtctgtgtgcgctttgcatatctcagac accatccccagtacagggacaggactatagctgagcttcttagaattctttaattatgaaa tttactgtgacttttctgctgattatttgcaccctatctgcgttttgttccccgacctccaagcctc aaagacatatatcatgcagattcactcgtatatggaatattccaagttgctacaatgaaaa aagcgatctttccgaagcctggttatatgcaatcatctctgttatggtgttctgcagtaccatc ttagccctagctatatatccctaccttgacattggctggaaacgaatagatgccatgaacc acccaactttccccgcgcccgctatgcttccactgcaacaagttgttgccggcggctttgtc ccagccaatcagcctcgccccacttctcccacccccactgaaatcagctactttaatcta acaggaggagatgactgacaccctagatctagaaatggacggaattattacagagca gcgcctgctagaaagacgcagggcagcggccgagcaacagcgcatgaatcaagag ctccaagacatggttaacttgcaccagtgcaaaaggggtatcttttgtctggtaaagcag gccaaagtcacctacgacagtaataccaccggacaccgccttagctacaagttgccaa ccaagcgtcagaaattggtggtcatggtgggagaaaagcccattaccataactcagca ctcggtagaaaccgaaggctgcattcactcaccttgtcaaggacctgaggatctctgca cccttattaagaccctgtgcggtctcaaagatcttattccctttaactaataaaaaaaaata ataaag |
| 22 | Poly A site sequence | aataaaatatctttattttcattacatctgtgtgttggttttttgtgtg |
| 23 | ITR sequence | ctacgtcacccgccccgttcccacgccccgcgccacgtcacaaactccaccccctcatt atcatattggcttcaatccaaaataaggtatattattgatgatg |
| 24 | VA RNA I sequences equence | agcgggcactcttccgtggtctggtggataaattcgcaagggtatcatggcggacgacc ggggttcgagccccgtatccggccgtccgccgtgatccatgcggttaccgcccgcgtgt cgaacccaggtgtgcgacgtcagacaacgggggagtgctcccttt |
| 25 | VA RNA II sequence | tggctcgctccctgtagccggagggttattttccaagggttgagtcgcgggacccccggtt cgagtctcggaccggccggactgcggcgaacgggggtttgcctccccgtcatgcaaga ccccgcttgcaaattcctccggaaacagggacgagcccctttt |
| 26 | VA RNA full-length sequence | ttgcgcagcggcaaaaagtgctccatggtcgggacgctctggccggtcaggcgcgcg caatcgttgacgctctagaccgtgcaaaaggagagcctgtaagcgggcactcttccgtg gtctggtggataaattcgcaagggtatcatggcggacgaccggggttcgagccccgtat ccggccgtccgccgtgatccatgcggttaccgcccgcgtgtcgaacccaggtgtgcga cgtcagacaacgggggagtgctccttttggcttccttccaggcgcggcggctgctgcgct agctttttggccactggccgcgcgcagcgtaagcggttaggctggaaagcgaaagcat taagtggctcgctccctgtagccggagggttattttccaagggttgagtcgcgggacccc cggttcgagtctcggaccggccggactgcggcgaacgggggtttgcctccccgtcatgc aagaccccgcttgcaaattcctccggaaacagggacgagcccctttttgcttttcccag |
| 27 | DBP nucleotide sequence | ttaaaaatcaaaggggttctgccgcgcatcgctatgcgccactggcagggacacgttgc gatactggtgtttagtgctccacttaaactcaggcacaaccatccgcggcagctcggtga agttttcactccacaggctgcgcaccatcaccaacgcgtttagcaggtcgggcgccgat atcttgaagtcgcagttggggcctccgccctgcgcgcgcgagttgcgatacacagggtt gcagcactggaacactatcagcgccgggtggtgcacgctggccagcacgctcttgtcg gagatcagatccgcgtccaggtcctccgcgttgctcagggcgaacggagtcaactttgg tagctgccttcccaaaaagggcgcgtgcccaggctttgagttgcactcgcaccgtagtg gcatcaaaaggtgaccgtgcccggtctgggcgttaggatacagcgcctgcataaaagc |
| | | cttgatctgcttaaaagccacctgagcctttgcgccttcagagaagaacatgccgcaag acttgccggaaaactgattggccggacaggccgcgtcgtgcacgcagcaccttgcgtc ggtgttggagatctgcaccacatttcggccccaccggttcttcacgatcttggccttgctag actgctccttcagcgcgcgctgcccgttttcgctcgtcacatccatttcaatcacgtgctcctt atttatcataatgcttccgtgtagacacttaagctcgccttcgatctcagcgcagcggtgca gccacaacgcgcagcccgtgggctcgtgatgcttgtaggtcacctctgcaaacgactgc aggtacgcctgcaggaatcgccccatcatcgtcacaaaggtcttgttgctggtgaaggtc agctgcaacccgcggtgctcctcgttcagccaggtcttgcatacggccgccagagcttc cacttggtcaggcagtagtttgaagttcgcctttagatcgttatccacgtggtacttgtccatc agcgcgcgcgcagcctccatgcccttctcccacgcagacacgatcggcacactcagc gggttcatcaccgtaatttcactttccgcttcgctgggctcttcctcttcctcttgcgtccgcat accacgcgccactgggtcgtcttcattcagccgccgcactgtgcgcttacctcctttgccat gcttgattagcaccggtgggttgctgaaacccaccatttgtagcgccacatcttctctttcttc ctcgctgtccacgattacctctggtgatggcgggcgctcgggcttgggagaagggcgctt ctttttcttcttgggcgcaatggccaaatccgccgccgaggtcgatggccgcgggctggg tgtgcgcggcaccagcgcgtcttgtgatgagtcttcctcgtcctcggactcgatacgccgc ctcatccgctttttttgggggcgcccggggaggcggcggcgacggggacggggacgac acgtcctccatggttgggggacgtcgcgccgcaccgcgtccgcgctcgggggtggtttc gcgctgctcctcttcccgactggccat |
| 28 | DBP protein | MASREEEQRETTPERGRGAARRPPTMEDVSSPSPSPPPPRA PPKKRMRRRIESEDEEDSSQDALVPRTPSPRPSTSAADLAIAP KKKKKRPSPKPERPPSPEVIVDSEEEREDVALQMVGFSNPPV LIKHGKGGKRTVRRLNEDDPVARGMRTQEEEEEPSEAESEIT VMNPLSVPIVSAWEKGMEAARALMDKYHVDNDLKANFKLLPD QVEALAAVCKTWLNEEHRGLQLTFTSNKTFVTMMGRFLQAYL QSFAEVTYKHHEPTGCALWLHRCAEIEGELKCLHGSIMINKEH VIEMDVTSENGQRALKEQSSKAKIVKNRWGRNVVQISNTDAR CCVHDAACPANQFSGKSCGMFFSEGAKAQVAFKQIKAFMQA LYPNAQTGHGHLLMPLRCECNSKPGHAPFLGRQLPKLTPFAL SNAEDLDADLISDKSVLASVHHPALIVFQCCNPVYRNSRAQGG GPNCDFKISAPDLLNALVMVRSLWSENFTELPRMVVPEFKWS TKHQYRNVSLPVAHSDARQNPFDF |
| 29 | UXP exon 3 sequence | aataaaggcaaatgcttttatttgtacactctcgggtgattatttacccccacccttgccgtct gcgccgtttaaaaatcaaaggggttctgccgcgcatcgctatgcgccactggcaggga cacgttgcgatactggtgtttagtgctccacttaaactcaggcacaaccatccgcggcag ctcggtgaagttttcactccacaggctgcgcaccatcaccaacgcgtttagcaggtcggg cgccgatatcttgaagtcgcagttggggcctccgccctgcgcgcgcgagttgcgataca cagggttgcagcactggaacactatcagcgccgggtggtgcacgctggccagcacgc tcttgtcggagatcagatccgcgtccaggtcctccgcgttgctcagggcgaacggagtc aactttggtagctgccttcccaaaaagggcgcgtgcccaggctttgagttgcactcgcac cgtagtggcatcaaaaggtgaccgtgcccggtctgggcgttaggatacagcgcctgcat aaaagccttgatctgcttaaaagccacctgagcctttgcgccttcagagaagaacatgc cgcaagacttgccggaaaactgattggccggacaggccgcgtcgtgcacgcagcacc ttgcgtcggtgttggagatctgcaccacatttcggccccaccggttcttcacgatcttggcct tgctagactgctccttcagcgcgcgctgcccgttttcgctcgtcacatccatttcaatcacgt gctccttatttatcataatgcttccgtgtagacacttaagctcgccttcgatctcagcgcagc ggtgcagccacaacgcgcagcccgtgggctcgtgatgcttgtaggtcacctctgcaaac gactgcaggtacgcctgcaggaatcgccccatcatcgtcacaaaggtcttgttgctggtg |
| | | aaggtcagctgcaacccgcggtgctcctcgttcagccaggtcttgcatacggccgccag agcttccacttggtcaggcagtagtttgaagttcgcctttagatcgttatccacgtggtacttg tccatcagcgcgcgcgcagcctccatgcccttctcccacgcagacacgatcggcacac tcagcgggttcatcaccgtaatttcactttccgcttcgctgggctcttcctcttcctcttgcgtcc gcataccacgcgccactgggtcgtcttcattcagccgccgcactgtgcgcttacctcctttg ccatgcttgattagcaccggtgggttgctgaaacccaccatttgtagcgccacatcttctctt tcttcctcgctgtccacgattacctctggtgatggcgggcgctcgggcttgggagaaggg cgcttctttttcttcttgggcgcaatggccaaatccgccgccgaggtcgatggccgcgggc tgggtgtgcgcggcaccagcgcgtcttgtgatgagtcttcctcgtcctcggactcgatacg ccgcctcatccgcttttttgggggcgcccggggaggcggcggcgacggggacgggga cgacacgtcctccatggttgggggacgtcgcgccgcaccgcgtccgcgctcgggggtg gtttcgcgctgctcctcttcccgactggccatttccttctc |
| 30 | E2a exon2/lead er sequence | ctgatatcgcctcgctcaacgaagtgccaaaaatctttgagggtcttggacgcgacgag aagcgcgcggcaaacgct |
| 31 | E2a late promoter sequence | tggacgtcggcttaccttcgcaaatttgtacctgaggactaccacgcccacgagattagg ttctacgaagaccaatcccgcccgccaaatgcggagcttaccgcctgcgtcattaccca gggccacattcttggccaattgcaagccatcaacaaagcccg |
| 32 | E2 exon 1 sequence | cgcacgctggaagacgcggaggctctctt |
| 33 | E2a early promoter sequence | tcagtaaatactgcgcgctgactcttaaggactagtttcgcgccctttctcaaatttaagcg cgaaaactacgtcatctcc |
| 34 | E2 late primary transcript | aataaaggcaaatgcttttatttgtacactctcgggtgattatttacccccacccttgccgtct gcgccgtttaaaaatcaaaggggttctgccgcgcatcgctatgcgccactggcaggga cacgttgcgatactggtgtttagtgctccacttaaactcaggcacaaccatccgcggcag ctcggtgaagttttcactccacaggctgcgcaccatcaccaacgcgtttagcaggtcggg cgccgatatcttgaagtcgcagttggggcctccgccctgcgcgcgcgagttgcgataca cagggttgcagcactggaacactatcagcgccgggtggtgcacgctggccagcacgc tcttgtcggagatcagatccgcgtccaggtcctccgcgttgctcagggcgaacggagtc aactttggtagctgccttcccaaaaagggcgcgtgcccaggctttgagttgcactcgcac cgtagtggcatcaaaaggtgaccgtgcccggtctgggcgttaggatacagcgcctgcat aaaagccttgatctgcttaaaagccacctgagcctttgcgccttcagagaagaacatgc cgcaagacttgccggaaaactgattggccggacaggccgcgtcgtgcacgcagcacc ttgcgtcggtgttggagatctgcaccacatttcggccccaccggttcttcacgatcttggcct tgctagactgctccttcagcgcgcgctgcccgttttcgctcgtcacatccatttcaatcacgt gctccttatttatcataatgcttccgtgtagacacttaagctcgccttcgatctcagcgcagc ggtgcagccacaacgcgcagcccgtgggctcgtgatgcttgtaggtcacctctgcaaac gactgcaggtacgcctgcaggaatcgccccatcatcgtcacaaaggtcttgttgctggtg aaggtcagctgcaacccgcggtgctcctcgttcagccaggtcttgcatacggccgccag agcttccacttggtcaggcagtagtttgaagttcgcctttagatcgttatccacgtggtacttg tccatcagcgcgcgcgcagcctccatgcccttctcccacgcagacacgatcggcacac tcagcgggttcatcaccgtaatttcactttccgcttcgctgggctcttcctcttcctcttgcgtcc gcataccacgcgccactgggtcgtcttcattcagccgccgcactgtgcgcttacctcctttg ccatgcttgattagcaccggtgggttgctgaaacccaccatttgtagcgccacatcttctctt tcttcctcgctgtccacgattacctctggtgatggcgggcgctcgggcttgggagaaggg cgcttctttttcttcttgggcgcaatggccaaatccgccgccgaggtcgatggccgcgggc |
| | | tgggtgtgcgcggcaccagcgcgtcttgtgatgagtcttcctcgtcctcggactcgatacg ccgcctcatccgctttttggggcgcccggggaggcggcggcgacggggacgggga cgacacgtcctccatggttgggggacgtcgcgccgcaccgcgtccgcgctcgggggtg gtttcgcgctgctcctcttcccgactggccatttccttctcctataggcagaaaaagatcata gtcagtcgagaagaaggacagcctaaccgccccctctgagttcgccaccaccgcctcc accgatgccgccaacgcgcctaccaccttccccgtcgaggcacccccgcttgaggag gaggaagtgattatcgagcaggacccaggttttgtaagcgaagacgacgaggaccgc tcagtaccaacagaggataaaaagcaagaccaggacaacgcagaggcaaacgag gaacaagtcgggcgggggacgaaaggcatggcgactacctagatgtgggagacg acgtgctgttgaagcatctgcagcgccagtgcgccattatctgcgacgcgttgcaagag cgcagcgatgtgcccctcgccatagcggatgtcagccttgcctacgaacgccacctatt ctcaccgcgcgtacccccaaacgccaagaaaacggcacatgcgagcccaacccg cgcctcaacttctaccccgtatttgccgtgccagaggtgcttgccacctatcacatctttttcc aaaactgcaagatacccctatcctgccgtgccaaccgcagccgagcggacaagcag ctggccttgcggcagggcgctgtcatacctgatatcgcctcgctcaacgaagtgccaaa aatctttgagggtcttggacgcgacgagaagcgcgcggcaaacgctctgcaacagga aaacagcgaaaatgaaagtcactctggagtgttggtggaactcgagggtgacaacgc gcgcctagccgtactaaaacgcagcatcgaggtcacccactttgcctacccggcactta acctacccccccaaggtcatgagcacagtcatgagtgagctgatcgtgcgccgtgcgca gcccctggagagggatgcaaatttgcaagaacaaacagaggagggcctacccgcag ttggcgacgagcagctagcgcgctggcttcaaacgcgcgagcctgccgacttggagg agcgacgcaaactaatgatggccgcagtgctcgttaccgtggagcttgagtgcatgcag cggttctttgctgacccggagatgcagcgcaagctagaggaaacattgcactacaccttt cgacagggctacgtacgccaggcctgcaagatctccaacgtggagctctgcaacctgg tctcctaccttggaattttgcacgaaaaccgccttgggcaaaacgtgcttcattccacgctc aagggcgaggcgcgccgcgactacgtccgcgactgcgtttacttatttctatgctacacct ggcagacggccatgggcgtttggcagcagtgcttggaggagtgcaacctcaaggagc tgcagaaactgctaaagcaaaacttgaaggacctatggacggccttcaacgagcgctc cgtggccgcgcacctggcggacatcattttccccgaacgcctgcttaaaaccctgcaac agggtctgccagacttcaccagtcaaagcatgttgcagaactttaggaactttatcctaga gcgctcaggaatcttgcccgccacctgctgtgcacttcctagcgactttgtgcccattaagt accgcgaatgccctccgccgctttggggccactgctaccttctgcagctagccaactacc ttgcctaccactctgacataatggaagacgtgagcggtgacggtctactggagtgtcact gtcgctgcaacctatgcaccccgcaccgctccctggtttgcaattcgcagctgcttaacg aaagtcaaattatcggtacctttgagctgcagggtccctcgcctgacgaaaagtccgcg gctccggggttgaaactcactccggggctg |
| 35 | E2 early primary transcript | aataaaggcaaatgcttttatttgtacactctcgggtgattatttacccccacccttgccgtct gcgccgtttaaaaatcaaaggggttctgccgcgcatcgctatgcgccactggcaggga cacgttgcgatactggtgtttagtgctccacttaaactcaggcacaaccatccgcggcag ctcggtgaagttttcactccacaggctgcgcaccatcaccaacgcgtttagcaggtcggg cgccgatatcttgaagtcgcagttggggcctccgccctgcgcgcgcgagttgcgataca cagggttgcagcactggaacactatcagcgccgggtggtgcacgctggccagcacgc tcttgtcggagatcagatccgcgtccaggtcctccgcgttgctcagggcgaacggagtc aactttggtagctgccttcccaaaaagggcgcgtgcccaggctttgagttgcactcgcac cgtagtggcatcaaaaggtgaccgtgcccggtctgggcgttaggatacagcgcctgcat aaaagccttgatctgcttaaaagccacctgagcctttgcgccttcagagaagaacatgc cgcaagacttgccggaaaactgattggccggacaggccgcgtcgtgcacgcagcacc ttgcgtcggtgttggagatctgcaccacatttcggccccaccggttcttcacgatcttggcct |
| | | tgctagactgctccttcagcgcgcgctgcccgttttcgctcgtcacatccatttcaatcacgt gctccttatttatcataatgcttccgtgtagacacttaagctcgccttcgatctcagcgcagc ggtgcagccacaacgcgcagcccgtgggctcgtgatgcttgtaggtcacctctgcaaac gactgcaggtacgcctgcaggaatcgccccatcatcgtcacaaaggtcttgttgctggtg aaggtcagctgcaacccgcggtgctcctcgttcagccaggtcttgcatacggccgccag agcttccacttggtcaggcagtagtttgaagttcgcctttagatcgttatccacgtggtacttg tccatcagcgcgcgcgcagcctccatgcccttctcccacgcagacacgatcggcacac tcagcgggttcatcaccgtaatttcactttccgcttcgctgggctcttcctcttcctcttgcgtcc gcataccacgcgccactgggtcgtcttcattcagccgccgcactgtgcgcttacctcctttg ccatgcttgattagcaccggtgggttgctgaaacccaccatttgtagcgccacatcttctctt tcttcctcgctgtccacgattacctctggtgatggcggcgctcgggcttgggagaaggg cgcttctttttcttcttgggcgcaatggccaaatccgccgccgaggtcgatggccgcgggc tgggtgtgcgcggcaccagcgcgtcttgtgatgagtcttcctcgtcctcggactcgatacg ccgcctcatccgcttttttgggggcgcccggggaggcggcggcgacggggacgggga cgacacgtcctccatggttgggggacgtcgcgccgcaccgcgtccgcgctcgggggtg gtttcgcgctgctcctcttcccgactggccatttccttctcctataggcagaaaaagatcata gtcagtcgagaagaaggacagcctaaccgcccctctgagttcgccaccaccgcctcc accgatgccgccaacgcgcctaccaccttccccgtcgaggcacccccgcttgaggag gaggaagtgattatcgagcaggacccaggttttgtaagcgaagacgacgaggaccgc tcagtaccaacagaggataaaaagcaagaccaggacaacgcagaggcaaacgag gaacaagtcgggcgggggacgaaaggcatggcgactacctagatgtgggagacg acgtgctgttgaagcatctgcagcgccagtgcgccattatctgcgacgcgttgcaagag cgcagcgatgtgcccctcgccatagcggatgtcagccttgcctacgaacgccacctatt ctcaccgcgcgtaccccccaaacgccaagaaaacggcacatgcgagcccaacccg cgcctcaacttctaccccgtatttgccgtgccagaggtgcttgccacctatcacatctttttcc aaaactgcaagatacccctatcctgccgtgccaaccgcagccgagcggacaagcag ctggccttgcggcagggcgctgtcatacctgatatcgcctcgctcaacgaagtgccaaa aatctttgagggtcttggacgcgacgagaagcgcgcggcaaacgctctgcaacagga aaacagcgaaaatgaaagtcactctggagtgttggtggaactcgagggtgacaacgc gcgcctagccgtactaaaacgcagcatcgaggtcacccactttgcctacccggcactta acctacccccccaaggtcatgagcacagtcatgagtgagctgatcgtgcgccgtgcgca gcccctggagagggatgcaaatttgcaagaacaaacagaggagggcctacccgcag ttggcgacgagcagctagcgcgctggcttcaaacgcgcgagcctgccgacttggagg agcgacgcaaactaatgatggccgcagtgctcgttaccgtggagcttgagtgcatgcag cggttctttgctgacccggagatgcagcgcaagctagaggaaacattgcactacaccttt cgacagggctacgtacgccaggcctgcaagatctccaacgtggagctctgcaacctgg tctcctaccttggaattttgcacgaaaaccgccttgggcaaaacgtgcttcattccacgctc aagggcgaggcgcgccgcgactacgtccgcgactgcgtttacttatttctatgctacacct ggcagacggccatgggcgtttggcagcagtgcttggaggagtgcaacctcaaggagc tgcagaaactgctaaagcaaaacttgaaggacctatggacggccttcaacgagcgctc cgtggccgcgcacctggcggacatcattttccccgaacgcctgcttaaaaccctgcaac agggtctgccagacttcaccagtcaaagcatgttgcagaactttaggaactttatcctaga gcgctcaggaatcttgcccgccacctgctgtgcacttcctagcgactttgtgcccattaagt accgcgaatgccctccgccgctttggggccactgctaccttctgcagctagccaactacc ttgcctaccactctgacataatggaagacgtgagcggtgacggtctactggagtgtcact gtcgctgcaacctatgcaccccgcaccgctccctggtttgcaattcgcagctgcttaacg aaagtcaaattatcggtacctttgagctgcagggtccctcgcctgacgaaaagtccgcg gctccggggttgaaactcactccggggctgtggacgtcggcttaccttcgcaaatttgtac |
| | | ctgaggactaccacgcccacgagattaggttctacgaagaccaatcccgcccgccaa atgcggagcttaccgcctgcgtcattacccagggccacattcttggccaattgcaagcca tcaacaaagcccgccaagagtttctgctacgaaagggacgggggtttacttggaccc ccagtccggcgaggagctcaacccaatccccccgccgccgcagccctatcagcagc agccgcgggcccttgcttcccaggatcacccaaaaagaagctgcagctgccgccgcc acccacggacgaggaggaatactgggacagtcaggcagaggaggttttggacgagg aggaggaggacatgatggaagactgggagagcctagacgaggaagcttccgaggtc gaagaggtgtcagacgaaacaccgtcaccctcggtcgcattcccctcgccggcgcccc agaaatcggcaaccggttccagcatggctacaacctccgctcctcaggcgccgccgg cactgcccgttcgccgacccaaccgtagatgggacaccactggaaccaggccggta agtccaagcagccgccgccgttagcccaagagcaacaacagcgccaaggctaccg ctcatggcgcgggcacaagaacgccatagttgcttgcttgcaagactgtgggggcaac atctccttcgcccgccgctttcttctctaccatcacggcgtggccttcccccgtaacatcctg cattactaccgtcatctctacagcccatactgcaccggcggcagcggcagcggcagca acagcagcggccacacagaagcaaaggcgaccggatagcaagactctgacaaag cccaagaaatccacagcggcggcagcagcaggaggaggagcgctgcgtctggcgc ccaacgaacccgtatcgacccgcgagcttagaaacaggatttttcccactctgtatgctat atttcaacagagcaggggccaagaacaagagctgaaataaaaaacaggtctctgc gatccctcacccgcagctgcctgtatcacaaaagcgaagatcagcttcggcgcacgct ggaagacgcggaggctctctt |
| 36 | E2 region full-length (pHAD1-5) | aataaaggcaaatgcttttatttgtacactctcgggtgattatttacccccacccttgccgtct gcgccgtttaaaaatcaaaggggttctgccgcgcatcgctatgcgccactggcaggga cacgttgcgatactggtgtttagtgctccacttaaactcaggcacaaccatccgcggcag ctcggtgaagttttcactccacaggctgcgcaccatcaccaacgcgtttagcaggtcggg cgccgatatcttgaagtcgcagttggggcctccgccctgcgcgcgcgagttgcgataca cagggttgcagcactggaacactatcagcgccgggtggtgcacgctggccagcacgc tcttgtcggagatcagatccgcgtccaggtcctccgcgttgctcagggcgaacggagtc aactttggtagctgccttcccaaaaagggcgcgtgcccaggctttgagttgcactcgcac cgtagtggcatcaaaaggtgaccgtgcccggtctgggcgttaggatacagcgcctgcat aaaagccttgatctgcttaaaagccacctgagcctttgcgccttcagagaagaacatgc cgcaagacttgccggaaaactgattggccggacaggccgcgtcgtgcacgcagcacc ttgcgtcggtgttggagatctgcaccacatttcggccccaccggttcttcacgatcttggcct tgctagactgctccttcagcgcgcgctgcccgttttcgctcgtcacatccatttcaatcacgt gctccttatttatcataatgcttccgtgtagacacttaagctcgccttcgatctcagcgcagc ggtgcagccacaacgcgcagcccgtgggctcgtgatgcttgtaggtcacctctgcaaac gactgcaggtacgcctgcaggaatcgccccatcatcgtcacaaaggtcttgttgctggtg aaggtcagctgcaacccgcggtgctcctcgttcagccaggtcttgcatacggccgccag agcttccacttggtcaggcagtagtttgaagttcgcctttagatcgttatccacgtggtacttg tccatcagcgcgcgcgcagcctccatgcccttctcccacgcagacacgatcggcacac tcagcgggttcatcaccgtaatttcactttccgcttcgctgggctcttcctcttcctcttgcgtcc gcataccacgcgccactgggtcgtcttcattcagccgccgcactgtgcgcttacctcctttg ccatgcttgattagcaccggtgggttgctgaaacccaccatttgtagcgccacatcttctctt tcttcctcgctgtccacgattacctctggtgatggcgggcgctcgggcttgggagaaggg cgcttctttttcttcttgggcgcaatggccaaatccgccgccgaggtcgatggccgcgggc tgggtgtgcgcggcaccagcgcgtcttgtgatgagtcttcctcgtcctcggactcgatacg ccgcctcatccgcttttttgggggcgcccggggaggcggcggcgacggggacgggga cgacacgtcctccatggttgggggacgtcgcgccgcaccgcgtccgcgctcgggggtg gtttcgcgctgctcctcttcccgactggccatttccttctcctataggcagaaaaagatcata |
| | | gtcagtcgagaagaaggacagcctaaccgccccctctgagttcgccaccaccgcctcc accgatgccgccaacgcgcctaccaccttccccgtcgaggcacccccgcttgaggag gaggaagtgattatcgagcaggacccaggttttgtaagcgaagacgacgaggaccgc tcagtaccaacagaggataaaaagcaagaccaggacaacgcagaggcaaacgag gaacaagtcgggcgggggacgaaaggcatggcgactacctagatgtgggagacg acgtgctgttgaagcatctgcagcgccagtgcgccattatctgcgacgcgttgcaagag cgcagcgatgtgcccctcgccatagcggatgtcagccttgcctacgaacgccacctatt ctcaccgcgcgtacccccaaacgccaagaaaacggcacatgcgagcccaacccg cgcctcaacttctaccccgtatttgccgtgccagaggtgcttgccacctatcacatctttttcc aaaactgcaagataccccctatcctgccgtgccaaccgcagccgagcggacaagcag ctggccttgcggcagggcgctgtcatacctgatatcgcctcgctcaacgaagtgccaaa aatctttgagggtcttggacgcgacgagaagcgcgcggcaaacgctctgcaacagga aaacagcgaaaatgaaagtcactctggagtgttggtggaactcgagggtgacaacgc gcgcctagccgtactaaaacgcagcatcgaggtcacccactttgcctacccggcactta acctacccccccaaggtcatgagcacagtcatgagtgagctgatcgtgcgccgtgcgca gcccctggagagggatgcaaatttgcaagaacaaacagaggagggcctacccgcag ttggcgacgagcagctagcgcgctggcttcaaacgcgcgagcctgccgacttggagg agcgacgcaaactaatgatggccgcagtgctcgttaccgtggagcttgagtgcatgcag cggttctttgctgacccggagatgcagcgcaagctagaggaaacattgcactacaccttt cgacagggctacgtacgccaggcctgcaagatctccaacgtggagctctgcaacctgg tctcctaccttggaattttgcacgaaaaccgccttgggcaaaacgtgcttcattccacgctc aagggcgaggcgcgccgcgactacgtccgcgactgcgtttacttatttctatgctacacct ggcagacggccatgggcgtttggcagcagtgcttggaggagtgcaacctcaaggagc tgcagaaactgctaaagcaaaacttgaaggacctatggacggccttcaacgagcgctc cgtggccgcgcacctggcggacatcattttccccgaacgcctgcttaaaaccctgcaac agggtctgccagacttcaccagtcaaagcatgttgcagaactttaggaactttatcctaga gcgctcaggaatcttgcccgccacctgctgtgcacttcctagcgactttgtgcccattaagt accgcgaatgccctccgccgctttggggccactgctaccttctgcagctagccaactacc ttgcctaccactctgacataatggaagacgtgagcggtgacggtctactggagtgtcact gtcgctgcaacctatgcaccccgcaccgctccctggtttgcaattcgcagctgcttaacg aaagtcaaattatcggtacctttgagctgcagggtccctcgcctgacgaaaagtccgcg gctccggggttgaaactcactccggggctgtggacgtcggcttaccttcgcaaatttgtac ctgaggactaccacgcccacgagattaggttctacgaagaccaatcccgcccgccaa atgcggagcttaccgcctgcgtcattacccagggccacattcttggccaattgcaagcca tcaacaaagcccgccaagagtttctgctacgaaagggacggggggtttacttggaccc ccagtccggcgaggagctcaacccaatcccccgccgccgcagccctatcagcagc agccgcgggcccttgcttcccaggatcacccaaaaagaagctgcagctgccgccgcc acccacggacgaggaggaatactgggacagtcaggcagaggaggttttggacgagg aggaggaggacatgatggaagactgggagagcctagacgaggaagcttccgaggtc gaagaggtgtcagacgaaacaccgtcaccctcggtcgcattcccctcgccggcgcccc agaaatcggcaaccggttccagcatggctacaacctccgctcctcaggcgccgccgg cactgcccgttcgccgacccaaccgtagatgggacaccactggaaccagggccggta agtccaagcagccgccgccgttagcccaagagcaacaacagcgccaaggctaccg ctcatggcgcgggcacaagaacgccatagttgcttgcttgcaagactgtgggggcaac atctccttcgcccgccgctttcttctctaccatcacggcgtggccttcccccgtaacatcctg cattactaccgtcatctctacagcccatactgcaccggcggcagcggcagcggcagca acagcagcggccacacagaagcaaaggcgaccggatagcaagactctgacaaag cccaagaaatccacagcggcggcagcagcaggaggaggagcgctgcgtctggcgc |
| | | ccaacgaacccgtatcgacccgcgagcttagaaacaggatttttcccactctgtatgctat atttcaacagagcaggggccaagaacaagagctgaaataaaaaacaggtctctgc gatccctcacccgcagctgcctgtatcacaaaagcgaagatcagcttcggcgcacgct ggaagacgcggaggctctcttcagtaaatactgcgcgctgactcttaaggactagtttcg cgccctttctcaaatttaagcgcgaaaactacgtcatctcc |
| 37 | E4orf6/7 nucleotide sequence | tcacagaaccctagtattcaacctgccacctccctcccaacacacagagtacacagtcc tttctccccggctggccttaaaaagcatcatatcatgggtaacagacatattcttaggtgtta tattccacacggtttcctgtcgagccaaacgctcatcagtgatattaataaactccccggg cagctcacttaagttcatgtcgctgtccagctgctgagccacaggctgctgtccaacttgc ggttgcttaacgggcggcgaaggagaagtccacgcctacatgggggtagagtcataat cgtgcatcaggatagggcggtggtgctgcagcagcgcgcgaataaactgctgccgcc gccgctccgtcctgcaggaatacaacatggcagtggtctcctcagcgatgattcgcacc gcccgcagcataaggcgccttgtcctccgggcacagcagcgcaccctgatctcactta aatcagcacagtaactgcagcacagcaccacaatattgttcaaaatcccacagtgcaa ggcgctgtatccaaagctcatggcggggaccacagaacccacgtggccatcatacca caagcgcaggtagattaagtggcgacccctcataaacacgctggacataaacattacc tcttttggcatgttgtaattcaccacctcccggtaccatataaacctctgattaaacatggcg ccatccaccaccatcctaaaccagctggccaaaacctgcccgccggctatacactgca gggaaccgggactggaacaatgacagtggagagcccaggactcgtaaccatggatc atcatgctcgtcatgatatcaatgttggcacaacacaggcacacgtgcatacacttcctc aggattacaagctcctcccgcgttagaaccatatcccagggaacaacccattcctgaat cagcgtaaatcccacactgcagggaagacctcgcacgtaactcacgttgtgcattgtca aagtgttacattcgggcagcagcggatgatcctccagtatggtagcgcgggtttctgtctc aaaaggaggtagacgatccctactgtacggagtgcgccgagacaaccgagatcgtgt tggtcgtagtgtcatgccaaatggaacgccggacgtagtcat |
| 38 | E4orf6/7 protein | MTTSGVPFGMTLRPTRSRLSRRTPYSRDRLPPFETETRATILE DHPLLPECNTLTMHNAWTSPSPPVKQPQVGQQPVAQQLDSD MNLSELPGEFINITDERLARQETVWNITPKNMSVTHDMMLFKA SRGERTVYSVCWEGGGRLNTRVL |
| 39 | E4orf6 (E4 34k protein) nucleotide sequence | MTTSGVPFGMTLRPTRSRLSRRTPYSRDRLPPFETETRATILE DHPLLPECNTLTMHNVSYVRGLPCSVGFTLIQEWVVPWDMVL TREELVILRKCMHVCLCCANIDIMTSMMIHGYESWALHCHCSS PGSLQCIAGGQVLASWFRMVVDGAMFNQRFIWYREVVNYNM PKEVMFMSSVFMRGRHLIYLRLWYDGHVGSVVPAMSFGYSA LHCGILNNIVVLCCSYCADLSEIRVRCCARRTRRLMLRAVRIIAE ETTAMLYSCRTERRRQQFIRALLQHHRPILMHDYDSTPM |
| 40 | E4orf4 nucleotide sequence | ctactgtacggagtgcgccgagacaaccgagatcgtgttggtcgtagtgtcatgccaaat ggaacgccggacgtagtcatatttcctgaagcaaaaccaggtgcgggcgtgacaaac agatctgcgtctccggtctcgccgcttagatcgctctgtgtagtagttgtagtatatccactct ctcaaagcatccaggcgccccctggcttcgggttctatgtaaactccttcatgcgccgctg ccctgataacatccaccaccgcagaataagccacacccagccaacctacacattcgtt ctgcgagtcacacacgggaggagcgggaagagctggaagaaccat |
| 41 | Eorf4 protein | MVLPALPAPPVCDSQNECVGWLGVAYSAVVDVIRAAAHEGVY IEPEARGRLDALREWIYYNYYTERSKRRDRRRRSVCHARTWF CFRKYDYVRRSIWHDTTTNTISVVSAHSVQ |
| 42 | E4orf3 nucleotide sequence | tattccaaaagattatccaaaacctcaaaatgaagatctattaagtgaacgcgctcccct ccggtggcgtggtcaaactctacagccaaagaacagataatggcatttgtaagatgttg cacaatggcttccaaaaggcaaacggccctcacgtccaagtggacgtaaaggctaaa |
| | | cccttcagggtgaatctcctctataaacattccagcaccttcaaccatgcccaaataattct catctcgccaccttctcaatatatctctaagcaaatcccgaatattaagtccggccattgta aaaatctgctccagagcgccctccaccttcagcctcaagcagcgaatcat |
| 43 | Eorf3 protein | MIRCLRLKVEGALEQIFTMAGLNIRDLLRDILRRWRDENYLGM VEGAGMFIEEIHPEGFSLYVHLDVRAVCLLEAIVQHLTNAIICSL AVEFDHATGGERVHLIDLHFEVLDNLLE |
| 44 | E4orf8 nucleotide sequence | tcatgattgcaaaaattcaggttcctcacagacctgtataagattcaaaagcggaacatt aacaaaaataccgcgatcccgtaggtcccttcgcagggccagctgaacataatcgtgc aggtctgcacggaccagcgcggccacttccccgccaggaaccatgacaaaagaacc cacactgattatgacacgcatactcggagctatgctaaccagcgtagccccgatgtaag cttgttgcatgggcggcgatataaaatgcaaggtgctgctcaaaaaatcaggcaaagc ctcgcgcaaaaaagaaagcacatcgtagtcatgctcatgcagataaaggcaggtaag ctccggaaccaccacagaaaaagacaccatttttctctcaaacat |
| 45 | Eorf8 protein | MFERKMVSFSVVVPELTCLYLHEHDYDVLSFLREALPDFLSST LHFISPPMQQAYIGATLVSIAPSMRVIISVGSFVMVPGGEVAAL VRADLHDYVQLALRRDLRDRGIFVNVPLLNLIQVCEEPEFLQS |
| 46 | E4orf1 nucleotide sequence | ttaaacattagaagcctgtcttacaacaggaaaaacaacccttataagcataagacgg actacggccatgccggcgtgaccgtaaaaaaactggtcaccgtgattaaaaagcacc accgacagctcctcggtcatgtccggagtcataatgtaagactcggtaaacacatcagg ttgattcacatcggtcagtgctaaaaagcgaccgaaatagcccgggggaatacatacc cgcaggcgtagagacaacattacagccccccataggaggtataacaaaattaatagga gagaaaaacacataaacacctgaaaaaccctcctgcctaggcaaaatagcaccctcc cgctccagaacaacatacagcgcttccacagcggcagccat |
| 47 | Eorf1 protein | MAAAVEALYVVLEREGAILPRQEGFSGVYVFFSPINFVIPPMGA VMLSLRLRVCIPPGYFGRFLALTDVNQPDVFTESYIMTPDMTE ELSVVLFNHGDQFFYGHAGMAVVRLMLIRVVFPVVRQASNV |
| 48 | E4 region full-length | tcacagaaccctagtattcaacctgccacctccctcccaacacacagagtacacagtcc tttctccccggctggccttaaaaagcatcatatcatgggtaacagacatattcttaggtgtta tattccacacggtttcctgtcgagccaaacgctcatcagtgatattaataaactccccggg cagctcacttaagttcatgtcgctgtccagctgctgagccacaggctgctgtccaacttgc ggttgcttaacgggcggcgaaggagaagtccacgcctacatgggggtagagtcataat cgtgcatcaggatagggcggtggtgctgcagcagcgcgcgaataaactgctgccgcc gccgctccgtcctgcaggaatacaacatggcagtggtctcctcagcgatgattcgcacc gcccgcagcataaggcgccttgtcctccgggcacagcagcgcaccctgatctcactta aatcagcacagtaactgcagcacagcaccacaatattgttcaaaatcccacagtgcaa ggcgctgtatccaaagctcatggcggggaccacagaacccacgtggccatcatacca caagcgcaggtagattaagtggcgacccctcataaacacgctggacataaacattacc tcttttggcatgttgtaattcaccacctcccggtaccatataaacctctgattaaacatggcg ccatccaccaccatcctaaaccagctggccaaaacctgcccgccggctatacactgca gggaaccgggactggaacaatgacagtggagagcccaggactcgtaaccatggatc atcatgctcgtcatgatatcaatgttggcacaacacaggcacacgtgcatacacttcctc aggattacaagctcctcccgcgttagaaccatatcccagggaacaacccattcctgaat cagcgtaaatcccacactgcagggaagacctcgcacgtaactcacgttgtgcattgtca aagtgttacattcgggcagcagcggatgatcctccagtatggtagcgcgggtttctgtctc aaaaggaggtagacgatccctactgtacggagtgcgccgagacaaccgagatcgtgt tggtcgtagtgtcatgccaaatggaacgccggacgtagtcatatttcctgaagcaaaacc aggtgcgggcgtgacaaacagatctgcgtctccggtctcgccgcttagatcgctctgtgt agtagttgtagtatatccactctctcaaagcatccaggcgccccctggcttcgggttctatg |
| | | taaactccttcatgcgccgctgccctgataacatccaccaccgcagaataagccacacc cagccaacctacacattcgttctgcgagtcacacacgggaggagcgggaagagctgg aagaaccatgtttttttttattccaaaagattatccaaaacctcaaaatgaagatctattaa gtgaacgcgctcccctccggtggcgtggtcaaactctacagccaaagaacagataatg gcatttgtaagatgttgcacaatggcttccaaaaggcaaacggccctcacgtccaagtg gacgtaaaggctaaacccttcagggtgaatctcctctataaacattccagcaccttcaac catgcccaaataattctcatctcgccaccttctcaatatatctctaagcaaatcccgaatatt aagtccggccattgtaaaaatctgctccagagcgccctccaccttcagcctcaagcagc gaatcatgattgcaaaaattcaggttcctcacagacctgtataagattcaaaagcggaa cattaacaaaaataccgcgatcccgtaggtcccttcgcagggccagctgaacataatc gtgcaggtctgcacggaccagcgcggccacttccccgccaggaaccatgacaaaag aacccacactgattatgacacgcatactcggagctatgctaaccagcgtagccccgatg taagcttgttgcatgggcggcgatataaaatgcaaggtgctgctcaaaaaatcaggcaa agcctcgcgcaaaaaagaaagcacatcgtagtcatgctcatgcagataaaggcaggt aagctccggaaccaccacagaaaaagacaccatttttctctcaaacatgtctgcgggttt ctgcataaacacaaaataaaataacaaaaaaacatttaaacattagaagcctgtcttac aacaggaaaaacaacccttataagcataagacggactacggccatgccggcgtgac cgtaaaaaaactggtcaccgtgattaaaaagcaccaccgacagctcctcggtcatgtc cggagtcataatgtaagactcggtaaacacatcaggttgattcacatcggtcagtgctaa aaagcgaccgaaatagcccgggggaatacatacccgcaggcgtagagacaacatta cagcccccataggaggtataacaaaattaataggagagaaaaacacataaacacct gaaaaaccctcctgcctaggcaaaatagcaccctcccgctccagaacaacatacagc gcttccacagcggcagccat |
| 49 | Protease protein | MGSSEQELKAIVKDLGCGPYFLGTYDKRFPGFVSPHKLACAIV NTAGRETGGVHWMAFAWNPHSKTCYLFEPFGFSDQRLKQVY QFEYESLLRRSAIASSPDRCITLEKSTQSVQGPNSAACGLFCC MFLHAFANWPQTPMDHNPTMNLITGVPNSMLNSPQVQPTLR RNQEQLYSFLERHSPYFRSHSAQIRSATSFCHLKNM |
| 50 | E1a 13S protein | MRHIICHGGVITEEMAASLLDQLIEEVLADNLPPPSHFEPPTLH ELYDLDVTAPEDPNEEAVSQIFPDSVMLAVQEGIDLLTFPPAP GSPEPPHLSRQPEQPEQRALGPVSMPNLVPEVIDLTCHEAGF PPSDDEDEEGEEFVLDYVEHPGHGCRSCHYHRRNTGDPDIM CSLCYMRTCGMFVYSPVSEPEPEPEPEPEPEPARPTRRPKMAP AILRRPTSPVSRECNSSTDSCDSGPSNTPPEIHPVVPLCPIKPV AVRVGGRRQAVECIEDLLNEPGQPLDLSCKRPRP |
| 51 | E1a 12S protein | MRHIICHGGVITEEMAASLLDQLIEEVLADNLPPPSHFEPPTLH ELYDLDVTAPEDPNEEAVSQIFPDSVMLAVQEGIDLLTFPPAP GSPEPPHLSRQPEQPEQRALGPVSMPNLVPEVIDLTCHEAGF PPSDDEDEEGPVSEPEPEPEPEPEPEPARPTRRPKMAPAILRRP TSPVSRECNSSTDSCDSGPSNTPPEIHPVVPLCPIKPVAVRVG GRRQAVECIEDLLNEPGQPLDLSCKRPRP |
| 52 | E1a 11S protein | MRHIICHGGVITEEMAASLLDQLIEEPEQPEQRALGPVSMPNL VPEVIDLTCHEAGFPPSDDEDEEGEEFVLDYVEHPGHGCRSC HYHRRNTGDPDIMCSLCYMRTCGMFVYSPVSEPEPEPEPEPE PARPTRRPKMAPAILRRPTSPVSRECNSSTDSCDSGPSNTPP EIHPVVPLCPIKPVAVRVGGRRQAVECIEDLLNEPGQPLDLSC KRPRP |
| 53 | E1a 10S protein | MRHIICHGGVITEEMAASLLDQLIEEPEQPEQRALGPVSMPNL VPEVIDLTCHEAGFPPSDDEDEEGPVSEPEPEPEPEPEPARPT RRPKMAPAILRRPTSPVSRECNSSTDSCDSGPSNTPPEIHPVV PLCPIKPVAVRVGGRRQAVECIEDLLNEPGQPLDLSCKRPRP |
| 54 | E1a 9S protein | MRHIICHGGVITEEMAASLLDQLIEEVLCLNLSLPSQNRSLQD LPAVLKWRLLS |
| 55 | E1a 19k protein | MEAWECLEDFSAVRNLLEQSSNSTSWFWRFLWGSSQAKLVC RIKEDYKWEFEELLKSCGELFDSLNLGHQALFQEKVIKTLDFST PGRAAAAVAFLSFIKDKWSEETHLSGGYLLDFLAMHLWRAVV RHKNRLLLLSSVRPAIIPTEEQQQQEEARRRRQEQSPWNPR AGLDPRE |
| 56 | E1b 55k protein | MERRNPSERGVPAGFSGHASVESGCETQESPATVVFRPPGD NTDGGAAAAAGGSQAAAAGAEPMEPESRPGPSGMNVVQVA ELYPELRRILTITEDGQGLKGVKRERGACEATEEARNLAFSLM TRHRPECITFQQIKDNCANELDLLAQKYSIEQLTTYWLQPGDD FEEAIRVYAKVALRPDCKYKISKLVNIRNCCYISGNGAEVEIDTE DRVAFRCSMINMWPGVLGMDGVVIMNVRFTGPNFSGTVFLAN TNLILHGVSFYGFNNTCVEAWTDVRVRGCAFYCCWKGVVCR PKSRASIKKCLFERCTLGILSEGNSRVRHNVASDCGCFMLVKS VAVIKHNMVCGNCEDRASQMLTCSDGNCHLLKTIHVASHSRK AWPVFEHNILTRCSLHLGNRRGVFLPYQCNLSHTKILLEPESM SKVNLNGVFDMTMKIWKVLRYDETRTRCRPCECGGKHIRNQP VMLDVTEELRPDHLVLACTRAEFGSSDEDTD |
| 57 | Hexon protein | MATPSMMPQWSYMHISGQDASEYLSPGLVQFARATETYFSL NNKFRNPTVAPTHDVTTDRSQRLTLRFIPVDREDTAYSYKARF TLAVGDNRVLDMASTYFDIRGVLDRGPTFKPYSGTAYNALAPK GAPNPCEWDEAATALEINLEEEDDDNEDEVDEQAEQQKTHVF GQAPYSGINITKEGIQIGVEGQTPKYADKTFQPEPQIGESQWY ETEINHAAGRVLKKTTPMKPCYGSYAKPTNENGGQGILVKQQ NGKLESQVEMQFFSTTEATAGNGDNLTPKVVLYSEDVDIETPD THISYMPTIKEGNSRELMGQQSMPNRPNYIAFRDNFIGLMYYN STGNMGVLAGQASQLNAVVDLQDRNTELSYQLLLDSIGDRTR YFSMWNQAVDSYDPDVRIIENHGTEDELPNYCFPLGGVINTET LTKVKPKTGQENGWEKDATEFSDKNEIRVGNNFAMEINLNAN LWRNFLYSNIALYLPDKLKYSPSNVKISDNPNTYDYMNKRVVA PGLVDCYINLGARWSLDYMDNVNPFNHHRNAGLRYRSMLLG NGRYVPFHIQVPQKFFAIKNLLLLPGSYTYEWNFRKDVNMVLQ SSLGNDLRVDGASIKFDSICLYATFFPMAHNTASTLEAMLRND TNDQSFNDYLSAANMLYPIPANATNVPISIPSRNWAAFRGWAF TRLKTKETPSLGSGYDPYYTYSGSIPYLDGTFYLNHTFKKVAIT FDSSVSWPGNDRLLTPNEFEIKRSVDGEGYNVAQCNMTKDW FLVQMLANYNIGYQGFYIPESYKDRMYSFFRNFQPMSRQVVD DTKYKDYQQVGILHQHNNSGFVGYLAPTMREGQAYPANFPYP LIGKTAVDSITQKKFLCDRTLWRIPFSSNFMSMGALTDLGQNLL YANSAHALDMTFEVDPMDEPTLLYVLFEVFDVVRVHRPHRGVI ETVYLRTPFSAGNATT |
| 58 | Peripenton al hexon-associated protein | MMQDATDPAVRAALQSQPSGLNSTDDWRQVMDRIMSLTARN PDAFRQQPQANRLSAILEAVVPARANPTHEKVLAIVNALAENR |
| | | AIRPDEAGLVYDALLQRVARYNSGNVQTNLDRLVGDVREAVA QRERAQQQGNLGSMVALNAFLSTQPANVPRGQEDYTNFVSA LRLMVTETPQSEVYQSGPDYFFQTSRQGLQTVNLSQAFKNLQ GLWGVRAPTGDRATVSSLLTPNSRLLLLLIAPFTDSGSVSRDT YLGHLLTLYREAIGQAHVDEHTFQEITSVSRALGQEDTGSLEA TLNYLLTNRRQKIPSLHSLNSEEERILRYVQQSVSLNLMRDGV TPSVALDMTARNMEPGMYASNRPFINRLMDYLHRAAAVNPEY FTNAILNPHWLPPPGFYTGGFEVPEGNDGFLWDDIDDSVFSP QPQTLLELQQREQAEAALRKESFRRPSSLSDLGAAAPRSDAS SPFPSLIGSLTSTRTTRPRLLGEEEYLNNSLLQPQREKNLPPAF PNNGIESLVDKMSRWKTYAQEHRDVPGPRPPTRRQRHDRQR GLVWEDDDSADDSSVLDLGGSGNPFAHLRPRLGRMF |
| 59 | E4orf6 (E4 34K) nucleotide sequence | ctacgtccggcgttccatttggcatgacactacgaccaacacgatctcggttgtctcggcg cactccgtacagtagggatcgtctacctccttttgagacagaaacccgcgctaccatact ggaggatcatccgctgctgcccgaatgtaacactttgacaatgcacaacgtgagttacgt gcgaggtcttccctgcagtgtgggatttacgctgattcaggaatgggttgttccctgggata tggttctaacgcgggaggagcttgtaatcctgaggaagtgtatgcacgtgtgcctgtgttgt gccaacattgatatcatgacgagcatgatgatccatggttacgagtcctgggctctccact gtcattgttccagtcccggttccctgcagtgtatagccggcgggcaggttttggccagctg gtttaggatggtggtggatggcgccatgtttaatcagaggtttatatggtaccgggaggtg gtgaattacaacatgccaaaagaggtaatgtttatgtccagcgtgtttatgaggggtcgcc acttaatctacctgcgcttgtggtatgatggccacgtgggttctgtggtccccgccatgagc tttggatacagcgccttgcactgtgggattttgaacaatattgtggtgctgtgctgcagttact gtgctgatttaagtgagatcagggtgcgctgctgtgcccggaggacaaggcgccttatg ctgcgggcggtgcgaatcatcgctgaggagaccactgccatgttgtattcctgcaggac ggagcggcggcggcagcagtttattcgcgcgctgctgcagcaccaccgccctatcctg atgcacgattatgactctacccccatgtaggcgtggacttctccttcgccgcccgttaagc aaccgcaagttggacagcagcctgtggctcagcagctggacagcgacatgaacttaa gtgagctgcccggggagtttattaatatcactgatgagcgtttggctcgacaggaaaccg tgtggaatataacacctaagaatatgtctgttacccatgatatgatgctttttaaggccagc cggggagaaaggactgtgtactctgtgtgttgggagggaggtggcaggttgaatactag ggttctgtga |
| 60 | E3 12k nucleotide | atgttaagtggagaggcagagcaactgcgcctgaaacacctggtccactgtcgccgcc acaagtgctttgcccgcgactccggtgagttttgctactttgaattgcccgaggatcatatc gagggcccggcgcacggcgtccggcttaccgcccagggagagcttgcccgtagcctg attcgggagtttacccagcgccccctgctagttgagcgggacaggggaccctgtgttctc actgtgatttgcaactgtcctaaccttggattacatcaagatctttgttgccatctctgtgctga gtataataaatacagaaattaa |
| 61 | E3 CR1-α nucleotide | atgaacaattcaagcaactctacgggctattctaattcaggtttctctagaatcggggttgg ggttattctctgtcttgtgattctctttattcttatactaacgcttctctgcctaaggctcgccgcct gctgtgtgcacatttgcatttattgtcagctttttaaacgctggggtcgccacccaagatga |
| 62 | E3 gp19 nucleotide | atgattaggtacataatcctaggtttactcacccttgcgtcagcccacggtaccacccaaa aggtggattttaaggagccagcctgtaatgttacattcgcagctgaagctaatgagtgca ccactcttataaaatgcaccacagaacatgaaaagctgcttattcgccacaaaaacaa aattggcaagtatgctgtttatgctatttggcagccaggtgacactacagagtataatgtta cagttttccagggtaaaagtcataaaacttttatgtatacttttccattttatgaaatgtgcgac attaccatgtacatgagcaaacagtataagttgtggcccccacaaaattgtgtggaaaa |
| | | cactggcactttctgctgcactgctatgctaattacagtgctcgctttggtctgtaccctactct atattaaatacaaaagcagacgcagctttattgaggaaaagaaaatgccttaa |
| 63 | E3 10.5K nucleotide | atgaccaacacaaccaacgcggccgccgctaccggacttacatctaccacaaataca ccccaagtttctgcctttgtcaataactgggataacttgggcatgtggtggttctccatagcg cttatgtttgtatgccttattattatgtggctcatctgctgcctaaagcgcaaacgcgcccga ccacccatctatagtcccatcattgtgctacacccaaacaatgatggaatccatagattg gacggactgaaacacatgttcttttctcttacagtatgat |
| 64 | E3 RID-α nucleotide | atgattcctcgagtttttatattactgacccttgttgcgctttttttgtgcgtgctccacattggctg cggtttctcacatcgaagtagactgcattccagccttcacagtctatttgctttacggatttgt caccctcacgctcatctgcagcctcatcactgtggtcatcgcctttatccagtgcattgact gggtctgtgtgcgctttgcatatctcagacaccatccccagtacagggacaggactatag ctgagcttcttagaattctttaa |
| 65 | E3 RID-β nucleotide | atgaaatttactgtgacttttctgctgattatttgcaccctatctgcgttttgttccccgacctcc aagcctcaaagacatatatcatgcagattcactcgtatatggaatattccaagttgctaca atgaaaaaagcgatctttccgaagcctggttatatgcaatcatctctgttatggtgttctgca gtaccatcttagccctagctatatatccctaccttgacattggctggaaacgaatagatgc catgaaccacccaactttccccgcgcccgctatgcttccactgcaacaagttgttgccgg cggctttgtcccagccaatcagcctcgccccacttctcccacccccactgaaatcagcta ctttaatctaacaggaggagatgactga |
| 66 | E3 14.7k nucleotide | atgactgacaccctagatctagaaatggacggaattattacagagcagcgcctgctag aaagacgcagggcagcggccgagcaacagcgcatgaatcaagagctccaagacat ggttaacttgcaccagtgcaaaaggggtatcttttgtctggtaaagcaggccaaagtcac ctacgacagtaataccaccggacaccgccttagctacaagttgccaaccaagcgtcag aaattggtggtcatggtgggagaaaagcccattaccataactcagcactcggtagaaa ccgaaggctgcattcactcaccttgtcaaggacctgaggatctctgcacccttattaagac cctgtgcggtctcaaagatcttattccctttaactaa |
| 67 | E3 CR1-β nucleotide | atgaccaacacaaccaacgcggccgccgctaccggacttacatctaccacaaataca ccccaagtttctgcctttgtcaataactgggataacttgggcatgtggtggttctccatagcg cttatgtttgtatgccttattattatgtggctcatctgctgcctaaagcgcaaacgcgcccga ccacccatctatagtcccatcattgtgctacacccaaacaatgatggaatccatagattg gacggactgaaacacatgttcttttctcttacagtatga |
| 68 | E3 CR1-β protein | MTNTTNAAAATGLTSTTNTPQVSAFVNNWDNLGMWWFSIAL MFVCLIIMWLICCLKRKRARPPIYSPIIVLHPNNDGIHRLDGLKH MFFSLTV |
| 69 | Sequence linking poly A site and VA RNA region | tcgacgagatcttccaacataaggcgatgatatccgtagatgtacctggacatcaggtg atgccggcggcggtggtggaggcgcgcggaaagtcgcggacgcggttccagatg |
| 70 | Sequence linking VA RNA region and E2 region | atcatccggtgctgcggcagatgcgccccctcctcagcagcggcaagagcaagagc agcggcagacatgcagggcaccctccatgcatccactttattttgtcacttgaaaacat gtaaaaataaatgtactagagacactttc |
| 71 | Sequence linking E2 and E4 (pHAD1) | agcggccacacccggcgccagcacctgtcgtcagcgccattatagcaaggaaattcc cacgccctacatgtggagttaccagccacaaatgggacttgcggctggagctgcccaa gactactcaacccgaataaactacatgagcgcgggaccccacatgatatcccgggtca acggaatccgcgcccttcgaaaccgaattctcttggaacaggcggctattaccaccaca |
| | | cctcgtaataaccttaatccccgtagttggcccgctgccctggtgtaccaggaaagtccc gctcccaccactgtggtacttcccagagacgcccaggccgaagttcagatgactaactc aggggcgcagcttgcgggcggctttcgtcacagggtgcggtcgcccgggcagggtata actcacctgacaatcagagggcgaggtattcagctcaacgacgagtcggtgagctcct cgcttggtctccgtccggacggacatttcagatcggcggcgccggccgtccttcattca cgcctcgtcaggcaatcctaactctgcagacctcgtcctctgagccgcgctctggaggc attggaactctgcaatttattgaggagtttgtgccatcggtctactttaacccccttctcgggac ctcccggccactatccggatcaatttattcctaactttgacgcggtaaaggactcggcgg acggctacgactgaatgttaagtggagaggcagagcaactgcgcctgaaacacctgg tccactgtcgccgccacaagtgctttgcccgcgactccggtgagttttgctactttgaattgc ccgaggatcatatcgagggcccggcgcacggcgtccggcttaccgcccagggagag cttgcccgtagcctgattcgggagtttacccagcgccccctgctagttgagcgggacagg ggaccctgtgttctcactgtgatttgcaactgtcctaaccttggattacatcaagatctttgttg ccatctctgtgctgagtataataaatacagaaattaaaatatactggggctcctatcgcca tcctgtaaacgccaccgtcttcacccgcccaagcaaaccaaggcgaaccttacctggt acttttaacatctctccctctgtgatttacaacagtttcaacccagacggagtgagtctacg agagaacctctccgagctcagctactccatcagaaaaacaccaccctccttacctgcc gggaacgtacgagtgcgtcaccggccgctgcaccacacctaccgcctgaccgtaaac cagactttttccggacagacctcaataactctgtttaccagaacaggaggtgagcttaga aaacccttagggtattaggccaaaggcgcagctactgtggggtttatgaacaattcaag caactctacgggctattctaattcaggtttctctagaatcggggttggggttattctctgtcttg tgattctctttattcttatactaacgcttctctgcctaaggctcgccgcctgctgtgtgcacattt gcatttattgtcagctttttaaacgctggggtcgccacccaagatgattaggtacataatcct aggtttactcaccctgcgtcagcccacggtaccacccaaaaggtggattttaaggagc cagcctgtaatgttacattcgcagctgaagctaatgagtgcaccactcttataaaatgcac cacagaacatgaaaagctgcttattcgccacaaaaacaaaattggcaagtatgctgttt atgctatttggcagccaggtgacactacagagtataatgttacagttttccagggtaaaag tcataaaacttttatgtatacttttccattttatgaaatgtgcgacattaccatgtacatgagca aacagtataagttgtggcccccacaaaattgtgtggaaaacactggcactttctgctgca ctgctatgctaattacagtgctcgctttggtctgtaccctactctatattaaatacaaaagca gacgcagctttattgaggaaaagaaaatgccttaatttactaagttacaaagctaatgtca ccactaactgctttactcgctgcttgcaaaacaaattcaaaaagttagcattataattaga ataggatttaaacccccccggtcatttcctgctcaataccattcccctgaacaattgactctat gtgggatatgctccagcgctacaaccttgaagtcaggcttcctggatgtcagcatctgact ttggccagcacctgtcccgcggatttgttccagtccaactacagcgacccaccctaaca gagatgaccaacacaaccaacgcggccgccgctaccggacttacatctaccacaaat acaccccaagtttctgcctttgtcaataactgggataacttgggcatgtggtggttctccata gcgcttatgtttgtatgccttattattatgtggctcatctgctgcctaaagcgcaaacgcgcc cgaccacccatctatagtcccatcattgtgctacacccaaacaatgatggaatccataga ttggacggactgaaacacatgttcttttctcttacagtatgattaaatgagacatgattcctc gagtttttatattactgacccttgttgcgctttttttgtgcgtgctccacattggctgcggtttctca catcgaagtagactgcattccagccttcacagtctatttgctttacggatttgtcaccctcac gctcatctgcagcctcatcactgtggtcatcgcctttatccagtgcattgactgggtctgtgt gcgctttgcatatctcagacaccatccccagtacagggacaggactatagctgagcttct tagaattctttaattatgaaatttactgtgacttttctgctgattatttgcaccctatctgcgttttgt tccccgacctccaagcctcaaagacatatatcatgcagattcactcgtatatggaatattc caagttgctacaatgaaaaaagcgatctttccgaagcctggttatatgcaatcatctctgtt atggtgttctgcagtaccatcttagccctagctatatatccctaccttgacattggctggaaa |
| | | cgaatagatgccatgaaccacccaactttccccgcgcccgctatgcttccactgcaaca agttgttgccggcggctttgtcccagccaatcagcctcgccccacttctcccacccccact gaaatcagctactttaatctaacaggaggagatgactgacaccctagatctagaaatgg acggaattattacagagcagcgcctgctagaaagacgcagggcagcggccgagcaa cagcgcatgaatcaagagctccaagacatggttaacttgcaccagtgcaaaaggggt atcttttgtctggtaaagcaggccaaagtcacctacgacagtaataccaccggacaccg ccttagctacaagttgccaaccaagcgtcagaaattggtggtcatggtgggagaaaag cccattaccataactcagcactcggtagaaaccgaaggctgcattcactcaccttgtcaa ggacctgaggatctctgcacccttattaagaccctgtgcggtctcaaagatcttattcccttt aactaataaaaaaaaataaagcatcacttacttaaaatcagttagcaaatttctgtcc agtttattcagcagcacctccttgccctcctcccagctctggtattgcagcttcctcctggctg caaactttctccacaatctaaatggaatgtcagtttcctcctgttcctgtccatccgcaccca ctatcttcatgttgttgcagataagcgcgcaagaccgtctgaagataccttcaaccccgtg tatccatatgacacggaaaccggtcctccaactgtgccttttcttactcctccctttgtatccc ccaatgggtttcaagagagtccccctggggtactctctttgcgcctatccgaacctctagtt acctccaatggcatgcttgcgctcaaaatgggcaacggcctctctctggacgaggccgg caaccttacctcccaaaatgtaaccactgtgagcccacctctcaaaaaaaccaagtca aacataaacctggaaatatctgcacccctcacagttacctcagaagccctaactgtggct gccgccgcacctctaatggtcgcgggcaacacactcaccatgcaatcacaggccccg ctaaccgtgcacgactccaaacttagcattgccacccaaggacccctcacagtgtcag aaggaaagctagccctgcaaacatcaggcccccctcaccaccatcgatagcagtaccc ttactatcactgcctcacccccctctaactactgccactggtagcttgggcattgacttgaaa gagcccatttatacacaaaatggaaaactaggactaaagtacggggctcctttgcatgt aacagacgacctaaacactttgaccgtagcaactggtccaggtgtgactattaataatac ttccttgcaaactaaagttactggagccttgggttttgattcacaaggcaatatgcaactta atgtagcaggaggactaaggattgattctcaaaacagacgccttatacttgatgttagttat ccgtttgatgctcaaaaccaactaaatctaagactaggacagggccctctttttataaact cagcccacaacttggatattaactacaacaaaggcctttacttgtttacagcttcaaacaa ttccaaaaagcttgaggttaacctaagcactgccaaggggttgatgtttgacgctacagc catagccattaatgcaggagatgggcttgaatttggttcacctaatgcaccaaacacaa atcccctcaaaacaaaaattggccatggcctagaatttgattcaaacaaggctatggttc ctaaactaggaactggccttagttttgacagcacaggtgccattacagtaggaaacaaa aataatgataagctaactttgtggaccacaccagctccatctcctaactgtagactaaatg cagagaaagatgctaaactcactttggtcttaacaaaatgtggcagtcaaatacttgcta cagtttcagttttggctgttaaaggcagtttggctccaatatctggaacagttcaaagtgctc atcttattataagatttgacgaaaatggagtgctactaaacaattccttcctggacccaga atattggaactttagaaatggagatcttattgaaggcacagcctatacaaacgctgttgga tttatgcctaacctatcagcttatccaaaatctcacggtaaaactgccaaaagtaacattgt cagtcaagtttacttaaacggagacaaaactaaacctgtaacactaaccattacactaa acggtacacaggaaacaggagacacaactccaagtgcatactctatgtcattttcatgg gactggtctggccacaactacatcgatgaaatatttgccacatcctcttacactttttcatac attgcccaagaataaagaatcgtttgtgttatgtttcaacgtgtttatttttcaattgcagaaa atttcaagtcatttttcattcagtagtatagccccaccaccacatagcttatacagatcacc gtaccttaatcaaac |
| 72 | Sequence linking E2 and E4 (pHAD2) | agcggccacacccggcgccagcacctgtcgtcagcgccattatagcaaggaaattcc cacgccctacatgtggagttaccagccacaaatgggacttgcggctggagctgcccaa gactactcaacccgaataaactacatgagcgcgggaccccacatgatatcccgggtca acggaatccgcgcccttcgaaaccgaattctcttggaacaggcggctattaccaccaca |
| | | cctcgtaataaccttaatccccgtagttggcccgctgccctggtgtaccaggaaagtccc gctcccaccactgtggtacttcccagagacgcccaggccgaagttcagatgactaactc aggggcgcagcttgcgggcggctttcgtcacagggtgcggtcgcccgggcagggtata actcacctgacaatcagagggcgaggtattcagctcaacgacgagtcggtgagctcct cgcttggtctccgtccggacggacatttcagatcggcggcgccggccgtccttcattca cgcctcgtcaggcaatcctaactctgcagacctcgtcctctgagccgcgctctggaggc attggaactctgcaatttattgaggagtttgtgccatcggtctactttaacccccttctcgggac ctcccggccactatccggatcaatttattcctaactttgacgcggtaaaggactcggcgg acggctacgactgaatgttaagtggagaggcagagcaactgcgcctgaaacacctgg tccactgtcgccgccacaagtgctttgcccgcgactccggtgagttttgctactttgaattgc ccgaggatcatatcgagggcccggcgcacggcgtccggcttaccgcccagggagag cttgcccgtagcctgattcgggagtttacccagcgccccctgctagttgagcgggacagg ggaccctgtgttctcactgtgatttgcaactgtcctaaccttggattacatcaagatctttgttg ccatctctgtgctgagtataataaatacagaaattaaaatatactggggctcctatcgcca tcctgtaaacgccaccgtcttcacccgcccaagcaaaccaaggcgaaccttacctggt acttttaacatctctccctctgtgatttacaacagtttcaacccagacggagtgagtctacg agagaacctctccgagctcagctactccatcagaaaaacaccaccctccttacctgcc gggaacgtacgagtgcgtcaccggccgctgcaccacacctaccgcctgaccgtaaac cagactttttccggacagacctcaataactctgtttaccagaacaggaggtgagcttaga aaacccttagggtattaggccaaaggcgcagctactgtggggtttatgaacaattcaag caactctacgggctattctaattcaggtttctctagaatcggggttggggttattctctgtcttg tgattctctttattcttatactaacgcttctctgcctaaggctcgccgcctgctgtgtgcacattt gcatttattgtcagctttttaaacgctggggtcgccacccaagatgattaggtacataatcct aggtttactcaccctgcgtcagcccacggtaccacccaaaaggtggattttaaggagc cagcctgtaatgttacattcgcagctgaagctaatgagtgcaccactcttataaaatgcac cacagaacatgaaaagctgcttattcgccacaaaaacaaaattggcaagtatgctgttt atgctatttggcagccaggtgacactacagagtataatgttacagttttccagggtaaaag tcataaaacttttatgtatacttttccattttatgaaatgtgcgacattaccatgtacatgagca aacagtataagttgtggcccccacaaaattgtgtggaaaacactggcactttctgctgca ctgctatgctaattacagtgctcgctttggtctgtaccctactctatattaaatacaaaagca gacgcagctttattgaggaaaagaaaatgccttaatttactaagttacaaagctaatgtca ccactaactgctttactcgctgcttgcaaaacaaattcaaaaagttagcattataattaga ataggatttaaaccccccggtcatttcctgctcaataccattcccctgaacaattgactctat gtgggatatgctccagcgctacaaccttgaagtcaggcttcctggatgtcagcatctgact ttggccagcacctgtcccgcggatttgttccagtccaactacagcgacccaccctaaca gagatgaccaacacaaccaacgcggccgccgctaccggacttacatctaccacaaat acaccccaagtttctgcctttgtcaataactgggataacttgggcatgtggtggttctccata gcgcttatgtttgtatgccttattattatgtggctcatctgctgcctaaagcgcaaacgcgcc cgaccacccatctatagtcccatcattgtgctacacccaaacaatgatggaatccataga ttggacggactgaaacacatgttcttttctcttacagtatgattaaatgagacatgattcctc gagtttttatattactgacccttgttgcgctttttgtgcgtgctccacattggctgcggtttctca catcgaagtagactgcattccagccttcacagtctatttgctttacggatttgtcaccctcac gctcatctgcagcctcatcactgtggtcatcgcctttatccagtgcattgactgggtctgtgt gcgctttgcatatctcagacaccatccccagtacagggacaggactatagctgagcttct tagaattctttaattatgaaatttactgtgacttttctgctgattatttgcaccctatctgcgttttgt tccccgacctccaagcctcaaagacatatatcatgcagattcactcgtatatggaatattc caagttgctacaatgaaaaaagcgatctttccgaagcctggttatatgcaatcatctctgtt atggtgttctgcagtaccatcttagccctagctatatatccctaccttgacattggctggaaa |
| | | cgaatagatgccatgaaccacccaactttccccgcgcccgctatgcttccactgcaaca agttgttgccggcggctttgtcccagccaatcagcctcgccccacttctcccaccccact gaaatcagctactttaatctaacaggaggagatgactgacaccctagatctagaaatgg acggaattattacagagcagcgcctgctagaaagacgcagggcagcggccgagcaa cagcgcatgaatcaagagctccaagacatggttaacttgcaccagtgcaaaaggggt atcttttgtctggtaaagcaggccaaagtcacctacgacagtaataccaccggacaccg ccttagctacaagttgccaaccaagcgtcagaaattggtggtcatggtgggagaaaag cccattaccataactcagcactcggtagaaaccgaaggctgcattcactcaccttgtcaa ggacctgaggatctctgcacccttattaagaccctgtgcggtctcaaagatcttattcccttt aactaataaaaaaaaataataaagcatcacttacttaaaatcagttagcaaatttctgtcc agtttattcagcagcacctccttgccctcctcccagctctggtattgcagcttcctcctggctg caaactttctccacaatctaaatggaatgtcagtttcctcctgttcctgtccatccgcaccca ctatcttcatgttgttgcagataagcgcgcaagaccgtctgaagataccttcaaccccgtg tatccatatgacacggaaaccggtcctccaactgtgccttttcttactcctccctttgtatccc ccaatgggtttcaagagagtccccctggggtactctctttgcgcctatccgaacctctagtt acctccaatggcatgcttgcgctcaaaatgggcaacggcctctctctggacgaggccgg caaccttacctcccaaaatgtaaccactgtgagcccacctctcaaaaaaaccaagtca aacataaacctggaaatatctgcacccctcacagttacctcagaagccctaactgtggct gccgccgcacctctaatggtcgcgggcaacacactcaccatgcaatcacaggccccg ctaaccgtgcacgactccaaacttagcattgccacccaaggacccctcacagtgtcag aaggaaagctagccctgcaaacatcaggcccccctcaccaccatcgatgaaatatttgc cacatcctcttacactttttcatacattgcccaagaataaagaatcgtttgtgttatgtttcaac gtgtttatttttcaattgcagaaaatttcaagtcatttttcattcagtagtatagccccaccacc acatagcttatacagatcaccgtaccttaatcaaac |
| 73 | Sequence linking E2 and E4 (pHAD3) | agcggccacacccggcgccagcacctgtcgtcagcgccattatagcaaggaaattcc cacgccctacatgtggagttaccagccacaaatgggacttgcggctggagctgcccaa gactactcaacccgaataaactacatgagcgcgggaccccacatgatatcccgggtca acggaatccgcgcccttcgaaaccgaattctttaattatgaaatttactgtgacttttctgctg attatttgcaccctatctgcgttttgttccccgacctccaagcctcaaagacatatatcatgc agattcactcgtatatggaatattccaagttgctacaatgaaaaaagcgatctttccgaag cctggttatatgcaatcatctctgttatggtgttctgcagtaccatcttagccctagctatatat ccctaccttgacattggctggaaacgaatagatgccatgaaccacccaactttccccgc gcccgctatgcttccactgcaacaagttgttgccggcggctttgtcccagccaatcagcct cgccccacttctcccacccccactgaaatcagctactttaatctaacaggaggagatga ctgacaccctagatctagaaatggacggaattattacagagcagcgcctgctagaaag acgcagggcagcggccgagcaacagcgcatgaatcaagagctccaagacatggtta acttgcaccagtgcaaaaggggtatcttttgtctggtaaagcaggccaaagtcacctacg acagtaataccaccggacaccgccttagctacaagttgccaaccaagcgtcagaaatt ggtggtcatggtgggagaaaagcccattaccataactcagcactcggtagaaaccga aggctgcattcactcaccttgtcaaggacctgaggatctctgcacccttattaagaccctgt gcggtctcaaagatcttattccctttaactaataaaaaaaaataataaagcatcacttactt aaaatcagttagcaaatttctgtccagtttattcagcagcacctccttgccctcctcccagct ctggtattgcagcttcctcctggctgcaaactttctccacaatctaaatggaatgtcagtttc ctcctgttcctgtccatccgcacccactatcttcatgttgttgcagataagcgcgcaagacc gtctgaagataccttcaaccccgtgtatccatatgacacggaaaccggtcctccaactgt gccttttcttactcctccctttgtatcccccaatgggtttcaagagagtccccctggggtactc tctttgcgcctatccgaacctctagttacctccaatggcatgcttgcgctcaaaatgggcaa cggcctctctctggacgaggccggcaaccttacctcccaaaatgtaaccactgtgagcc |
| | | cacctctcaaaaaaaccaagtcaaacataaacctggaaatatctgcacccctcacagtt acctcagaagccctaactgtggctgccgccacctctaatggtcgcgggcaacacac tcaccatgcaatcacaggccccgctaaccgtgcacgactccaaacttagcattgccacc caaggacccctcacagtgtcagaaggaaagctagccctgcaaacatcaggccccctc accaccatcgatagcagtacccttactatcactgcctcacccccctctaactactgccactg gtagcttgggcattgacttgaaagagcccatttatacacaaaatggaaaactaggacta aagtacggggctcctttgcatgtaacagacgacctaaacactttgaccgtagcaactggt ccaggtgtgactattaataatacttccttgcaaactaaagttactggagccttgggttttgatt cacaaggcaatatgcaacttaatgtagcaggaggactaaggattgattctcaaaacag acgccttatacttgatgttagttatccgtttgatgctcaaaaccaactaaatctaagactag gacagggccctctttttataaactcagcccacaacttggatattaactacaacaaaggcc tttacttgtttacagcttcaaacaattccaaaaagcttgaggttaacctaagcactgccaag gggttgatgtttgacgctacagccatagccattaatgcaggagatgggcttgaatttggttc acctaatgcaccaaacacaaatcccctcaaaacaaaaattggccatggcctagaattt gattcaaacaaggctatggttcctaaactaggaactggccttagttttgacagcacaggt gccattacagtaggaaacaaaaataatgataagctaactttgtggaccacaccagctcc atctcctaactgtagactaaatgcagagaaagatgctaaactcactttggtcttaacaaa atgtggcagtcaaatacttgctacagtttcagttttggctgttaaaggcagtttggctccaat atctggaacagttcaaagtgctcatcttattataagatttgacgaaaatggagtgctactaa acaattccttcctggacccagaatattggaactttagaaatggagatcttattgaaggcac agcctatacaaacgctgttggatttatgcctaacctatcagcttatccaaaatctcacggta aaactgccaaaagtaacattgtcagtcaagtttacttaaacggagacaaaactaaacct gtaacactaaccattacactaaacggtacacaggaaacaggagacacaactccaagt gcatactctatgtcattttcatgggactggtctggccacaactacatcgatgaaatatttgcc acatcctcttacactttttcatacattgcccaagaataaagaatcgtttgtgttatgtttcaacg tgtttatttttcaattgcagaaaatttcaagtcatttttcattcagtagtatagccccaccacca catagcttatacagatcaccgtaccttaatcaaac |
| 74 | Sequence linking E2 and E4 (pHAD4) | agcggccacacccggcgccagcacctgtcgtcagcgccattatagcaaggaaattcc cacgccctacatgtggagttaccagccacaaatgggacttgcggctggagctgcccaa gactactcaacccgaataaactacatgagcgcgggaccccacatgatatcccgggtca acggaatccgcgcccttcgaaaccgaattctttaattatgaaatttactgtgacttttctgctg attatttgcaccctatctgcgttttgttccccgacctccaagcctcaaagacatatatcatgc agattcactcgtatatggaatattccaagttgctacaatgaaaaaagcgatctttccgaag cctggttatatgcaatcatctctgttatggtgttctgcagtaccatcttagccctagctatatat ccctaccttgacattggctggaaacgaatagatgccatgaaccacccaactttccccgc gcccgctatgcttccactgcaacaagttgttgccggcggctttgtcccagccaatcagcct cgccccacttctcccacccccactgaaatcagctactttaatctaacaggaggagatga ctgacaccctagatctagaaatggacggaattattacagagcagcgcctgctagaaag acgcagggcagcggccgagcaacagcgcatgaatcaagagctccaagacatggtta acttgcaccagtgcaaaaggggtatcttttgtctggtaaagcaggccaaagtcacctacg acagtaataccaccggacaccgccttagctacaagttgccaaccaagcgtcagaaatt ggtggtcatggtgggagaaaagcccattaccataactcagcactcggtagaaaccga aggctgcattcactcaccttgtcaaggacctgaggatctctgcacccttattaagaccctgt gcggtctcaaagatcttattccctttaactaataaaaaaaaataataaagcatcacttactt aaaatcagttagcaaatttctgtccagtttattcagcagcacctccttgccctcctcccagct ctggtattgcagcttcctcctggctgcaaactttctccacaatctaaatggaatgtcagtttc ctcctgttcctgtccatccgcacccactatcttcatgttgttgcagataagcgcgcaagacc gtctgaagataccttcaaccccgtgtatccatatgacacggaaaccggtcctccaactgt |
| | | gccttttcttactcctccctttgtatcccccaatgggtttcaagagagtccccctggggtactc tctttgcgcctatccgaacctctagttacctccaatggcatgcttgcgctcaaaatgggcaa cggcctctctctggacgaggccggcaaccttacctcccaaaatgtaaccactgtgagcc cacctctcaaaaaaaccaagtcaaacataaacctggaaatatctgcacccctcacagtt acctcagaagccctaactgtggctgccgccgcacctctaatggtcgcgggcaacacac tcaccatgcaatcacaggccccgctaaccgtgcacgactccaaacttagcattgccacc caaggacccctcacagtgtcagaaggaaagctagccctgcaaacatcaggcccccctc accaccatcgatagcagtacccttactatcactgcctcacccccctctaactactgccactg gtagcttgggcattgacttgaaagagcccatttatacacaaaatggaaaactaggacta aagtacggggctcctttgcatgtaacagacgacctaaacactttgaccgtagcaactggt ccaggtgtgactattaataatacttccttgcaaactaaagttactggagccttgggttttgatt cacaaggcaatatgcaacttaatgtagcaggaggactaaggattgattctcaaaacag acgccttatacttgatgttagttatccgtttgatgctcaaaaccaactaaatctaagactag gacagggccctctttttataaactcagcccacaacttggatattaactacaacaaaggcc tttacttgtttacagcttcaaacaattccaaaaagcttgaggttaacctaagcactgccaag gggttgatgtttgacgctacagccatagccattaatgcaggagatgggcttgaatttggttc acctaatgcaccaaacacaaatcccctcaaaacaaaaattggccatggcctagaattt gattcaaacaaggctatggttcctaaactaggaactggccttagttttgacagcacaggt gccattacagtaggaaacaaaaataatgataagctaactttgtggaccacaccagctcc atctcctaactgtagactaaatgcagagaaagatgctaaactcactttggtcttaacaaa atgtggcagtcaaatacttgctacagtttcagttttggctgttaaaggcagtttggctccaat atctggaacagttcaaagtgctcatcttattataagatttgacgaaaatggagtgctactaa acaattccttcctggacccagaatattggaactttagaaatggagatcttattgaaggcac agcctatacaaacgctgttggatttatgcctaacctatcagcttatccaaaatctcacggta aaactgccaaaagtaacattgtcagtcaagtttacttaaacggagacaaaactaaacct gtaacactaaccattacactaaacggtacacaggaaacaggagacacaactccaagt gcatactctatgtcattttcatgggactggtctggccacaactacatcgatgaaatatttgcc acatcctcttacactttttcatacattgcccaagaataaagaatcgtttgtgttatgtttcaacg tgtttatttttcaattgcagaaaatttcaagtcatttttcattcagtagtatagccccaccacca catagcttatacagatcaccgtaccttaatcaaac |
| 75 | Sequence linking E2 and E4 (pHAD5) | agcggccacacccggcgccagcacctgtcgtcagcgccattatagcaaggaaattcc cacgccctacatgtggagttaccagccacaaatgggacttgcggctggagctgcccaa gactactcaacccgaataaactacatgagcgcggaccccacatgatatcccgggtca acggaatccgcgcccttcgtgaaatatttgccacatcctcttacactttttcatacattgccc aagaataaagaatcgtttgtgttatgtttcaacgtgtttatttttcaattgcagaaaatttcaag tcatttttcattcagtagtatagccccaccaccacatagcttatacagatcaccgtaccttaa tcaaac |
| 76 | E2 region full-length (pHAD6) | aataaaggcaaatgcttttatttgtacactctcgggtgattatttacccccacccttgccgtct gcgccgtttaaaaatcaaaggggttctgccgcgcatcgctatgcgccactggcaggga cacgttgcgatactggtgtttagtgctccacttaaactcaggcacaaccatccgcggcag ctcggtgaagttttcactccacaggctgcgcaccatcaccaacgcgtttagcaggtcggg cgccgatatcttgaagtcgcagttggggcctccgccctgcgcgcgcgagttgcgataca cagggttgcagcactggaacactatcagcgccgggtggtgcacgctggccagcacgc tcttgtcggagatcagatccgcgtccaggtcctccgcgttgctcagggcgaacggagtc aactttggtagctgccttcccaaaaagggcgcgtgcccaggctttgagttgcactcgcac cgtagtggcatcaaaaggtgaccgtgcccggtctgggcgttaggatacagcgcctgcat aaaagccttgatctgcttaaaagccacctgagcctttgcgccttcagagaagaacatgc cgcaagacttgccggaaaactgattggccggacaggccgcgtcgtgcacgcagcacc |
| | | ttgcgtcggtgttggagatctgcaccacatttcggccccaccggttcttcacgatcttggcct tgctagactgctccttcagcgcgcgctgcccgttttcgctcgtcacatccatttcaatcacgt gctccttatttatcataatgcttccgtgtagacacttaagctcgccttcgatctcagcgcagc ggtgcagccacaacgcgcagcccgtgggctcgtgatgcttgtaggtcacctctgcaaac gactgcaggtacgcctgcaggaatcgccccatcatcgtcacaaaggtcttgttgctggtg aaggtcagctgcaacccgcggtgctcctcgttcagccaggtcttgcatacggccgccag agcttccacttggtcaggcagtagtttgaagttcgcctttagatcgttatccacgtggtacttg tccatcagcgcgcgcgcagcctccatgcccttctcccacgcagacacgatcggcacac tcagcgggttcatcaccgtaatttcactttccgcttcgctgggctcttcctcttcctcttgcgtcc gcataccacgcgccactgggtcgtcttcattcagccgccgcactgtgcgcttacctcctttg ccatgcttgattagcaccggtgggttgctgaaacccaccatttgtagcgccacatcttctctt tcttcctcgctgtccacgattacctctggtgatggcggcgctcgggcttgggagaaggg cgcttctttttcttcttgggcgcaatggccaaatccgccgccgaggtcgatggccgcgggc tgggtgtgcgcggcaccagcgcgtcttgtgatgagtcttcctcgtcctcggactcgatacg ccgcctcatccgcttttttgggggcgcccggggaggcggcggcgacggggacgggga cgacacgtcctccatggttgggggacgtcgcgccgcaccgcgtccgcgctcgggggtg gtttcgcgctgctcctcttcccgactggccatttccttctcctataggcagaaaaagatcata gtcagtcgagaagaaggacagcctaaccgcccccctctgagttcgccaccaccgcctcc accgatgccgccaacgcgcctaccaccttccccgtcgaggcacccccgcttgaggag gaggaagtgattatcgagcaggacccaggttttgtaagcgaagacgacgaggaccgc tcagtaccaacagaggataaaaagcaagaccaggacaacgcagaggcaaacgag gaacaagtcgggcgggggacgaaaggcatggcgactacctagatgtgggagacg acgtgctgttgaagcatctgcagcgccagtgcgccattatctgcgacgcgttgcaagag cgcagcgatgtgcccctcgccatagcggatgtcagccttgcctacgaacgccacctatt ctcaccgcgcgtaccccccaaacgccaagaaaacggcacatgcgagcccaacccg cgcctcaacttctaccccgtatttgccgtgccagaggtgcttgccacctatcacatctttttcc aaaactgcaagatacccctatcctgccgtgccaaccgcagccgagcggacaagcag ctggccttgcggcagggcgctgtcatacctgatatcgcctcgctcaacgaagtgccaaa aatctttgagggtcttggacgcgacgagaagcgcgcggcaaacgctctgcaacagga aaacagcgaaaatgaaagtcactctggagtgttggtggaactcgagggtgacaacgc gcgcctagccgtactaaaacgcagcatcgaggtcacccactttgcctacccggcactta acctacccccccaaggtcatgagcacagtcatgagtgagctgatcgtgcgccgtgcgca gcccctggagagggatgcaaatttgcaagaacaaacagaggagggcctacccgcag ttggcgacgagcagctagcgcgctggcttcaaacgcgcgagcctgccgacttggagg agcgacgcaaactaatgatggccgcagtgctcgttaccgtggagcttgagtgcatgcag cggttctttgctgacccggagatgcagcgcaagctagaggaaacattgcactacaccttt cgacagggctacgtacgccaggcctgcaagatctccaacgtggagctctgcaacctgg tctcctaccttggaattttgcacgaaaaccgccttgggcaaaacgtgcttcattccacgctc aagggcgaggcgcgccgcgactacgtccgcgactgcgtttacttatttctatgctacacct ggcagacggccatgggcgtttggcagcagtgcttggaggagtgcaacctcaaggagc tgcagaaactgctaaagcaaaacttgaaggacctatggacggccttcaacgagcgctc cgtggccgcgcacctggcggacatcattttccccgaacgcctgcttaaaaccctgcaac agggtctgccagacttcaccagtcaaagcatgttgcagaactttaggaactttatcctaga gcgctcaggaatcttgcccgccacctgctgtgcacttcctagcgactttgtgcccattaagt accgcgaatgccctccgccgctttggggccactgctaccttctgcagctagccaactacc ttgcctaccactctgacataatggaagacgtgagcggtgacggtctactggagtgtcact gtcgctgcaacctatgcaccccgcaccgctccctggtttgcaattcgcagctgcttaacg aaagtcaaattatcggtacctttgagctgcagggtccctcgcctgacgaaaagtccgcg |
| | | gctccggggttgaaactcactccggggctgtggacgtcggcttaccttcgcaaatttgtac ctgaggactaccacgcccacgagattaggttctacgaagaccaatcccgcccgccaa atgcggagcttaccgcctgcgtcattacccagggccacattcttggccaattgcaagcca tcaacaaagcccgccaagagtttctgctacgaaagggacggggggtttacttggaccc ccagtccggcgaggagctcaacccaatccccccgccgccgcagccctatcagcagc agccgcgggcccttgcttcccaggatggcacccaaaaagaagctgcagctgccgccg ccacccacggacgaggaggaatactgggacagtcaggcagaggaggttttggacga ggaggaggaggacatgatggaagactgggagagcctagacgaggaagcttccgag gtcgaagaggtgtcagacgaaacaccgtcaccctcggtcgcattcccctcgccggcgc cccagaaatcggcaaccggttccagcatggctacaacctccgctcctcaggcgccgcc ggcactgcccgttcgccgacccaaccgtagatgggacaccactggaaccagggccg gtaagtccaagcagccgccgccgttagcccaagagcaacaacagcgccaaggctac cgctcatggcgcgggcacaagaacgccatagttgcttgcttgcaagactgtgggggca acatctccttcgcccgccgctttcttctctaccatcacggcgtggccttccccgtaacatcc tgcattactaccgtcatctctacagcccatactgcaccggcggcagcggcagcggcag caacagcagcggccacacagaagcaaaggcgaccggatagcaagactctgacaa agcccaagaaatccacagcggcggcagcagcaggaggaggagcgctgcgtctggc gcccaacgaacccgtatcgacccgcgagcttagaaacaggatttttcccactctgtatgc tatatttcaacagagcaggggccaagaacaagagctgaaaataaaaaacaggtctct gcgatccctcacccgcagctgcctgtatcacaaaagcgaagatcagcttcggcgcacg ctggaagacgcggaggctctcttcagtaaatactgcgcgctgactcttaaggactagtttc gcgccctttctcaaatttaagcgcgaaaactacgtcatctcc |
| 77 | ptr_lux2a-gfp | gccattcgccattcaggctgcgcaactgttgggaagggcgatcggtgcgggcctcttcg ctattacgccagctggcgaaagggggatgtgctgcaaggcgattaagttgggtaacgc cagggttttcccagtcacgacgttgtaaaacgacggccagtgaattcctcgagatttaaat gacgttggccactccctctctgcgcgctcgctcgctcactgaggccgggcgaccaaag gtcgcccgacgcccgggctttgcccgggcggcctcagtgagcgagcgagcgcgcag agagggagtggccaactccatcactaggggttcctgagtttaaacttcgtcgaggccgc acgcgtctagttattaatagtaatcaattacggggtcattagttcatagcccatatatggagt tccgcgttacataacttacggtaaatggcccgcctggctgaccgcccaacgacccccgc ccattgacgtcaataatgacgtatgttcccatagtaacgccaatagggactttccattgac gtcaatgggtggagtatttacggtaaactgcccacttggcagtacatcaagtgtatcatat gccaagtacgccccctattgacgtcaatgacggtaaatggcccgcctggcattatgccc agtacatgaccttatgggactttcctacttggcagtacatctacgtattagtcatcgctattac catggtgatgcggttttggcagtacatcaatgggcgtggatagcggtttgactcacgggg atttccaagtctccaccccattgacgtcaatgggagtttgttttggcaccaaaatcaacgg gactttccaaaatgtcgtaacaactccgccccattgacgcaaatgggcggtaggcgtgt acggtgggaggtctatataagcagagctcgtttagtgaaccgtcagatcgcctggagac gccatccacgctgttttgacctccatagaagacaccgggaccgatccagcctccgcgg attcgaatcccggccgggaacggtgcattggaacgcggattccccgtgccaagagtga cgtaagtaccgcctatagagtctataggcccacaaaaaatgctttcttcttttaatatactttttt tgtttatcttatttctaatactttccctaatctctttctttcagggcaataatgatacaatgtatcat gcctctttgcaccattctaaagaataacagtgataatttctgggttaaggcaatagcaatat ttctgcatataaatatttctgcatataaattgtaactgatgtaagaggtttcatattgctaatag cagctacaatccagctaccattctgcttttattttatggttgggataaggctggattattctga gtccaagctaggcccttttgctaatcatgttcatacctcttatcttcctcccacagctcctggg caacgtgctggtctgtgtgctggcccatcactttggcaaagaattgggataagcttgaga ccgccaccatggactacaaagaccatgacggtgattataaagatcatgacatcgattac |
| | | aaggatgacgatgacaagagcggcggcagcatggaggacgccaagaacatc aagaagggaccagcccccagataccccctggaggacggcacagccggcgagcag ctgcacaaggccatgaagcggtacgcccaggtgccaggcaccatcgccttcaccgac gcccacatcgaggtgaacatcacctacgccgagtacttcgagatgagcgtgcggctgg ccgaggccatgaagcggtacggcctgaacaccaaccaccggatcgtggtgtgcagc gagaacagcctgcagttcttcatgcccgtgctgggagccctgttcatcggcgtggccgtg gccccagccaacgacatctacaacgagcgggagctgctgaacagcatgaacatcag ccagcccaccgtggtgttcgtgagcaagaagggcctgcagaagatcctgaatgtgcag aagaagctgcccatcatccagaagatcatcatcatggacagcaagaccgattaccag ggcttccagagcatgtacaccttcgtgaccagccacctgcccccaggcttcaacgagta cgacttcaagcccgagagcttcgaccgggacaagaccatcgccctgatcatgaacag cagcggcagcaccggcctgcccaagggcgtggccctgccccaccggaccgcctgcg tgcggttcagccacgccagagaccccatcttcggcaaccagatcaagcccgacaccg ccatcctgagcgtggtgcccttccaccacggcttcggcatgttcaccaccctgggctacct gatctgcggcttccgggtggtgctgatgtacaggttcgaggaggagctgttcctgcggag cctgcaggactacaagatccagagcgccctgctggtgcccaccctgttcagcttcttcgc caagagcaccctgatcgacaagtacgacctgagcaacctgcacgagatcgcctctgg cggagccccactgagcaaggaggtgggcgaggccgtggccaagcggttccacctgc caggcatccggcagggctacggcctgaccgagaccaccagcgccatcctgatcaccc ccgagggcgacgacaagcccggagccgtgggcaaggtggtgcccttcttcgaggcc aaggtggtggacctggacaccggcaagaccctgggcgtgaaccagagaggcgagct gtgcgtgagaggccccatgatcatgagcggctacgtgaacaaccccgaggccacca acgccctgatcgacaaggacggctggctgcacagcggcgacatcgcctactgggacg aggacgagcacttcttcatcgtggaccggctgaagagcctgatcaagtacaagggcta ccaggtggccccagccgagctggagagcatcctgctgcagcaccccaacatccggg acgccggagtggccggactgcccgacgacgacgccggagagctgccagccgccgt ggtggtgctggagcacggcaagaccatgaccgagaaggagatcgtggactacgtgg ccagccaggtgaccaccgccaagaagctgagaggcggcgtggtgttcgtggacgag gtgcccaagggcctgaccggcaagctggacgccagaaagatccgggagatcctgat caaggccaagaagggcggcaagatccgcgcagagggccggggctcattgctgacct gtggagatgtcgaggaaaatcccggccccatggtgagcaagggcgaggagctgttca ccggggtggtgcccatcctggtcgagctggacggcgacgtaaacggccacaagttca gcgtgtccggcgagggcgagggcgatgccacctacggcaagctgaccctgaagttca tctgcaccaccggcaagctgcccgtgccctggcccaccctcgtgaccaccctgacctac ggcgtgcagtgcttcagccgctaccccgaccacatgaagcagcacgacttcttcaagtc cgccatgcccgaaggctacgtccaggagcgcaccatcttcttcaaggacgacggcaa ctacaagacccgcgccgaggtgaagttcgagggcgacaccctggtgaaccgcatcg agctgaagggcatcgacttcaaggaggacggcaacatcctggggcacaagctggag tacaactacaacagccacaacgtctatatcatggccgacaagcagaagaacggcatc aaggtgaacttcaagatccgccacaacatcgaggacggcagcgtgcagctcgccga ccactaccagcagaacacccccatcggcgacggccccgtgctgctgcccgacaacc actacctgagcacccagtccgccctgagcaaagaccccaacgagaagcgcgatcac atggtcctgctggagttcgtgaccgccgccgggatcactctcggcatggacgagctgta caagtaaacgcgtggtctcgaattcgggtggcatccctgtgacccctccccagtgcctct cctggccctggaagttgccactccagtgcccaccagccttgtcctaataaaattaagttgc atcattttgtctgactaggtgtccttctataatattatggggtggaggggggtggtatggagc aaggggcaagttgggaagacaacctgtagggcctgcggggtctattgggaaccaagc tggagtgcagtggcacaatcttggctcactgcaatctccgcctcctgggttcaagcgattc |
| | | tcctgcctcagcctcccgagttgttgggattccaggcatgcatgaccaggctcagctaattt ttgtttttttggtagagacggggtttcaccatattggccaggctggtctccaactcctaatctc aggtgatctacccaccttggcctcccaaattgctgggattacaggcgtgaaccactgctc ccttccctgtccttctgattttgtaggtaaccacgtgcggaccgagcggcctaagctcgcg aaggaacccctagtgatggagttggccactccctctctgcgcgctcgctcgctcactgag gccgggcgaccaaaggtcgcccgacgcccgggctttgcccgggcggcctcagtgag cgagcgagcgcgcagagagggagtggccaagacgatttaaatgacaagcttggcgt aatcatggtcatagctgtttcctgtgtgaaattgttatccgctcacaattccacacaacatac gagccggaagcataaagtgtaaagcctggggtgcctaatgagtgagctaactcacatt aattgcgttgcgctcactgcccgctttccagtcgggaaacctgtcgtgccagctgcattaat gaatcggccaacgcgcggggagaggcggtttgcgtattgggcgctcttccgcttcctcg ctcactgactcgctgcgctcggtcgttcggctgcggcgagcggtatcagctcactcaaag gcggtaatacggttatccacagaatcaggggataacgcaggaaagaacatgtgagca aaaggccagcaaaaggccaggaaccgtaaaaaggccgcgttgctggcgtttttccata ggctccgcccccctgacgagcatcacaaaaatcgacgctcaagtcagaggtggcgaaa cccgacaggactataaagataccaggcgtttccccctggaagctccctcgtgcgctctc ctgttccgaccctgccgcttaccggatacctgtccgcctttctcccttcgggaagcgtggcg ctttctcatagctcacgctgtaggtatctcagttcggtgtaggtcgttcgctccaagctgggc tgtgtgcacgaaccccccgttcagcccgaccgctgcgccttatccggtaactatcgtcttg agtccaacccggtaagacacgacttatcgccactggcagcagccactggtaacagga ttagcagagcgaggtatgtaggcggtgctacagagttcttgaagtggtggcctaactacg gctacactagaagaacagtatttggtatctgcgctctgctgaagccagttaccttcggaaa aagagttggtagctcttgatccggcaaacaaaccaccgctggtagcggtggtttttttgttt gcaagcagcagattacgcgcagaaaaaaaggatctcaagaagatcctttgatcttttct acggggtctgacgctcagtggaacgaaaactcacgttaagggattttggtcatgagatta tcaaaaaggatcttcacctagatccttttaaattaaaaatgaagttttaaatcaatctaaagt atatatgagtaaacttggtctgacagttagaaaaactcatcgagcatcaaatgaaactgc aatttattcatatcaggattatcaataccatatttttgaaaaagccgtttctgtaatgaagga gaaaactcaccgaggcagttccataggatggcaagatcctggtatcggtctgcgattcc gactcgtccaacatcaatacaacctattaatttcccctcgtcaaaaataaggttatcaagt gagaaatcaccatgagtgacgactgaatccggtgagaatggcaaaagtttatgcatttct ttccagacttgttcaacaggccagccattacgctcgtcatcaaaatcactcgcatcaacc aaaccgttattcattcgtgattgcgcctgagcgagacgaaatacgcgatcgctgttaaaa ggacaattacaaacaggaatcgaatgcaaccggcgcaggaacactgccagcgcatc aacaatattttcacctgaatcaggatattcttctaatacctggaatgctgtttttccggggatc gcagtggtgagtaaccatgcatcatcaggagtacggataaaatgcttgatggtcggaag aggcataaattccgtcagccagtttagtctgaccatctcatctgtaacatcattggcaacg ctacctttgccatgtttcagaaacaactctggcgcatcgggcttcccatacaagcgatag attgtcgcacctgattgcccgacattatcgcgagcccatttatacccatataaatcagcatc catgttggaatttaatcgcggcctcgacgtttcccgttgaatatggctcatactcttcctttttc aatattattgaagcatttatcagggttattgtctcatgagcggatacatatttgaatgtatttag aaaaataaacaaataggggttccgcgcacatttccccgaaaagtgccacctgacgtct aagaaaccattattatcatgacattaacctataaaaataggcgtatcacgaggccctttc gtctcgcgcgtttcggtgatgacggtgaaaacctctgacacatgcagctcccggagacg gtcacagcttgtctgtaagcggatgccgggagcagacaagcccgtcagggcgcgtca gcgggtgttggcgggtgtcggggctggcttaactatgcggcatcagagcagattgtactg agagtgcaccatatgcggtgtgaaataccgcacagatgcgtaaggagaaaataccgc atcaggc |
| | | |
| 78 | Sequence linking E4 and ITR | aacagtcagccttaccagtaaaaaagaaaacctattaaaaaacaccactcgacacg gcaccagctcaatcagtcacagtgtaaaaaagggccaagtgcagagcgagtatatat aggactaaaaaatgacgtaacggttaaagtccacaaaaaacacccagaaaaccgc acgcgaacctacgcccagaaacgaaagccaaaaaacccacaacttcctcaaatcgt cacttccgttttcccacgttacgtaacttcccattttaagaaaactacaattcccaacacata caagttactccgccctaaaac |
| 79 | Sequence at 3'-end of ITR | aacagtcagccttaccagtaaaaagaaaacctattaaaaaacaccactcgacacg gcaccagctcaatcagtcacagtgtaaaaaagggccaagtgcagagcgagtatatat aggactaaaaaatgacgtaacggttaaagtccacaaaaaacacccagaaaaccgc acgcgaacctacgcccagaaacgaaagccaaaaaacccacaacttcctcaaatcgt cacttccgttttcccacgttacgtaacttcccattttaagaaaactacaattcccaacacata caagttactccgccctaaaac |
| 80 | Sequence at 5'-end of poly A site | atcgaagtactcggggaaatgtgcgcggaacccctatttgtttatttttctaaatacattcaa atatgtatccgctcatgagacaataaccctgataaatgcttcaataatattgaaaaagga agagtatgagccatattcaacgggaaacgtcgaggccgcgattaaattccaacatggat gctgatttatatgggtataaatgggctcgcgataatgtcgggcaatcaggtgcgacaatct atcgcttgtatgggaagcccgatgcgccagagttgtttctgaaacatggcaaaggtagcg ttgccaatgatgttacagatgagatggtcagactaaactggctgacggaatttatgcctctt ccgaccatcaagcattttatccgtactcctgatgatgcatggttactcaccactgcgatccc cggaaaaacagcattccaggtattagaagaatatcctgattcaggtgaaaatattgttgat gcgctggcagtgttcctgcgccggttgcattcgattcctgtttgtaattgtccttttaacagcg atcgcgtatttcgtctcgctcaggcgcaatcacgaatgaataacggtttggttgatgcgagt gattttgatgacgagcgtaatggctggcctgttgaacaagtctggaaagaaatgcataaa cttttgccattctcaccggattcagtcgtcactcatggtgatttctcacttgataaccttatttttg acgaggggaaattaataggttgtattgatgttggacgagtcggaatcgcagaccgatac caggatcttgccatcctatggaactgcctcggtgagttttctccttcattacagaaacggcttt ttcaaaaatatggtattgataatcctgatatgaataaattgcagtttcatttgatgctcgatga gtttttctaactgtcagaccaagtttactcatatatactttagattgatttaaaacttcatttttaat ttaaaaggatctaggtgaagatcctttttgataatctcatgaccaaaatcccttaacgtgag ttttcgttccactgagcgtcagaccccgtagaaaagatcaaaggatcttcttgagatcctttt tttctgcgcgtaatctgctgcttgcaaacaaaaaaaccaccgctaccagcggtggtttgttt gccggatcaagagctaccaactctttttccgaaggtaactggcttcagcagagcgcaga taccaaatactgttcttctagtgtagccgtagttaggccaccacttcaagaactctgtagca ccgcctacatacctcgctctgctaatcctgttaccagtggctgctgccagtggcgataagt cgtgtcttaccgggttggactcaagacgatagttaccggataaggcgcagcggtcgggc tgaacggggggttcgtgcacacagcccagcttggagcgaacgacctacaccgaactg agatacctacagcgtgagctatgagaaagcgccacgcttcccgaagggagaaaggc ggacaggtatccggtaagcggcagggtcggaacaggagagcgcacgagggagcttc caggggggaaacgcctggtatctttatagtcctgtcgggtttcgccacctctgacttgagcgt cgatttttgtgatgctcgtcaggggggcggagcctatggaaaaacgccagcaacgcgg cctttttacggttcctggccttttttcctgcagcccgggggatccgattaattaagacgtttaa ac |
| 81 | ITR of AAV2 | gaggtcctgtattagaggtcacgtgagtgttttgcgacattttgcgacaccatgtggtcacg ctgggtatttaagcccgagtgagcacgcagggtctccattttgaagcgggaggtttgaac gcgcagccgcc |
| 82 | 5'ITR, full-length (flip) | ttggccactccctctctgcgcgctcgctcgctcactgaggccgggcgaccaaaggtcgc ccgacgcccgggctttgcccgggcggcctcagtgagcgagcgagcgcgcagagagg gagtggccaactccatcactaggggttcct |
| 83 | 5'ITR, full-length (flop) | ttggccactccctctctgcgcgctcgctcgctcactgaggccgcccgggcaaagcccgg gcgtcgggcgacctttggtcgcccggcctcagtgagcgagcgagcgcgcagagaggg agtggccaactccatcactaggggttcct |
| 84 | 5'ITR, short-stemmed (flip) | ctgcgcgctcgctcgctcactgaggccgggcgaccaaaggtcgcccgacgcccgggc tttgcccgggcggcctcagtgagcgagcgagcgcgcagagagggagtggccaactcc atcactaggggttcct |
| 85 | 5'ITR, short-stemmed | ctgcgcgctcgctcgctcactgaggccgcccgggcaaagcccgggcgtcgggcgacc tttggtcgcccggcctcagtgagcgagcgagcgcgcagagagggagtggccaactcc atcactaggggttcct |
| 86 | 3'ITR, full-length (flop) | aggaacccctagtgatggagttggccactccctctctgcgcgctcgctcgctcactgagg ccgcccgggcaaagcccgggcgtcgggcgacctttggtcgcccggcctcagtgagcg agcgagcgcgcagagagggagtggccaa |
| 87 | 3'ITR, full-length (flop) | aggaacccctagtgatggagttggccactccctctctgcgcgctcgctcgctcactgagg ccgggcgaccaaaggtcgcccgacgcccgggctttgcccgggcggcctcagtgagcg agcgagcgcgcagagagggagtggccaa |
| 88 | 3'ITR, short-stemmed (flip) | aggaacccctagtgatggagttggccactccctctctgcgcgctcgctcgctcactgagg ccgcccgggcaaagcccgggcgtcgggcgacctttggtcgcccggcctcagtgagcg agcgagcgcgcag |
| 89 | 3'ITR, short-stemmed (flop) | aggaacccctagtgatggagttggccactccctctctgcgcgctcgctcgctcactgagg ccgggcgaccaaaggtcgcccgacgcccgggctttgcccgggcggcctcagtgagcg agcgagcgcgcag |
| 90 | Truncated L4-33k protein (start at residue 35 of L4-33K) | MMEDWESLDEEASEVEEVSDETPSPSVAFPSPAPQKSATGSS MATTSAPQAPPALPVRRPNRRWDTTGTRAAHTAPAAAAAAAT AAATQKQRRPDSKTLTKPKKSTAAAAAGGGALRLAPNEPVST RELRNRIFPTLYAIFQQSRGQEQELKIKNRSLRSLTRSCLYHKS EDQLRRTLEDAEALFSKYCALTLKD |
| 91 | Truncated L4-33k protein (start at residue 77 of L4-33K) | MATTSAPQAPPALPVRRPNRRWDTTGTRAAHTAPAAAAAAAT AAATQKQRRPDSKTLTKPKKSTAAAAAGGGALRLAPNEPVST RELRNRIFPTLYAIFQQSRGQEQELKIKNRSLRSLTRSCLYHKS EDQLRRTLEDAEALFSKYCALTLKD |
| 92 | Truncated L4-22k protein (start at residue 35 of L4-22K) | MMEDWESLDEEASEVEEVSDETPSPSVAFPSPAPQKSATGSS MATTSAPQAPPALPVRRPNRRWDTTGTRAGKSKQPPPLAQE QQQRQGYRSWRGHKNAIVACLQDCGGNISFARRFLLYHHGV AFPRNILHYYRHLYSPYCTGGSGSGSNSSGHTEAKATG |
| 93 | Truncated L4-22k protein (start at residue 77 of L4-22K) | MATTSAPQAPPALPVRRPNRRWDTTGTRAGKSKQPPPLAQE QQQRQGYRSWRGHKNAIVACLQDCGGNISFARRFLLYHHGV AFPRNILHYYRHLYSPYCTGGSGSGSNSSGHTEAKATG |
| 94 | Deleted portion of L4-22K and L4-33K | MAPKKKLQLPPPPTDEEEYWDSQAEEVLDEEEED |
| 95 | Deleted portion of L4-22K and L4-33K | MAPKKKLQLPPPPTDEEEYWDSQAEEVLDEEEEDMMEDWES LDEEASEVEEVSDETPSPSVAFPSPAPQKSATGSS |
| 96 | XX680 neDNA | tctagagctagcatatggatccatcgatttagggataacagggtaattatcagcacacaa ttgcccattatacgcgcgtataatggactattgtgtgctgataggcgcgcccgacagaag caccatgtccttgggtccggcctgctgaatgcgcaggcggtcggccatgccccaggcttc gttttgacatcggcgcaggtctttgtagtagtcttgcatgagcctttctaccggcacttcttcttc tccttcctcttgtcctgcatctcttgcatctatcgctgcggcggcggcggagtttggccgtag gtggcgccctcttcctcccatgcgtgtgaccccgaagcccctcatcggctgaagcaggg ctaggtcggcgacaacgcgctcggctaatatggcctgctgcacctgcgtgagggtagac tggaagtcatccatgtccacaaagcggtggtatgcgcccgtgttgatggtgtaagtgcagt tggccataacggaccagttaacggtctggtgacccggctgcgagagctcggtgtacctg agacgcgagtaagccctcgagtcaaatacgtagtcgttgcaagtccgcaccaggtact ggtatcccaccaaaaagtgcggcggcggctggcggtagaggggccagcgtagggtg gccggggctccgggggcgagatcttccaacataaggcgatgatatccgtagatgtacct ggacatccaggtgatgccggcggcggtggtggaggcgcgcggaaagtcgcggacgc ggttccagatgttgcgcagcggcaaaaagtgctccatggtcgggacgctctggccggtc aggcgcgcgcaatcgttgacgctctagcgtgcaaaaggagagcctgtaagcgggcac tcttccgtggtctggtggataaattcgcaagggtatcatggcggacgaccggggttcgag ccccgtatccggccgtccgccgtgatccatgcggttaccgcccgcgtgtcgaacccagg tgtgcgacgtcagacaacgggggagtgctccttttggcttccttccaggcgcggcggctg ctgcgctagcttttttggccactggccgcgcgcagcgtaagcggttaggctggaaagcg aaagcattaagtggctcgctccctgtagccggagggttattttccaagggttgagtcgcgg gacccccggttcgagtctcggaccggccggactgcggcgaacgggggtttgcctcccc gtcatgcaagaccccgcttgcaaattcctccggaaacagggacgagcccctttttgctttt cccagatgcatccggtgctgcggcagatgcgcccccctcctcagcagcggcaagagc aagagcagcggcagacatgcagggcaccctcccctcctcctaccgcgtcaggaggg gcgacatccgcggttgacgcggcagcagatggtgattacgaaccccgcggcgccgg gcccggcactacctggacttggaggagggcgagggcctggcgcggctaggagcgcc ctctcctgagcggcacccaagggtgcagctgaagcgtgatacgcgtgaggcgtacgtg ccgcggcagaacctgtttcgcgaccgcgagggagaggagcccgaggagatgcggg atcgaaagttccacgcagggcgcgagctgcggcatggcctgaatcgcgagcggttgct gcgcgaggaggactttgagcccgacgcgcgaaccgggattagtcccgcgcgcgcac acgtggcggccgccgacctggtaaccgcatacgagcagacggtgaaccaggagatt aactttcaaaaaagctttaacaaccacgtgcgtacgcttgtggcgcgcgaggaggtggc tataggactgatgcatctgtgggactttgtaagcgcgctggagcaaaacccaaatagca agccgctcatggcgcagctgttccttatagtgcagcacagcagggacaacgaggcattc agggatgcgctgctaaacatagtagagcccgagggccgctggctgctcgatttgataaa |
| | | catcctgcagagcatagtggtgcaggagcgcagcttgagcctggctgacaaggtggcc gccatcaactattccatgcttagcctgggcaagttttacgcccgcaagatataccataccc cttacgttcccatagacaaggaggtaaagatcgaggggttctacatgcgcatggcgctg aaggtgcttaccttgagcgacgacctgggcgtttatcgcaacgagcgcatccacaaggc cgtgagcgtgagccggcggcgcgagctcagcgaccgcgagctgatgcacagcctgc aaagggccctggctggcacgggcagcggcgatagagaggccgagtcctactttgacg cgggcgctgacctgcgctgggccccaagccgacgcgccctggaggcagctggggcc ggacctgggctggcggtggcacccgcgcgcgctggcaacgtcggcggcgtggagga atatgacgaggacgatgagtacgagccagaggacggcgagtactaagcggtgatgttt ctgatcagatgatgcaagacgcaacggacccggcggtgcgggcggcgctgcagagc cagccgtccggccttaactccacggacgactggcgccaggtcatggaccgcatcatgtc gctgactgcgcgcaatcctgacgcgttccggcagcagccgcaggccaaccggctctcc gcaattctggaagcggtggtcccggcgcgcgcaaaccccacgcacgagaaggtgctg gcgatcgtaaacgcgctggccgaaaacagggccatccggcccgacgaggccggcct ggtctacgacgcgctgcttcagcgcgtggctcgttacaacagcggcaacgtgcagacc aacctggaccggctggtgggggatgtgcgcgaggccgtggcgcagcgtgagcgcgc gcagcagcagggcaacctgggctccatggttgcactaaacgccttcctgagtacacag cccgccaacgtgccgcggggacaggaggactacaccaactttgtgagcgcactgcgg ctaatggtgactgagacaccgcaaagtgaggtgtaccagtctggccagactattttttcc agaccagtagacaaggcctgcagaccgtaaacctgagccaggctttcaaaaacttgc aggggctgtggggggtgcgggctcccacaggcgaccgcgcgaccgtgtctagcttgct gacgcccaactcgcgcctgttgctgctgctaatagcgcccttcacggacagtggcagcg tgtcccgggacacatacctaggtcacttgctgacactgtaccgcgaggccataggtcag gcgcatgtggacgagcatactttccaggagattacaagtgtcagccgcgcgctggggc aggaggacacgggcagcctggaggcaaccctaaactacctgctgaccaaccggcgg cagaagatcccctcgttgcacagtttgcaccctttggcgcatcccattctccagtaactttat gtccatgggcgcactcacagacctgggccaaaaccttctctacgccaactccgcccac gcgctagacatgacttttgaggtggatcccatggacgagcccacccttctttatgttttgtttg aagtctttgacgtggtccgtgtgcaccagccgcaccgcggcgtcatcgaaaccgtgtac ctgcgcacgcccttctcggccggcaacgccacaacataaagaagcaagcaacatca acaacagctgccgccatgggctccagtgagcaggaactgaaagccattgtcaaagat cttggttgtgggccatatttttgggcacctatgacaagcgctttccaggctttgtttctccaca caagctcgcctgcgccatagtcaatacggccggtcgcgagactgggggcgtacactgg atggcctttgcctggaacccgcactcaaaaacatgctacctctttgagccctttggcttttct gaccagcgactcaagcaggtttaccagtttgagtacgagtcactcctgcgccgtagcgc cattgcttcttcccccgaccgctgtataacgctggaaaagtccacccaaagcgtacagg ggcccaactcggccgcctgtggactattctgctgcatgtttctccacgcctttgccaactgg ccccaaactcccatggatcacaaccccaccatgaaccttattaccggggtacccaactc catgctcaacagtccccaggtacagcccaccctgcgtcgcaaccaggaacagctctac agcttcctggagcgccactcgccctacttccgcagccacagtgcgcagattaggagcgc cacttctttttgtcacttgaaaaacatgtaaaaataatgtactagagacactttcaataaag gcaaatgcttttatttgtacactctcgggtgattatttacccccacccttgccgtctgcgccgtt taaaaatcaaaggggttctgccgcgcatcgctatgcgccactggcagggacacgttgc gatactggtgtttagtgctccacttaaactcaggcacaaccatccgcggcagctcggtga agttttcactccacaggctgcgcaccatcaccaacgcgtttagcaggtcgggcgccgat atcttgaagtcgcagttggggcctccgccctgcgcgcgcgagttgcgatacacagggttg cagcactggaacactatcagcgccgggtggtgcacgctggccagcacgctcttgtcgg agatcagatccgcgtccaggtcctccgcgttgctcagggcgaacggagtcaactttggta |
| | | gctgccttcccaaaagggcgcgtgcccaggctttgagttgcactcgcaccgtagtggc atcaaaggtgaccgtgcccggtctgggcgttaggatacagcgcctgcataaaagcctt gatctgcttaaaagccacctgagcctttgcgccttcagagaagaacatgccgcaagactt gccggaaaactgattggccggacaggccgcgtcgtgcacgcagcaccttgcgtcggtg ttggagatctgcaccacatttcggccccaccggttcttcacgatcttggccttgctagactg ctccttcagcgcgcgctgcccgttttcgctcgtcacatccatttcaatcacgtgctccttattta tcataatgcttccgtgtagacacttaagctcgccttcgatctcagcgcagcggtgcagcca caacgcgcagcccgtgggctcgtgatgcttgtaggtcacctctgcaaacgactgcaggt acgcctgcaggaatcgccccatcatcgtcacaaaggtcttgttgctggtgaaggtcagct gcaacccgcggtgctcctcgttcagccaggtcttgcatacggccgccagagcttccactt ggtcaggcagtagtttgaagttcgcctttagatcgttatccacgtggtacttgtccatcagcg cgcgcgcagcctccatgcccttctcccacgcagacacgatcggcacactcagcgggtt catcaccgtaatttcactttccgcttcgctgggctcttcctcttcctcttgcgtccgcatacccac gcgccactgggtcgtcttcattcagccgccgcactgtgcgcttacctcctttgccatgcttga ttagcaccggtgggttgctgaaacccaccatttgtagcgccacatcttctctttcttcctcgct gtccacgattacctctggtgatggcgggcgctcgggcttgggagaagggcgcttctttttct tcttgggcgcaatggccaaatccgccgccgaggtcgatggccgcgggctgggtgtgcg cggcaccagcgcgtcttgtgatgagtcttcctcgtcctcggactcgatacgccgcctcatc cgcttttttgggggcgcccggggaggcggcggcgacggggacggggacgacacgtc ctccatggttgggggacgtcgcgccgcaccgcgtccgcgctcgggggtggtttcgcgct gctcctcttcccgactggccatttccttctcctataggcagaaaaagatcatggagtcagtc gagaagaaggacagcctaaccgccccctctgagttcgccaccaccgcctccaccgat gccgccaacgcgcctaccaccttccccgtcgaggcacccccgcttgaggaggaggaa gtgattatcgagcaggacccaggttttgtaagcgaagacgacgaggaccgctcagtac caacagaggataaaaagcaagaccaggacaacgcagaggcaaacgaggaacaa gtcgggcgggggggacgaaaggcatggcgactacctagatgtgggagacgacgtgct gttgaagcatctgcagcgccagtgcgccattatctgcgacgcgttgcaagagcgcagcg atgtgcccctcgccatagcggatgtcagccttgcctacgaacgccacctattctcaccgc gcgtaccccccaaacgccaagaaaacggcacatgcgagcccaacccgcgcctcaa cttctaccccgtatttgccgtgccagaggtgcttgccacctatcacatctttttccaaaactgc aagatacccctatcctgccgtgccaaccgcagccgagcggacaagcagctggccttgc ggcagggcgctgtcatacctgatatcgcctcgctcaacgaagtgccaaaaatctttgag ggtcttggacgcgacgagaagcgcgcggcaaacgctctgcaacaggaaaacagcg aaaatgaaagtcactctggagtgttggtggaactcgagggtgacaacgcgcgcctagc cgtactaaaacgcagcatcgaggtcacccactttgcctacccggcacttaacctacccc ccaaggtcatgagcacagtcatgagtgagctgatcgtgcgccgtgcgcagcccctgga gagggatgcaaatttgcaagaacaaacagaggagggcctacccgcagttggcgacg agcagctagcgcgctggcttcaaacgcgcgagcctgccgacttggaggagcgacgca aactaatgatggccgcagtgctcgttaccgtggagcttgagtgcatgcagcggttctttgct gacccggagatgcagcgcaagctagaggaaacattgcactacacctttcgacagggc tacgtacgccaggcctgcaagatctccaacgtggagctctgcaacctggtctcctacctt ggaattttgcacgaaaaccgccttgggcaaaacgtgcttcattccacgctcaagggcga ggcgcgccgcgactacgtccgcgactgcgtttacttatttctatgctacacctggcagacg gccatgggcgtttggcagcagtgcttggaggagtgcaacctcaaggagctgcagaaac tgctaaagcaaaacttgaaggacctatggacggccttcaacgagcgctccgtggccgc gcacctggcggacatcattttccccgaacgcctgcttaaaaccctgcaacagggtctgcc agacttcaccagtcaaagcatgttgcagaactttaggaactttatcctagagcgctcagg aatcttgcccgccacctgctgtgcacttcctagcgactttgtgcccattaagtaccgcgaat |
| | | gccctccgccgctttggggccactgctaccttctgcagctagccaactaccttgcctacca ctctgacataatggaagacgtgagcggtgacggtctactggagtgtcactgtcgctgcaa cctatgcaccccgcaccgctccctggtttgcaattcgcagctgcttaacgaaagtcaaatt atcggtacctttgagctgcagggtccctcgcctgacgaaagtccgcggctccggggttg aaactcactccggggctgtggacgtcggcttaccttcgcaaatttgtacctgaggactacc acgcccacgagattaggttctacgaagaccaatcccgcccgcctaatgcggagcttac cgcctgcgtcattacccagggccacattcttggccaattgcaagccatcaacaaagccc gccaagagtttctgctacgaaagggacgggggtttacttggacccccagtccggcga ggagctcaacccaatcccccgccgccgcagccctatcagcagcagccgcgggccct tgcttcccaggatggcacccaaaaagaagctgcagctgccgccgccacccacggacg aggaggaatactggacagtcaggcagaggaggttttggacgaggaggaggagga catgatggaagactgggagagcctagacgaggaagcttccgaggtcgaagaggtgtc agacgaaacaccgtcaccctcggtcgcattcccctcgccggcgccccagaaatcggc aaccggttccagcatggctacaacctccgctcctcaggcgccgccggcactgcccgttc gccgacccaaccgtagatggacaccactggaaccagggccggtaagtccaagcag ccgccgccgttagcccaagagcaacaacagcgccaaggctaccgctcatggcgcgg gcacaagaacgccatagttgcttgcttgcaagactgtgggggcaacatctccttcgcccg ccgctttcttctctaccatcacggcgtggccttcccccgtaacatcctgcattactaccgtca tctctacagcccatactgcaccggcggcagcggcagcaacagcagcggccacacag aagcaaaggcgaccggatagcaagactctgacaaagcccaagaaatccacagcgg cggcagcagcaggaggaggagcgctgcgtctggcgcccaacgaacccgtatcgacc cgcgagcttagaaacaggatttttcccactctgtatgctatatttcaacagagcaggggcc aagaacaagagctgaaaataaaaaacaggtctctgcgatccctcacccgcagctgcct gtatcacaaaagcgaagatcagcttcggcgcacgctggaagacgcggaggctctcttc agtaaatactgcgcgctgactcttaaggactagtttcgcgccctttctcaaatttaagcgcg aaaactacgtcatctccagcggccacacccggcgccagcacctgttgtcagcgccatta tgagcaaggaaattcccacgccctacatgtggagttaccagccacaaatgggacttgc ggctggagctgcccaagactactcaacccgaataaactacatgagcgcggacccca catgatatcccgggtcaacggaatacgcgcccaccgaaaccgaattctcctggaacag gcggctattaccaccacacctcgtaataaccttaatccccgtagttggcccgctgccctgg tgtaccaggaaagtcccgctcccaccactgtggtacttcccagagacgcccaggccga agttcagatgactaactcaggggcgcagcttgcgggcggctttcgtcacagggtgcggt cgcccgggcagggtataactcacctgacaatcagagggcgaggtattcagctcaacg acgagtcggtgagctcctcgcttggtctccgtccggacgggacatttcagatcggcggcg ccggccgctcttcattcacgcctcgtcaggcaatcctaactctgcagacctcgtcctctga gccgcgctctggaggcattggaactctgcaatttattgaggagtttgtgccatcggtctactt taaccccttctcgggacctcccggccactatccggatcaatttattcctaactttgacgcggt aaaggactcggcggacggctacgactgaatgttaagtggagaggcagagcaactgc gcctgaaacacctggtccactgtcgccgccacaagtgctttgcccgcgactccggtgagt tttgctactttgaattgcccgaggatcatatcgagggcccggcgcacggcgtccggcttac cgcccagggagagcttgcccgtagcctgattcgggagtttacccagcgcccccctgctag ttgagcgggacaggggaccctgtgttctcactgtgatttgcaactgtcctaaccctggatta catcaagatcctctagttaattaactagagtacccggggatcttattccctttaactaataaa aaaaaataataaagcatcacttacttaaaatcagttagcaaatttctgtccagtttattcag cagcacctccttgccctcctcccagctctggtattgcagcttcctcctggctgcaaactttctc cacaatctaaatggaatgtcagtttcctcctgttcctgtccatccgcacccactatcttcatgt tgttgcagatgaagcgcgcaagaccgtctgaagataccttcaaccccgtgtatccatatg acacggaaaccggtcctccaactgtgccttttcttactcctccctttgtatcccccaatgggtt |
| | | tcaagagagtccccctggggtactctctttgcgcctatccgaacctctagttacctccaatg gcatgcttgcgctcaaaatgggcaacggcctctctctggacgaggccggcaaccttacc tcccaaaatgtaaccactgtgagcccacctctcaaaaaaccaagtcaaacataaacc tggaaatatctgcacccctcacagttacctcagaagccctaactgtggctgccgccgcac ctctaatggtcgcgggcaacacactcaccatgcaatcacaggccccgctaaccgtgca cgactccaaacttagcattgccacccaaggacccctcacagtgtcagaaggaaagcta gccctgcaaacatcaggcccccctcaccaccaccgatagcagtacccttactatcactgc ctcacccccctctaactactgccactggtagcttgggcattgacttgaaagagcccatttata cacaaaatggaaaactaggactaaagtacggggctcctttgcatgtaacagacgacct aaacactttgaccgtagcaactggtccaggtgtgactattaataatacttccttgcaaacta aagttactggagccttgggttttgattcacaaggcaatatgcaacttaatgtagcaggagg actaaggattgattctcaaaacagacgccttatacttgatgttagttatccgtttgatgctcaa aaccaactaaatctaagactaggacagggccctcttttataaactcagcccacaacttg gatattaactacaacaaaggcctttacttgtttacagcttcaaacaattccaaaaagcttga ggttaacctaagcactgccaaggggttgatgtttgacgctacagccatagccattaatgc aggagatgggcttgaatttggttcacctaatgcaccaaacacaaatcccctcaaaacaa aaattggccatggcctagaatttgattcaaacaaggctatggttcctaaactaggaactg gccttagttttgacagcacaggtgccattacagtaggaaacaaaaataatgataagcta actttgtggaccacaccagctccatctcctaactgtagactaaatgcagagaaagatgct aaactcactttggtcttaacaaaatgtggcagtcaaatacttgctacagtttcagttttggctg ttaaaggcagtttggctccaatatctggaacagttcaaagtgctcatcttattataagatttg acgaaaatggagtgctactaaacaattccttcctggacccagaatattggaactttagaa atggagatcttactgaaggcacagcctatacaaacgctgttggatttatgcctaacctatc agcttatccaaaatctcacggtaaaactgccaaaagtaacattgtcagtcaagtttactta aacggagacaaaactaaacctgtaacactaaccattacactaaacggtacacaggaa acaggagacacaactccaagtgcatactctatgtcattttcatgggactggtctggccac aactacattaatgaaatatttgccacatcctcttacacttttcatacattgcccaagaataa agaatcgtttgtgttatgtttcaacgtgtttatttttcaattgcagaaaatttcaagtcatttttcatt cagtagtatagccccaccaccacatagcttatacagatcaccgtaccttaatcaaactca cagaaccctagtattcaacctgccacctccctcccaacacacagagtacacagtcctttc tccccggctggccttaaaaagcatcatatcatgggtaacagacatattcttaggtgttatatt ccacacggtttcctgtcgagccaaacgctcatcagtgatattaataaactccccgggcag ctcacttaagttcatgtcgctgtccagctgctgagccacaggctgctgtccaacttgcggtt gcttaacgggcggcgaaggagaagtccacgcctacatgggggtagagtcataatcgtg catcaggatagggcggtggtgctgcagcagcgcgcgaataaactgctgccgccgccg ctccgtcctgcaggaatacaacatggcagtggtctcctcagcgatgattcgcaccgccc gcagcataaggcgccttgtcctccgggcacagcagcgcaccctgatctcacttaaatca gcacagtaactgcagcacagcaccacaatattgttcaaaatcccacagtgcaaggcgc tgtatccaaagctcatggcggggaccacagaacccacgtggccatcataccacaagc gcaggtagattaagtggcgacccctcataaacacgctggacataaacattacctcttttg gcatgttgtaattcaccacctcccggtaccatataaacctctgattaaacatggcgccatc caccaccatcctaaaccagctggccaaaacctgcccgccggctatacactgcaggga accgggactggaacaatgacagtggagagcccaggactcgtaaccatggatcatcat gctcgtcatgatatcaatgttggcacaacacaggcacacgtgcatacacttcctcaggatt acaagctcctcccgcgttagaaccatatcccagggaacaacccattcctgaatcagcgt aaatcccacactgcagggaagacctcgcacgtaactcacgttgtgcattgtcaaagtgtt acattcgggcagcagcggatgatcctccagtatggtagcgcgggtttctgtctcaaaagg aggtagacgatccctactgtacggagtgcgccgagacaaccgagatcgtgttggtcgta |
| | | gtgtcatgccaaatggaacgccggacgtagtcatatttcctgaagcaaaccaggtgcg ggcgtgacaaacagatctgcgtctccggtctcgccgcttagatcgctctgtgtagtagttgt agtatatccactctctcaaagcatccaggcgcccctggcttcgggttctatgtaaactcct tcatgcgccgctgccctgataacatccaccaccgcagaataagccacacccagccaa cctacacattcgttctgcgagtcacacacgggaggagcgggaagagctggaagaacc atgtttttttttattccaaaagattatccaaaacctcaaaatgaagatctattaagtgaacg cgctcccctccggtggcgtggtcaaactctacagccaaagaacagataatggcatttgta agatgttgcacaatggcttccaaaaggcaaacggccctcacgtccaagtggacgtaaa ggctaaacccttcagggtgaatctcctctataaacattccagcaccttcaaccatgccca aataattctcatctcgccaccttctcaatatatctctaagcaaatcccgaatattaagtccgg ccattgtaaaaatctgctccagagcgccctccaccttcagcctcaagcagcgaatcatg attgcaaaaattcaggttcctcacagacctgtataagattcaaaagcggaacattaacaa aaataccgcgatcccgtaggtcccttcgcagggccagctgaacataatcgtgcaggtct gcacggaccagcgcggccacttccccgccaggaaccatgacaaaagaacccacact gattatgacacgcatactcggagctatgctaaccagcgtagccccgatgtaagcttgttg catgggcggcgatataaaatgcaaggtgctgctcaaaaaatcaggcaaagcctcgcg caaaaaagaaagcacatcgtagtcatgctcatgcagataaaggcaggtaagctccgg aaccaccacagaaaaagacaccatttttctctcaaacatgtctgcgggtttctgcataaac acaaaataaaataacaaaaaaacatttaaacattagaagcctgtcttacaacaggaaa aacaacccttataagcataagacggactacggccatgccggcgtgaccgtaaaaaa ctggtcaccgtgattaaaaagcaccaccgacagctcctcggtcatgtccggagtcataat gtaagactcggtaaacacatcaggttgattcacatcggtcagtgctaaaaagcgaccga aatagcccgggggaatacatacccgcaggcgtagagacaacattacagcccccatag gaggtataacaaaattaataggagagaaaaacacataaacacctgaaaaaccctcct gcctaggcaaaatagcaccctcccgctccagaacaacatacagcgcttccacagcgg cagccataacagtcagccttaccagtaaaaaagaaaacctattaaaaaaacaccact cgacacggcaccagctcaatcagtcacagtgtaaaaaagggccaagtgcagagcga gtatatataggactaaaaaatgacgtaacggttaaagtccacaaaaaacacccagaa aaccgcacgcgaacctacgcccagaaacgaaagccaaaaaacccacaacttcctca aatcgtcacttccgttttcccacgttacgtcacttcccattttaagaaaactacaattcccaa cacatacaagttactccgccctaaaacctacgtcacccgccccgttcccacgccccgcg ccacgtcacaaactccaccccctcattatcatattggcttcaatccaaaataaggtatatt cactagttatcagcacacaattgcccattatacgcgcgtataatggactattgtgtgctgat atagggataacagggtaattctagagctagcatatggatccatcgattttcggggaaatgt gcgcggaacccctatttgtttatttttctaaatacattcaaatatgtatccgctcatgagacaa taaccctgataaatgcttcaataatattgaaaaaggaagagtatgagccatattcaacgg gaaacgtcgaggccgcgattaaattccaacatggatgctgatttatatgggtataaatgg gctcgcgataatgtcgggcaatcaggtgcgacaatctatcgcttgtatgggaagcccgat gcgccagagttgtttctgaaacatggcaaaggtagcgttgccaatgatgttacagatgag atggtcagactaaactggctgacggaatttatgcctcttccgaccatcaagcattttatccg tactcctgatgatgcatggttactcaccactgcgatccccggaaaaacagcattccaggt attagaagaatatcctgattcaggtgaaaatattgttgatgcgctggcagtgttcctgcgcc ggttgcattcgattcctgtttgtaattgtccttttaacagcgatcgcgtatttcgtctcgctcagg cgcaatcacgaatgaataacggtttggttgatgcgagtgattttgatgacgagcgtaatgg ctggcctgttgaacaagtctggaaagaaatgcataaacttttgccattctcaccggattca gtcgtcactcatggtgatttctcacttgataaccttatttttgacgaggggaaattaataggtt gtattgatgttggacgagtcggaatcgcagaccgataccaggatcttgccatcctatgga actgcctcggtgagttttctccttcattacagaaacggctttttcaaaaatatggtattgataat |
| | | cctgatatgaataaattgcagtttcatttgatgctcgatgagtttttctaagcgtataatggtct agagctagcatatggatccatcgattccattatacgcctgtcagaccaagtttactcatata tactttagattgatttaaaacttcatttttaatttaaaaggatctaggtgaagatcctttttgata atctcatgaccaaaatcccttaacgtgagttttcgttccactgagcgtcagaccccgtaga aaagatcaaaggatcttcttgagatcctttttttctgcgcgtaatctgctgcttgcaaacaaa aaaaccaccgctaccagcggtggtttgtttgccggatcaagagctaccaactctttttccg aaggtaactggcttcagcagagcgcagataccaaatactgttcttctagtgtagccgtagt taggccaccacttcaagaactctgtagcaccgcctacatacctcgctctgctaatcctgtta ccagtggctgctgccagtggcgataagtcgtgtcttaccgggttggactcaagacgatag ttaccggataaggcgcagcggtcgggctgaacggggggttcgtgcacacagcccagc ttggagcgaacgacctacaccgaactgagatacctacagcgtgagctatgagaaagc gccacgcttcccgaagggagaaaggcggacaggtatccggtaagcggcagggtcgg aacaggagagcgcacgagggagcttccagggggaaacgcctggtatctttatagtcct gtcgggtttcgccacctctgacttgagcgtcgatttttgtgatgctcgtcaggggggcggag cctatggaaaaacgccagcaacgcggcctttttacggttcctggccttttgctggccttttgc tcacatgt |
| 97 | XX680 plasmid DNA sequence | tcttccgcttcctcgctcactgactcgctgcgctcggtcgttcggctgcggcgagcggtatc agctcactcaaaggcggtaatacggttatccacagaatcaggggataacgcaggaaa gaacatgtgagcaaaaggccagcaaaaggccaggaaccgtaaaaaggccgcgttg ctggcgtttttccataggctccgcccccctgacgagcatcacaaaaatcgacgctcaagt cagaggtggcgaaacccgacaggactataaagataccaggcgtttccccctggaagc tccctcgtgcgctctcctgttccgaccctgccgcttaccggatacctgtccgcctttctccctt cgggaagcgtggcgctttctcatagctcacgctgtaggtatctcagttcggtgtaggtcgtt cgctccaagctgggctgtgtgcacgaaccccccgttcagcccgaccgctgcgccttatc cggtaactatcgtcttgagtccaacccggtaagacacgacttatcgccactggcagcag ccactggtaacaggattagcagagcgaggtatgtaggcggtgctacagagttcttgaag tggtggcctaactacggctacactagaagaacagtatttggtatctgcgctctgctgaagc cagttaccttcggaaaaagagttggtagctcttgatccggcaaacaaaccaccgctggt agcggtggtttttttgtttgcaagcagcagattacgcgcagaaaaaaaggatctcaagaa gatcctttgatcttttctacggggtctgacgctcagtggaacgaaaactcacgttaagggat tttggtcatgagattatcaaaaaggatcttcacctagatccttttaaattaaaaatgaagtttt aaatcaatctaaagtatatatgagtaaacttggtctgacagttattagaaaaactcatcga gcatcaaatgaaactgcaatttattcatatcaggattatcaataccatatttttgaaaaagc cgtttctgtaatgaaggagaaaactcaccgaggcagttccataggatggcaagatcctg gtatcggtctgcgattccgactcgtccaacatcaatacaacctattaatttcccctcgtcaa aaataaggttatcaagtgagaaatcaccatgagtgacgactgaatccggtgagaatgg caaaagtttatgcatttctttccagacttgttcaacaggccagccattacgctcgtcatcaaa atcactcgcatcaaccaaaccgttattcattcgtgattgcgcctgagcgagacgaaatac gcgatcgctgttaaaaggacaattacaaacaggaatcgaatgcaaccggcgcaggaa cactgccagcgcatcaacaatattttcacctgaatcaggatattcttctaatacctggaatg ctgtttttccggggatcgcagtggtgagtaaccatgcatcatcaggagtacggataaaat gcttgatggtcggaagaggcataaattccgtcagccagtttagtctgaccatctcatctgta acatcattggcaacgctacctttgccatgtttcagaaacaactctggcgcatcgggcttcc catacaagcgatagattgtcgcacctgattgcccgacattatcgcgagcccatttataccc atataaatcagcatccatgttggaatttaatcgcggcctcgacgtttcccgttgaatatggct catactcttcctttttcaatattattgaagcatttatcagggttattgtctcatgagcggatacat atttgaatgtatttagaaaaataaacaaataggggttccgcgcacatttccccgaaaagt gccacctgacgtctaagaaaccattattatcatgacattaacctataaaaataggcgtatc |
| | | acgaggccctttcgtctcgcgcgtttcggtgatgacggtgaaaacctctgacacatgcag ctcccggagacggtcacagcttgtctgtaagcggatgccgggagcagacaagcccgtc agggcgcgtcagcgggtgttggcgggtgtcggggctggcttaactatgcggcatcagag cagattgtactgagagtgcaccataaaattgtaaacgttaatattttgttaaaattcgcgtta aatttttgttaaatcagctcattttttaaccaataggccgaaatcggcaaaatcccttataaat caaaagaatagcccgagatagggttgagtgttgttccagtttggaacaagagtccactat taaagaacgtggactccaacgtcaaagggcgaaaaaccgtctatcagggcgatggcc cactacgtgaaccatcacccaaatcaagttttttggggtcgaggtgccgtaaagcactaa atcggaaccctaaagggagcccccgatttagagcttgacggggaaagccggcgaac gtggcgagaaaggaagggaagaaagcgaaaggagcgggcgctagggcgctggca agtgtagcggtcacgctgcgcgtaaccaccacacccgccgcgcttaatgcgccgctac agggcgcgtactatggttgctttgacgtatgcggtgtgaaataccgcacagatgcgtaag gagaaaataccgcatcaggcgccattcgccattcaggctgcgcaactgttgggaaggg cgatcggtgcgggcctcttcgctattacgccagctggcgaaagggggatgtgctgcaag gcgattaagttgggtaacgccagggttttcccagtcacgacgttgtaaaacgacggcca gtgccaagcttaaggtgcacggcccacgtggccactagtacttctcgacagaagcacc atgtccttgggtccggcctgctgaatgcgcaggcggtcggccatgccccaggcttcgtttt gacatcggcgcaggtctttgtagtagtcttgcatgagcctttctaccggcacttcttcttctcct tcctcttgtcctgcatctcttgcatctatcgctgcggcggcggcggagtttggccgtaggtgg cgccctcttcctcccatgcgtgtgaccccgaagcccctcatcggctgaagcagggctag gtcggcgacaacgcgctcggctaatatggcctgctgcacctgcgtgagggtagactgg aagtcatccatgtccacaaagcggtggtatgcgcccgtgttgatggtgtaagtgcagttgg ccataacggaccagttaacggtctggtgacccggctgcgagagctcggtgtacctgag acgcgagtaagccctcgagtcaaatacgtagtcgttgcaagtccgcaccaggtactggt atcccaccaaaaagtgcggcggcggctggcggtagaggggccagcgtagggtggcc ggggctccgggggcgagatcttccaacataaggcgatgatatccgtagatgtacctgga catccaggtgatgccggcggcggtggtggaggcgcgcggaaagtcgcggacgcggtt ccagatgttgcgcagcggcaaaaagtgctccatggtcgggacgctctggccggtcagg cgcgcgcaatcgttgacgctctagcgtgcaaaaggagagcctgtaagcgggcactcttc cgtggtctggtggataaattcgcaagggtatcatggcggacgaccggggttcgagcccc gtatccggccgtccgccgtgatccatgcggttaccgcccgcgtgtcgaacccaggtgtgc gacgtcagacaacgggggagtgctccttttggcttccttccaggcgcggcggctgctgcg ctagcttttttggccactggccgcgcgcagcgtaagcggttaggctggaaagcgaaagc attaagtggctcgctccctgtagccggagggttattttccaagggttgagtcgcgggaccc ccggttcgagtctcggaccggccggactgcggcgaacgggggtttgcctccccgtcatg caagaccccgcttgcaaattcctccggaaacagggacgagcccctttttttgcttttcccag atgcatccggtgctgcggcagatgcgcccccctcctcagcagcggcaagagcaagag cagcggcagacatgcagggcaccctcccctcctcctaccgcgtcaggaggggcgaca tccgcggttgacgcggcagcagatggtgattacgaacccccgcggcgccgggcccgg cactacctggacttggaggagggcgagggcctggcgcggctaggagcgccctctcctg agcggcacccaagggtgcagctgaagcgtgatacgcgtgaggcgtacgtgccgcgg cagaacctgtttcgcgaccgcgagggagaggagcccgaggagatgcgggatcgaaa gttccacgcagggcgcgagctgcggcatggcctgaatcgcgagcggttgctgcgcgag gaggactttgagcccgacgcgcgaaccgggattagtcccgcgcgcgcacacgtggcg gccgccgacctggtaaccgcatacgagcagacggtgaaccaggagattaactttcaa aaaagctttaacaaccacgtgcgtacgcttgtggcgcgcgaggaggtggctataggact gatgcatctgtgggactttgtaagcgcgctggagcaaaacccaaatagcaagccgctc atggcgcagctgttccttatagtgcagcacagcagggacaacgaggcattcagggatg |
| | | cgctgctaaacatagtagagcccgagggccgctggctgctcgatttgataaacatcctgc agagcatagtggtgcaggagcgcagcttgagcctggctgacaaggtggccgccatca actattccatgcttagcctgggcaagttttacgcccgcaagatataccataccccttacgtt cccatagacaaggaggtaaagatcgaggggttctacatgcgcatggcgctgaaggtgc ttaccttgagcgacgacctgggcgtttatcgcaacgagcgcatccacaaggccgtgagc gtgagccggcggcgcgagctcagcgaccgcgagctgatgcacagcctgcaaagggc cctggctggcacgggcagcggcgatagagaggccgagtcctactttgacgcgggcgct gacctgcgctgggccccaagccgacgcgccctggaggcagctggggccggacctgg gctggcggtggcacccgcgcgcgctggcaacgtcggcggcgtggaggaatatgacg aggacgatgagtacgagccagaggacggcgagtactaagcggtgatgtttctgatcag atgatgcaagacgcaacggacccggcggtgcgggcggcgctgcagagccagccgtc cggccttaactccacggacgactggcgccaggtcatggaccgcatcatgtcgctgactg cgcgcaatcctgacgcgttccggcagcagccgcaggccaaccggctctccgcaattct ggaagcggtggtcccggcgcgcgcaaaccccacgcacgagaaggtgctggcgatcg taaacgcgctggccgaaaacagggccatccggcccgacgaggccggcctggtctac gacgcgctgcttcagcgcgtggctcgttacaacagcggcaacgtgcagaccaacctgg accggctggtgggggatgtgcgcgaggccgtggcgcagcgtgagcgcgcgcagcag cagggcaacctgggctccatggttgcactaaacgccttcctgagtacacagcccgcca acgtgccgcggggacaggaggactacaccaactttgtgagcgcactgcggctaatggt gactgagacaccgcaaagtgaggtgtaccagtctgggccagactatttttccagacca gtagacaaggcctgcagaccgtaaacctgagccaggctttcaaaaacttgcaggggct gtgggggtgcgggctcccacaggcgaccgcgaccgtgtctagcttgctgacgccc aactcgcgcctgttgctgctgctaatagcgcccttcacggacagtggcagcgtgtcccgg gacacatacctaggtcacttgctgacactgtaccgcgaggccataggtcaggcgcatgt ggacgagcatactttccaggagattacaagtgtcagccgcgcgctggggcaggagga cacgggcagcctggaggcaaccctaaactacctgctgaccaaccggcggcagaaga tcccctcgttgcacagtttgcaccctttggcgcatcccattctccagtaactttatgtccatgg gcgcactcacagacctgggccaaaaccttctctacgccaactccgcccacgcgctaga catgacttttgaggtggatcccatggacgagcccacccttctttatgttttgtttgaagtctttg acgtggtccgtgtgcaccagccgcaccgcggcgtcatcgaaaccgtgtacctgcgcac gcccttctcggccggcaacgccacaacataaagaagcaagcaacatcaacaacagc tgccgccatgggctccagtgagcaggaactgaaagccattgtcaaagatcttggttgtgg gccatatttttgggcacctatgacaagcgctttccaggctttgtttctccacacaagctcgc ctgcgccatagtcaatacggccggtcgcgagactgggggcgtacactggatggcctttg cctggaacccgcactcaaaaacatgctacctctttgagccctttggcttttctgaccagcg actcaagcaggtttaccagtttgagtacgagtcactcctgcgccgtagcgccattgcttctt cccccgaccgctgtataacgctggaaaagtccacccaaagcgtacaggggcccaact cggccgcctgtggactattctgctgcatgtttctccacgcctttgccaactggccccaaact cccatggatcacaaccccaccatgaaccttattaccggggtacccaactccatgctcaa cagtccccaggtacagcccaccctgcgtcgcaaccaggaacagctctacagcttcctg gagcgccactcgccctacttccgcagccacagtgcgcagattaggagcgccacttctttt gtcacttgaaaaacatgtaaaaataatgtactagagacactttcaataaaggcaaatgct tttatttgtacactctcgggtgattatttacccccacccttgccgtctgcgccgtttaaaaatca aaggggttctgccgcgcatcgctatgcgccactggcagggacacgttgcgatactggtg tttagtgctccacttaaactcaggcacaaccatccgcggcagctcggtgaagttttcactc cacaggctgcgcaccatcaccaacgcgtttagcaggtcgggcgccgatatcttgaagtc gcagttggggcctccgccctgcgcgcgcgagttgcgatacacagggttgcagcactgg aacactatcagcgccgggtggtgcacgctggccagcacgctcttgtcggagatcagatc |
| | | cgcgtccaggtcctccgcgttgctcagggcgaacggagtcaactttggtagctgccttcc caaaaagggcgcgtgcccaggctttgagttgcactcgcaccgtagtggcatcaaaagg tgaccgtgcccggtctgggcgttaggatacagcgcctgcataaaagccttgatctgctta aaagccacctgagcctttgcgccttcagagaagaacatgccgcaagacttgccggaaa actgattggccggacaggccgcgtcgtgcacgcagcaccttgcgtcggtgttggagatct gcaccacatttcggccccaccggttcttcacgatcttggccttgctagactgctccttcagc gcgcgctgcccgttttcgctcgtcacatccatttcaatcacgtgctccttatttatcataatgct tccgtgtagacacttaagctcgccttcgatctcagcgcagcggtgcagccacaacgcgc agcccgtgggctcgtgatgcttgtaggtcacctctgcaaacgactgcaggtacgcctgca ggaatcgccccatcatcgtcacaaaggtcttgttgctggtgaaggtcagctgcaacccgc ggtgctcctcgttcagccaggtcttgcatacggccgccagagcttccacttggtcaggca gtagtttgaagttcgcctttagatcgttatccacgtggtacttgtccatcagcgcgcgcgca gcctccatgcccttctcccacgcagacacgatcggcacactcagcgggttcatcaccgt aatttcactttccgcttcgctgggctcttcctcttcctcttgcgtccgcataccacgcgccact gggtcgtcttcattcagccgccgcactgtgcgcttacctcctttgccatgcttgattagcacc ggtgggttgctgaaacccaccatttgtagcgccacatcttctctttcttcctcgctgtccacg attacctctggtgatggcgggcgctcgggcttgggagaagggcgcttctttttcttcttgggc gcaatggccaaatccgccgccgaggtcgatggccgcgggctgggtgtgcgcggcacc agcgcgtcttgtgatgagtcttcctcgtcctcggactcgatacgccgcctcatccgcttttttg ggggcgcccggggaggcggcggcgacggggacggggacgacacgtcctccatggt tgggggacgtcgcgccgcaccgcgtccgcgctcgggggtggtttcgcgctgctcctcttc ccgactggccatttccttctcctataggcagaaaaagatcatggagtcagtcgagaaga aggacagcctaaccgcccccctctgagttcgccaccaccgcctccaccgatgccgccaa cgcgcctaccaccttccccgtcgaggcacccccgcttgaggaggaggaagtgattatc gagcaggacccaggttttgtaagcgaagacgacgaggaccgctcagtaccaacaga ggataaaaagcaagaccaggacaacgcagaggcaaacgaggaacaagtcgggc gggggacgaaaggcatggcgactacctagatgtgggagacgacgtgctgttgaagc atctgcagcgccagtgcgccattatctgcgacgcgttgcaagagcgcagcgatgtgccc ctcgccatagcggatgtcagccttgcctacgaacgccacctattctcaccgcgcgtaccc cccaaacgccaagaaaacggcacatgcgagcccaacccgcgcctcaacttctaccc cgtatttgccgtgccagaggtgcttgccacctatcacatctttttccaaaactgcaagatac ccctatcctgccgtgccaaccgcagccgagcggacaagcagctggccttgcggcagg gcgctgtcatacctgatatcgcctcgctcaacgaagtgccaaaaatctttgagggtcttgg acgcgacgagaagcgcgcggcaaacgctctgcaacaggaaaacagcgaaaatga aagtcactctggagtgttggtggaactcgagggtgacaacgcgcgcctagccgtactaa aacgcagcatcgaggtcacccactttgcctacccggcacttaacctaccccccaaggtc atgagcacagtcatgagtgagctgatcgtgcgccgtgcgcagcccctggagagggatg caaatttgcaagaacaaacagaggagggcctacccgcagttggcgacgagcagcta gcgcgctggcttcaaacgcgcgagcctgccgacttggaggagcgacgcaaactaatg atggccgcagtgctcgttaccgtggagcttgagtgcatgcagcggttctttgctgacccgg agatgcagcgcaagctagaggaaacattgcactacacctttcgacagggctacgtacg ccaggcctgcaagatctccaacgtggagctctgcaacctggtctcctaccttggaattttg cacgaaaaccgccttgggcaaaacgtgcttcattccacgctcaagggcgaggcgcgc cgcgactacgtccgcgactgcgtttacttatttctatgctacacctggcagacggccatgg gcgtttggcagcagtgcttggaggagtgcaacctcaaggagctgcagaaactgctaaa gcaaaacttgaaggacctatggacggccttcaacgagcgctccgtggccgcgcacctg gcggacatcattttccccgaacgcctgcttaaaaccctgcaacagggtctgccagacttc accagtcaaagcatgttgcagaactttaggaactttatcctagagcgctcaggaatcttgc |
| | | ccgccacctgctgtgcacttcctagcgactttgtgcccattaagtaccgcgaatgccctcc gccgctttggggccactgctaccttctgcagctagccaactaccttgcctaccactctgac ataatggaagacgtgagcggtgacggtctactggagtgtcactgtcgctgcaacctatgc accccgcaccgctccctggtttgcaattcgcagctgcttaacgaaagtcaaattatcggta cctttgagctgcagggtccctcgcctgacgaaaagtccgcggctccggggttgaaactc actccggggctgtggacgtcggcttaccttcgcaaatttgtacctgaggactaccacgcc cacgagattaggttctacgaagaccaatcccgcccgcctaatgcggagcttaccgcctg cgtcattacccagggccacattcttggccaattgcaagccatcaacaaagcccgccaa gagtttctgctacgaaagggacgggggtttacttggacccccagtccggcgaggagct caacccaatcccccgccgccgcagccctatcagcagcagccgcgggcccttgcttcc caggatggcacccaaaaagaagctgcagctgccgccgccacccacggacgaggag gaatactgggacagtcaggcagaggaggttttggacgaggaggaggaggacatgat ggaagactgggagagcctagacgaggaagcttccgaggtcgaagaggtgtcagacg aaacaccgtcaccctcggtcgcattcccctcgccggcgccccagaaatcggcaaccg gttccagcatggctacaacctccgctcctcaggcgccgccggcactgcccgttcgccga cccaaccgtagatgggacaccactggaaccagggccggtaagtccaagcagccgcc gccgttagcccaagagcaacaacagcgccaaggctaccgctcatggcgcgggcaca agaacgccatagttgcttgcttgcaagactgtgggggcaacatctccttcgcccgccgctt tcttctctaccatcacggcgtggccttcccccgtaacatcctgcattactaccgtcatctcta cagcccatactgcaccggcggcagcggcagcaacagcagcggccacacagaagc aaaggcgaccggatagcaagactctgacaaagcccaagaaatccacagcggcggc agcagcaggaggaggagcgctgcgtctggcgcccaacgaacccgtatcgacccgcg agcttagaaacaggatttttcccactctgtatgctatatttcaacagagcaggggccaaga acaagagctgaaaataaaaaacaggtctctgcgatccctcacccgcagctgcctgtatc acaaaagcgaagatcagcttcggcgcacgctggaagacgcggaggctctcttcagta aatactgcgcgctgactcttaaggactagtttcgcgccctttctcaaatttaagcgcgaaa actacgtcatctccagcggccacacccggcgccagcacctgttgtcagcgccattatga gcaaggaaattcccacgccctacatgtggagttaccagccacaaatgggacttgcggct ggagctgcccaagactactcaacccgaataaactacatgagcgcgggaccccacatg atatcccgggtcaacggaatacgcgcccaccgaaaccgaattctcctggaacaggcg gctattaccaccacacctcgtaataaccttaatccccgtagttggcccgctgccctggtgta ccaggaaagtcccgctcccaccactgtggtacttcccagagacgcccaggccgaagtt cagatgactaactcaggggcgcagcttgcgggcggctttcgtcacagggtgcggtcgcc cgggcagggtataactcacctgacaatcagagggcgaggtattcagctcaacgacga gtcggtgagctcctcgcttggtctccgtccggacgggacatttcagatcggcggcgccgg ccgctcttcattcacgcctcgtcaggcaatcctaactctgcagacctcgtcctctgagccg cgctctggaggcattggaactctgcaatttattgaggagtttgtgccatcggtctactttaac cccttctcgggacctcccggccactatccggatcaatttattcctaactttgacgcggtaaa ggactcggcggacggctacgactgaatgttaagtggagaggcagagcaactgcgcct gaaacacctggtccactgtcgccgccacaagtgctttgcccgcgactccggtgagttttg ctactttgaattgcccgaggatcatatcgagggcccggcgcacggcgtccggcttaccg cccagggagagcttgcccgtagcctgattcgggagtttacccagcgcccccctgctagttg agcgggacaggggaccctgtgttctcactgtgatttgcaactgtcctaaccctggattaca tcaagatcctctagttaattaactagagtacccggggatcttattccctttaactaataaaaa aaaataataaagcatcacttacttaaaatcagttagcaaatttctgtccagtttattcagca gcacctccttgccctcctcccagctctggtattgcagcttcctcctggctgcaaactttctcca caatctaaatggaatgtcagtttcctcctgttcctgtccatccgcacccactatcttcatgttgt tgcagatgaagcgcgcaagaccgtctgaagataccttcaaccccgtgtatccatatgac |
| | | acggaaaccggtcctccaactgtgccttttcttactcctccctttgtatcccccaatgggtttc aagagagtccccctggggtactctctttgcgcctatccgaacctctagttacctccaatgg catgcttgcgctcaaaatgggcaacggcctctctctggacgaggccggcaaccttacct cccaaaatgtaaccactgtgagcccacctctcaaaaaaaccaagtcaaacataaacct ggaaatatctgcacccctcacagttacctcagaagccctaactgtggctgccgccgcac ctctaatggtcgcgggcaacacactcaccatgcaatcacaggccccgctaaccgtgca cgactccaaacttagcattgccacccaaggacccctcacagtgtcagaaggaaagcta gccctgcaaacatcaggcccccctcaccaccaccgatagcagtacccttactatcactgc ctcacccccctctaactactgccactggtagcttgggcattgacttgaaagagcccatttata cacaaaatggaaaactaggactaaagtacggggctcctttgcatgtaacagacgacct aaacactttgaccgtagcaactggtccaggtgtgactattaataatacttccttgcaaacta aagttactggagccttgggttttgattcacaaggcaatatgcaacttaatgtagcaggagg actaaggattgattctcaaaacagacgccttatacttgatgttagttatccgtttgatgctcaa aaccaactaaatctaagactaggacagggccctctttttataaactcagcccacaacttg gatattaactacaacaaaggcctttacttgtttacagcttcaaacaattccaaaaagcttga ggttaacctaagcactgccaaggggttgatgtttgacgctacagccatagccattaatgc aggagatgggcttgaatttggttcacctaatgcaccaaacacaaatcccctcaaaacaa aaattggccatggcctagaatttgattcaaacaaggctatggttcctaaactaggaactg gccttagttttgacagcacaggtgccattacagtaggaaacaaaaataatgataagcta actttgtggaccacaccagctccatctcctaactgtagactaaatgcagagaaagatgct aaactcactttggtcttaacaaaatgtggcagtcaaatacttgctacagtttcagttttggctg ttaaaggcagtttggctccaatatctggaacagttcaaagtgctcatcttattataagatttg acgaaaatggagtgctactaaacaattccttcctggacccagaatattggaactttagaa atggagatcttactgaaggcacagcctatacaaacgctgttggatttatgcctaacctatc agcttatccaaaatctcacggtaaaactgccaaaagtaacattgtcagtcaagtttactta aacggagacaaaactaaacctgtaacactaaccattacactaaacggtacacaggaa acaggagacacaactccaagtgcatactctatgtcattttcatgggactggtctggccac aactacattaatgaaatatttgccacatcctcttacacttttcatacattgcccaagaataa agaatcgtttgtgttatgtttcaacgtgtttatttttcaattgcagaaaatttcaagtcatttttcatt cagtagtatagccccaccaccacatagcttatacagatcaccgtaccttaatcaaactca cagaaccctagtattcaacctgccacctccctcccaacacacagagtacacagtcctttc tccccggctggccttaaaaagcatcatatcatgggtaacagacatattcttaggtgttatatt ccacacggtttcctgtcgagccaaacgctcatcagtgatattaataaactccccgggcag ctcacttaagttcatgtcgctgtccagctgctgagccacaggctgctgtccaacttgcggtt gcttaacgggcggcgaaggagaagtccacgcctacatgggggtagagtcataatcgtg catcaggatagggcggtggtgctgcagcagcgcgcgaataaactgctgccgccgccg ctccgtcctgcaggaatacaacatggcagtggtctcctcagcgatgattcgcaccgccc gcagcataaggcgccttgtcctccgggcacagcagcgcaccctgatctcacttaaatca gcacagtaactgcagcacagcaccacaatattgttcaaaatcccacagtgcaaggcgc tgtatccaaagctcatggcggggaccacagaacccacgtggccatcataccacaagc gcaggtagattaagtggcgacccctcataaacacgctggacataaacattacctctttg gcatgttgtaattcaccacctcccggtaccatataaacctctgattaaacatggcgccatc caccaccatcctaaaccagctggccaaaacctgcccgccggctatacactgcaggga accgggactggaacaatgacagtggagagcccaggactcgtaaccatggatcatcat gctcgtcatgatatcaatgttggcacaacacaggcacacgtgcatacacttcctcaggatt acaagctcctcccgcgttagaaccatatcccagggaacaacccattcctgaatcagcgt aaatcccacactgcagggaagacctcgcacgtaactcacgttgtgcattgtcaaagtgtt acattcgggcagcagcggatgatcctccagtatggtagcgcgggtttctgtctcaaaagg |
| | | aggtagacgatccctactgtacggagtgcgccgagacaaccgagatcgtgttggtcgta gtgtcatgccaaatggaacgccggacgtagtcatatttcctgaagcaaaaccaggtgcg ggcgtgacaaacagatctgcgtctccggtctcgccgcttagatcgctctgtgtagtagttgt agtatatccactctctcaaagcatccaggcgcccctggcttcgggttctatgtaaactcct tcatgcgccgctgccctgataacatccaccaccgcagaataagccacacccagccaa cctacacattcgttctgcgagtcacacacgggaggagcgggaagagctggaagaacc atgtttttttttattccaaaagattatccaaaacctcaaaatgaagatctattaagtgaacg cgctcccctccggtggcgtggtcaaactctacagccaaagaacagataatggcatttgta agatgttgcacaatggcttccaaaaggcaaacggccctcacgtccaagtggacgtaaa ggctaaacccttcagggtgaatctcctctataaacattccagcaccttcaaccatgccca aataattctcatctcgccaccttctcaatatatctctaagcaaatcccgaatattaagtccgg ccattgtaaaaatctgctccagagcgccctccaccttcagcctcaagcagcgaatcatg attgcaaaaattcaggttcctcacagacctgtataagattcaaaagcggaacattaacaa aaataccgcgatcccgtaggtcccttcgcagggccagctgaacataatcgtgcaggtct gcacggaccagcgcggccacttccccgccaggaaccatgacaaaagaacccacact gattatgacacgcatactcggagctatgctaaccagcgtagccccgatgtaagcttgttg catgggcggcgatataaaatgcaaggtgctgctcaaaaaatcaggcaaagcctcgcg caaaaaagaaagcacatcgtagtcatgctcatgcagataaaggcaggtaagctccgg aaccaccacagaaaaagacaccatttttctctcaaacatgtctgcgggtttctgcataaac acaaaataaaataacaaaaaaacatttaaacattagaagcctgtcttacaacaggaaa aacaacccttataagcataagacggactacggccatgccggcgtgaccgtaaaaaa ctggtcaccgtgattaaaaagcaccaccgacagctcctcggtcatgtccggagtcataat gtaagactcggtaaacacatcaggttgattcacatcggtcagtgctaaaaagcgaccga aatagcccgggggaatacatacccgcaggcgtagagacaacattacagcccccatag gaggtataacaaaattaataggagagaaaaacacataaacacctgaaaaaccctcct gcctaggcaaaatagcaccctcccgctccagaacaacatacagcgcttccacagcgg cagccataacagtcagccttaccagtaaaaaagaaaacctattaaaaaaacaccact cgacacggcaccagctcaatcagtcacagtgtaaaaaagggccaagtgcagagcga gtatatataggactaaaaaatgacgtaacggttaaagtccacaaaaaacacccagaa aaccgcacgcgaacctacgcccagaaacgaaagccaaaaaacccacaacttcctca aatcgtcacttccgttttcccacgttacgtcacttcccattttaagaaaactacaattcccaa cacatacaagttactccgccctaaaacctacgtcacccgccccgttcccacgccccgcg ccacgtcacaaactccaccccctcattatcatattggcttcaatccaaaataatcatcaat aatataccttattttggattgaagccaatatgataatgagggggtggagtttgtgacgtggc gcggggcgtgggaacggggcgggtgacgtagtagtgtggcggaagtgtgatgttgcaa gtgtggcggaacacatgtaagcgacggatgtggcaaaagtgacgtttttggtgtgcgcc ggatccacaggacgggtgtggtcgccatgatcgcgtagtcgatagtggctccaagtagc gaagcgagcaggactgggcggcggccaaagcggtcggacagtgctccgagaacgg gtgcgcatagaaattgcatcaacgcatatagcgctagcagcacgccatagtgactggc gatgctgtcggaatggacgatatcccgcaagaggcccggcagtaccggcataaccaa gcctatgcctacagcatccagggtgacggtgccgaggatgacgatgagcgcattgttag atttcatacacggtgcctgactgcgttagcaatttaactgtgataaactaccgcattaaagc ttatcgaattcgtaatcatgtcatagctgtttcctgtgtgaaattgttatccgctcacaattcca cacaacatacgagccggaagcataaagtgtaaagcctggggtgcctaatgagtgagct aactcacattaattgcgttgcgctcactgcccgctttccagtcgggaaacctgtcgtgcca gctgcattaatgaatcggccaacgcgcggggagaggcggtttgcgtattgggcgc |
| 98 | XX85 neDNA sequence | gcgtataatggactattgtgtgctgataggcgcgccttggattgaagccaatatgataatg agggggtggagtttgtgacgtggcgcggggcgtgggaacggggcgggtgacgtaggtt ttagggcggagtaacttgtatgtgttgggaattgtagttttcttaaaatgggaagttacgtaa cgtgggaaaacggaagtgacgatttgaggaagttgtgggtttttggctttcgtttctgggcg taggttcgcgtgcggttttctgggtgtttttgtggactttaaccgttacgtcatttttagtcctat atatactcgctctgcacttggcccttttttacactgtgactgattgagctggtgccgtgtcgag tggtgtttttttaataggttttctttttactggtaaggctgactgttatggctgccgctgtggaag cgctgtatgttgttctggagcgggagggtgctattttgcctaggcaggagggtttttcaggtg tttatgtgtttttctctcctattaatttgttatacctcctatgggggctgtaatgttgtctctacgcct gcgggtatgtattcccccgggctatttcggtcgctttttagcactgaccgatgtgaatcaacc tgatgtgtttaccgagtcttacattatgactccggacatgaccgaggagctgtcggtggtgc tttttaatcacggtgaccagtttttttacggtcacgccggcatggccgtagtccgtcttatgctt ataagggttgtttttcctgttgtaagacaggcttctaatgtttaaatgtttttttgttattttattttgtg tttatgcagaaacccgcagacatgtttgagagaaaaatggtgtctttttctgtggtggttccg gagcttacctgcctttatctgcatgagcatgactacgatgtgctttctttttgcgcgaggcttt gcctgatttttgagcagcaccttgcattttatatcgccgcccatgcaacaagcttacatcgg ggctacgctggttagcatagctccgagtatgcgtgtcataatcagtgtgggttcttttgtcatg gttcctggcggggaagtggccgcgctggtccgtgcagacctgcacgattatgttcagctg gccctgcgaagggacctacgggatcgcggtatttttgttaatgttccgcttttgaatcttatac aggtctgtgaggaacctgaattttgcaatcatgattcgctgcttgaggctgaaggtggag ggcgctctggagcagatttttacaatggccggacttaatattcgggatttgcttagagatat attgagaaggtggcgagatgagaattatttgggcatggttgaaggtgctggaatgtttata gaggagattcaccctgaagggtttagcctttacgtccacttggacgtgagggccgtttgcc ttttggaagccattgtgcaacatcttacaaatgccattatctgttctttggctgtagagtttgac cacgccaccggagggggagcgcgttcacttaatagatcttcattttgaggttttggataatctt ttggaataaaaaaaaaaacatggttcttccagctcttcccgctcctcccgtgtgtgactcgc agaacgaatgtgtaggttggctgggtgtggcttattctgcggtggtggatgttatcagggca gcggcgcatgaaggagtttacatagaacccgaagccaggggggcgcctggatgctttga gagagtggatatactacaactactacacagagcgatctaagcggcgagaccggagac gcagatctgtttgtcacgcccgcacctggttttgcttcaggaaatatgactacgtccggcgtt ccatttggcatgacactacgaccaacacgatctcggttgtctcggcgcactccgtacagt agggatcgtctacctccttttgagacagaaacccgcgctaccatactggaggatcatccg ctgctgcccgaatgtaacactttgacaatgcacaacgtgagttacgtgcgaggtcttccct gcagtgtgggatttacgctgattcaggaatgggttgttccctgggatatggttctaacgcgg gaggagcttgtaatcctgaggaagtgtatgcacgtgtgcctgtgttgtgccaacattgatat catgacgagcatgatgatccatggttacgagtcctgggctctccactgtcattgttccagtc ccggttccctgcagtgtatagccggcgggcaggttttggccagctggtttaggatggtggt ggatggcgccatgtttaatcagaggtttatatggtaccgggaggtggtgaattacaacatg ccaaaagaggtaatgtttatgtccagcgtgtttatgaggggtcgccacttaatctacctgcg cttgtggtatgatggccacgtgggttctgtggtccccgccatgagctttggatacagcgcct tgcactgtgggattttgaacaatattgtggtgctgtgctgcagttactgtgctgatttaagtga gatcagggtgcgctgctgtgcccggaggacaaggcgccttatgctgcgggcggtgcga atcatcgctgaggagaccactgccatgttgtattcctgcaggacggagcggcggcggc agcagtttattcgcgcgctgctgcagcaccaccgccctatcctgatgcacgattatgactc tacccccatgtaggcgtggacttctccttcgccgcccgttaagcaaccgcaagttggaca gcagcctgtggctcagcagctggacagcgacatgaacttaagtgagctgcccgggga gtttattaatatcactgatgagcgtttggctcgacaggaaaccgtgtggaatataacacct aagaatatgtctgttacccatgatatgatgctttttaaggccagccggggagaaaggact |
| | | gtgtactctgtgtgttgggagggaggtggcaggttgaatactagggttctgtgagtttgatta aggtacggtgatctgtataagctatgtggtggtggggctatactactgaatgaaaaatgac ttgaaattttctgcaattgaaaaataaacacgttgaaacataacacaaacgattctttattct tgggcaatgtatgaaaaagtgtaagaggatgtggcaaatatttcattaatgtagttgtgggt ttaaacggtcaggcgcgcgcaatcgttgacgctctgtagaccgtgcaaaaggagagcc tgtaagcgggcactcttccgtggtctggtggataaattcgcaagggtatcatggcggacg accggggttcgagccccgtatccggccgtccgccgtgatccatgcggttaccgcccgcg tgtcgaacccaggtgtgcgacgtcagacaacgggggagtgctccttttggcttccttccag gcgcggcggctgctgcgctagctttttggccactggccgcgcgcagcgtaagcggttag gctggaaagcgaaagcattaagtggctcgctccctgtagccggagggttattttccaagg gttgagtcgcgggacccccggttcgagtctcggaccggccggactgcggcgaacggg ggtttgcctccccgtcatgcaagaccccgcttgcaaattcctccggaaacagggacgag cccctttttgcttttcccagatgcatccggtgctgcggcagatttaattaaaatggcgctgac gacaggtgctggcgccgggtgtggccgctggagatgacgtagttttcgcgcttaaatttga gaaagggcgcgaaactagtccttaagagtcagcgcgcagtatttactgaagagagcct ccgcgtcttccagcgtgcgccgaagctgatcttcgcttttgtgatacaggcagctgcgggt gagggatcgcagagacctgtttttattttcagctcttgttcttggcccctgctctgttgaaatat agcatacagagtgggaaaaatcctgtttctaagctcgcgggtcgatacgggttcgttggg cgccagacgcagcgctcctcctcctgctgctgccgccgctgtggatttcttgggctttgtca gagtcttgctatccggtcgcctttgcttctgtgtggccgctgctgttgctgccgctgccgctgc cgccggtgcagtatgggctgtagagatgacggtagtaatgcaggatgttacgggggaa ggccacgccgtgatggtagagaagaaagcggcgggcgaaggagatgttgcccccac agtcttgcaagcaagcaactatggcgttcttgtgcccgcgccatgagcggtagccttggc gctgttgttgctcttgggctaacggcggcggctgcttggacttaccggccctggttccagtg gtgtcccatctacggttgggtcggcgaacgggcagtgccggcggcgcctgaggagcg gaggttgtagccatgctggaaccggttgccgatttctggggcgccggcgaggggaatgc gaccgagggtgacggtgtttcgtctgacacctcttcgacctcggaagcttcctcgtctagg ctctcccagtcttccatcatgtcctcctcctcctcgtccaaaacctcctctgcctgactgtccc agtattcctcctcgtccgtgggtggcggcggcagctgcagcttctttttgggtgccatcctgg gaagcaagggcccgcggctgctgctgatagggctgcggcggcgggggggattgggttg agctcctcgccggactgggggtccaagtaaacccccccgtccctttcgtagcagaaactct tggcgggctttgttgatggcttgcaattggccaagaatgtggccctgggtaatgacgcag gcggtaagctccgcatttggcgggcgggattggtcttcgtagaacctaatctcgtgggcgt ggtagtcctcaggtacaaatttgcgaaggtaagccgacgtccacagccccggagtgag tttcaaccccggagccgcggacttttcgtcaggcgagggaccctgcagctcaaaggtac cgataatttgactttcgttaagcagctgcgaattgcaaaccagggagcggtgcggggtgc ataggttgcagcgacagtgacactccagtagaccgtcaccgctcacgtcttccattatgtc agagtggtaggcaaggtagttggctagctgcagaaggtagcagtggccccaaagcgg cggagggcattcgcggtacttaatgggcacaaagtcgctaggaagtgcacagcaggtg gcgggcaagattcctgagcgctctaggataaagttcctaaagttctgcaacatgctttgac tggtgaagtctggcagaccctgttgcagggttttaagcaggcgttcggggaaaatgatgt ccgccaggtgcgcggccacggagcgctcgttgaaggccgtccataggtccttcaagtttt gctttagcagtttctgcagctccttgaggttgcactcctccaagcactgctgccaaacgccc atggccgtctgccaggtgtagcatagaaataagtaaacgcagtcgcggacgtagtcgc gccgcgcctcgcccttgagcgtggaatgaagcacgttttgcccaaggcggttttcgtgca aaattccaaggtaggagaccaggttgcagagctccacgttggagatcttgcaggcctgg cgtacgtagccctgtcgaaaggtgtagtgcaatgtttcctctagcttgcgctgcatctccgg gtcagcaaagaaccgctgcatgcactcaagctccacggtaacgagcactgcggccat |
| | | cattagtttgcgtcgctcctccaagtcggcaggctcgcgcgtttgaagccagcgcgctag ctgctcgtcgccaactgcgggtaggccctcctctgtttgttcttgcaaatttgcatccctctcc aggggctgcgcacggcgcacgatcagctcactcatgactgtgctcatgaccttgggggg taggttaagtgccgggtaggcaaagtgggtgacctcgatgctgcgttttagtacggctag gcgcgcgttgtcaccctcgagttccaccaacactccagagtgactttcatttcgctgttttcc tgttgcagagcgtttgccgcgcgcttctcgtcgcgtccaagaccctcaaagatttttggcac ttcgttgagcgaggcgatatcaggtatgacagcgccctgccgcaaggccagctgcttgtc cgctcggctgcggttggcacggcaggataggggtatcttgcagttttggaaaaagatgtg ataggtggcaagcacctctggcacggcaaatacggggtagaagttgaggcgcgggtt gggctcgcatgtgccgttttcttggcgtttggggggtacgcgcggtgagaataggtggcgt tcgtaggcaaggctgacatccgctatggcgagggcacatcgctgcgctcttgcaacgc gtcgcagataatggcgcactggcgctgcagatgcttcaacagcacgtcgtctcccacat ctaggtagtcgccatgcctttcgtcccccgcccgacttgttcctcgtttgcctctgcgttgtc ctggtcttgctttttatcctctgttggtactgagcggtcctcgtcgtcttcgcttacaaaacctgg gtcctgctcgataatcacttcctcctcctcaagcgggggtgcctcgacggggaaggtggt aggcgcgttggcggcatcggtggaggcggtggtggcgaactcagagggggcggttag gctgtccttcttctcgactgactccatgatctttttctgcctataggagaaggaaatggccag tcgggaagaggagcagcgcgaaaccacccccgagcgcggacgcggtgcggcgcg acgtcccccaaccatggaggacgtgtcgtccccgtccccgtcgccgccgcctccccgg gcgcccccaaaaaagcggatgaggcggcgtatcgagtccgaggacgaggaagact catcacaagacgcgctggtgccgcgcacacccagcccgcggccatcgacctcggcg gcggatttggccattgcgcccaagaagaaaaagaagcgcccttctcccaagcccgag cgcccgccatcaccagaggtaatcgtggacagcgaggaagaaagagaagatgtggc gctacaaatggtgggtttcagcaacccaccggtgctaatcaagcatggcaaaggaggt aagcgcacagtgcggcggctgaatgaagacgacccagtggcgcgtggtatgcggac gcaagaggaagaggaagagcccagcgaagcggaaagtgaaattacggtgatgaac ccgctgagtgtgccgatcgtgtctgcgtgggagaagggcatggaggctgcgcgcgcgc tgatggacaagtaccacgtggataacgatctaaaggcgaacttcaaactactgcctgac caagtggaagctctggcggccgtatgcaagacctggctgaacgaggagcaccgcgg gttgcagctgaccttcaccagcaacaagacctttgtgacgatgatggggcgattcctgca ggcgtacctgcagtcgtttgcagaggtgacctacaagcatcacgagcccacgggctgc gcgttgtggctgcaccgctgcgctgagatcgaaggcgagcttaagtgtctacacggaag cattatgataaataaggagcacgtgattgaaatggatgtgacgagcgaaaacgggcag cgcgcgctgaaggagcagtctagcaaggccaagatcgtgaagaaccggtggggccg aaatgtggtgcagatctccaacaccgacgcaaggtgctgcgtgcacgacgcggcctgt ccggccaatcagttttccggcaagtcttgcggcatgttcttctctgaaggcgcaaaggctc aggtggcttttaagcagatcaaggcttttatgcaggcgctgtatcctaacgcccagaccg ggcacggtcaccttttgatgccactacggtgcgagtgcaactcaaagcctgggcacgcg cccttttgggaaggcagctaccaaagttgactccgttcgccctgagcaacgcggagga cctggacgcggatctgatctccgacaagagcgtgctggccagcgtgcaccacccggc gctgatagtgttccagtgctgcaaccctgtgtatcgcaactcgcgcgcgcagggcggag gccccaactgcgacttcaagatatcggcgcccgacctgctaaacgcgttggtgatggtg cgcagcctgtggagtgaaaacttcaccgagctgccgcggatggttgtgcctgagtttaag tggagcactaaacaccagtatcgcaacgtgtccctgccagtggcgcatagcgatgcgc ggcagaaccccttttgatttttaaacggcgcagacggcaagggtgggggtaaataatcac ccgagagtgtacaaataaaagcatttgcctttattgaaagtgtctctagtacattatttttaca tgtttttcaagtgacaaaaagaagtgggcggccgcactagttatcagcacacaattgccc attatacgc |
| 99 | XX85 plasmid DNA sequence | tctagagctagcatatggatccatcgatttagggataacagggtaattatcagcacacaa ttgcccattatacgcgcgtataatggactattgtgtgctgataggcgcgccttggattgaag ccaatatgataatgagggggtggagtttgtgacgtggcgcggggcgtgggaacggggc gggtgacgtaggttttagggcggagtaacttgtatgtgttgggaattgtagttttcttaaaatg ggaagttacgtaacgtgggaaaacggaagtgacgatttgaggaagttgtgggtttttgg ctttcgtttctgggcgtaggttcgcgtgcggttttctgggtgtttttgtggactttaaccgttacgt catttttagtcctatatatactcgctctgcacttggcccttttttacactgtgactgattgagctg gtgccgtgtcgagtggtgtttttttaataggttttctttttactggtaaggctgactgttatggctg ccgctgtggaagcgctgtatgttgttctggagcgggagggtgctatttgcctaggcagga gggtttttcaggtgtttatgtgtttttctctcctattaatttgttatacctcctatgggggctgtaat gttgtctctacgcctgcgggtatgtattcccccgggctatttcggtcgctttttagcactgacc gatgtgaatcaacctgatgtgtttaccgagtcttacattatgactccggacatgaccgagg agctgtcggtggtgctttttaatcacggtgaccagtttttttacggtcacgccggcatggccg tagtccgtcttatgcttataagggttgtttttcctgttgtaagacaggcttctaatgtttaaatgttt ttttgttattttattttgtgtttatgcagaaacccgcagacatgtttgagagaaaaatggtgtcttt ttctgtggtggttccggagcttacctgcctttatctgcatgagcatgactacgatgtgctttcttt tttgcgcgaggctttgcctgatttttgagcagcaccttgcattttatatcgccgcccatgcaa caagcttacatcggggctacgctggttagcatagctccgagtatgcgtgtcataatcagtg tgggttcttttgtcatggttcctggcggggaagtggccgcgctggtccgtgcagacctgca cgattatgttcagctggccctgcgaagggacctacgggatcgcggtattttgttaatgttcc gcttttgaatcttatacaggtctgtgaggaacctgaatttttgcaatcatgattcgctgcttga ggctgaaggtggagggcgctctggagcagatttttacaatggccggacttaatattcggg atttgcttagagatatattgagaaggtggcgagatgagaattatttgggcatggttgaaggt gctggaatgtttatagaggagattcaccctgaagggtttagcctttacgtccacttggacgt gagggccgtttgccttttggaagccattgtgcaacatcttacaaatgccattatctgttctttg gctgtagagtttgaccacgccaccggagggagcgcgttcacttaatagatcttcattttg aggttttggataatcttttggaataaaaaaaaaaacatggttcttccagctcttcccgctcct cccgtgtgtgactcgcagaacgaatgtgtaggttggctgggtgtggcttattctgcggtggt ggatgttatcagggcagcggcgcatgaaggagtttacatagaacccgaagccagggg gcgcctggatgctttgagagagtggatatactacaactactacacagagcgatctaagc ggcgagaccggagacgcagatctgtttgtcacgcccgcacctggttttgcttcaggaaat atgactacgtccggcgttccatttggcatgacactacgaccaacacgatctcggttgtctc ggcgcactccgtacagtagggatcgtctacctccttttgagacagaaacccgcgctacc atactggaggatcatccgctgctgcccgaatgtaacactttgacaatgcacaacgtgagt tacgtgcgaggtcttccctgcagtgtgggatttacgctgattcaggaatgggttgttccctgg gatatggttctaacgcgggaggagcttgtaatcctgaggaagtgtatgcacgtgtgcctgt gttgtgccaacattgatatcatgacgagcatgatgatccatggttacgagtcctgggctctc cactgtcattgttccagtcccggttccctgcagtgtatagccggcgggcaggttttggccag ctggtttaggatggtggtggatggcgccatgtttaatcagaggtttatatggtaccgggagg tggtgaattacaacatgccaaaagaggtaatgtttatgtccagcgtgtttatgaggggtcg ccacttaatctacctgcgcttgtggtatgatggccacgtgggttctgtggtccccgccatga gctttggatacagcgccttgcactgtgggattttgaacaatattgtggtgctgtgctgcagtt actgtgctgatttaagtgagatcagggtgcgctgctgtgcccggaggacaaggcgcctta tgctgcgggcggtgcgaatcatcgctgaggagaccactgccatgttgtattcctgcagga cggagcggcggcggcagcagtttattcgcgcgctgctgcagcaccaccgccctatcctg atgcacgattatgactctacccccatgtaggcgtggacttctccttcgccgcccgttaagc aaccgcaagttggacagcagcctgtggctcagcagctggacagcgacatgaacttaa gtgagctgcccggggagtttattaatatcactgatgagcgtttggctcgacaggaaaccgt |
| | | gtggaatataacacctaagaatatgtctgttacccatgatatgatgctttttaaggccagcc ggggagaaaggactgtgtactctgtgtgttgggagggaggtggcaggttgaatactagg gttctgtgagtttgattaaggtacggtgatctgtataagctatgtggtggtggggctatactac tgaatgaaaaatgacttgaaattttctgcaattgaaaaataaacacgttgaaacataaca caaacgattctttattcttgggcaatgtatgaaaaagtgtaagaggatgtggcaaatatttc attaatgtagttgtgggtttaaacggtcaggcgcgcaatcgttgacgctctgtagaccgt gcaaaaggagagcctgtaagcgggcactcttccgtggtctggtggataaattcgcaagg gtatcatggcggacgaccggggttcgagccccgtatccggccgtccgccgtgatccatg cggttaccgcccgcgtgtcgaacccaggtgtgcgacgtcagacaacgggggagtgctc cttttggcttccttccaggcgcggcggctgctgcgctagcttttttggccactggccgcgcgc agcgtaagcggttaggctggaaagcgaaagcattaagtggctcgctccctgtagccgg agggttattttccaagggttgagtcgcgggacccccggttcgagtctcggaccggccgg actgcggcgaacgggggtttgcctccccgtcatgcaagaccccgcttgcaaattcctccg gaaacagggacgagcccctttttgcttttcccagatgcatccggtgctgcggcagattta attaaaatggcgctgacgacaggtgctggcgccgggtgtggccgctggagatgacgta gttttcgcgcttaaatttgagaaagggcgcgaaactagtccttaagagtcagcgcgcagt atttactgaagagagcctccgcgtcttccagcgtgcgccgaagctgatcttcgcttttgtgat acaggcagctgcgggtgagggatcgcagagacctgtttttattttcagctcttgttcttggc ccctgctctgttgaaatatagcatacagagtgggaaaaatcctgtttctaagctcgcgggt cgatacgggttcgttgggcgccagacgcagcgctcctcctcctgctgctgccgccgctgt ggatttcttgggctttgtcagagtcttgctatccggtcgcctttgcttctgtgtggccgctgctgt tgctgccgctgccgctgccgccggtgcagtatgggctgtagagatgacggtagtaatgc aggatgttacgggggaaggccacgccgtgatggtagagaagaaagcggcgggcga aggagatgttgcccccacagtcttgcaagcaagcaactatggcgttcttgtgcccgcgcc atgagcggtagccttggcgctgttgttgctcttgggctaacggcggcggctgcttggactta ccggccctggttccagtggtgtcccatctacggttgggtcggcgaacggcagtgccgg cggcgcctgaggagcggaggttgtagccatgctggaaccggttgccgatttctggggcg ccggcgaggggaatgcgaccgagggtgacggtgtttcgtctgacacctcttcgacctcg gaagcttcctcgtctaggctctcccagtcttccatcatgtcctcctcctcctcgtccaaaacct cctctgcctgactgtcccagtattcctcctcgtccgtgggtggcggcggcagctgcagcttc tttttgggtgccatcctgggaagcaagggcccgcggctgctgctgatagggctgcggcg gcgggggggattgggttgagctcctcgccggactggggtccaagtaaacccccccgtcc ctttcgtagcagaaactcttggcgggctttgttgatggcttgcaattggccaagaatgtggc cctgggtaatgacgcaggcggtaagctccgcatttggcgggcgggattggtcttcgtaga acctaatctcgtgggcgtggtagtcctcaggtacaaatttgcgaaggtaagccgacgtcc acagccccggagtgagtttcaaccccggagccgcggacttttcgtcaggcgagggacc ctgcagctcaaaggtaccgataatttgactttcgttaagcagctgcgaattgcaaaccag ggagcggtgcggggtgcataggttgcagcgacagtgacactccagtagaccgtcacc gctcacgtcttccattatgtcagagtggtaggcaaggtagttggctagctgcagaaggta gcagtggccccaaagcggcggagggcattcgcggtacttaatgggcacaaagtcgct aggaagtgcacagcaggtggcgggcaagattcctgagcgctctaggataaagttccta aagttctgcaacatgctttgactggtgaagtctggcagaccctgttgcagggttttaagcag gcgttcggggaaaatgatgtccgccaggtgcgcggccacggagcgctcgttgaaggc cgtccataggtccttcaagttttgctttagcagtttctgcagctccttgaggttgcactcctcca agcactgctgccaaacgcccatggccgtctgccaggtgtagcatagaaataagtaaac gcagtcgcggacgtagtcgcgccgcgcctcgcccttgagcgtggaatgaagcacgtttt gcccaaggcggttttcgtgcaaaattccaaggtaggagaccaggttgcagagctccac gttggagatcttgcaggcctggcgtacgtagccctgtcgaaaggtgtagtgcaatgtttcct |
| | | ctagcttgcgctgcatctccgggtcagcaaagaaccgctgcatgcactcaagctccacg gtaacgagcactgcggccatcattagtttgcgtcgctcctccaagtcggcaggctcgcgc gtttgaagccagcgcgctagctgctcgtcgccaactgcgggtaggccctcctctgtttgttc ttgcaaatttgcatccctctccaggggctgcgcacggcgcacgatcagctcactcatgac tgtgctcatgaccttgggggtaggttaagtgccgggtaggcaaagtgggtgacctcgat gctgcgttttagtacggctaggcgcgcgttgtcaccctcgagttccaccaacactccaga gtgactttcattttcgctgttttcctgttgcagagcgtttgccgcgcttctcgtcgcgtccaa gaccctcaaagatttttggcacttcgttgagcgaggcgatatcaggtatgacagcgccct gccgcaaggccagctgcttgtccgctcggctgcggttggcacggcaggataggggtatc ttgcagttttggaaaaagatgtgataggtggcaagcacctctggcacggcaaatacggg gtagaagttgaggcgcgggttgggctcgcatgtgccgttttcttggcgtttgggggtacg cgcggtgagaataggtggcgttcgtaggcaaggctgacatccgctatggcgagggca catcgctgcgctcttgcaacgcgtcgcagataatggcgcactggcgctgcagatgcttca acagcacgtcgtctcccacatctaggtagtcgccatgcctttcgtcccccgcccgacttg ttcctcgtttgcctctgcgttgtcctggtcttgctttttatcctctgttggtactgagcggtcctcgt cgtcttcgcttacaaaacctgggtcctgctcgataatcacttcctcctcctcaagcgggggt gcctcgacggggaaggtggtaggcgcgttggcggcatcggtggaggcggtggtggcg aactcagagggggcggttaggctgtccttcttctcgactgactccatgatctttttctgcctat aggagaaggaaatggccagtcgggaagaggagcagcgcgaaaccacccccgagc gcggacgcggtgcggcgcgacgtcccccaaccatggaggacgtgtcgtccccgtccc cgtcgccgccgcctccccgggcgcccccaaaaaagcggatgaggcggcgtatcgagt ccgaggacgaggaagactcatcacaagacgcgctggtgccgcgcacacccagccc gcggccatcgacctcggcggcggatttggccattgcgcccaagaagaaaaagaagc gcccttctcccaagcccgagcgcccgccatcaccagaggtaatcgtggacagcgagg aagaaagagaagatgtggcgctacaaatggtgggtttcagcaacccaccggtgctaat caagcatggcaaaggaggtaagcgcacagtgcggcggctgaatgaagacgaccca gtggcgcgtggtatgcggacgcaagaggaagaggaagagcccagcgaagcggaa agtgaaattacggtgatgaacccgctgagtgtgccgatcgtgtctgcgtgggagaaggg catggaggctgcgcgcgcgctgatggacaagtaccacgtggataacgatctaaaggc gaacttcaaactactgcctgaccaagtggaagctctggcggccgtatgcaagacctggc tgaacgaggagcaccgcgggttgcagctgaccttcaccagcaacaagacctttgtgac gatgatggggcgattcctgcaggcgtacctgcagtcgtttgcagaggtgacctacaagc atcacgagcccacgggctgcgcgttgtggctgcaccgctgcgctgagatcgaaggcga gcttaagtgtctacacggaagcattatgataaataaggagcacgtgattgaaatggatgt gacgagcgaaaacgggcagcgcgcgctgaaggagcagtctagcaaggccaagatc gtgaagaaccggtggggccgaaatgtggtgcagatctccaacaccgacgcaaggtgc tgcgtgcacgacgcggcctgtccggccaatcagttttccggcaagtcttgcggcatgttctt ctctgaaggcgcaaaggctcaggtggcttttaagcagatcaaggcttttatgcaggcgct gtatcctaacgcccagaccgggcacggtcaccttttgatgccactacggtgcgagtgca actcaaagcctgggcacgcgcccttttgggaaggcagctaccaaagttgactccgttcg ccctgagcaacgcggaggacctggacgcggatctgatctccgacaagagcgtgctgg ccagcgtgcaccacccggcgctgatagtgttccagtgctgcaaccctgtgtatcgcaact cgcgcgcgcagggcggaggccccaactgcgacttcaagatatcggcgcccgacctgc taaacgcgttggtgatggtgcgcagcctgtggagtgaaaacttcaccgagctgccgcgg atggttgtgcctgagtttaagtggagcactaaacaccagtatcgcaacgtgtccctgcca gtggcgcatagcgatgcgcggcagaacccctttgatttttaaacggcgcagacggcaa gggtgggggtaaataatcacccgagagtgtacaaataaaagcatttgcctttattgaaag tgtctctagtacattatttttacatgtttttcaagtgacaaaaagaagtgggcggccgcacta |
| | | gttatcagcacacaattgcccattatacgcgtataatggactattgtgtgctgatatagg gataacagggtaattctagagctagcatatggatccatcgattttcggggaaatgtgcgc ggaacccctatttgtttatttttctaaatacattcaaatatgtatccgctcatgagacaataac cctgataaatgcttcaataatattgaaaaaggaagagtatgagccatattcaacgggaa acgtcgaggccgcgattaaattccaacatggatgctgatttatatgggtataaatgggctc gcgataatgtcgggcaatcaggtgcgacaatctatcgcttgtatgggaagcccgatgcg ccagagttgtttctgaaacatggcaaaggtagcgttgccaatgatgttacagatgagatg gtcagactaaactggctgacggaatttatgcctcttccgaccatcaagcattttatccgtact cctgatgatgcatggttactcaccactgcgatccccggaaaaacagcattccaggtatta gaagaatatcctgattcaggtgaaaatattgttgatgcgctggcagtgttcctgcgccggtt gcattcgattcctgtttgtaattgtccttttaacagcgatcgcgtatttcgtctcgctcaggcgc aatcacgaatgaataacggtttggttgatgcgagtgattttgatgacgagcgtaatggctg gcctgttgaacaagtctggaaagaaatgcataaacttttgccattctcaccggattcagtc gtcactcatggtgatttctcacttgataaccttatttttgacgaggggaaattaataggttgtat tgatgttggacgagtcggaatcgcagaccgataccaggatcttgccatcctatggaactg cctcggtgagttttctccttcattacagaaacggctttttcaaaaatatggtattgataatcctg atatgaataaattgcagtttcatttgatgctcgatgagtttttctaagcgtataatggtctagag ctagcatatggatccatcgattccattatacgcctgtcagaccaagtttactcatatatacttt agattgatttaaaacttcatttttaatttaaaaggatctaggtgaagatcctttttgataatctca tgaccaaaatcccttaacgtgagttttcgttccactgagcgtcagaccccgtagaaaaga tcaaaggatcttcttgagatcctttttttctgcgcgtaatctgctgcttgcaaacaaaaaaac caccgctaccagcggtggtttgtttgccggatcaagagctaccaactctttttccgaaggta actggcttcagcagagcgcagataccaaatactgttcttctagtgtagccgtagttaggcc accacttcaagaactctgtagcaccgcctacatacctcgctctgctaatcctgttaccagtg gctgctgccagtggcgataagtcgtgtcttaccgggttggactcaagacgatagttaccg gataaggcgcagcggtcgggctgaacggggggttcgtgcacacagcccagcttggag cgaacgacctacaccgaactgagatacctacagcgtgagctatgagaaagcgccacg cttcccgaagggagaaaggcggacaggtatccggtaagcggcagggtcggaacagg agagcgcacgagggagcttccagggggaaacgcctggtatctttatagtcctgtcgggtt tcgccacctctgacttgagcgtcgatttttgtgatgctcgtcaggggggcggagcctatgg aaaaacgccagcaacgcggcctttttacggttcctggccttttgctggccttttgctcacatg t |
| 100 | E4 mini promoter | gggaaaacggaagtgacgatttgaggaagttgtgggtttttttggctttcgtttctgggcgta ggttcgcgtgcggttttctgggtgtttttttgtggactttaaccgttacgtcatttttttagtcctatat atactcgctctgcacttggcccttttttacactgtgactgattgagctggtgccgtgtcgagtg gtgtttttttaataggttttctttttttactggtaaggctgactgt |
| 101 | UXP exon promoter | gaagataccttcaaccccgtgtatccatatgacacggaaaccggtcctccaactgtgcct tttcttactcctccctttgtatcccccaatgggtttcaagagagtccccctggggtactctcttt gcgcctatccgaacctctagttacctccaatggcatgcttgcgctcaaaatgggcaacgg cctctctctggacgaggccggcaaccttacctcccaaaatgtaaccactgtgagcccac ctctcaaaaaaaccaagtcaaacataaacctggaaatatctgcacccctcacagttacc tcagaagccctaactgtggctgccgccgcacctctaatggtcgcgggcaacacactcac catgcaatcacaggccccgctaaccgtgc |
| 102 | UXP exon 1 | ttaaaatcagttagcaaatttctgtccagtttattcagcagcacctccttgccctcctcccag ctctggtattgcagcttcctcctggctgcaaactttctccacaatctaaatggaatgtcagttt cctcctgttcctgtccatccgcacccactatcttcat |
| 103 | L22K nt sequence | atggcacccaaaaagaagctgcagctgccgccgccacccacggacgaggaggaat actgggacagtcaggcagaggaggttttggacgaggaggaggaggacatgatggaa |
| | | gactgggagagcctagacgaggaagcttccgaggtcgaagaggtgtcagacgaaac accgtcaccctcggtcgcattcccctcgccggcgccccagaaatcggcaaccggttcca gcatggctacaacctccgctcctcaggcgccgccggcactgcccgttcgccgacccaa ccgtagatgggacaccactggaaccagggccggtaagtccaagcagccgccgccgtt agcccaagagcaacaacagcgccaaggctaccgctcatggcgcgggcacaagaac gccatagttgcttgcttgcaagactgtgggggcaacatctccttcgcccgccgctttcttctc taccatcacggcgtggccttcccccgtaacatcctgcattactaccgtcatctctacagcc catactgcaccggcggcagcggcagcggcagcaacagcagcggccacacagaag caaaggcgaccggatag |
| 104 | L33K nt sequence | Atggcacccaaaaagaagctgcagctgccgccacccacggacgaggaggaat actgggacagtcaggcagaggaggtttttggacgaggaggaggaggacatgatggaa gactgggagagcctagacgaggaagcttccgaggtcgaagaggtgtcagacgaaac accgtcaccctcggtcgcattcccctcgccggcgccccagaaatcggcaaccggttcca gcatggctacaacctccgctcctcaggcgccgccggcactgcccgttcgccgacccaa ccgtagatgggacaccactggaaccagggccggtaagtccaagcagccgccgccgtt agcccaagagcaacaacagcgccaaggctaccgctcatggcgcgggcacaagaac gccatagttgcttgcttgcaagactgtgggggcaacatctccttcgcccgccgctttcttctc taccatcacggcgtggccttcccccgtaacatcctgcattactaccgtcatctctacagcc catactgcaccggcggcagcggcagcggcagcaacagcagcggccacacagaag caaaggcgaccggatagcaagactctgacaaagcccaagaaatccacagcggcgg cagcagcaggaggaggagcgctgcgtctggcgcccaacgaacccgtatcgacccgc gagcttagaaacaggatttttcccactctgtatgctatatttcaacagagcaggggccaag aacaagagctgaaaataaaaaacaggtctctgcgatccctcacccgcagctgcctgtat cacaaaagcgaagatcagcttcggcgcacgctggaagacgcggaggctctcttcagt aaatactgcgcgctgactcttaaggactag |
| 105 | 100k nt sequence | atggagtcagtcgagaagaaggacagcctaaccgccccctctgagttcgccaccacc gcctccaccgatgccgccaacgcgcctaccaccttccccgtcgaggcacccccgcttg aggaggaggaagtgattatcgagcaggacccaggttttgtaagcgaagacgacgagg accgctcagtaccaacagaggataaaaagcaagaccaggacaacgcagaggcaa acgaggaacaagtcgggcgggggacgaaaggcatggcgactacctagatgtggg agacgacgtgctgttgaagcatctgcagcgccagtgcgccattatctgcgacgcgttgca agagcgcagcgatgtgcccctcgccatagcggatgtcagccttgcctacgaacgccac ctattctcaccgcgcgtacccccaaacgccaagaaaacggcacatgcgagcccaac ccgcgcctcaacttctaccccgtatttgccgtgccagaggtgcttgccacctatcacatcttt ttccaaaactgcaagataccccctatcctgccgtgccaaccgcagccgagcggacaagc agctggccttgcggcagggcgctgtcatacctgatatcgcctcgctcaacgaagtgcca aaaatctttgagggtcttggacgcgacgagaagcgcgcggcaaacgctctgcaacag gaaaacagcgaaaatgaaagtcactctggagtgttggtggaactcgagggtgacaac gcgcgcctagccgtactaaaacgcagcatcgaggtcacccactttgcctacccggcact taacctacccccccaaggtcatgagcacagtcatgagtgagctgatcgtgcgccgtgcgc agcccctggagagggatgcaaatttgcaagaacaaacagaggagggcctacccgca gttggcgacgagcagctagcgcgctggcttcaaacgcgcgagcctgccgacttggagg agcgacgcaaactaatgatggccgcagtgctcgttaccgtggagcttgagtgcatgcag cggttctttgctgacccggagatgcagcgcaagctagaggaaacattgcactacaccttt cgacagggctacgtacgccaggcctgcaagatctccaacgtggagctctgcaacctgg tctcctaccttggaattttgcacgaaaaccgccttgggcaaaacgtgcttcattccacgctc aagggcgaggcgcgccgcgactacgtccgcgactgcgtttacttatttctatgctacacct ggcagacggccatgggcgtttggcagcagtgcttggaggagtgcaacctcaaggagct |
| | | gcagaaactgctaaagcaaaacttgaaggacctatggacggccttcaacgagcgctc cgtggccgcgcacctggcggacatcattttccccgaacgcctgcttaaaccctgcaac agggtctgccagacttcaccagtcaaagcatgttgcagaactttaggaactttatcctaga gcgctcaggaatcttgcccgccacctgctgtgcacttcctagcgactttgtgcccattaagt accgcgaatgccctccgccgctttggggccactgctaccttctgcagctagccaactacc ttgcctaccactctgacataatggaagacgtgagcggtgacggtctactggagtgtcact gtcgctgcaacctatgcaccccgcaccgctccctggtttgcaattcgcagctgcttaacga aagtcaaattatcggtacctttgagctgcagggtccctcgcctgacgaaaagtccgcggc tccggggttgaaactcactccggggctgtggacgtcggcttaccttcgcaaatttgtacctg aggactaccacgcccacgagattaggttctacgaagaccaatcccgcccgccaaatg cggagcttaccgcctgcgtcattacccagggccacattcttggccaattgcaagccatca acaaagcccgccaagagtttctgctacgaaagggacgggggtttacttggacccccca gtccggcgaggagctcaacccaatcccccgccgccgcagccctatcagcagcagcc gcgggcccttgcttcccaggatggcacccaaaaagaagctgcagctgccgccgccac ccacggacgaggaggaatactgggacagtcaggcagaggaggttttggacgaggag gaggaggacatgatggaagactgggagagcctagacgaggaagcttccgaggtcga agaggtgtcagacgaaacaccgtcaccctcggtcgcattcccctcgccggcgccccag aaatcggcaaccggttccagcatggctacaacctccgctcctcaggcgccgccggcac tgcccgttcgccgacccaaccgtag |
| 106 | pVIII nt sequence | atgagcaaggaaattcccacgccctacatgtggagttaccagccacaaatgggacttg cggctggagctgcccaagactactcaacccgaataaactacatgagcgcggacccc acatgatatcccgggtcaacggaatccgcgcccaccgaaaccgaattctcttggaaca ggcggctattaccaccacacctcgtaataaccttaatccccgtagttggcccgctgccctg gtgtaccaggaaagtcccgctcccaccactgtggtacttcccagagacgcccaggccg aagttcagatgactaactcaggggcgcagcttgcgggcggctttcgtcacagggtgcgg tcgcccgggcagggtataactcacctgacaatcagagggcgaggtattcagctcaacg acgagtcggtgagctcctcgcttggtctccgtccggacgggacatttcagatcggcggcg ccggccgtccttcattcacgcctcgtcaggcaatcctaactctgcagacctcgtcctctga gccgcgctctggaggcattggaactctgcaatttattgaggagtttgtgccatcggtctactt taaccccttctcgggacctcccggccactatccggatcaatttattcctaactttgacgcggt aaaggactcggcggacggctacgactga |
| 107 | Fiber nt sequence | atgaagcgcgcaagaccgtctgaagataccttcaaccccgtgtatccatatgacacgga aaccggtcctccaactgtgccttttcttactcctccctttgtatcccccaatgggtttcaagag agtcccccctggggtactctctttgcgcctatccgaacctctagttacctccaatggcatgctt gcgctcaaaatgggcaacggcctctctctggacgaggccggcaaccttacctcccaaa atgtaaccactgtgagcccacctctcaaaaaaaccaagtcaaacataaacctggaaat atctgcacccctcacagttacctcagaagccctaactgtggctgccgccgcacctctaat ggtcgcgggcaacacactcaccatgcaatcacaggccccgctaaccgtgcacgactc caaacttagcattgccacccaaggacccctcacagtgtcagaaggaaagctagccctg caaacatcaggcccccctcaccaccaccgatagcagtacccttactatcactgcctcacc ccctctaactactgccactggtagcttgggcattgacttgaaagagcccatttatacacaa aatggaaaactaggactaaagtacggggctcctttgcatgtaacagacgacctaaaca ctttgaccgtagcaactggtccaggtgtgactattaataatacttccttgcaaactaaagtta ctggagccttgggttttgattcacaaggcaatatgcaacttaatgtagcaggaggactaa ggattgattctcaaaacagacgccttatacttgatgttagttatccgtttgatgctcaaaacc aactaaatctaagactaggacagggccctcttttataaactcagcccacaacttggatat taactacaacaaaggcctttacttgtttacagcttcaaacaattccaaaaagcttgaggtta acctaagcactgccaaggggttgatgtttgacgctacagccatagccattaatgcagga |
| | | gatgggcttgaatttggttcacctaatgcaccaaacacaaatcccctcaaaacaaaaatt ggccatggcctagaatttgattcaaacaaggctatggttcctaaactaggaactggcctta gttttgacagcacaggtgccattacagtaggaaacaaaaataatgataagctaactttgt ggaccacaccagctccatctcctaactgtagactaaatgcagagaaagatgctaaactc actttggtcttaacaaaatgtggcagtcaaatacttgctacagtttcagttttggctgttaaag gcagtttggctccaatatctggaacagttcaaagtgctcatcttattataagatttgacgaa aatggagtgctactaaacaattccttcctggacccagaatattggaactttagaaatgga gatcttactgaaggcacagcctatacaaacgctgttggatttatgcctaacctatcagctta tccaaaatctcacggtaaaactgccaaaagtaacattgtcagtcaagtttacttaaacgg agacaaaactaaacctgtaacactaaccattacactaaacggtacacaggaaacagg agacacaactccaagtgcatactctatgtcattttcatgggactggtctggccacaactac attaatgaaatatttgccacatcctcttacacttttcatacattgcccaagaataa |
| | | |
| | | |
| ***In SEQ ID NO: 1-6, bold is poly A site, bold and underlined is VA RNA region, *bold in italics is E2 region, bold in italics and underlined is E4 region, and* bold and double underlined is ITR** | | |

### Definitions

For convenience, certain terms employed herein, in the specification, examples and appended claims are collected herein. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Further, unless otherwise required by context, singular terms shall include pluralities and plural terms shall include the singular.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein. Definitions of common terms in immunology and molecular biology can be found in The Merck Manual of Diagnosis and Therapy, 20th Edition, published by Merck Sharp & Dohme Corp., 2018 (ISBN 0911910190, 978-0911910421); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), W. W. Norton & Company, 2016 (ISBN 0815345054, 978-0815345053); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737), the contents of which are all incorporated by reference herein in their entireties.

Further, the practice of the present invention can employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988); "Phage Display: A Laboratory Manual" (Barbas et al., 2001).

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Other than in the operating examples, or where otherwise indicated, all numbers expressing quantities of ingredients or reaction conditions used herein should be understood as modified in all instances by the term "about." The term "about" when used in connection with percentages can mean ±1%. In some embodiments of the various aspects described herein, the term "about" when used in connection with percentages can mean ±5%. The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

As used herein the term "comprising" or "comprises" is used in reference to compositions, methods, and respective component(s) thereof, that are essential to the invention, yet open to the inclusion of unspecified elements, whether essential or not.

The term "consisting of" refers to compositions, methods, and respective components thereof as described herein, which are exclusive of any element not recited in that description of the embodiment.

As used herein the term "consisting essentially of" refers to those elements required for a given embodiment. The term permits the presence of additional elements that do not materially affect the basic and novel or functional characteristic(s) of that embodiment of the invention.

The singular terms "a," "an," and "the" include plural referents unless context clearly indicates otherwise. Similarly, the word "or" is intended to include "and" unless the context clearly indicates otherwise. It is further noted that the claims can be drafted to exclude any optional element. As such, this statement is intended to serve as antecedent basis for use of such exclusive terminology as "solely," "only" and the like in connection with the recitation of claim elements, or use of a "negative" limitation.

The abbreviation, "e.g." is derived from the Latin exempli gratia, and is used herein to indicate a non-limiting example. Thus, the abbreviation "e.g." is synonymous with the term "for example."

As used herein, the terms "identity", "identical" and the like refer to the sequence similarity between two polymeric molecules, e.g., between two nucleic acid molecules or between two polypeptide molecules. Sequence alignments and determination of sequence identity can be done, e.g., using the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250). Methods for aligning sequences for comparison are well-known in the art. Various programs and alignment algorithms are described in, for example: Smith and Waterman (1981) Adv. Appl. Math. 2:482; Needleman and Wunsch (1970) J. Mol. Biol. 48:443; Pearson and Lipman (1988) Proc. Natl. Acad. Sci. U.S.A. 85:2444; Higgins and Sharp (1988) Gene 73:237-44; Higgins and Sharp (1989) CABIOS 5:151-3; Corpet et al. (1988) Nucleic Acids Res. 16:10881-90; Huang et al. (1992) Comp. Appl. Biosci. 8:155-65; Pearson et al. (1994) Methods Mol. Biol. 24:307-31; Tatiana et al. (1999) FEMS Microbiol. Lett. 174:247-50. A detailed consideration of sequence alignment methods and homology calculations can be found in, e.g., Altschul et al. (1990) J. Mol. Biol. 215:403-10. The National Center for Biotechnology Information (NCBI) Basic Local Alignment Search Tool (BLAST^{™}; Altschul et al. (1990)) is available from several sources, including the National Center for Biotechnology Information (Bethesda, MD), and on the internet, for use in connection with several sequence analysis programs. A description of how to determine sequence identity using this program is available on the internet under the "help" section for BLAST^{™}. For comparisons of nucleic acid sequences, the "Blast 2 sequences" function of the BLAST^{™} (Blastn) program may be employed using the default parameters. Nucleic acid sequences with even greater similarity to the reference sequences will show increasing percentage identity when assessed by this method. Typically, the percentage sequence identity is calculated over the entire length of the sequence. For example, a global optimal alignment is suitably found by the Needleman-Wunsch algorithm with the following scoring parameters: Match score: +2, Mismatch score: -3; Gap penalties: gap open 5, gap extension 2. The percentage identity of the resulting optimal global alignment is suitably calculated by the ratio of the number of aligned bases to the total length of the alignment, where the alignment length includes both matches and mismatches, multiplied by 100. Preferably sequence identity is assessed over the length of the shorter of the two sequences being compared.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions may include: 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

As used herein, the term "substantially complementary", with respect to a nucleotide sequence in relation to a reference nucleotide sequence means a nucleotide sequence having a percentage of identity between the substantially complementary nucleotide sequence and the exact complementary sequence of said reference of at least at least 80%. e.g., at least 85%, at least 90%, at least 93%, at least 95%, at least 96%, at least 97%, at least 98%, at least 99% or 100% (i.e., exactly complementary). Preferably complementarity is assessed over the length of the shorter of the two sequences being compared.

A "functional equivalent" or a "functional homologue" of a given nucleotide sequence includes similar regions derived from a heterologous adenovirus serotype, nucleotide regions derived from another virus or from a cellular source, and recombinantly produced or chemically synthesized polynucleotides which function in a manner similar to the reference nucleotide region to achieve a desired result. For example, a functional homologue of an adenoviral VA RNA gene region or an adenoviral E2A gene region encompasses derivatives and analogues of such gene regions-including derivatives and analogues with any single or multiple nucleotide base additions, substitutions and/or deletions occurring within the regions, so long as the homologue retains the ability to provide its inherent accessory function to support AAV virion production at levels detectable above background.

A "functional equivalent" or a "functional homologue" of a given polypeptide or amino acid sequence includes molecules derived from the native polypeptide sequence, as well as recombinantly produced or chemically synthesized polypeptides which function in a manner similar to the reference polypeptide to achieve a desired result. For example, a functional equivalent of DBP encompasses derivatives and analogues of those polypeptides, including derivatives and analogues with any single or multiple amino acid additions, substitutions and/or deletions occurring internally or at the amino or carboxy termini thereof, so long as DBP activity remains.

As used herein, the phrase "transgene" refers to an exogenous nucleic acid sequence. In one example, a transgene is a gene encoding an industrially or pharmaceutically useful compound, or a gene encoding a desirable trait. In yet another example, the transgene encodes an antisense nucleic acid sequence, wherein expression of the antisense nucleic acid sequence inhibits expression of a target nucleic acid sequence.

The term "operably linked", "operably connected" or equivalent expressions as used herein refer to the arrangement of various nucleic acid elements relative to each such that the elements are functionally connected and are able to interact with each other in the manner intended. Such elements may include, without limitation, a promoter, an enhancer and/or a regulatory element, a polyadenylation sequence, one or more introns and/or exons, and a coding sequence of a gene of interest to be expressed. The nucleic acid sequence elements, when properly oriented or operably linked, act together to modulate the activity of one another, and ultimately may affect the level of expression of an expression product. By modulate is meant increasing, decreasing, or maintaining the level of activity of a particular element. The position of each element relative to other elements may be expressed in terms of the 5' terminus and the 3' terminus of each element, and the distance between any particular elements may be referenced by the number of intervening nucleotides, or base pairs, between the elements. As understood by the skilled person, operably linked implies functional activity, and is not necessarily related to a natural positional link. Indeed, when used in a vector, cis-regulatory elements will typically be located immediately upstream of the promoter (although this is generally the case, it should definitely not be interpreted as a limitation or exclusion of positions within the vector, but this needs not be the case *in vivo,* e.g., a regulatory element sequence naturally occurring downstream of a gene whose transcription it affects is able to function in the same way when located upstream of the promoter. Hence, in some embodiments, the regulatory or enhancing effect of the regulatory element is position- independent.

It should be understood that this disclosure is not limited to the particular methodology, protocols, and reagents, etc., provided herein and as such may vary. The terminology used herein is for the purpose of describing particular embodiments only, and is not intended to limit the scope of the present disclosure, which is defined solely by the claims. The invention is further illustrated by the following example, which should not be construed as further limiting.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

The technology described herein is further illustrated by the following examples which in no way should be construed as being further limiting.

### EXAMPLES

### Example 1: Helper adenovirus plasmids HAD1-6

Adeno-associated virus is a dependovirus, that naturally needs co-infection with a helper virus, such as adenovirus or herpes virus, to efficiently complete its life cycle. However, only a small portion of the helper virus genes are needed to achieve efficient AAV genome replication and encapsidation (see Meier et al., Viruses (2020), 12(6):662 for review).

Recombinant AAV vectors used for gene transfer application are made of a DNA sequence of interest (the transgene) flanked by AAV inverted terminal repeats (ITR), which are packaged into an AAV capsid made of viral structural proteins. The currently most used method for rAAV production consists in transfection of HEK293 cells with three plasmids, the first one containing the transgene flanked by AAV ITR, the second one encoding the AAV Rep and capsid proteins, and the third one encoding the adenovirus type 5 (Ad5) VA RNA (virus-associated small non-coding RNA), E2A (single-stranded DNA binding protein), and E4 helper functions.

Adenovirus helper plasmids available up to now are the following (not necessarily exhaustive list):
- pXX6-80, which was created through successive deletions of the Ad5 genome, to maintain expression of the small virus-associated RNA (VA RNA I and VA RNA II), the single-stranded DNA binding protein (E2A or DBP) and the E4 proteins, while avoiding expression of the adenovirus capsid structural proteins (Xiao et al. J Virol 1998). Thus, the plasmid keeps the relative orientation of the different sequence elements from the Ad5 viral genome. It contains the following nucleotides positions of Ad5 (RefSeq # AC_000008): 9847-13258, 21444-28119, and 30819-35919. One drawback of this plasmid is its large size (almost 19 kb), which leads to significant cost for production and quality control. In addition, this plasmid still contains still contains the full-length coding sequence of some structural proteins, in particular the adenovirus fiber which could lead to toxicity and immune response if expressed in treated patients.
- pAdDeltaF6, , briefly described in patent application WO2017180861A1. The plasmid is slightly smaller than pXX6-80 (15.4 kb), but it contains the fiber coding sequence as well. It was also obtained through successive deletion of the Ad5 genome and thus keeps the original relative orientation of the different sequence elements. It contains the following nucleotides positions of Ad5 (RefSeq # AC_000008): 9846-10947, 21867-28137, and 30819-35937.
- pHelper, Genbank # AF369965, distributed by Agilent Technologies, that has a significant smaller size (11.6kb), and it doesn't contain the full-length fiber coding sequence. However, internal experimental results have shown that this plasmid was less efficient than pXX6-80, leading to lower AAV yields.. Another pHelper plasmid of 11.6 kbderived from plasmid pladeno5 is also known in the art. (see PCT patent publication WO1997017458A1). In contrast to the two above plasmids, the relative orientation of the Ad5 sequence elements has been changed in this plasmid. It contains the following nucleotides positions of Ad5 (RefSeq # AC_000008): 22188-27527, 32799-35835, and 10436-11166.
- pXX85 **(SEQ ID NO: 99),** described in PCT publication no. WO2023250416, content of which is incorporated herein by reference in its entirety, has a smaller size (10.6 kb), and it doesn't contain the fiber sequence. The relative orientation of the Ad5 sequence elements has been also changed in this plasmid compared to the viral genome. It contains the following nucleotides positions of Ad5 (RefSeq # AC_000008): 32721-35915, 10562-11072, and 22316-27173.
- pDG helper plasmid and derivatives, , is described in patent application EP1412510B1. These plasmids are larger than the pXX6-80 plasmid (all around 22 kb), since in addition to regions of the Ad5 required for AAV production, they also contain the AAV Rep and Cap genes, thus allowing AAV vector production using 2 plasmids instead of 3. These plasmids also contain some Ad5 late gene sequences including the full-length fiber coding sequence.

In the current invention, a series of adenovirus helper plasmids of varying size and sequence content have been developed, according to the following strategy:
1. A DNA sequence of 14,440 nucleotides was designed to include the following features, in the 5' to 3' direction:
   - Three unique (BamHl, Pmel and Pacl) restriction sites offering the possibility to clone other sequence elements upstream the adenovirus-derived sequence.
   - A synthetic polyadenylation signal
   - A unique Sall restriction site
   - Nucleotides 10409-11134 of Ad5 (RefSeq # AC_000008), that include the VA RNA helper genes, and 5' end of protein 52K CDS with mutation of the start codon.
   - A unique Nsil restriction site
   - Nucleotides 22316-35938 of Ad5 (RefSeq # AC_000008), that include the whole right end of the Ad5 genome. This region contains the well characterized AAV helper genes, early E2A and E4 genes, as well as other genes not known to be involved in AAV life cycle, encoded by early region E3, and late regions L4 and L5. The purpose was to evaluate if some adenovirus non-structural proteins not strictly required for AAV production could have helper functions, such as (i) the U exon protein (UXP), for which promoter and coding sequence are present in pXX6-80 but part of the pre-mRNA is missing, and (ii) the E3-10.5K protein, or adenovirus death protein (ADP). Indeed, the UXP interacts with E2A within the adenovirus replication centers and may thus enhance E2A helper activity (Tollefson et al. J Virol 2007) and ADP is expressed at late stages of adenovirus infection to induce cell death and virus release (Tollefson et al. Virology 1996), which may improve rAAV vector recovery from the Pro10 cells. In addition, the sequence also includes the Ad5 right ITR downstream E4, since it contains some transcription factors binding sites and might be involved in E4 genes regulation.
   - Two unique Swal and Nrul restriction sites

Since AAV preparations can be contaminated by residual helper plasmid DNA, the presence of late adenovirus proteins coding sequences, in particular the fiber structural protein, has raised safety concerns for the patients. To limit the risk linked to expression of these proteins, mutations have been introduced in the coding sequences of all of them, namely 100K, 22K/33K, pVIII, and fiber. These mutations are detailed in the **Table 4.**

| **Table 4: Mutations introduced into the initial Ad5-derived sequence included in a synthetic 14,400 bp DNA fragment.** | | |
|---|---|---|
| CDS | mutation | outcome |
| 100K | G₃G₄ deletion | Removal of start codon and shift of the reading frame |
| 22K/33K | G₃G₄ deletion | Removal of start codon and shift of the reading frame |
| pVIII | G₃ deletion | Removal of start codon and shift of the reading frame |
| | A₁₄₉C₁₅₀ > TT | Introduction of a BstBl restriction site |
| Fiber | G₃ deletion | Removal of start codon and shift of the reading frame |
| | C₅₀₃ >T | Introduction of a Clal restriction site |
| | T₁₆₉₂A₁₆₉₃ > CG | Introduction of a Clal restriction site |

2. This 14.4 kb fragment was generated through gene synthesis and cloned into pUC57-Brick at GenScript, resulting in plasmid pUC57-Brick_HAD1.
3. For construction of pHAD1 **(SEQ ID NO: 1),** the 14.4 kb fragment was excised from pUC57-Brick_HAD1 through BamHI-Nrul digestion, and cloned into an in-house pUC19-Kan backbone digested with BamHI and EcoRV. The final plasmid sequence is 16.2 kb.
4. For construction of pHAD2 **(SEQ ID NO:** 2), pHAD1 was digested with Clal and re-ligated. This resulted in deletion of 1,188 bp from the remaining 1,745 bp of the fiber CDS. The final plasmid sequence is 15.0 kb.
5. For construction of pHAD3 **(SEQ ID NO:** 3), pHAD1 was digested with EcoRI and re-ligated. This resulted in deletion of 2,718 bp from the 3,474 bp E3 transcription unit, 3'end of pVIII CDS, and part of U exon protein intron 1. The final plasmid sequence is 13.5 kb.
6. For construction of pHAD4 **(SEQ ID NO: 4),** pHAD2 was digested with EcoRI and re-ligated. This resulted in combined fiber sequence deletion of pHAD2 and E3 region deletion of pHAD3. The final plasmid sequence is 12.3 kb.
7. For construction of pHAD5 **(SEQ ID NO: 5),** pHAD4 was digested with BstBl and Clal then re-ligated. This resulted in complete removal of the remaining fiber and E3 region sequences, as well as deletion of U exon protein promoter and exon 1. The final plasmid sequence is 10.8 kb.
8. Plasmid pHAD6 was generated as followed: a Apal-Spel fragment was excised from plasmid pLDB_XX85 **(SEQ ID NO: 99),** described in PCT publication no. WO2023250416, content of which is incorporated herein by reference in its entirety, which contains the 22K/33K coding sequence, and was cloned between Apal and Spel into pHAD5 to replace the mutated 22K/33K sequence. 22K/33K is described in PCT publication no. WO2023250416; Su et al, scientific reports (2023), 13:21670; Adsero et al, Human Gene therapy (2024), 35(1-2): 59. Thus, pHAD6 **(SEQ ID NO: 6)** is identical to pHAD5 except the 2-base pair deletion (start codon and frame-shift mutation) was corrected. The final plasmid sequence is 10.8 kb.

The plasmids pHAD5 and pAHD6 contain only the functional coding sequences for E2A and E4 transcription units, with a full length 22K/33K coding sequence (pHAD6) or without a full length 22K/33K coding sequence (pHAD5), and the VA RNA transcription unit. All the sequences encoding adenovirus structural proteins are deleted, and the only remaining full length sequence without an AAV helper function, 100K, has a knock-out mutation **(Table 4).**

***Plasmids:*** A 14.4 kb fragment containing a synthetic polyadenylation signal, nucleotides positions 10409-11134 and nucleotides positions 22316-35938 of human adenovirus type 5 (RefSeq #AC_000008), was generated through gene synthesis and cloned into pUC57-Brick at GenScript, resulting in plasmid pUC57-Brick_HAD1. Then, all different plasmids were obtained through standard molecular biology technics and grown into E. coli Stbl3^{™} competent cells (Thermo Fisher Scientific). For construction of pHAD1, the 14.4 kb fragment was excised from pUC57-Brick_HAD1 through BamHI-Nrul digestion and cloned into an in-house pUC19-Kan backbone digested with BamHI and EcoRV. For construction of pHAD2, pHAD1 was digested with Clal to delete a 1,188 bp fragment and re-ligated. For construction of pHAD3 and pHAD4, pHAD1 and pHAD2, respectively, were digested with EcoRI to delete a 2,718 bp fragment and re-ligated. For construction of pHAD4, pHAD2 was digested with EcoRI to delete a 2,718 bp fragment and re-ligated. For construction of pHAD5, pHAD4 was digested with BstBl and Clal to deleted a 1,502 bp fragment and re-ligated. For construction of pHAD6, a Spel-Apal 909 bp fragment was excised from pLDB_XX85 plasmid, described in PCT publication no. WO2023250416, and cloned between Apal and Spel restriction sites in pHAD5.

***Small-scale AAVs production and purification:*** Small-scale rAAV production was achieved through replicates polyethyleneimine-mediated transfection using three plasmids (Grieger et al., "Production of Recombinant Adeno-associated Virus Vectors Using Suspension HEK293 Cells and Continuous Harvest of Vector From the Culture Media for GMP FIX and FLT1 Clinical Vector." Mol Ther. 2016 Feb; 24(2): 287-297, content of which is incorporated herein by reference in its entirety), one encoding the AAV-2 rep gene and the cap of different AAV serotypes, one encoding the adenovirus helper functions, and one containing a dual firefly luciferase and eGFP expression cassette flanked with AAV-2 inverted terminal repeats. Transfections were performed into the Pro10 cell line (Pro10 cell line is described in US Patent No: 9,441,206, content of which is incorporated herein by reference in its entirety) grown in 62 or 125 mL cell culture medium. After three days, cells were chemically lysed by adding Triton X-100. Cell lysates were clarified, and rAAV was purified through affinity chromatography using POROS CaptureSelect AAVX or AAV9 resin (Thermo Scientific). For 62 mL transfections, purification was performed using AcroPrep filter plates (Cytivia), while for 125 mL transfections, this was done with a TECAN Freedom EVO automatic liquid handler. Affinity eluates were adjusted to neutral pH using 1M Tris buffer at pH 9.

***Bioreactor-scale AAVs production and purification:*** Larger volumes rAAV production was also achieved through triple plasmids transfection using polyethyleneimine (Grieger et al., "Production of Recombinant Adeno-associated Virus Vectors Using Suspension HEK293 Cells and Continuous Harvest of Vector From the Culture Media for GMP FIX and FLT1 Clinical Vector." Mol Ther. 2016 Feb; 24(2): 287-297, content of which is incorporated herein by reference in its entirety)) into the Pro10 cell line grown in 2-liters suspension culture. After three days of production, cells were chemically lysed by adding Triton X-100. Cell lysates were clarified, and rAAV was purified first through POROS CaptureSelect AAVX or AAV9 affinity chromatography. Then, affinity eluates were adjusted to neutral pH using 1M Tris buffer at pH 9 and submitted to discontinuous iodixanol gradient ultra-centrifugation (Zolotukhin et al., "Recombinant adeno-associated virus purification using novel methods improves infectious titer and yield." Gene Ther. 1999 Jun;6(6):973-85 1999, content of which is incorporated herein by reference in its entirety). Full AAV particles were collected from the interface with the 40 to 60% iodixanol layer then submitted to ion exchange chromatography using POROS 50HQ resin. Finally, purified vectors were formulated into phosphate buffer saline containing from about 0.0001% to about 0.01% Poloxamer 188 through dialysis.

***Vector genomes titrations by ITR qPCR:*** Samples were treated with DNase I at 37°C, then digested with proteinase K at 55°C followed by enzyme heat inactivation at 95°C. AAV vector genomes concentrations (VG/mL) were measured by quantitative PCR using primers and probe targeting the AAV2 ITR, based on the amplification signal of their ITR sequences (Arriaga et al., "Cellular and Structural Characterization of VP1 and VP2 Knockout Mutants of AAV3B Serotype and Implications for AAV Manufacturing." Hum Gene Ther. 2022 Nov;33(21-22):1142-1156, content of which is incorporated herein by reference in its entirety).

***Vector genomes titration by ITR ddPCR:*** Samples were treated with DNase I at 37°C, then digested with proteinase K at 55°C followed by enzyme heat inactivation at 80°C. AAV vector genomes concentrations (VG/mL) were measured by droplet digital PCR (ddPCR, Bio-Rad) using primers and probe targeting the AAV2 ITR (Aurnhammer et al., "Universal real-time PCR for the detection and quantification of adeno-associated virus serotype 2-derived inverted terminal repeat sequences." Hum Gene Ther Methods 2012 Feb;23(1):18-28, content of which is incorporated herein by reference in its entirety), based on the number of positive droplets where an ITR amplification signal is detected.

***Vector particles titration by ELISA:*** Samples were analyzed using capsid serotype-specific AAV titration ELISA kits (PROGEN) to determine the concentration of vector capsid.

***Analysis by size exclusion high pressure liquid chromatography (SEC-HPLC):*** Samples were analyzed in an Agilent HPLC 1290 Infinity II system with DAD detector equipped with an Agilent BioSEC-5 column (500 Å). To determine the viral particles (VP/mL) concentration, the analysis was performed using an isocratic elution at 0.4mL/min of mobile phase (3× phosphate buffered saline, 10% isopropyl alcohol in water), and AAV particles were detected by measuring absorbance at 214 nm. (Arriaga et al., "Cellular and Structural Characterization of VP1 and VP2 Knockout Mutants of AAV3B Serotype and Implications for AAV Manufacturing." Hum Gene Ther. 2022 Nov;33(21-22):1142-1156, content of which is incorporated herein by reference in its entirety).

***Analytical ultracentrifugation (AUC):*** Quantification of percent full, intermediate, and empty AAV capsids was performed using an Optima AUC XLI (Beckman Coulter). Analysis of AAV samples by the SEDFIT 16.36 c(S) model yields a distribution of sedimentation coefficients with each peak in the distribution representing capsids with an intact AAV genome, with packaged intermediate AAV genomes or residual nucleic acid impurities, or empty capsids. Integration of the individual peaks yields the sedimentation coefficient (S) and the relative concentration of each species in the distribution (Burnham et al., "Analytical Ultracentrifugation as an Approach to Characterize Recombinant Adeno-Associated Viral Vectors." Hum Gene Ther Methods 2015 Dec;26(6):228-42, content of which is incorporated herein by reference in its entirety).

***Residual plasmid quantification by ddPCR:*** Final product samples were digested with proteinase K at 55°C followed by enzyme heat inactivation at 80°C, and then analyzed by ddPCR (Bio-Rad). Three different assays were performed using primers and probes targeting (1) the kanamycin resistance gene from transposon Tn903 (named KanR1) to determine the total quantity of residual plasmid DNA, the adenovirus type 5 E4-orf6/7 gene to quantify residual helper plasmid DNA, and (3) the AAV2 rep gene to quantify residual rep-cap plasmid DNA.

***Sodium dodecyl-sulfate polyacrylamide gel electrophoresis (SDS-PAGE) and Silver Staining:*** For each purified rAAV sample, 5E+9 vector genomes were heat-denatured for 5 min at 95°C in Laemmli buffer with β-mercaptoethanol, then separated into a 10% Tris-Glycine SDS-PAGE gel (Invitrogen) followed by silver staining using the Pierce^{™} Silver Stain Kit (Arriaga et al., "Cellular and Structural Characterization of VP1 and VP2 Knockout Mutants of AAV3B Serotype and Implications for AAV Manufacturing." Hum Gene Ther. 2022 Nov;33(21-22):1142-1156, content of which is incorporated herein by reference in its entirety).

***Luciferase transgene expression:*** Cells, GM16095 (NIGMS Human Genetic Cell Repository, the Coriell Institute for Medical Research) or HeLa (ATCC^{®} CCL-2^{™}), were seeded in 96-wells plates at a density of 2E+04 cells/well in DMEM (Dulbecco's Modified Eagle's Medium) supplemented with 10% FBS (Fetal Bovine Serum) and incubated at 37°C under 5% CO₂. The next day, cells were transduced with the vector samples at multiplicities ranging from 1E+05 to 1E+06 vg/cell. After 48 hours incubation, cells were harvested, total proteins were extracted using RIPA buffer, and luciferase protein expression was analyzed using the Pierce^{™} Firefly Luciferase Glow Assay Kit. The luminescence signal was normalized to the total amount of proteins as measured with the Pierce^{™} BCA Protein Assay Kit.

***Western blotting.*** HEK293 cells (ATCC^{®} CRL-1573^{™}), were seeded into 6-wells plates at a density of 6E+05 cells/well in DMEM (Dulbecco's Modified Eagle's Medium) supplemented with 10% FBS (Fetal Bovine Serum) and incubated at 37°C under 5% CO₂. The next day, cells were transfected with one of the adenovirus helper plasmid, pXX680 **(SEQ ID NO: 96),** pLDB-XX85 **(SEQ ID NO:** 99), pHAD5 **(SEQ ID NO: 5)** or pHAD6 **(SEQ ID NO: 6),** using Lipofectamine 3000 (Thermo Fisher Scientific). Cells transfected with a pUC19 empty plasmid, or infected with adenovirus type 5 at a multiplicity of 5 pfu/cell, were used as negative and positive controls, respectively. After 48 hours incubation, cells were harvested, total proteins were extracted using RIPA buffer, and protein concentration was measured with the Pierce^{™} BCA Protein Assay Kit. Protein extracts (10 µg) were heat treated for 5 min at 95C in Laemmli buffer with β-mercaptoethanol, and loaded onto a 10% Tris-Glycine sodium dodecyl-sulfate polyacrylamide gel electrophoresis (SDS-PAGE) gel (Invitrogen). After electrophoresis, proteins were transferred onto a nitrocellulose membrane using the Trans-Blot Turbo kit following supplier's recommendations (Bio-Rad). Membranes were probed with an anti-Adenovirus 5 L4-33K rabbit polyclonal antibody (ABF105, Merck-Millipore), then with an anti-rabbit HRP-conjugated goat antibody, and proteins were detected using SuperSignal^{™} West Femto Maximum Sensitivity Substrate (Thermo Fisher Scientific).

### Results and discussion

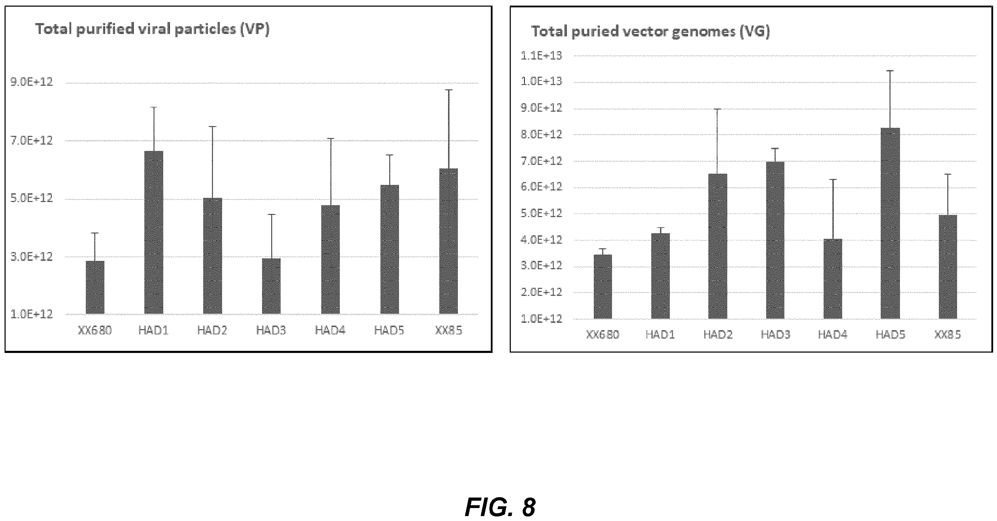

Results are shown in **FIGS. 8-30****.**

**FIG. 8** shows the quantity of VP was ranging from 1.9E+12 with pHAD3 to 8.0E+12 with pXX85, and the quantity of VG from 3.3E+12 with pXX680 to 9.8E+12 with pHAD5. The results show that overall, pHAD1 through pHAD5 helper plasmids allow production of equal or higher quantities of AAV8 capsids and encapsidated AAV vector genomes compared to pXX680 and pXX85 control plasmids.

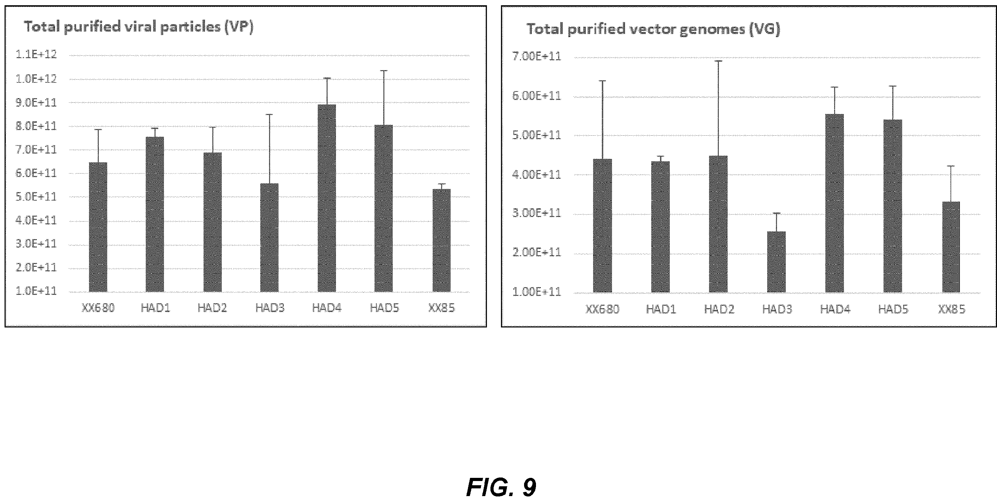

**FIG. 9** shows the quantity of VP was ranging from 3.5E+11 with pHAD3 to 9.7E+11 with pHAD4, and the quantity of VG from 2.2E+11 with pHAD3 to 6.2E+11 with pHAD2. The results show that overall, pHAD1 through pHAD5 helper plasmids allow production of equal or higher quantities of AAV6 capsids and encapsidated AAV vector genomes compared to pXX680 and pXX85 control plasmids.

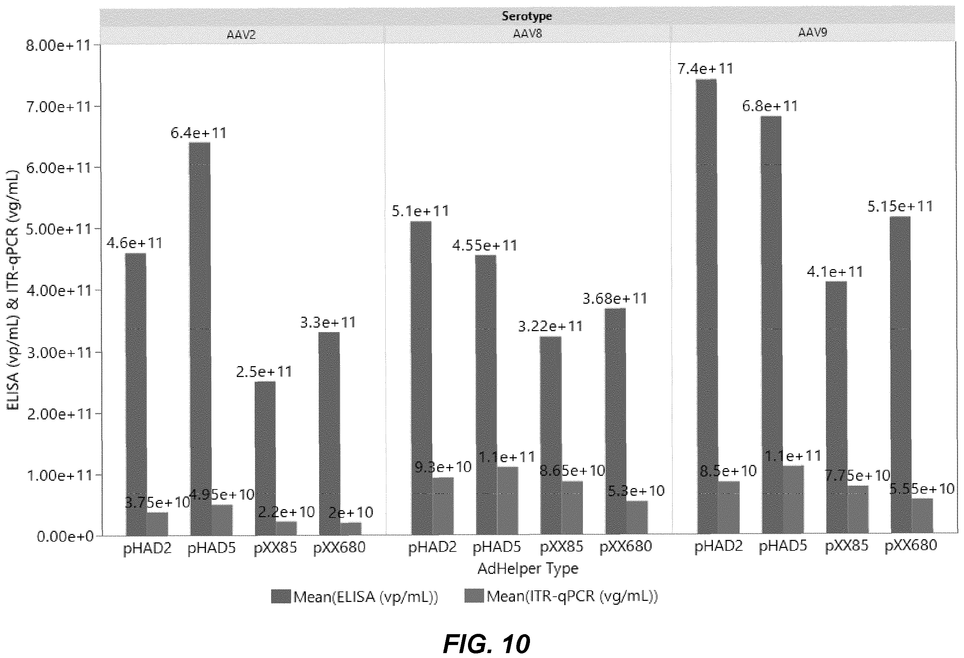

**FIG. 10** shows the results show that overall, pHAD2 and pHAD5 helper plasmids allow production of higher quantities of AAV2, AAV8 and AAV9 capsids and encapsidated AAV vector genomes compared to pXX680 and pXX85 control plasmids.

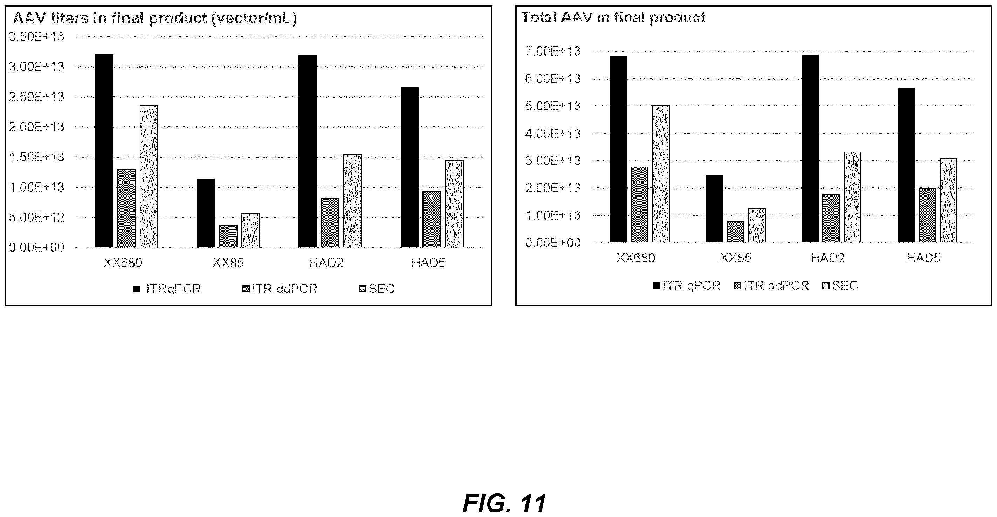

**FIG. 11** shows with all three methods, the titers and total vector yields produced using pHAD2 and pHAD5 helper plasmids appear slightly lower but still close compared to production performed with pXX680, and higher when compared to pXX85.

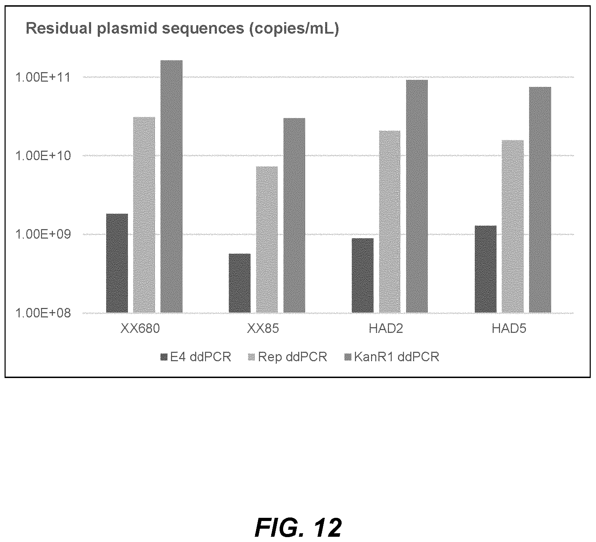

**FIG. 12** shows, as expected, the residual helper plasmid is found at a lower concentration compared to the rep-cap plasmid which is present at a higher copy number in the transfection cocktail, and the KanR sequence also present in the AAV vector plasmid is found at a higher concentration. Overall, the results show the presence of similar concentrations of residual plasmid DNA in all the samples. PCR targets concentrations (copies/mL) are presented in logarithmic scale.

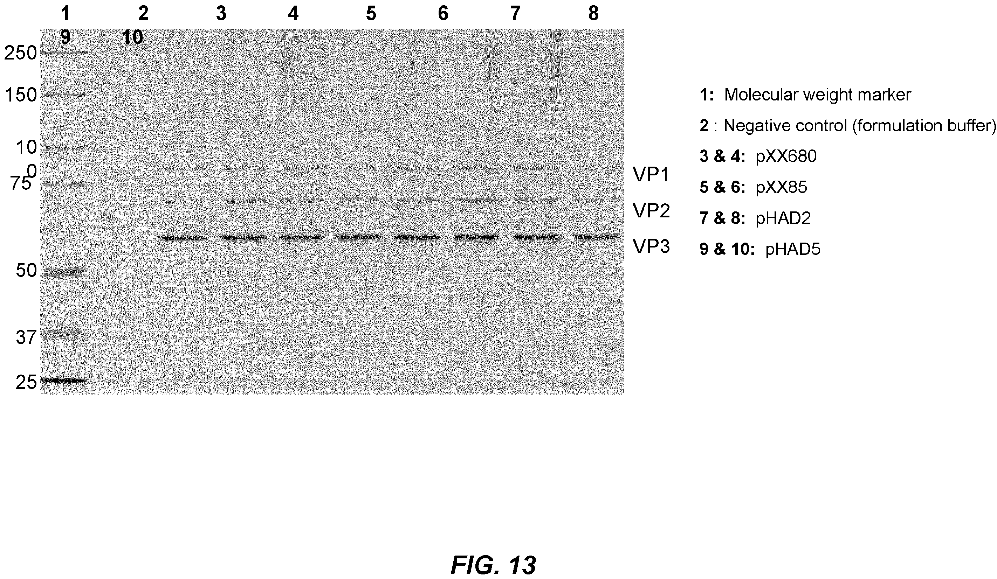

**FIG. 13** shows in all the samples, only three bands corresponding to AAV8 VP1, VP2, and VP3 capsid proteins were detected with similar relative bands intensities, showing high vector purity independently of the helper plasmid used for production.

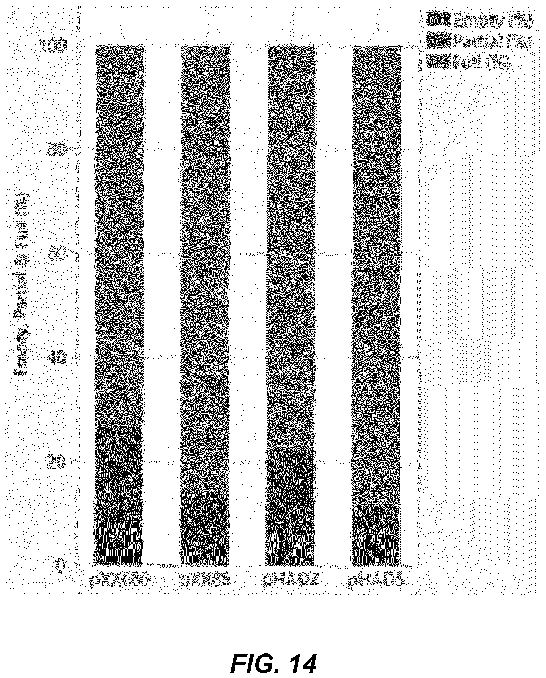

**FIG. 14** shows the AUC results show that AAV8 vectors produced with the different helper plasmids contain a majority (≥ 73%) of vector particles filled with full-length vector genomes (full particles). Production with pXX680 helper plasmid yielded the highest proportion of both intermediate particles (19%) filled with partial vector genomes or DNA impurities, and empty capsids (8%). Production with pHAD5 resulted in only 5% of partial particles and 6% of empty capsids.

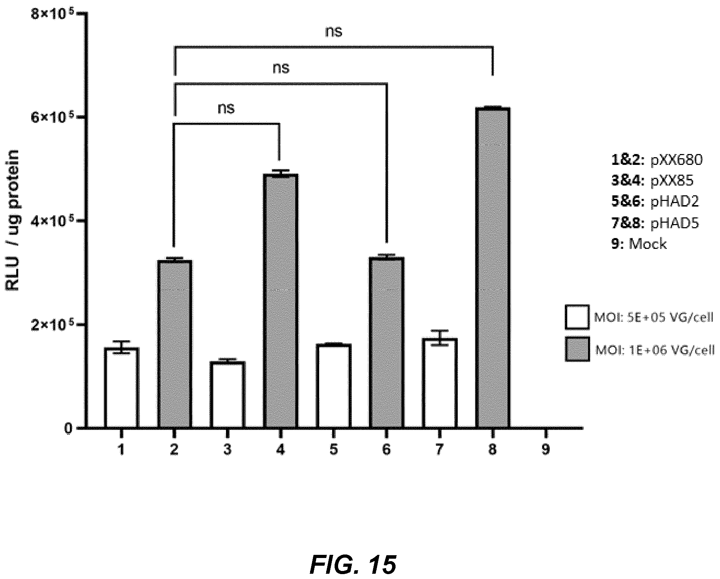

**FIG. 15** shows the statistical analysis by one-way ANOVA with Kruskal-Wallis test show no significant difference between the four samples, indicating that AAV8 vectors produced with any of the four helper plasmid, pXX680, pXX85, pHAD2, or pHAD5 had similar potency.

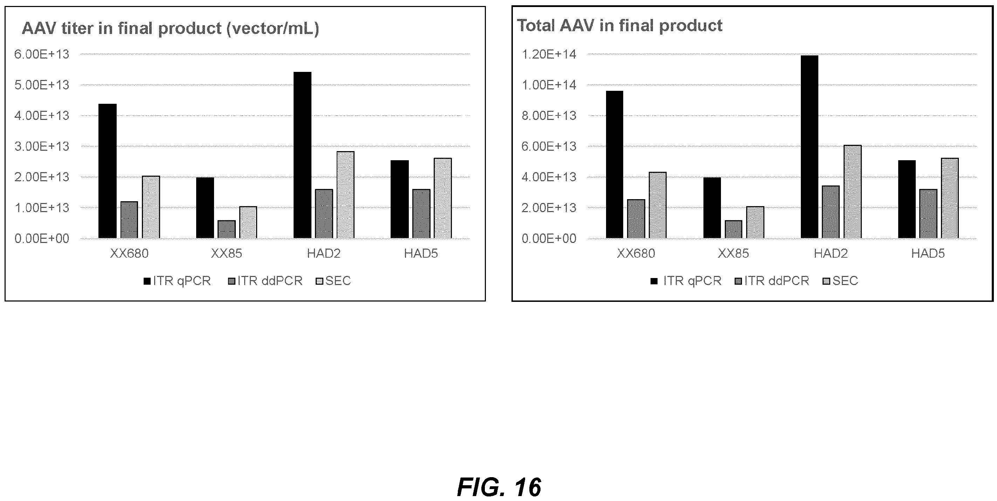

**FIG. 16** shows higher titers were found by ITR qPCR for vectors produced with pHAD2 and pXX680 compared to pHAD5. However, slightly higher titers were found with both pHAD2 and pHAD5 compared to pXX680 by ITR ddPCR and SEC-HPLC. Lower vector titers were detected with pXX85 compared to the other helper plasmids using all three methods.

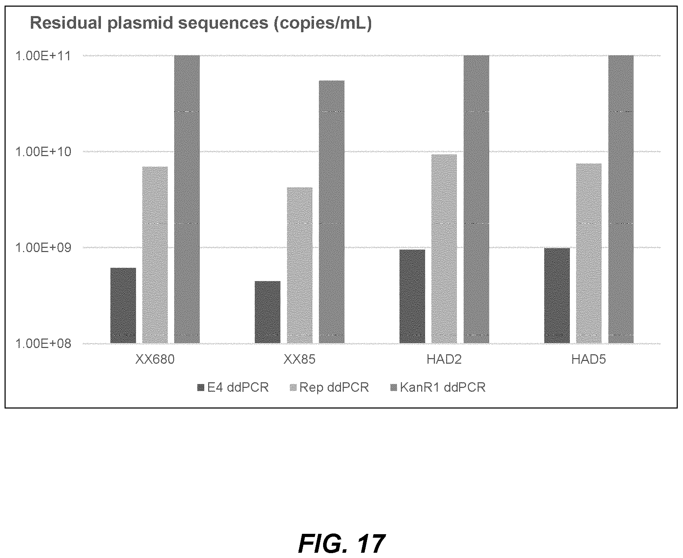

**FIG. 17** shows, as expected, the residual helper plasmid is found at a lower concentration compared to the rep-cap plasmid which is present at a higher copy number in the transfection cocktail, and the KanR sequence also present in the AAV vector plasmid is found at a higher concentration. Overall, the results show the presence of similar concentrations of residual plasmid DNA in all the samples.

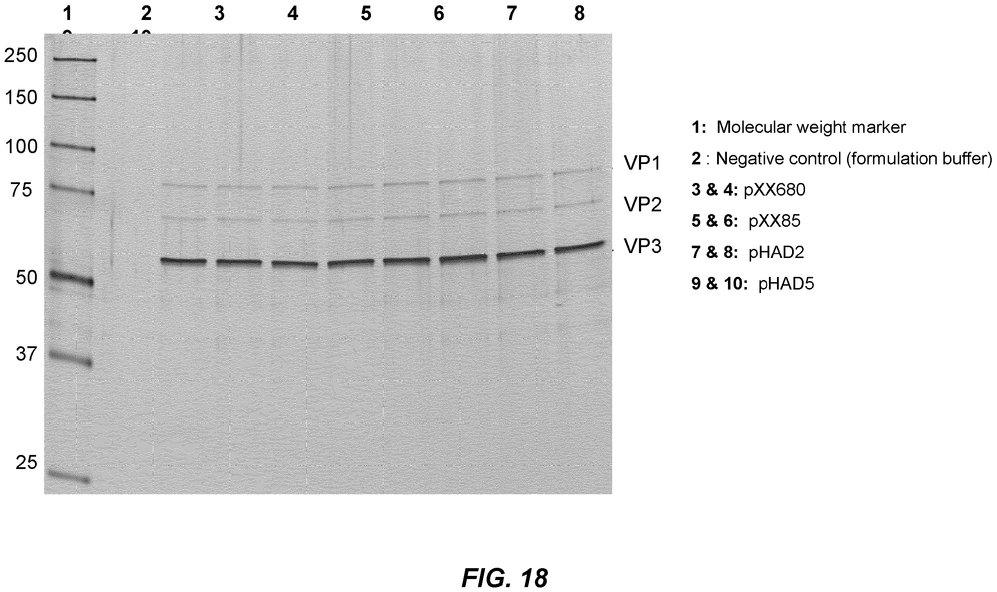

**FIG. 18** shows, in all the samples, only three bands corresponding to AAV6 VP1, VP2, and VP3 capsid proteins were detected with similar relative bands intensities, showing high vector purity independently of the helper plasmid used for production.

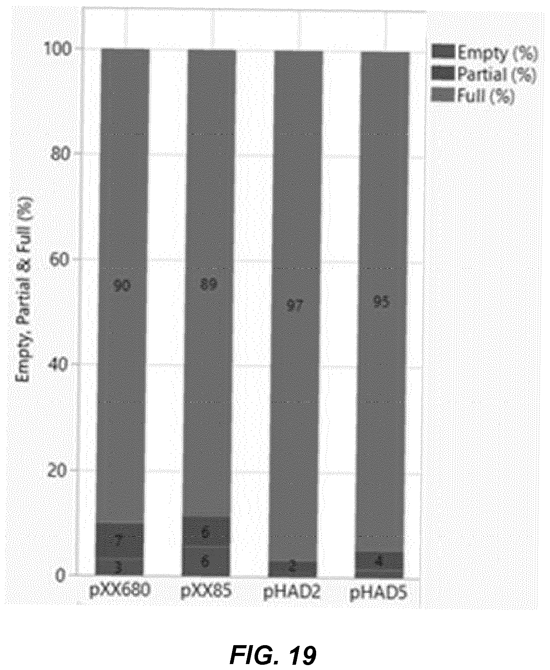

**FIG. 19** shows the AUC results show that AAV6 vectors produced with the different helper plasmids contain a majority (≥ 89%) of vector particles filled with full-length vector genomes (full particles). Production with pXX680 and pXX85 helper plasmids yielded a higher proportion of intermediate particles (7 and 6%) filled with partial vector genomes or DNA impurities, and empty capsids (3 and 6%). Production with pHAD2 and pHAD5 resulted in only 2 and 4% of partial particles and 1% of empty capsids.

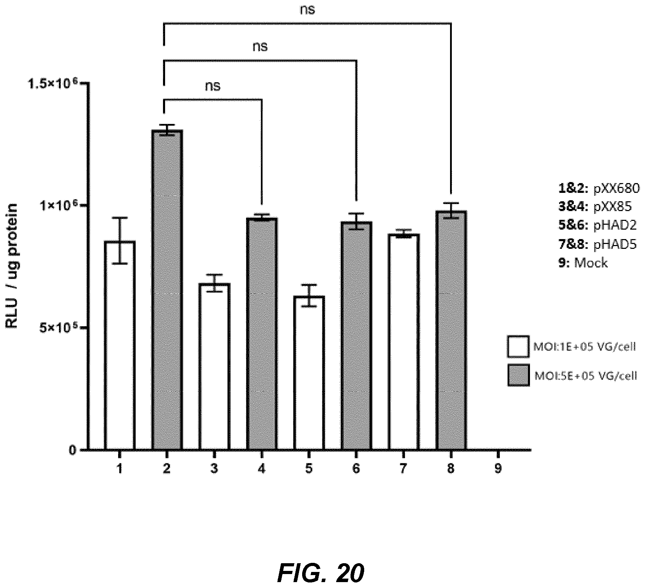

**FIG. 20** shows the statistical analysis by one-way ANOVA with Kruskal-Wallis test show no significant difference between the four samples, indicating that AAV6 vectors produced with any of the four helper plasmid, pXX680, pXX85, pHAD2, or pHAD5 had similar potency.

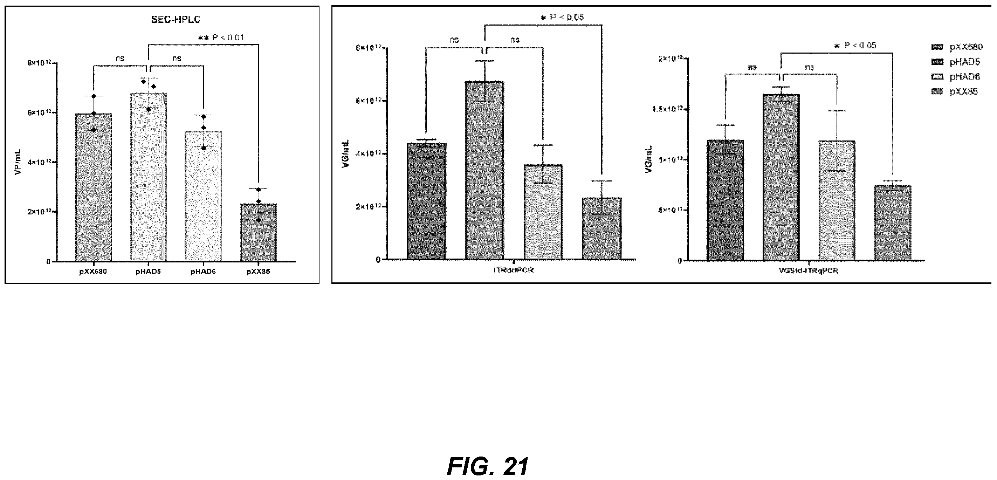

**FIG. 21** shows with the three titration methods, the results show no significant difference between vectors produced with pXX680, pHAD5 and pHAD6 helper plasmids, while the titers were found significantly lower with pXX85.

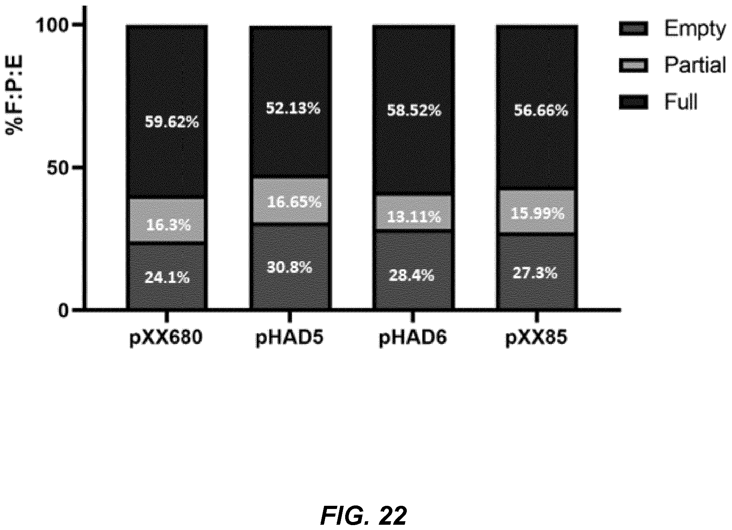

**FIG. 22** shows the results show that all the vector preparation contain between 52 and 60% of full particles. The proportion of partially filled particles was around 16% with pXX680, pHAD5 or pXX85 used as the helper plasmid, and only 13% with pHAD6, but overall, packaging efficiency with all four helper plasmids appears to be very similar.

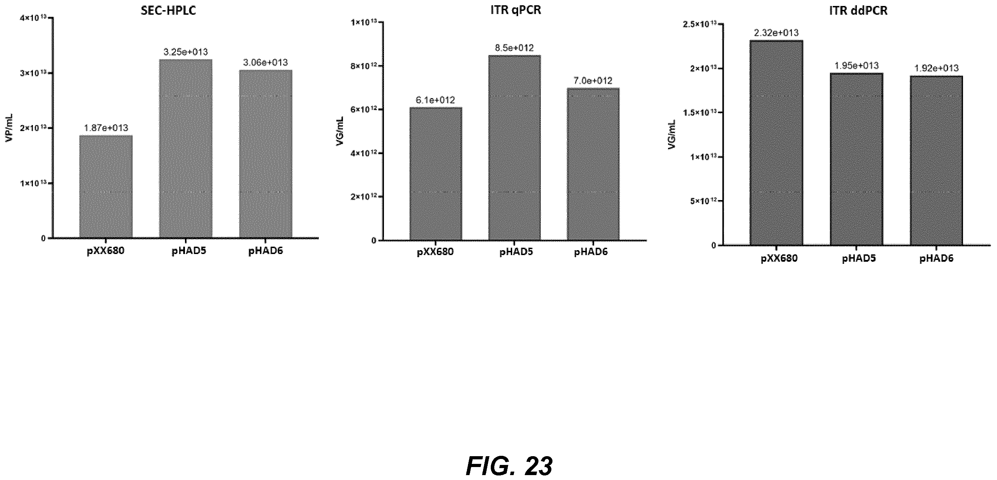

**FIG. 23** shows, by ITR qPCR and SEC-HPLC, a higher titer was found for the batch produced with pHAD5 compared to pXX680 and pHAD6, while slightly higher titer was found with pXX680 by ITR ddPCR. Overall, the three helper plasmids yielded similar vector productivity.

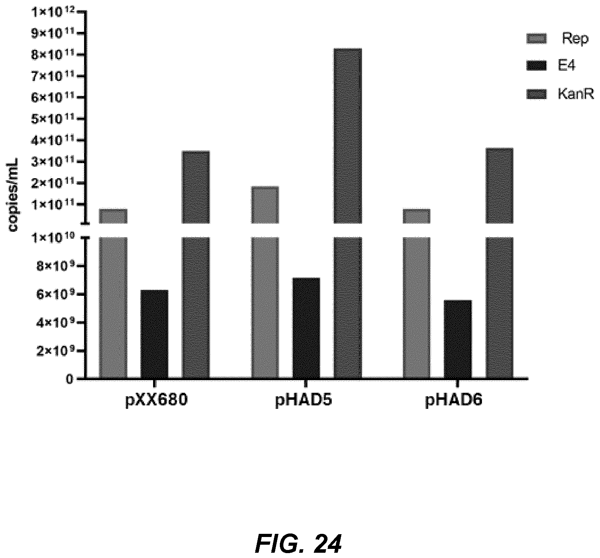

**FIG. 24** shows, as expected, the residual helper plasmid is found at a lower concentration compared to the rep-cap plasmid which is present at a higher copy number in the transfection cocktail, and the KanR sequence also present in the AAV vector plasmid is found at a higher concentration. Overall, the results show the presence of similar concentrations of residual plasmid DNA in all the samples.

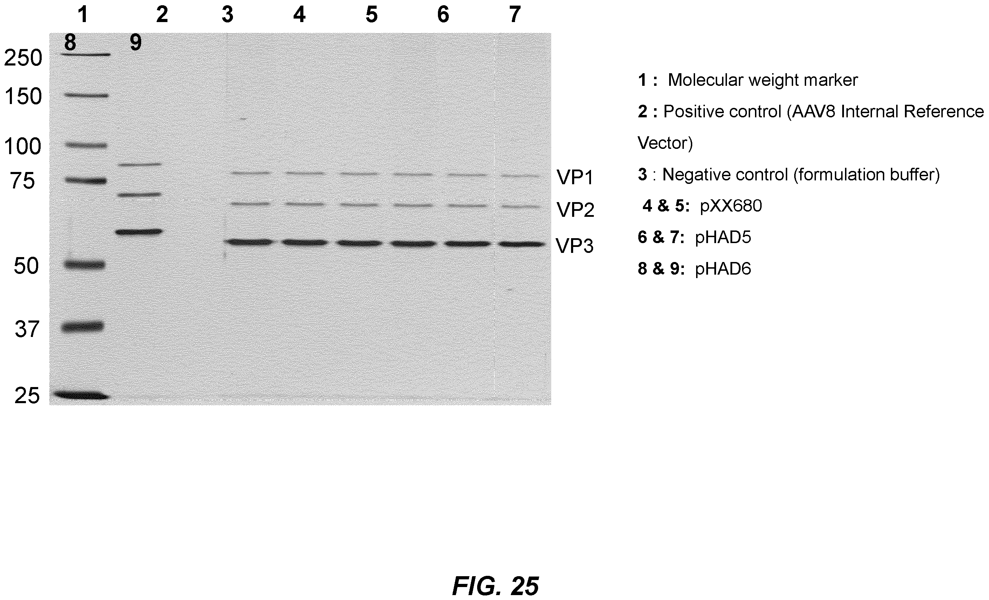

**FIG. 25** shows, in all the samples, only three bands corresponding to AAV9 VP1, VP2, and VP3 capsid proteins were detected with similar relative bands intensities, showing high vector purity independently of the helper plasmid used for production.

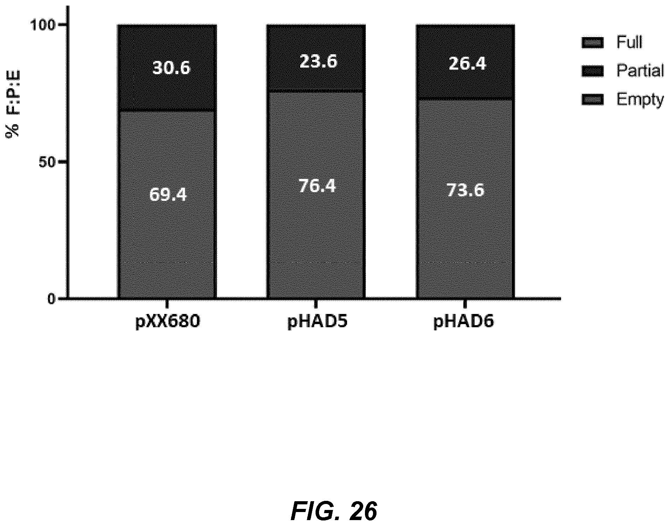

**FIG. 26** shows the AUC results show that AAV9 production with all three helper plasmids yielded similar packaging efficiency. The best results, i.e. the lowest proportion of intermediate particles filled with partial vector genomes and the highest proportion of full particles was obtained with pHAD5, and the poorest results with pXX680. Empty capsids were not detected in any of the three batches.

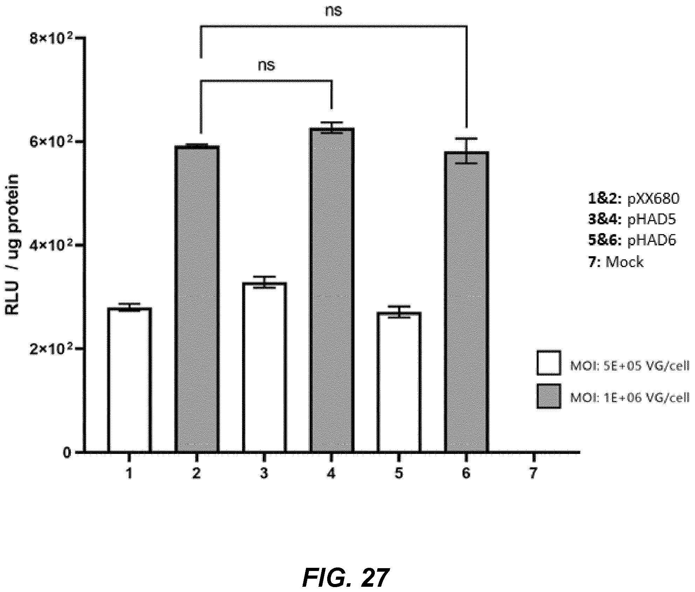

**FIG. 27** shows the statistical analysis by one-way ANOVA with Kruskal-Wallis test showed no significant difference between the three batches, indicating that AAV9 vectors produced with pXX680, pHAD5, and pHAD6 as the helper plasmid had similar potency.

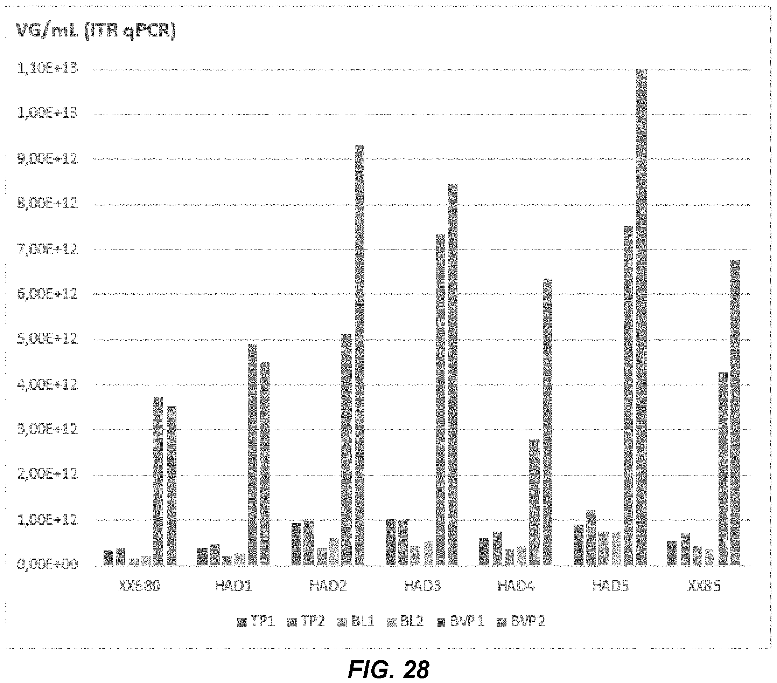

**FIG. 28** shows the results show that the titers obtained after affinity purification are consistent with the titers obtained at cells harvest, and vector recovery across the process is similar in all the productions, independently of the helper plasmid. The highest titers were obtained using pHAD5 as the helper plasmid, followed by pHAD2 and pHAD3, and lower titers were obtained with pXX680 and pHAD1, the two larger plasmids.

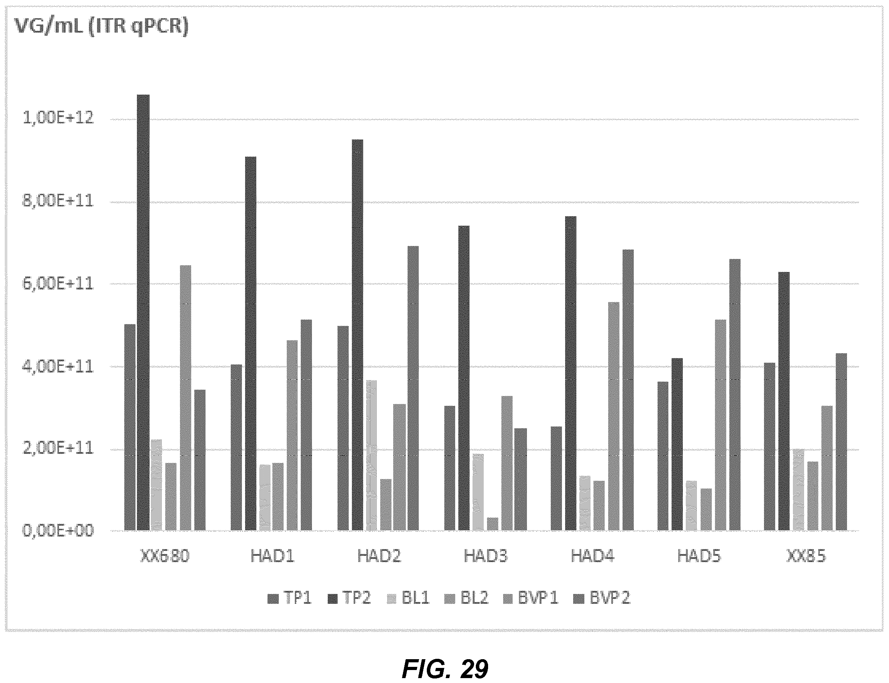

**FIG. 29** shows, in contrast to what is observed with AAV8 (FIG. 28), vector recovery across the process is variable, with a tendency to lose vectors between cells harvest and affinity purification. This is likely due to properties of the AAV6 capsid, which as affinity for both sialic acid and heparan sulfate, which makes it more difficult to recover since it can remain associated with cellular debris through interaction with glycoproteins. However, the titers obtained after affinity purification are very similar between productions performed with the different helper plasmids overall.

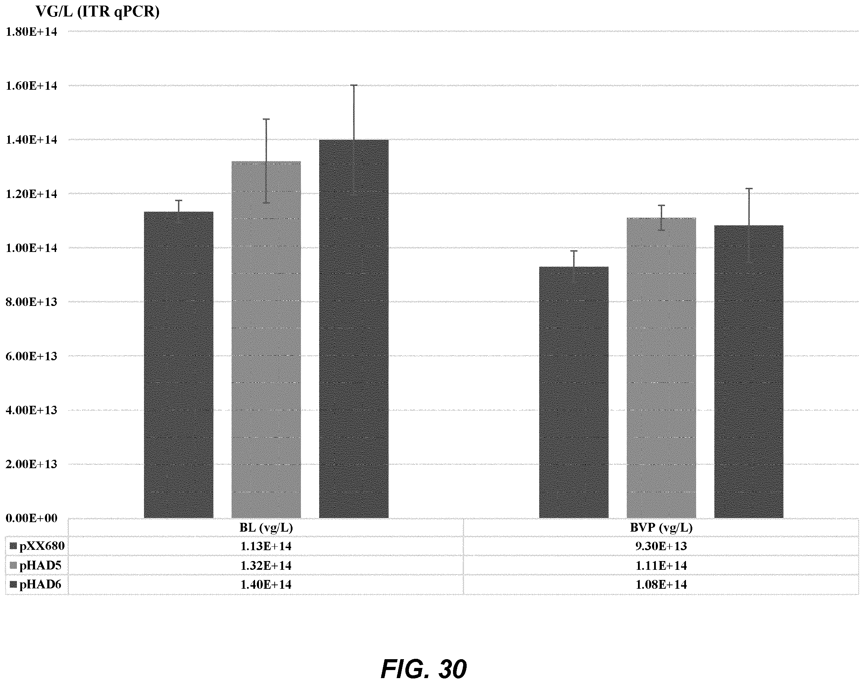

**FIG. 30** shows pHAD5 and pHAD6 helper plasmids yielded similar AAV9 vector concentrations compared to pXX680, both at the bulk lysate step and after affinity purification where the titers were all reaching around 1E+14 VG/L (1E+11 VG/mL).

All data cumulatively show the helper plasmids of the invention successfully produce AAV vectors as tested across different serotypes. Furthermore, the AAVs produced with the helper plasmids of invention e.g. HAD5 and HAD6 lead to successful transduction as tested with reporter luciferase gene expression.

All patents and other publications; including literature references, issued patents, published patent applications, and co-pending patent applications; cited throughout this application are expressly incorporated herein by reference for the purpose of describing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### CLAUSES

1. A polynucleotide comprising a nucleotide sequence encoding:
   a. an adenoviral E2a region;
   b. an adenoviral E4 region; and
   c. an adenoviral virus associated (VA) RNA region, and
      wherein the polynucleotide does not comprise a nucleotide sequence encoding
      at least 1 (e.g., 2, 3, 4, or all 5) of:
         a full length adenoviral L4-100K protein or a portion thereof;
         a full length adenoviral L5 fiber protein or a portion thereof;
         a full length adenoviral pVIII protein or a portion thereof;
         a full length adenoviral L1-52K/55K protein or a portion thereof; and
         a full length adenoviral precursor terminal protein (pTP) or a portion thereof; and
      wherein the polynucleotide comprises at least one (e.g., 1, 2, 3, 4, 5, 6 or all 7) of:
         a deletion of G₃ and G₄ in a nucleotide sequence encoding an adenoviral L-22K protein (e.g., **SEQ ID NO: 103)** and/or a L-33K protein (e.g., **SEQ ID NO: 104);**
         a deletion of G₃ and G₄ in a nucleotide sequence encoding an adenoviral L4-100K protein (e.g., **SEQ ID NO: 105);**
         a deletion of G₃ in a nucleotide sequence encoding adenoviral pVIII protein (e.g., **SEQ ID NO: 106);**
         a AC → TT mutation at positions 149-150 of a nucleotide sequence encoding adenoviral pVIII protein (e.g., **SEQ ID NO: 106);**
         a deletion of G₃ in a nucleotide sequence encoding adenoviral fiber protein (e.g., **SEQ ID NO: 107);**
         a C → T mutation at position 503 of a nucleotide encoding adenoviral L5 fiber protein (e.g., **SEQ ID NO: 107);** and
         a TA → CG mutation at positions 1692-1693 of a nucleotide encoding adenoviral L5 fiber protein (e.g., **SEQ ID NO: 107).**
2. The polynucleotide of clause 1, wherein the polynucleotide comprises:
   a. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 5;**
   b. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 6;**
   c. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 4;**
   d. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 3;**
   e. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 2;** or
   f. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 1**.
3. The polynucleotide of any one of clauses 1-2, wherein:
   a. the polynucleotide does not comprise nucleotides 26197-26198 relative to Genbank Accession Number AC_000008;
   b. the polynucleotide does not comprise nucleotides 24063-24064 relative to Genbank Accession Number AC_000008;
   c. the polynucleotide does not comprise nucleotide 27176 relative to Genbank Accession Number AC_000008;
   d. the polynucleotide comprises AC → TT mutation at positions 27322-27324 relative to Genbank Accession Number AC_000008;
   e. the polynucleotide does not comprise nucleotide 31024 relative to Genbank Accession Number AC_000008;
   f. the polynucleotide comprises C → T mutation at positions 31544 relative to Genbank Accession Number AC_000008; and/or
   g. the polynucleotide comprises TA → CG mutation at positions 32733-32734 relative to Genbank Accession Number AC_000008.
4. The polynucleotide of any one of clauses 1-3, wherein the polynucleotide does not comprise a nucleotide sequence encoding a protein comprising, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95**.
5. The polynucleotide of clause 1, wherein the polynucleotide does not comprise a nucleotide sequence encoding: (i) full length adenoviral L4-22K protein or a portion thereof; and/or (ii) full length adenoviral L4-33K protein or a portion thereof.
6. The polynucleotide of clause 5, wherein the polynucleotide comprises a nucleotide sequence encoding a less than full length fragment of adenoviral L4-22K protein and/or a less than full length fragment of adenoviral L4-33K protein, i.e., polynucleotide comprises a nucleotide sequence encoding a truncated adenoviral L4-22K protein and/or a nucleotide sequence encoding a truncated adenoviral L4-33K protein.
7. The polynucleotide of clause 5 or 6, wherein:
   a. the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the protein does not comprise, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95;**
   b. the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a full length adenoviral L4-22K protein having the amino acid sequence **SEQ ID NO: 7;**
   c. the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the protein does not comprise at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95;** or
   d. wherein the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the polynucleotide does not comprise a nucleotide sequence encoding a full-length adenoviral L4-33K protein having the amino acid sequence **SEQ ID NO: 8.**
8. The polynucleotide of clause 1, wherein:
   a. the polynucleotide does not comprise a nucleotide sequence encoding a full length L4-100K protein or a portion thereof, optionally, the full-length L4-100K protein has the amino acid sequence **SEQ ID NO: 9;**
   b. the polynucleotide does not comprise a nucleotide sequence encoding a full-length L5 fiber protein or a portion thereof, optionally, the full-length L5 fiber protein has the amino acid sequence **SEQ ID NO: 10;**
   c. the polynucleotide does not comprise a nucleotide sequence encoding a full length pVIII protein or a portion thereof, optionally, the full-length pVIII protein has the amino acid sequence **SEQ ID NO: 11;**
   d. the polynucleotide does not comprise a nucleotide sequence encoding aL1-52K/55K protein or a portion thereof, optionally, the full length L1-52K/55K protein has the amino acid sequence **SEQ ID NO: 12;** and/or
   e. the polynucleotide does not comprise a nucleotide sequence encoding a pTP or a portion thereof, optionally the full-length pTP has the amino acid sequence **SEQ ID NO: 13.**
9. The polynucleotide of clause 1, wherein:
   a. the polynucleotide does not comprise a nucleotide sequence encoding:
      a full length adenoviral E3 region or a portion thereof;
      a full length adenoviral U exon protein promoter or a portion thereof;
      a full length adenoviral U exon protein exon 1 or a portion thereof;
      a full length L4-100K protein or a portion thereof;
      a full length L1-52K/55K protein or a portion thereof;
      a full length L4-33K protein or a portion thereof;
      a full length L4-22K protein or a portion thereof;
      a full length L5 fiber protein or a portion thereof;
      a full length pVIII protein or a portion thereof; and
      a full length pTP or a portion thereof,
      optionally
      the polynucleotide does not comprise a nucleotide sequence encoding:
      a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
      a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion thereof;
      a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
      a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
      a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
      a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
      a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
      a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
      a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
      a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof;
   b. the polynucleotide does not comprise a nucleotide sequence encoding:
      a full length L4-100K protein or a portion thereof;
      a full length L1-52K/55K protein or a portion thereof;
      a full length L4-33K protein or a portion thereof;
      a full length L4-22K protein or a portion thereof;
      a full length L5 fiber protein or a portion thereof;
      a full length pVIII protein or a portion thereof; and
      a full length pTP or a portion thereof,
      optionally
      the polynucleotide does not comprise a nucleotide sequence encoding:
      a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
      a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
      a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
      a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
      a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
      a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
      a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof;
   c. the polynucleotide does not comprise a nucleotide sequence encoding:
      a full length adenoviral E3 region or a portion thereof;
      a full length adenoviral U exon protein (UXP) exon 1 or a portion thereof;
      a full length L4-100K protein or a portion thereof;
      a full length L1-55K protein or a portion thereof;
      a full length L4-33K protein or a portion thereof;
      a full length L4-22K protein or a portion thereof;
      a full length L5 fiber protein or a portion thereof;
      a full length pVIII protein or a portion thereof; and
      a full length pTP or a portion thereof,
      optionally,
      the polynucleotide does not comprise a nucleotide sequence encoding:
      a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
      a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
      a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
      a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
      a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
      a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
      a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
      a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
      a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof; or
   d. the polynucleotide comprises a nucleotide sequence encoding:
      a full-length L4-33K protein; and
      a full-length L4-22K protein, and
         wherein the wherein the polynucleotide does not comprise a nucleotide sequence encoding:
         a full length adenoviral E3 region or a portion thereof;
         a full length adenoviral U exon protein promoter or a portion thereof;
         a full length adenoviral U exon protein exon 1 or a portion thereof;
         a full length L4-100K protein or a portion thereof;
         a full length L1-55K protein or a portion thereof;
         a full length L5 fiber protein or a portion thereof;
         a full length pVIII protein or a portion thereof; and
         a full length pTP or a portion thereof,
         optionally,
         the polynucleotide comprises a nucleotide sequence encoding:
            a L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof; and
            a L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof; and
         the polynucleotide does not comprise a nucleotide sequence encoding:
            a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
            a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion thereof;
            a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
            a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
            a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
            a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
            a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
            a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof.
10. The polynucleotide of any one of clauses 1-9, wherein:
   a. the polynucleotide does not comprise a nucleotide sequence encoding an adenoviral E3 region or a portion thereof,
      optionally
      the polynucleotide does not comprise a nucleotide sequence encoding:
      i. an adenoviral E3 12.5k protein or a portion thereof, e.g., a protein having the amino acid sequence **SEQ ID NO: 14;**
      ii. encoding an adenoviral E3 CR1-alpha protein or a portion thereof, e.g., a protein having the amino acid sequence **SEQ ID NO: 15;**
      iii. an adenoviral E3 gp19k protein or a portion thereof, e.g., a protein having the amino acid sequence **SEQ ID NO: 16;**
      iv. an adenoviral E3 10.5kd protein or a portion thereof, e.g., a protein having the amino acid sequence **SEQ ID NO: 17;**
      v. an adenoviral E3 RID-alpha protein or a portion thereof, e.g., a protein having the amino acid sequence **SEQ ID NO: 18;**
      vi. an adenoviral E3 RID-beta protein, e.g., a protein having the amino acid sequence **SEQ ID NO: 19;** and/or
      vii. an adenoviral E3 14.7k protein or a portion thereof, e.g., a protein having the amino acid sequence **SEQ ID NO: 20;**
   b. the polynucleotide comprises a nucleotide sequence encoding an adenoviral E3 region, and wherein the E3 region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21;**
      and/or
   c. the polynucleotide does not comprise a nucleotide sequence encoding:
      i. an adenoviral E1 region or a portion thereof;
      ii. an adenoviral protease or a portion thereof, optionally the protease has the amino acid sequence **SEQ ID NO: 49;**
      iii. an adenoviral E1a protein 13S or a portion thereof, optionally the E1a protein 13S has the amino acid sequence **SEQ ID NO: 50;**
      iv. an adenoviral E1a protein 12S or a portion thereof, optionally the E1a protein 12S has the amino acid sequence **SEQ ID NO: 51;**
      v. an adenoviral E1a protein 11S or a portion thereof, optionally the E1a protein 11S has the amino acid sequence **SEQ ID NO: 52;**
      vi. the polynucleotide does not comprise a nucleotide sequence encoding an adenoviral E1a protein 10S or a portion thereof, optionally the E1a protein 10S has the amino acid sequence **SEQ ID NO: 53;**
      vii. an adenoviral E1a protein 9S or a portion thereof, optionally the E1a protein 9S has the amino acid sequence **SEQ ID NO: 54;**
      viii. an adenoviral E1b protein 19K or a portion thereof, optionally the E1b protein 19K has the amino acid sequence **SEQ ID NO: 55;**
      ix. an adenoviral E1b protein 55K or a portion thereof, optionally the E1b protein 55K has the amino acid sequence **SEQ ID NO: 56;**
      x. an adenoviral hexon protein or a portion thereof, optionally the hexon protein has the amino acid sequence **SEQ ID NO: 57;** and/or
      xi. an adenoviral peripentonal hexon-associated protein or a portion thereof, optionally the peripentonal hexon-associated protein has the amino acid sequence **SEQ ID NO: 58.**
11. The polynucleotide of any one of clauses 1-10, wherein the VA RNA region is flanked on each side by at least one restriction site, optionally
   a. the polynucleotide comprises at least 2, e.g., 3, 4, 5 or more restriction sites upstream of the VA RNA region;
   b. the polynucleotide comprises at least one restriction site downstream of the VA RNA region, and wherein the restriction site is between the VA region and the E2a region; and/or
   c. the polynucleotide comprises at least one restriction site downstream of the E4 region.
12. The polynucleotide of any one of clauses 1-11, wherein:
   a. the polynucleotide comprises a nucleotide sequence encoding a polyadenylation (poly A) site, optionally
      i. the poly A site is upstream of the VA RNA region;
      ii. the poly A site comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 22** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 22;**
      iii. the poly A site is flanked by at least one restriction site on each side;
      iv. the polynucleotide comprises at least three restriction sites upstream of the poly A site; and/or
      v. the polynucleotide comprises at least one restriction site downstream of the poly A site, and wherein the at least one restriction site is between the poly A site and the VA region.; and/or
   b. the polynucleotide comprises at least one inverted terminal repeat (ITR) sequence, optionally
      i. the polynucleotide comprises at least one inverted terminal repeat (ITR) sequence upstream of the E4 region; and/or
      ii. the ITR comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 23** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 23.**
13. The polynucleotide of any one of clauses 1-12, wherein:
   a. the VA RNA region comprises a VA RNA I region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 24** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 24;**
   b. the VA RNA region comprises a VA RNA II region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 25** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 25** and/or
   c. the VA region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 26** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 26.**
14. The polynucleotide of any one of clauses 1-13, wherein:
   a. the E2a region comprises a nucleotide sequence encoding an adenoviral DNA binding protein (DBP), optionally
      i. the E2a region comprises a nucleotide sequence encoding an adenoviral DNA binding protein (DBP) and having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 27**or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 27;** and/or
      ii. the E2a region comprises a nucleotide sequence encoding a DNA binding protein (DBP) having the amino acid sequence **SEQ ID NO: 28;**
   b. the E2a region comprises an adenoviral U exon protein (UXP) exon 3 sequence, optionally the E2a region comprises an adenoviral UXP exon 3 sequence having at least 8% identity to **SEQ ID NO: 29** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 29;**
   c. the E2a region comprises an adenoviral E2a exon2/leader sequence, optionally the E2a region comprises an adenoviral E2a exon2/leader sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30;**
   d. the E2a region comprises an adenoviral UXP exon 2 sequence, optionally the E2a region comprises an adenoviral UXP exon 2 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30;**
   e. the E2a region comprises an adenoviral E2a late promoter sequence, optionally the E2a region comprises an adenoviral E2a late promoter sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 31** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 31;**
   f. the E2a region comprises an adenoviral E2a exon 1 sequence, optionally the E2a region comprises an adenoviral E2a exon 1 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 32** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 32;**
   g. the E2a region is under the control of one or more of promoters, optionally the promoter is not a chicken β-actin promoter or a SV40 promoter;
   h. the E2a region comprises an adenoviral E2a early promoter sequence, optionally
   i. the E2a region comprises an adenoviral E2a early promoter sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 33** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 33;**
   j. the E2a region comprises an adenoviral E2a late primary transcript sequence, optionally
   k. the E2a region comprises an adenoviral E2a late primary transcript sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 34** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 34;**
   l. the E2a region comprises an adenoviral E2a early primary transcript sequence, optionally the E2a region comprises an adenoviral E2a early primary transcript sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 35** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 35;** and/or
   m. the E2a region comprises a nucleotide having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 36** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 36.**
15. The polynucleotide of any one of clauses 1-14, wherein:
   a. the E4 region comprises an adenoviral E4 orf6/7 sequence, optionally
      i. the E4 region comprises an adenoviral E4 orf6/7 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 37** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 37;**
         and/or
      ii. the E4 region comprises a nucleotide sequence encoding an adenoviral E4 orf6/7 protein having the amino acid sequence **SEQ ID NO: 38;**
   b. the E4 region comprises a nucleotide sequence encoding an adenoviral E4 34K protein having the amino acid sequence **SEQ ID NO: 39;**
   c. the E4 region comprises an adenoviral E4 orf4 sequence, optionally
      i. the E4 region comprises an adenoviral E4 orf4 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 40** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 40** and/or
      ii. the E4 region comprises a nucleotide sequence encoding an adenoviral E4 orf4 protein having the amino acid sequence **SEQ ID NO: 41;**
   d. the E4 region comprises an adenoviral E4 orf3 sequence, optionally
      i. the E4 region comprises an adenoviral E4 orf3 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 42** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 42;** and/or
      ii. the E4 region comprises a nucleotide sequence encoding an adenoviral E4 orf3 protein having the amino acid sequence **SEQ ID NO: 43;**
   e. the E4 region comprises an adenoviral E4 orf8 sequence, optionally
      i. the E4 region comprises an adenoviral E4 orf4 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 44** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 44;** and/or
      ii. the E4 region comprises a nucleotide sequence encoding an adenoviral E4 orf4 protein having the amino acid sequence **SEQ ID NO: 45;**
   f. the E4 region comprises an adenoviral E4 orf1 sequence, optionally
      i. the E4 region comprises an adenoviral E4 orf1 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 46** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 46;** and/or
      ii. the E4 region comprises a nucleotide sequence encoding an adenoviral E4 orf1 protein having the amino acid sequence **SEQ ID NO: 47;**
   g. the E4 region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 48** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 48;** and/or
   h. the E4 region is operably linked to a promoter, optionally the promoter is not a chicken β-actin promoter or a SV40 promoter.
16. The polynucleotide of any one of clauses 1-15, wherein:
   a. the polynucleotide is about 16,500 nucleotides or less in size, e.g., about 15,500 nucleotides or less in size, about 14,000 nucleotide or less in size, about 12,500 nucleotides or less in size, about 12,000 nucleotides or less in size, or about 11,000 nucleotides or less in size;
   b. the polynucleotide is double-stranded;
   c. the polynucleotide is single-stranded;
   d. the polynucleotide is linear;
   e. the polynucleotide is circular; and/or
   f. the polynucleotide is a close ended linear duplexed DNA (clDNA).
17. The polynucleotide of any one of clauses 1-16, wherein:
   a. the polynucleotide is capable of producing recombinant adeno-associated viral (rAAV) particles in an amount that is at least 80% of an amount produced under similar conditions with a similar polynucleotide that further comprises a nucleotide sequence encoding a full-length L-22K protein and/or a full-length L-33K protein; and/or a packaging efficiency of the polynucleotide in a method of producing rAAV is at least 80% of a packaging efficiency of a similar polypeptide that further comprises a nucleotide sequence encoding a full-length L-22K protein and/or a full-length L-33K protein.
18. A cell, plasmid or vector comprising a polynucleotide of any one of clauses 1-17, optionally
   a. the cell, plasmid or vector does not comprise a nucleotide sequence encoding a full length L-22K and/or a full length L-33K protein, e.g., the cell, plasmid or vector does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8.**
   b. vector is a viral vector, e.g., a retroviral vector, a lentiviral vector, an adenoviral vector, an adeno-associated viral vector, or a herpes simplex viral vector, preferably an adenoviral vector;
   c. the cell
      i. is an insect cell or a mammalian cell, preferably the cell is a mammalian cell;
      ii. is a HeLa cell, COS cell, COS-1 cell, COS-7 cell, HEK293 cell, A549 cell, BHK cell, BSC-1 cell, BSC-40 cell, Vero cell, Sf"c9 cell, Sf -21 cell, Tn-368 cell, BTI-Tn-5B1-4 (High-Five) cell, Saos cell, C2C12 cell, L cell, HT1080 cell, HepG2 cell, WEHI cell, 3T3 cell, 10T1/2 cell, MDCK cell, BMT-10 cell, WI38 cell, or a primary fibroblast, hepatocyte or myoblast cells derived from a mammal, preferably the cell is a HEK293 cell or HeLa cell;
      iii. is a suspension adapted cell; and/or
      iv. is a producer cell;
      v. further comprises a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR), and wherein the L-ITR and R-ITR are selected independently from AAV ITRs and adenoviral ITRs;
      vi. further comprises a polynucleotide encoding viral capsid (e.g., AAV capsid) and/or non-structural replication (e.g., AAV Rep) genes.
19. Use of a polynucleotide of any one of clauses 1-17, or a cell, plasmid or vector of vector of clause 18, for producing recombinant adeno-associated viral (rAAV) particles.
20. A method for producing recombinant adeno-associated viral (rAAV) particles, the method comprising: culturing a cell of clause 18 in a culture medium under conditions in which rAAV particles are produced.
21. A method for producing recombinant adeno-associated viral (rAAV) particles, the method comprising: culturing a host cell in a culture medium under conditions in which rAAV particles are produced, where the host cell comprises: (i) a polynucleotide of any one of clauses 1-17, or a plasmid or vector of clause 18; (ii) a polynucleotide encoding a virus genome (e.g., an AAV endogenous genome, optionally flanked by left inverted terminal repeat (L-AAV ITR) and/or right ITR (R-AAV ITR), or a recombinant AAV genome comprising polynucleotide encoding a transgene, optionally flanked by L-AAVITR and/or R-AAV ITR); and (iii) a polynucleotide encoding viral capsid (e.g., AAV capsid) and/or non-structural replication (e.g., AAV Rep) genes.
22. The method of any one of clauses 20 or 21, wherein:
   a. the cell culture is substantially free of a polynucleotide encoding a full-length L-22K protein and/or a full-length L-33K protein, e.g., the cell culture is substantially free of a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8;** and/or
   b. a full-length L-22K protein and/or a full-length L-33K protein is not expressed during the culturing of the cell, e.g., a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 and/or 8** is not expressed during the culturing of the cell.

## Claims

1. A polynucleotide comprising a nucleotide sequence encoding:
a. an adenoviral E2a region;
b. an adenoviral E4 region; and
c. an adenoviral virus associated (VA) RNA region, and
wherein the polynucleotide does not comprise a nucleotide sequence encoding
at least 1 (e.g., 2, 3, 4, or all 5) of:
a full length adenoviral L4-100K protein or a portion thereof;
a full length adenoviral L5 fiber protein or a portion thereof;
a full length adenoviral pVIII protein or a portion thereof;
a full length adenoviral L1-52K/55K protein or a portion thereof; and
a full length adenoviral precursor terminal protein (pTP) or a portion
thereof; and
wherein the polynucleotide comprises at least one (e.g., 1, 2, 3, 4, 5, 6 or all 7) of:
a deletion of G₃ and G₄ in a nucleotide sequence encoding an adenoviral L-22K protein (e.g., **SEQ ID NO: 103)** and/or a L-33K protein (e.g., **SEQ ID NO: 104);**
a deletion of G₃ and G₄ in a nucleotide sequence encoding an adenoviral L4-100K protein (e.g., **SEQ ID NO: 105);**
a deletion of G₃ in a nucleotide sequence encoding adenoviral pVIII protein (e.g., **SEQ ID NO: 106);**
a AC → TT mutation at positions 149-150 of a nucleotide sequence encoding adenoviral pVIII protein (e.g., **SEQ ID NO: 106);**
a deletion of G₃ in a nucleotide sequence encoding adenoviral fiber protein (e.g., **SEQ ID NO: 107);**
a C → T mutation at position 503 of a nucleotide encoding adenoviral L5 fiber protein (e.g., **SEQ ID NO: 107);** and
a TA → CG mutation at positions 1692-1693 of a nucleotide encoding adenoviral L5 fiber protein (e.g., **SEQ ID NO: 107).**

2. The polynucleotide of claim 1, wherein the polynucleotide comprises:
a. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 5;**
b. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 6;**
c. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 4;**
d. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 3;**
e. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 2;** or
f. a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 1.**

3. The polynucleotide of any one of claims 1-2, wherein:
a. the polynucleotide does not comprise nucleotides 26197-26198 relative to Genbank Accession Number AC_000008;
b. the polynucleotide does not comprise nucleotides 24063-24064 relative to Genbank Accession Number AC_000008;
c. the polynucleotide does not comprise nucleotide 27176 relative to Genbank Accession Number AC_000008;
d. the polynucleotide comprises AC → TT mutation at positions 27322-27324 relative to Genbank Accession Number AC_000008;
e. the polynucleotide does not comprise nucleotide 31024 relative to Genbank Accession Number AC_000008;
f. the polynucleotide comprises C → T mutation at positions 31544 relative to Genbank Accession Number AC_000008; and/or
g. the polynucleotide comprises TA → CG mutation at positions 32733-32734 relative to Genbank Accession Number AC_000008, and/or
wherein the polynucleotide does not comprise a nucleotide sequence encoding a protein comprising, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95.**

4. The polynucleotide of claim 1, wherein the polynucleotide does not comprise a nucleotide sequence encoding: (i) full length adenoviral L4-22K protein or a portion thereof; and/or (ii) full length adenoviral L4-33K protein or a portion thereof, optionally
wherein the polynucleotide comprises a nucleotide sequence encoding a less than full length fragment of adenoviral L4-22K protein and/or a less than full length fragment of adenoviral L4-33K protein, i.e., polynucleotide comprises a nucleotide sequence encoding a truncated adenoviral L4-22K protein and/or a nucleotide sequence encoding a truncated adenoviral L4-33K protein.

5. The polynucleotide of claim 4, wherein:
a. the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the protein does not comprise, e.g., at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95;**
b. the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 92 or 93,** and wherein the polynucleotide does not comprise a nucleotide sequence encoding a full length adenoviral L4-22K protein having the amino acid sequence **SEQ ID NO: 7;**
c. the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the protein does not comprise at its N-terminus an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 94 or 95;** or
d. wherein the polynucleotide comprises a nucleotide sequence encoding a protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 90 or 91,** and wherein the polynucleotide does not comprise a nucleotide sequence encoding a full-length adenoviral L4-33K protein having the amino acid sequence **SEQ ID NO: 8.**

6. The polynucleotide of claim 1, wherein:
a. the polynucleotide does not comprise a nucleotide sequence encoding a full length L4-100K protein or a portion thereof, optionally, the full-length L4-100K protein has the amino acid sequence **SEQ ID NO: 9;**
b. the polynucleotide does not comprise a nucleotide sequence encoding a full-length L5 fiber protein or a portion thereof, optionally, the full-length L5 fiber protein has the amino acid sequence **SEQ ID NO: 10;**
c. the polynucleotide does not comprise a nucleotide sequence encoding a full length pVIII protein or a portion thereof, optionally, the full-length pVIII protein has the amino acid sequence **SEQ ID NO: 11;**
d. the polynucleotide does not comprise a nucleotide sequence encoding aL1-52K/55K protein or a portion thereof, optionally, the full length L1-52K/55K protein has the amino acid sequence **SEQ ID NO: 12;** and/or
e. the polynucleotide does not comprise a nucleotide sequence encoding a pTP or a portion thereof, optionally the full-length pTP has the amino acid sequence **SEQ ID NO: 13,** or
wherein:
a. the polynucleotide does not comprise a nucleotide sequence encoding:
a full length adenoviral E3 region or a portion thereof;
a full length adenoviral U exon protein promoter or a portion thereof;
a full length adenoviral U exon protein exon 1 or a portion thereof;
a full length L4-100K protein or a portion thereof;
a full length L1-52K/55K protein or a portion thereof;
a full length L4-33K protein or a portion thereof;
a full length L4-22K protein or a portion thereof;
a full length L5 fiber protein or a portion thereof;
a full length pVIII protein or a portion thereof; and
a full length pTP or a portion thereof,
optionally
the polynucleotide does not comprise a nucleotide sequence encoding:
a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion thereof;
a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof;
b. the polynucleotide does not comprise a nucleotide sequence encoding:
a full length L4-100K protein or a portion thereof;
a full length L1-52K/55K protein or a portion thereof;
a full length L4-33K protein or a portion thereof;
a full length L4-22K protein or a portion thereof;
a full length L5 fiber protein or a portion thereof;
a full length pVIII protein or a portion thereof; and
a full length pTP or a portion thereof,
optionally
the polynucleotide does not comprise a nucleotide sequence encoding:
a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof;
c. the polynucleotide does not comprise a nucleotide sequence encoding:
a full length adenoviral E3 region or a portion thereof;
a full length adenoviral U exon protein (UXP) exon 1 or a portion thereof;
a full length L4-100K protein or a portion thereof;
a full length L1-55K protein or a portion thereof;
a full length L4-33K protein or a portion thereof;
a full length L4-22K protein or a portion thereof;
a full length L5 fiber protein or a portion thereof;
a full length pVIII protein or a portion thereof; and
a full length pTP or a portion thereof,
optionally,
the polynucleotide does not comprise a nucleotide sequence encoding:
a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
a full length L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof;
a full length L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof;
a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof; or
d. the polynucleotide comprises a nucleotide sequence encoding:
a full-length L4-33K protein; and
a full-length L4-22K protein, and wherein the wherein the polynucleotide does not comprise a nucleotide
sequence encoding:
a full length adenoviral E3 region or a portion thereof;
a full length adenoviral U exon protein promoter or a portion thereof;
a full length adenoviral U exon protein exon 1 or a portion thereof;
a full length L4-100K protein or a portion thereof;
a full length L1-55K protein or a portion thereof;
a full length L5 fiber protein or a portion thereof;
a full length pVIII protein or a portion thereof; and
a full length pTP or a portion thereof,
optionally.
the polynucleotide comprises a nucleotide sequence encoding:
a L4-33K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 8,** or a portion thereof; and
a L4-22K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7,** or a portion thereof; and
the polynucleotide does not comprise a nucleotide sequence encoding:
a full length adenoviral E3 region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21,** or a portion thereof;
a full length adenoviral U exon protein promoter 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 101** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 101,** or a portion thereof;
a full length adenoviral UXP exon 1 comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 102** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 102,** or a portion thereof;
a full length L4-100K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 9,** or a portion thereof;
a full length L1-52K/55K protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 13,** or a portion thereof;
a full length L5 fiber protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 10,** or a portion thereof;
a full length pVIII protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 11,** or a portion thereof; and
a full length pTP protein comprising an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO:13,** or a portion thereof.

7. The polynucleotide of any one of claims 1-6, wherein:
a. the polynucleotide does not comprise a nucleotide sequence encoding an adenoviral E3 region or a portion thereof,
optionally
the polynucleotide does not comprise a nucleotide sequence encoding:
i. an adenoviral E3 12.5k protein or a portion thereof, e.g., a protein having the amino acid sequence **SEQ ID NO: 14;**
ii. encoding an adenoviral E3 CR1-alpha protein or a portion thereof, e.g., a protein having the amino acid sequence **SEQ ID NO: 15;**
iii. an adenoviral E3 gp19k protein or a portion thereof, e.g., a protein having the amino acid sequence **SEQ ID NO: 16;**
iv. an adenoviral E3 10.5kd protein or a portion thereof, e.g., a protein having the amino acid sequence **SEQ ID NO: 17;**
v. an adenoviral E3 RID-alpha protein or a portion thereof, e.g., a protein having the amino acid sequence **SEQ ID NO: 18;**
vi. an adenoviral E3 RID-beta protein, e.g., a protein having the amino acid sequence **SEQ ID NO: 19;** and/or
vii. an adenoviral E3 14.7k protein or a portion thereof, e.g., a protein having the amino acid sequence **SEQ ID NO: 20;**
b. the polynucleotide comprises a nucleotide sequence encoding an adenoviral E3 region, and wherein the E3 region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 21** or having at least 80% (e.g., at least 85%, at least 90%, at least 91%, at least 92%, at least 93%, at least 94%, at least 95%, at least 96%, at least 97%, at least 98%, at least 99% or 100%) identity to a nucleotide sequence complementary to **SEQ ID NO: 21;**
and/or
c. the polynucleotide does not comprise a nucleotide sequence encoding:
i. an adenoviral E1 region or a portion thereof;
ii. an adenoviral protease or a portion thereof, optionally the protease has the amino acid sequence **SEQ ID NO: 49;**
iii. an adenoviral E1a protein 13S or a portion thereof, optionally the E1a protein 13S has the amino acid sequence **SEQ ID NO: 50;**
iv. an adenoviral E1a protein 12S or a portion thereof, optionally the E1a protein 12S has the amino acid sequence **SEQ ID NO: 51;**
v. an adenoviral E1a protein 11S or a portion thereof, optionally the E1a protein 11S has the amino acid sequence **SEQ ID NO: 52;**
vi. the polynucleotide does not comprise a nucleotide sequence encoding an adenoviral E1a protein 10S or a portion thereof, optionally the E1a protein 10S has the amino acid sequence **SEQ ID NO: 53;**
vii. an adenoviral E1a protein 9S or a portion thereof, optionally the E1a protein 9S has the amino acid sequence **SEQ ID NO: 54;**
viii. an adenoviral E1b protein 19K or a portion thereof, optionally the E1b protein 19K has the amino acid sequence **SEQ ID NO: 55;**
ix. an adenoviral E1b protein 55K or a portion thereof, optionally the E1b protein 55K has the amino acid sequence **SEQ ID NO: 56;**
x. an adenoviral hexon protein or a portion thereof, optionally the hexon protein has the amino acid sequence **SEQ ID NO: 57;** and/or
xi. an adenoviral peripentonal hexon-associated protein or a portion thereof, optionally the peripentonal hexon-associated protein has the amino acid sequence **SEQ ID NO: 58,** and/or
wherein the VA RNA region is flanked on each side by at least one restriction site, optionally
a. the polynucleotide comprises at least 2, e.g., 3, 4, 5 or more restriction sites upstream of the VA RNA region;
b. the polynucleotide comprises at least one restriction site downstream of the VA RNA region, and wherein the restriction site is between the VA region and the E2a region; and/or
c. the polynucleotide comprises at least one restriction site downstream of the E4 region, and/or
wherein:
a. the polynucleotide comprises a nucleotide sequence encoding a polyadenylation (poly A) site, optionally
i. the poly A site is upstream of the VA RNA region;
ii. the poly A site comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 22** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 22;**
iii. the poly A site is flanked by at least one restriction site on each side;
iv. the polynucleotide comprises at least three restriction sites upstream of the poly A site; and/or
v. the polynucleotide comprises at least one restriction site downstream of the poly A site, and wherein the at least one restriction site is between the poly A site and the VA region.; and/or
b. the polynucleotide comprises at least one inverted terminal repeat (ITR) sequence, optionally
i. the polynucleotide comprises at least one inverted terminal repeat (ITR) sequence upstream of the E4 region; and/or
ii. the ITR comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 23** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 23,** and/or
wherein:
a. the VA RNA region comprises a VA RNA I region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 24** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 24;**
b. the VA RNA region comprises a VA RNA II region comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 25** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 25** and/or
c. the VA region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 26** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 26.**

8. The polynucleotide of any one of claims 1-7, wherein:
a. the E2a region comprises a nucleotide sequence encoding an adenoviral DNA binding protein (DBP), optionally
i. the E2a region comprises a nucleotide sequence encoding an adenoviral DNA binding protein (DBP) and having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 27**or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 27;** and/or
ii. the E2a region comprises a nucleotide sequence encoding a DNA binding protein (DBP) having the amino acid sequence **SEQ ID NO: 28;**
b. the E2a region comprises an adenoviral U exon protein (UXP) exon 3 sequence, optionally the E2a region comprises an adenoviral UXP exon 3 sequence having at least 8% identity to **SEQ ID NO: 29** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 29;**
c. the E2a region comprises an adenoviral E2a exon2/leader sequence, optionally the E2a region comprises an adenoviral E2a exon2/leader sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30;**
d. the E2a region comprises an adenoviral UXP exon 2 sequence, optionally the E2a region comprises an adenoviral UXP exon 2 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 30** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 30;**
e. the E2a region comprises an adenoviral E2a late promoter sequence, optionally the E2a region comprises an adenoviral E2a late promoter sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 31** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 31;**
f. the E2a region comprises an adenoviral E2a exon 1 sequence, optionally the E2a region comprises an adenoviral E2a exon 1 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 32** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 32;**
g. the E2a region is under the control of one or more of promoters, optionally the promoter is not a chicken β-actin promoter or a SV40 promoter;
h. the E2a region comprises an adenoviral E2a early promoter sequence, optionally
i. the E2a region comprises an adenoviral E2a early promoter sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 33** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 33;**
j. the E2a region comprises an adenoviral E2a late primary transcript sequence, optionally
k. the E2a region comprises an adenoviral E2a late primary transcript sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 34** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 34;**
l. the E2a region comprises an adenoviral E2a early primary transcript sequence, optionally the E2a region comprises an adenoviral E2a early primary transcript sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 35** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 35;** and/or
m. the E2a region comprises a nucleotide having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 36** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 36,** and/or
wherein:
a. the E4 region comprises an adenoviral E4 orf6/7 sequence, optionally
i. the E4 region comprises an adenoviral E4 orf6/7 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 37** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 37;**
and/or
ii. the E4 region comprises a nucleotide sequence encoding an adenoviral E4 orf6/7 protein having the amino acid sequence **SEQ ID NO: 38;**
b. the E4 region comprises a nucleotide sequence encoding an adenoviral E4 34K protein having the amino acid sequence **SEQ ID NO: 39;**
c. the E4 region comprises an adenoviral E4 orf4 sequence, optionally
i. the E4 region comprises an adenoviral E4 orf4 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 40** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 40** and/or
ii. the E4 region comprises a nucleotide sequence encoding an adenoviral E4 orf4 protein having the amino acid sequence **SEQ ID NO: 41;**
d. the E4 region comprises an adenoviral E4 orf3 sequence, optionally
i. the E4 region comprises an adenoviral E4 orf3 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 42** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 42**; and/or
ii. the E4 region comprises a nucleotide sequence encoding an adenoviral E4 orf3 protein having the amino acid sequence **SEQ ID NO: 43**;
e. the E4 region comprises an adenoviral E4 orf8 sequence, optionally
i. the E4 region comprises an adenoviral E4 orf4 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 44** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 44**; and/or
ii. the E4 region comprises a nucleotide sequence encoding an adenoviral E4 orf4 protein having the amino acid sequence **SEQ ID NO: 45**;
f. the E4 region comprises an adenoviral E4 orf1 sequence, optionally
i. the E4 region comprises an adenoviral E4 orf1 sequence comprising a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 46** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 46**; and/or
ii. the E4 region comprises a nucleotide sequence encoding an adenoviral E4 orf1 protein having the amino acid sequence **SEQ ID NO: 47**;
g. the E4 region comprises a nucleotide sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 48** or having at least 80% (e.g., at least 85%, at least 90%, at least 91%, at least 92%, at least 93%, at least 94%, at least 95%, at least 96%, at least 97%, at least 98%, at least 99% or 100%) identity to a sequence complementary to **SEQ ID NO: 48**; and/or
h. the E4 region is operably linked to a promoter, optionally the promoter is not a chicken β-actin promoter or a SV40 promoter.

9. The polynucleotide of any one of claims 1-8, wherein:
a. the polynucleotide is about 16,500 nucleotides or less in size, e.g., about 15,500 nucleotides or less in size, about 14,000 nucleotide or less in size, about 12,500 nucleotides or less in size, about 12,000 nucleotides or less in size, or about 11,000 nucleotides or less in size;
b. the polynucleotide is double-stranded;
c. the polynucleotide is single-stranded;
d. the polynucleotide is linear;
e. the polynucleotide is circular; and/or
f. the polynucleotide is a close ended linear duplexed DNA (cIDNA).

10. The polynucleotide of any one of claims 1-9, wherein:
a. the polynucleotide is capable of producing recombinant adeno-associated viral (rAAV) particles in an amount that is at least 80% of an amount produced under similar conditions with a similar polynucleotide that further comprises a nucleotide sequence encoding a full-length L-22K protein and/or a full-length L-33K protein; and/or a packaging efficiency of the polynucleotide in a method of producing rAAV is at least 80% of a packaging efficiency of a similar polypeptide that further comprises a nucleotide sequence encoding a full-length L-22K protein and/or a full-length L-33K protein.

11. A cell, plasmid or vector comprising a polynucleotide of any one of claims 1-10, optionally
a. the cell, plasmid or vector does not comprise a nucleotide sequence encoding a full length L-22K and/or a full length L-33K protein, e.g., the cell, plasmid or vector does not comprise a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8**.
b. vector is a viral vector, e.g., a retroviral vector, a lentiviral vector, an adenoviral vector, an adeno-associated viral vector, or a herpes simplex viral vector, preferably an adenoviral vector;
c. the cell
i. is an insect cell or a mammalian cell, preferably the cell is a mammalian cell;
ii. is a HeLa cell, COS cell, COS-1 cell, COS-7 cell, HEK293 cell, A549 cell, BHK cell, BSC-1 cell, BSC-40 cell, Vero cell, Sf"c9 cell, Sf -21 cell, Tn-368 cell, BTI-Tn-5B1-4 (High-Five) cell, Saos cell, C2C12 cell, L cell, HT1080 cell, HepG2 cell, WEHI cell, 3T3 cell, 10T1/2 cell, MDCK cell, BMT-10 cell, WI38 cell, or a primary fibroblast, hepatocyte or myoblast cells derived from a mammal, preferably the cell is a HEK293 cell or HeLa cell;
iii. is a suspension adapted cell; and/or
iv. is a producer cell;
v. further comprises a polynucleotide encoding a virus genome (e.g., an AAV endogenous genome, optionally flanked by left inverted terminal repeat (L-ITR) and/or right ITR (R-ITR), or a recombinant AAV genome comprising polynucleotide encoding a transgene, optionally flanked by L-ITR and/or R-ITR), and wherein the L-ITR and R-ITR are selected independently from AAV ITRs and adenoviral ITRs;
vi. further comprises a polynucleotide encoding viral capsid (e.g., AAV capsid) and/or non-structural replication (e.g., AAV Rep) genes.

12. Use of a polynucleotide of any one of claims 1-10, or a cell, plasmid or vector of vector of claim 11, for producing recombinant adeno-associated viral (rAAV) particles.

13. A method for producing recombinant adeno-associated viral (rAAV) particles, the method comprising: culturing a cell of claim 11 in a culture medium under conditions in which rAAV particles are produced.

14. A method for producing recombinant adeno-associated viral (rAAV) particles, the method comprising: culturing a host cell in a culture medium under conditions in which rAAV particles are produced, where the host cell comprises: (i) a polynucleotide of any one of claims 1-10, or a plasmid or vector of claim 11; (ii) a polynucleotide encoding a virus genome (e.g., an AAV endogenous genome, optionally flanked by left inverted terminal repeat (L-AAV ITR) and/or right ITR (R-AAV ITR), or a recombinant AAV genome comprising polynucleotide encoding a transgene, optionally flanked by L-AAVITR and/or R-AAV ITR); and (iii) a polynucleotide encoding viral capsid (e.g., AAV capsid) and/or non-structural replication (e.g., AAV Rep) genes.

15. The method of any one of claims 13 or 14, wherein:
a. the cell culture is substantially free of a polynucleotide encoding a full-length L-22K protein and/or a full-length L-33K protein, e.g., the cell culture is substantially free of a polynucleotide encoding a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 or 8;** and/or
b. a full-length L-22K protein and/or a full-length L-33K protein is not expressed during the culturing of the cell, e.g., a protein having an amino acid sequence having at least 80% (e.g., at least 85%, at least 90%, at least 91%, at least 92%, at least 93%, at least 94%, at least 95%, at least 96%, at least 97%, at least 98%, at least 99% or 100%) identity to **SEQ ID NO: 7 and/or 8** is not expressed during the culturing of the cell.
